# EUROPEAN PATENT SPECIFICATION

(11) **EP 2 029 543 B1**
(45) Date of publication and mention of the grant of the patent: **18.11.2015**
(21) Application number: 07796104.3
(22) Date of filing: 13.06.2007
(51) Int. Cl.: C07D 213/85, A61K 31/465, C07D 401/12, C07D 401/14, C07D 405/12, C07D 405/14, C07D 409/14, C07D 413/14, C07D 417/12, C07D 417/14, C07D 471/04

(54) **SUBSTITUTED 3-CYANOPYRIDINES AS PROTEIN KINASE INHIBITORS**
SUBSTITUTIERTE 3-CYANOPYRIDINE ALS PROTEIN KINASE INHIBITOREN
3-CYANOPYRIDINES SUBSTITUÉES EN TANT QU'INHIBITEURS DE PROTÉINE KINASES

(30) Priority: 13.06.2006 US 813060 P
(43) Date of publication of application: 04.03.2009
(73) Proprietor: Wyeth LLC, New York, NY 10017-5755 (US)
(72) Inventor: COLE, Derek, Cecil, New City, NY 10956 (US); BOSCHELLI, Diane, Harris, New City, NY 10956 (US); WANG, Yanong, Daniel, Warren, NJ 07059 (US); ASSELIN, Magda, Mahwah, NJ 07430 (US); JOSEPH-MCARTHY, Diane, Marie, Belmont, MA 02478 (US); PRASHAD, Amarnauth, Shastrie, New City, NY 10956 (US); WISSNER, Allan, Ardsley, NY 10502 (US); DUSHIN, Russell, Garrison, NY 10524 (US); WU, Biqi, Nanuet, NY 10954 (US); TUMEY, Lawrence, Nathan, New Windsor, NY 12553 (US); NIU, Chuan, S., Cheshire, CT 06410 (US); CHEN, Joan, Flushing, NY 11354 (US)
(74) Representative: Pfizer
(86) International application number: PCT/US2007/013954
(87) International publication number: WO 2007/146376

(56) References cited:
- WO-A-00/18761
- WO-A-03/050090
- WO-A-2004/054505
- US-A1- 2006 264 460

## Description

### Field

The present teachings relate to substituted 3-cyanopyridines (also known as nicotinonitriles) that are capable of inhibiting protein kinases. The present teachings also relate to methods for the preparation of the substituted cyanopyridines and methods of their use. For example, the compounds of the present teachings can be useful for the treatment of autoimmune and inflammatory diseases such as asthma and arthritis.

### Introduction

Protein kinases are enzymes that catalyze the transfer of a phosphate group from adenosine triphosphate (ATP) to an amino acid residue (e.g., tyrosine, serine, threonine or histidine) on a protein. Regulation of these protein kinases is essential for the control of a wide variety of cellular events including proliferation and migration. A large number of diseases including various inflammatory diseases and autoimmune diseases such as asthma, colitis, multiple sclerosis, psoriasis, arthritis, rheumatoid arthritis, osteoarthritis, and joint inflammation, are associated with abnormal cellular events that are mediated by these kinases. *See*, *e.g.*, Salek-Ardakami, S. et al. (2004), J. Immunology, 173(10): 6440-47; Marsland, B. et al. (2004), J. Exp. Med., 200(2): 181-89; Tan, S, et al. (2006), J. Immunology, 176: 2872-79; Salek-Ardakami, S. et al. (2005), J. Immunology, 175(11): 7635-41; Anderson, K. et al. (2006), Autoimmunity, 39(6): 469-78; Healy, A. et al. (2006), J. Immunology, 177(3): 1886-93; Sun, Z. et al. (2000), Nature, 404: 402-7; and Pfeifhofer, C. et al. (2003), J. Exp. Med., 197(11): 1525-35.

One class of serine/threonine kinases is the protein kinase C (PKC) family. This group of kinases consists of 10 members that share sequence and structural homology. The PKCs are divided into 3 groups and include the classic, the novel, and the atypical isoforms. The theta isoform (PKCθ) is a member of the novel calcium-independent class of PKCs (Baier, G. et al. (1993), J. Biol. Chem., 268: 4997-5004). PKCθ is highly expressed in T cells (Mischak, H. et al. (1993), FEBS Lett., 326: 51-5), with some expression reported in mast cells (Liu, Y. et al. (2001), J. Leukoc. Biol., 69: 831-40), endothelial cells (Mattila, P. et al. (1994), Life Sci., 55: 1253-60), and skeletal muscles (Baier, G. et al. (1994), Eur. J. Biochem., 225: 195-203). It has been shown that PKCθ plays an essential role in T cell receptor (TCR)-mediated signaling (Tan, S.L. et al. (2003), Biochem. J., 376: 545-52). Specifically, it has been observed that inhibiting PKCθ signal transduction, as demonstrated with two independent PKCθ knockout mouse lines, will result in defects in T cell activation and interleukin-2 (IL-2) production (Sun, Z. et al. (2000), Nature, 404: 402-7; Pfeifhofer, C. et al. (2003), J. Exp. Med., 197: 1525-35). It also has been shown that PKCθ-deficient mice show impaired pulmonary inflammation and airway hyperresponsiveness (AHR) in a Th2-dependent murine asthma model, with no defects in viral clearance and Th1-dependent cytotoxic T cell function (Berg-Brown, N.N. et al. (2004), J. Exp. Med., 199: 743-52; Marsland, B.J. et al. (2004), J. Exp. Med., 200: 181-9). The impaired Th2 cell responses result in reduced levels of interleukin-4 (IL-4) and immunoglobulin E (IgE), contributing to the AHR and inflammatory pathophysiology.

Evidence also exists that PKCθ participates in the IgE receptor (FceRI)-mediated response of mast cells (Liu, Y. et al. (2001), J. Leukoc. Biol., 69: 831-840). In human-cultured mast cells (HCMC), it has been demonstrated that PKC kinase activity rapidly localizes (in less than five minutes) to the membrane following FceRI cross-Iinking (Kimata, M. et al. (1999), Biochem. Biophys. Res. Commun., 257(3): 895-900). A recent study examining *in vitro* activation of bone marrow mast cells (BMMCs) derived from wild-type and PKCθ-deficient mice shows that upon FceRI cross-linking, BMMCs from PKCθ-deficient mice produced reduced levels of interleukin-6 (IL-6), tumor necrosis factor-alpha (TNFα), and interleukin-13 (IL-13) in comparison with BMMCs from wild-type mice, suggesting a potential role for PKCθ in mast cell cytokine production in addition to T cell activation (Ciarletta, A.B. et al. (2005), poster presentation at the 2005 American Thorasic Society International Conference).

Other serine/threonine kinases include those of the mitogen-activated protein kinase (MAPK) pathway which consists of the MAP kinases (MAPK) (e.g., erk) and the MAPK kinases (MAPKK) (e.g., mek and their substrates). Members of the raf family of kinases phosphorylate residues on mek. The cyclin-dependent kinases (cdks), including cdc2/cyclin B, cdk2/cyclin A, cdk2/cyclin E and cdk4/cyclin D, and others, are serine/threonine kinases that regulate mammalian cell division. Additional serine/threonine kinases include the protein kinases A and B. These kinases, known as PKA or cyclic AMP-dependent protein kinase and PKB (Akt), play key roles in signal transduction pathways.

Tyrosine kinases (TKs) are divided into two classes: the non-transmembrane TKs and transmembrane growth factor receptor TKs (RTKs). Growth factors, such as epidermal growth factor (EGF), bind to the extracellular domain of their partner RTK on the cell surface which activates the RTK, initiating a signal transduction cascade that controls a wide variety of cellular responses. In addition to EGF, there are several other RTKs including FGFR (the receptor for fibroblast growth factor (FGF)); flk-1 (also known as KDR), and flt-1 (the receptors for vascular endothelial growth factor (VEGF)); and PDGFR (the receptor for platelet derived growth factor (PDGF)). Other RTKs include tie-1 and tie-2, colony stimulating factor receptor, the nerve growth factor receptor, and the insulin-like growth factor receptor. In addition to the RTKs there is another family of TKs termed the cytoplasmic protein or non-receptor TKs. The cytoplasmic protein TKs have intrinsic kinase activity, are present in the cytoplasm and nucleus, and participate in diverse signaling pathways. There are a large number of non-receptor TKs including Abl, Jak, Fak, Syk, Zap-70 and Csk, and the Src family of kinases (SFKs) which include Src, Lck, Lyn, Fyn and others.

Certain pyridine and pyrimidine derivatives have been noted as kinase inhibitors. These compounds differ both in nature and placement of substituents at various positions when compared to the compounds of the present teachings. For example, international patent applications WO 00/18761 and WO 03/050090 relate to 3-cyanoquinoline derivatives, and international patent application WO 2004/054505 relates to 2-amino-3-cyanopyridine derivatives.

The present teachings relate to substituted 3-cyanopyridines of formula I: 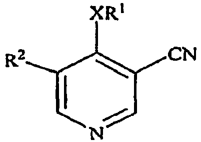 and their pharmaceutically acceptable salts, hydrates, and esters, wherein R¹, R², and X are defined as described herein.

The present teachings also relate to pharmaceutical compositions that include a pharmaceutically effective amount of one or more compounds of formula I (including their pharmaceutically acceptable salts, hydrates, and esters) and a pharmaceutically acceptable carrier or excipient. Another aspect of the present teachings relates to methods of preparing the compounds of formula I and their pharmaceutically acceptable salts, hydrates, and esters. The present teachings also provide methods of using the compounds of formula I and their pharmaceutically acceptable salts, hydrates, and esters. In some embodiments, the present teachings provide compounds of formula I (or their pharmaceutically acceptable salts, hydrates, or esters) for use in the treatment of autoimmune and inflammatory diseases, such as asthma, colitis, multiple sclerosis, psoriasis, arthritis, rheumatoid arthritis, osteoarthritis, and joint inflammation.

### Detailed Description

The present teachings provide compounds of formula **I**: 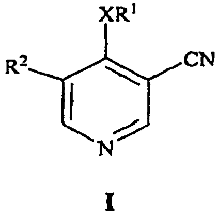 and their pharmaceutically acceptable salts, hydrates, and esters, wherein:
X is selected from a) NR³-Y-, b) -O-Y-, c) -S(O)ₘ-Y-, d)-S(O)ₘNR³-Y-, e) -NR³S(O)ₘ-Y-, f) -C(O)NR³-Y-, g) -C(S)NR³-Y-, h) -NR³C(O)-Y-, i) -NR³C(S)-Y-, j) -C(O)O-Y-, k) -OC(O)-Y-, and 1) a covalent bond;
Y, at each occurrence, independently is selected from a) a divalent C₁₋₁₀ alkyl group, b) a divalent C₂₋₁₀ alkenyl group, c) a divalent C₂₋₁₀ alkynyl group, d) a divalent C₁₋₁₀ haloalkyl group, and e) a covalent bond;
R¹ is selected from a) a C₁₋₁₀alkyl group, b) a C₃₋₁₄ cycloalkyl group, c) a 3-14 membered cycloheteroalkyl group, d) a C₈₋₁₄ polycyclic aryl group, and e) a 5-14 membered heteroaryl group, wherein each group optionally is substituted with 1-4-Y-R⁴;
R² is a C₆₋₁₄ aryl group or a 5-14 membered heteroaryl group, wherein
each group optionally is substituted with 1-4 groups independently selected from -Y-R⁴ or -O-Y-R⁴;
R³, at each occurrence, independently is selected from a) H, b) a C₁₋₁₀ alkyl group, c) a C₂₋₁₀ alkenyl group, d) a C₂₋₁₀ alkynyl group, and e) a C₁₋₁₀ haloalkyl group;
R⁴, at each occurrence, independently is selected from a) halogen, b)-CN, c) NO₂, d) oxo, e) -O-Y-R⁵, f) -NR⁶-Y-R⁷, g) -N(O)R⁶-Y-R⁷, h) -S(O)ₘ-Y-R⁵, i) -S(O)ₘO-Y-R⁵, j) -S(O)ₘNR⁶-Y-R⁷, k) -C(O)-Y-R⁵, 1) -C(O)O-Y-R⁵, m)-C(O)NR⁶-Y-R⁷, n) -C(S)NR⁶-Y-R⁷, o) a C₁₋₁₀ alkyl group, p) a C₂₋₁₀ alkenyl group, q) a C₂₋₁₀ alkynyl group, r) a C₁₋₁₀ haloalkyl group, s) a C₃₋₁₄ cycloalkyl group, t) a C₆₋₁₄ aryl group, u) a 3-14 membered cycloheteroalkyl group, and v) a 5-14 membered heteroaryl group, wherein each of o) - v) optionally is substituted with 1-4 -Y-R⁸ groups;
R⁵, at each occurrence, independently is selected from a) H, b) -C(O)R⁹, c) -C(O)OR⁹, d) a C₁₋₁₀ alkyl group, e) a C₂₋₁₀ alkenyl group, f) a C₂₋₁₀ alkynyl group, g) a C₁₋₁₀ haloalkyl group, h) a C₃₋₁₄ cycloalkyl group, i) a C₆₋₁₄ aryl group, j) a 3-14 membered cycloheteroalkyl group, and k) a 5-14 membered heteroaryl group, wherein each of d) - k) optionally is substituted with 1-4 -Y-R⁸ groups;
R⁶ and R⁷, at each occurrence, independently are selected from a) H, b)-O-Y-R⁹, c) -S(O)ₘ-Y-R⁹, d) -S(O)ₘO-Y-R⁹, e) -C(O)-Y-R⁹, f)-C(O)O-Y-R⁹, g) -C(O)NR¹⁰-Y-R¹¹, h) -C(S)NR¹⁰-Y-R¹¹, i) a C₁₋₁₀ alkyl group, j) a C₂₋₁₀ alkenyl group, k) a C₂₋₁₀ alkynyl group, l) a C₁₋₁₀ haloalkyl group, m) a C₃₋₁₄ cycloalkyl group, n) a C₆₋₁₄ aryl group, o) a 3-14 membered cycloheteroalkyl group, and p) a 5-14 membered heteroaryl group, wherein each of i) - p) optionally is substituted with 1-4 -Y-R⁸ groups;
R⁸, at each occurrence, independently is selected from a) halogen, b)-CN, c) -NO₂, d) oxo, e) -O-Y-R⁹, f) -NR¹⁰-Y-R¹¹, g) -N(O)R¹⁰-Y-R¹¹, h) -S(O)ₘ-Y-R⁹, i) -S(O)ₘO-Y-R⁹, j) -S(O)ₘNR¹⁰-Y-R¹¹, k)-C(O)-Y-R⁹, l) -C(O)O-Y-R⁹, m) -C(O)NR¹⁰-Y-R¹¹, n) -C(S)NR¹⁰-Y-R¹¹, o) a C₁₋₁₀ alkyl group, p) a C₂₋₁₀ alkenyl group, q) a C₂₋₁₀ alkynyl group, r) a C₁₋₁₀ haloalkyl group, s) a C₃₋₁₄ cycloalkyl group, t) a C₆₋₁₄ aryl group, u) a 3-14 membered cycloheteroalkyl group, and v) a 5-14 membered heteroaryl group, wherein each of o) - v) optionally is substituted with 1-4 -Y-R¹² groups;
R⁹, at each occurrence, independently is selected from a) H, b) -C(O)-C₁₋₁₀alkyl, c) -C(O)OH, d) -C(O)O-C₁₋₁₀alkyl, e) a C₁₋₁₀ alkyl group, f) a C₂₋₁₀ alkenyl group, g) a C₂₋₁₀ alkynyl group, h) a C₁₋₁₀ haloalkyl group, i) a C₃₋₁₄ cycloalkyl group, j) a C₆₋₁₄ aryl group, k) a 3-14 membered cycloheteroalkyl group, and 1) a 5-14 membered heteroaryl group, wherein each of the C₁₋₁₀ alkyl group, the C₂₋₁₀ alkenyl group, the C₂₋₁₀ alkynyl group, the C₁₋₁₀ haloalkyl group, the C₃₋₁₄ cycloalkyl group, the C₆₋₁₄ aryl group, the 3-14 membered cycloheteroalkyl group, and the 5-14 membered heteroaryl group optionally is substituted with 1-4 -Y-R¹² groups;
R¹⁰ and R¹¹, at each occurrence, independently are selected from a) H, b) -OH, c) -SH, d) NH₂, e) -NH-C₁₋₁₀ alkyl, f) -N(C₁₋₁₀ alkyl)₂, g)-S(O)ₘ-C₁₋₁₀ alkyl, h) -S(O)₂OH, i) -S(O)ₘ-OC₁₋₁₀ alkyl, j) -C(O)-C₁₋₁₀ alkyl, k) -C(O)OH, l) -C(O)-OC₁₋₁₀ alkyl, m) -C(O)NH₂, n) -C(O)NH-C₁₋₁₀ alkyl, o) -C(O)N(C₁₋₁₀ alkyl)₂, p) -C(S)NH₂, q) -C(S)NH-C₁₋₁₀ alkyl, r) -C(S)N(C₁₋₁₀ alkyl)₂, s) a C₁₋₁₀ alkyl group, t) a C₂₋₁₀ alkenyl group, u) a C₂₋₁₀ alkynyl group, v) a C₁₋₁₀ alkoxy group, w) a C₁₋₁₀ haloalkyl group, x) a C₃₋₁₄ cycloalkyl group, y) a C₆₋₁₄ aryl group, z) a 3-14 membered cycloheteroalkyl group, and aa) a 5-14 membered heteroaryl group, wherein each of the C₁₋₁₀ alkyl group, the C₂₋₁₀ alkenyl group, the C₂₋₁₀ alkynyl group, the C₁₋₁₀ alkoxy group, the C₁₋₁₀ haloalkyl group, the C₃₋₁₄ cycloalkyl group, the C₆₋₁₄ aryl group, the 3-14 membered cycloheteroalkyl group, and the 5-14 membered heteroaryl group optionally is substituted with 1-4-Y-R¹² groups;
R¹², at each occurrence, independently is selected from a) halogen, b)-CN, c) NO₂, d) oxo, e) -OH, f) NH₂, g) NH(C₁₋₁₀ alkyl), h) -N(C₁₋₁₀ alkyl)₂, i) -SH, j) -S(O)ₘ-C₁₋₁₀ alkyl, k) -S(O)₂OH, l) -S(O)ₘ-OC₁₋₁₀ alkyl, m) -C(O)-C₁₋₁₀ alkyl, n) -C(O)OH, o) -C(O)-OC₁₋₁₀ alkyl, p)-C(O)NH₂, q) -C(O)NH-C₁₋₁₀ alkyl, r) -C(O)N(C₁₋₁₀ alkyl)₂, s)-C(S)NH₂, t) -C(S)NH-C₁₋₁₀ alkyl, u) -C(S)N(C₁₋₁₀ alkyl)₂, v) a C₁₋₁₀ alkyl group, w) a C₂₋₁₀ alkenyl group, x) a C₂₋₁₀ alkynyl group, y) a C₁₋₁₀ alkoxy group, z) a C₁₋₁₀ haloalkyl group, aa) a C₃₋₁₄ cycloalkyl group, ab) a C₆₋₁₄ aryl group, ac) a 3-14 membered cycloheteroalkyl group, and ad) a 5-14 membered heteroaryl group; and
m is 0, 1, or 2.

In some embodiments, the pyridine ring can be oxidized on the nitrogen atom to provide the corresponding N-oxide having the formula **I'**: 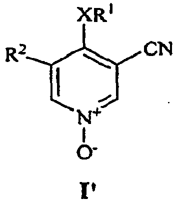 wherein R¹, R², and X are as defined herein.

In some embodiments, X can be selected from -NR³-Y-, -O-Y-, and a covalent bond. For example, X can be selected from -NH-, -N(CH₃)-, NH-CH₂-, -NH-CH₂CH₂-, -NH-CH₂CH₂CH₂-, -O-, and a covalent bond. In particular embodiments, X can be NH-.

In certain embodiments, R¹ can be a C₈₋₁₄ polycyclic (e.g., bicyclic or tricyclic) aryl group or a 5-14 membered heteroaryl group, wherein each of these groups can be optionally substituted with 1-4 -Y-R⁴ groups, wherein Y and R⁴ are as defined herein. For example, R¹ can be selected from a benzimidazolyl group, a benzodioxolyl group, a benzodioxinyl group, a benzodioxanyl group, a benzofuranyl group, a benzothienyl group, a benzoxadiazolyl group, a benzoxazolyl group, a benzisoxazolyl group, a benzothiadiazolyl group, a benzothiazolyl group, a benzisothiazolyl group, a benzo[c]isothiazolyl group, a benzo[c]thienyl group, a benzotriazolyl group, an indazolyl group, an indenyl group, an indanyl group, an indolyl group, an isobenzofuranyl group, an isoindolyl group, an isoquinolinyl group, a naphthyl group, an indolinyl group, a pyrazolyl group, a pyridinyl group, a pyrrolopyridinyl group, a pyrrolyl group, a quinolinyl group, and a tetrahydronaphthalenyl group, wherein each of these groups can be optionally substituted with 1-4 -Y-R⁴ groups, wherein Y and R⁴ are as defined herein. In particular embodiments, R¹ can be an indolyl group or a pyrrolopyridinyl group, wherein each of these groups can be optionally substituted with 1-4 -Y-R⁴ groups, wherein Y and R⁴ are as defined herein. For example, R¹ can be a 1*H*-indol-4-yl group, a 1*H*-indol-5-yl group, a 1*H*-indol-6-yl group, or a 1*H*-indol-7-yl group, wherein each of these groups can be optionally substituted with 1-4 groups independently selected from a halogen, a C₁₋₄ alkyl group, and a C₁₋₄ alkoxy group.

In other embodiments, R¹ can be a C₃₋₁₄ cycloalkyl group or a 3-14 membered cycloheteroalkyl group, wherein each of these groups can be optionally substituted with 1-4 -Y-R⁴ groups, wherein Y and R⁴ are as defined herein. For example, R¹ can be selected from a cyclobutyl group, a cyclopentyl group, a cyclohexyl group, a cycloheptyl group, a pyrrolidinyl group, a piperidinyl group, a piperazinyl group, a morpholinyl group, and a thiomorpholinyl group, wherein each of these groups can be optionally substituted with 1-4 -Y-R⁴ groups, wherein Y and R⁴ are as defined herein. In some embodiments, Y, at each occurrence, can be independently a divalent C₁₋₄ alkyl group or a covalent bond, and R⁴, at each occurrence, can be independently -(CH₂)ₙ-NR⁶-Y-R⁷ or a C₁₋₄ alkyl group, wherein n can be 0, 1, 2, 3, or 4, and Y, R⁶, and R⁷ are as defined herein.

In some embodiments, R² can be selected from a phenyl group, a C₈₋₁₄ aryl group, and a 5-14 membered heteroaryl group, wherein each of these groups can be optionally substituted with 1-4 groups independently selected from -Y-R⁴ and-O-Y-R⁴, wherein Y and R⁴ are as defined herein. For example, R² can be selected from a phenyl group, a pyridyl group, a pyrimidyl group, a pyrazinyl group, a furyl group, a thienyl group, a thiazolyl group, an oxazolyl group, a benzofuranyl group, a benzothienyl group, an indolyl group, a benzodioxinyl group, a benzodioxolyl group, a benzodioxanyl group, a dibenzofuranyl group, a dibenzothienyl group, a benzoindolyl group, an indanyl group, an indenyl group, an isothiazolyl group, a pyridazinyl group, a pyrazolyl group, a tetrahydronaphthyl group, an isoxazolyl group, a quinolinyl group, a naphthyl group, an imidazolyl group, and a pyrrolyl group, wherein each of these groups can be optionally substituted with 1-4 groups independently selected from -Y-R⁴ or -O-Y-R⁴, wherein Y and R⁴ are as defined herein.

In certain embodiments, R² can be 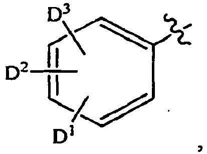 wherein D¹, D², and D³ independently can be H, a-Y-R⁴ group, or an -O-Y-R⁴ group, wherein Y and R⁴ are as defined herein.

For example, at least one of D¹, D², and D³ can be a -Y-R⁴ group or an-O-Y-R⁴ group, wherein Y, at each occurrence, can be independently a divalent C₁₋₄ alkyl group or a covalent bond, and R⁴, at each occurrence, can be independently selected from a halogen, -CN, -NO₂, -O-Y-R⁵, -NR⁶-Y-R⁷, -S(O)₂-Y-R⁵,-S(O)₂NR⁶-Y-R⁷, -C(O)-Y-R⁵, -C(O)O-Y-R⁵, -C(O)NR⁶-Y-R⁷, a C₁₋₁₀ alkyl group, a C₁₋₁₀ haloalkyl group, a C₃₋₁₄ cycloalkyl group, a C₆₋₁₄ aryl group, a 3-14 membered cycloheteroalkyl group, and a 5-14 membered heteroaryl group, wherein each of the C₁₋₁₀ alkyl group, the C₁₋₁₀ haloalkyl group, the C₃₋₁₄ cycloalkyl group, the C₆₋₁₄ aryl group, the 3-14 membered cycloheteroalkyl group, and the 5-14 membered heteroaryl group can be optionally substituted with 1-4 -Y-R⁸ groups, wherein Y, R⁵, R⁶, R⁷, and R⁸ are as defined herein.

In certain embodiments, at least one of D¹, D², and D³ can be an -O-(CH₂)ₙ-R⁴ group, wherein n, at each occurrence, independently can be 0, 1, 2, 3, or 4, and R⁴, at each occurrence, can be independently selected from F, Cl, Br, -NO₂,-O-Y-R⁵, -NR⁶-Y-R⁷, S(O)₂-Y-R⁵, -S(O)₂NR⁶-Y-R⁷, -C(O)NR⁶-Y-R⁷, a C₁₋₁₀ alkyl group, a C₃₋₁₄ cycloalkyl group, a C₆₋₁₄ aryl group, a 3-14 membered cycloheteroalkyl group, and a 5-14 membered heteroaryl group, wherein each of the C₁₋₁₀ alkyl group, the C₃₋₁₄ cycloalkyl group, the C₆₋₁₄ aryl group, the 3-14 membered cycloheteroalkyl group, and the 5-14 membered heteroaryl group can be optionally substituted with 1-4 -Y-R⁸ groups, wherein Y, R⁵, R⁶, R⁷, and R⁸ are as defined herein. In particular embodiments, at least one of D¹, D², and D³ can be-O-(CH₂)ₙNR⁶-Y-R⁷ or an -O-(CH₂)ₙ-3-14 membered cycloheteroalkyl group, wherein the 3-14 membered cycloheteroalkyl group can be optionally substituted with 1-4 -Y-R⁸ groups, wherein Y, R⁶, R⁷, and R⁸ are as defined herein, and n, at each occurrence, independently can be 0, 1, 2, 3, or 4.

In some embodiments, at least one of D¹, D², and D³ can be - (CH₂)ₙNR⁶-Y-R⁷ or a -(CH₂)ₙ-3-14 membered cycloheteroalkyl group, wherein the 3-14 membered cycloheteroalkyl group can be optionally substituted with 1-4 -Y-R⁸ groups, Y, R⁶, R⁷, and R⁸ are as defined herein, and n, at each occurrence, independently can be 0, 1, 2, 3, or 4.

In embodiments where at least one of D¹, D², and D³ can be an -O-(CH₂)ₙNR⁶-Y-R⁷ group or a -(CH₂)ₙNR⁶-Y-R⁷ group, the -O-(CH₂)ₙNR⁶-Y-R⁷ group and the -(CH₂)ₙNR⁶-Y-R⁷ group can be -O-(CH₂)ₙNH-Y-R⁷ or -O-(CH₂)ₙN(CH₃)-Y-R⁷, and -(CH₂)ₙNH-Y-R⁷ or -(CH₂)ₙN(CH₃)-Y-R⁷, respectively, wherein Y, at each occurrence, can be independently a divalent C₁₋₄ alkyl group or a covalent bond, and R⁷, at each occurrence, can be independently selected from -O-Y-R⁹, -C(O)-Y-R⁹, -C(O)O-Y-R⁹, -C(O)NR¹⁰-Y-R¹¹, a C₁₋₁₀ alkyl group, a C₃₋₁₄ cycloalkyl group, a C₆₋₁₄ aryl group, a 3-14 membered cycloheteroalkyl group, and a 5-14 membered heteroaryl group, wherein each of the C₁₋₁₀ alkyl group, the C₃₋₁₄ cycloalkyl group, the C₆₋₁₄ aryl group, the 3-14 membered cycloheteroalkyl group, and the 5-14 membered heteroaryl group can be optionally substituted with 1-4 -Y-R¹² groups, wherein Y and R¹² are as defined herein. For example, the C₃₋₁₄ cycloalkyl group, the C₆₋₁₄ aryl group, the 3-14 membered cycloheteroalkyl group, and the 5-14 membered heteroaryl group can be selected from a cyclopentyl group, a cyclohexyl group, a phenyl group, a pyrrolidinyl group, a morpholinyl group, a piperazinyl group, a piperidinyl group, an azepanyl group, a diazepanyl group, a thiomorpholinyl group, a furyl group, an imidazolyl group, and a pyridinyl group, wherein each of these groups can be optionally substituted with 1-4-Y-R¹² groups, wherein Y and R¹² are as defined herein.

In embodiments where at least one of D¹, D², and D³ can be an -0-(CH₂)ₙ-3-14 membered cycloheteroalkyl group or a -(CH₂)ₙ-3-14 membered cycloheteroalkyl group, the 3-14 membered cycloheteroalkyl group can be selected from a pyrrolidinyl group, a morpholinyl group, a piperazinyl group, a piperidinyl group, an azepanyl group, a diazepanyl group, and a thiomorpholinyl group, wherein each of these groups can be optionally substituted with 1-4 -Y-R⁸ groups, wherein Y and R⁸ are as defined herein. For example, Y, at each occurrence, can be independently a divalent C₁₋₄ alkyl group or a covalent bond, and R⁸, at each occurrence, can be independently an oxo group, -O-Y-R⁹, -NR¹⁰-Y-R¹¹, -S(O)ₘ-Y-R⁹, -C(O)O-Y-R⁹, a C₁₋₁₀ alkyl group, a C₃₋₁₄ cycloalkyl group, a C₆₋₁₄ aryl group, a 3-14 membered cycloheteroalkyl group, and a 5-14 membered heteroaryl group, wherein each of the C₁₋₁₀ alkyl group, the C₃₋₁₄ cycloalkyl group, the C₆₋₁₄ aryl group, the 3-14 membered cycloheteroalkyl group, and the 5-14 membered heteroaryl group can be optionally substituted with 1-4 -Y-R¹² groups, wherein Y and R¹² are as defined herein. For example, the C₃₋₁₄ cycloalkyl group, the C₆₋₁₄ aryl group, the 3-14 membered cycloheteroalkyl group, and the 5-14 membered heteroaryl group can be selected from a cyclopentyl group, a cyclohexyl group, a phenyl group, a pyrrolidinyl group, a morpholinyl group, a piperazinyl group, a piperidinyl group, an azepanyl group, a diazepanyl group, a thiomorpholinyl group, a furyl group, an imidazolyl group, and a pyridinyl group, wherein each of these groups can be optionally substituted with 1-4 -Y-R¹² groups, wherein Y and R¹² are as defined herein.

Alternatively or concurrently, at least one of D¹, D², and D³ can be selected from halogen, -CN, -NO₂, -S(O)₂-Y-R⁵, -S(O)₂NR⁶-Y-R⁷, -C(O)O-Y-R⁵, -C(O)NR⁶-Y-R⁷, a C₁₋₁₀ alkyl group, and a C₁₋₁₀ haloalkyl group, wherein Y, R⁵, R⁶, and R⁷ are as defined herein.

In some embodiments, at least two of D¹, D², and D³ can be -O-(CH₂)ₙ-R⁴ groups, wherein n, at each occurrence, independently can be 0, 1, 2, 3, or 4, and R⁴, at each occurrence, can be independently selected from F, Cl, Br, -NO₂, -O-Y-R⁵, -NR⁶-Y-R⁷, -S(O)₂-Y-R⁵, -S(O)₂NR⁶-Y-R⁷, -C(O)NR⁶-Y-R⁷, a C₁₋₁₀ alkyl group, a C₃₋₁₄ cycloalkyl group, a C₆₋₁₄ aryl group, a 3-14 membered cycloheteroalkyl group, and a 5-14 membered heteroaryl group, wherein each of the C₁₋₁₀ alkyl group, the C₃₋₁₄ cycloalkyl group, the C₆₋₁₄ aryl group, the 3-14 membered cycloheteroalkyl group, and the 5-14 membered heteroaryl group can be optionally substituted with 1-4 -Y-R⁸ groups, wherein Y, R⁵, R⁶, R⁷, and R⁸ are as defined herein.

In certain embodiments, at least two of D¹, D², and D³ can be independently an -O-CH₃ group or an -O-(CH₂)ₙ-O-Y-R⁵ group, wherein Y and R⁵ are as defined herein, and n, at each occurrence, independently can be 0, 1, 2, 3, or 4. In certain embodiments, two of D¹, D², and D³ can be -O-CH₃ groups. In other embodiments, two of D¹, D², and D³ can be -O-(CH₂)ₙO-Y-R⁵ groups or alternatively, an -O-CH₃ group and an -O-(CH₂)ₙ-O-Y-R⁵ group, wherein Y and R⁵ are as defined herein, and n, at each occurrence, independently can be 0, 1, 2, 3, or 4.

In certain embodiments, at least one of D¹, D², and D³ can be -O-CH₃, and at least one of D¹, D², and D³ can be an -O-(CH₂)ₙNR⁶-Y-R⁷ group or an -0-(CH₂)ₙ-3-14 membered cycloheteroalkyl group, wherein the 3-14 membered cycloheteroalkyl group can be optionally substituted with 1-4 -Y-R⁸ groups, wherein Y, R⁶, R⁷, and R⁸ are as defined herein, and n, at each occurrence, independently can be 0, 1, 2, 3, or 4.

In some embodiments, one of D¹, D², and D³ can be 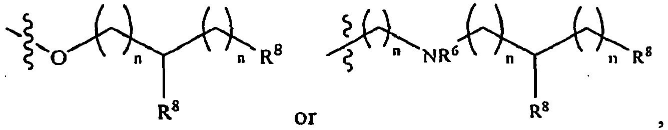 wherein R⁸, at each occurrence, independently can be selected from -O-Y-R⁹,-NR¹⁰-Y-R¹¹, a C₆₋₁₄ aryl group, and a 5-14 membered heteroaryl group, wherein each of the C₆₋₁₄ aryl group and the 5-14 membered heteroaryl group can be optionally substituted with 1-4-Y-R¹² groups, wherein Y, R⁹, R¹⁰, R¹¹, and R¹² are as defined herein, and n, at each occurrence, independently can be 0, 1, 2, 3, or 4.

In certain embodiments, at least one of D¹, D², and D³ can be a C₆₋₁₄ aryl group or a 5-14 membered heteroaryl group, wherein each of these groups can be optionally substituted with 1-4 Y-R⁸ groups, wherein Y and R⁸ are as defined herein. For example, at least one of D¹, D², and D³ can be selected from a benzothienyl group, a benzofuryl group, a furyl group, a pyridyl group, a pyrimidinyl group, a pyrrolyl group, and a thienyl group, wherein each of these groups can be optionally substituted with 1-4-Y-R⁸ groups, wherein Y and R⁸ are as defined herein. In particular embodiments, Y, at each occurrence, can be independently a C₁₋₄ alkyl group or a covalent bond, and R⁸ can be independently selected from a halogen, -CN, -NO₂, -O-Y-R⁹, -NR¹⁰-Y-R¹¹, -C(O)-Y-R⁹,-C(O)NR¹⁰-Y-R¹¹, -S(O)₂-Y-R⁹, -S(O)₂NR¹⁰-Y-R¹¹, and a 3-14 membered cycloheteroalkyl group optionally substituted with a C₁₋₄ alkyl group, wherein Y, R⁹, R¹⁰, and R¹¹ are as defined herein.

In other embodiments, R² can be a C₈₋₁₄ bicyclic aryl group or a 5-14 membered heteroaryl group, where each of these groups can be optionally substituted with 1-4 groups independently selected from -Y-R⁴ groups and -O-Y-R⁴ groups, wherein Y and R⁴ are as defined herein.

In particular embodiments, R² can be selected from a benzothienyl group, a benzofuryl group, a furyl group, a pyridyl group, a pyrimidinyl group, a pyrazinyl group, a thienyl group, an imidazolyl group, an isoxazolyl group, a thiazolyl group, an oxazolyl group, an indolyl group, a benzodioxolyl group, a benzodioxanyl group, and a dibenzofuranyl group, wherein each of these groups can be optionally substituted with 1-4 groups independently selected from a -(CH₂)ₙ-R⁴ group and an -O-(CH₂)ₙ-R⁴ group, wherein n, at each occurrence, independently can be 0, 1, 2, 3, or 4, and R⁴, at each occurrence, can be independently -NR⁶-Y-R⁷ or a 3-14 membered cycloheteroalkyl group optionally substituted with 1-4 -Y-R⁸ group, wherein Y, R⁶, R⁷ and R⁸ are as defined herein.

For example, R⁴ can be -O-(CH₂)ₙNH-Y-R⁷, -O-(CH₂)ₙN(CH₃)-Y-R⁷, -(CH₂)ₙNH-Y-R⁷, or -(CH₂)ₙN(CH₃)-Y-R⁷, wherein Y, at each occurrence, can be independently a divalent C₁₋₄ alkyl group or a covalent bond, and R⁷, at each occurrence, can be independently selected from -O-Y-R⁹, -C(O)-Y-R⁹, -C(O)O-Y-R⁹, -C(O)NR¹⁰-Y-R¹¹, a C₁₋₁₀ alkyl group, a C₃₋₁₄ cycloalkyl group, a C₆₋₁₄ aryl group, a 3-14 membered cycloheteroalkyl group, and a 5-14 membered heteroaryl group, wherein each of the C₁₋₁₀ alkyl group, the C₃₋₁₄ cycloalkyl group, the C₆₋₁₄ aryl group, the 3-14 membered cycloheteroalkyl group, and the 5-14 membered heteroaryl group can be optionally substituted with 1-4 -Y-R¹² groups, wherein Y and R¹² are as defined herein. In particular embodiments, R⁷ can be a C₃₋₁₄ cycloalkyl group, a C₆₋₁₄ aryl group, a 3-14 membered cycloheteroalkyl group, or a 5-14 membered heteroaryl group selected from a cyclopentyl group, a cyclohexyl group, a phenyl group, a pyrrolidinyl group, a morpholinyl group, a piperazinyl group, a piperidinyl group, an azepanyl group, a diazepanyl group, a thiomorpholinyl group, a furyl group, an imidazolyl group, and a pyridinyl group, wherein each of these groups can be optionally substituted with 1-4 -Y-R¹² groups, wherein Y and R¹² are as defined herein.

Alternatively, R⁴ can be an -O-(CH₂)ₙ-3-14 membered cycloheteroalkyl group or a a -(CH₂)ₙ-3-14 membered cycloheteroalkyl group, wherein the 3-14 membered cycloheteroalkyl group can be selected from a pyrrolidinyl group, a morpholinyl group, a piperazinyl group, a piperidinyl group, an azepanyl group, a diazepanyl group, and a thiomorpholinyl group, wherein each of these groups can be optionally substituted with 1-4 -Y-R⁸ groups, wherein Y and R⁸ are as defined herein. For example, Y, at each occurrence, can be independently a divalent C₁₋₄ alkyl group or a covalent bond, and R⁸, at each occurrence, can be independently an oxo group, -O-Y-R⁹, -NR¹⁰-Y-R¹¹, -S(O)ₘ-Y-R⁹, -C(O)O-Y-R⁹, a C₁₋₁₀ alkyl group, a C₃₋₁₄ cycloalkyl group, a C₆₋₁₄ aryl group, a 3-14 membered cycloheteroalkyl group, and a 5-14 membered heteroaryl group, wherein each of the C₁₋₁₀ alkyl group, the C₃₋₁₄ cycloalkyl group, the C₆₋₁₄ aryl group, the 3-14 membered cycloheteroalkyl group, and the 5-14 membered heteroaryl group can be optionally substituted with 1-4 -Y-R¹² groups, wherein Y and R¹² are as defined herein. For example, R⁸ can be a C₃₋₁₄ cycloalkyl group, a C₆₋₁₄ aryl group, a 3-14 membered cycloheteroalkyl group, and a 5-14 membered heteroaryl group selected from a cyclopentyl group, a cyclohexyl group, a phenyl group, a pyrrolidinyl group, a morpholinyl group, a piperazinyl group, a piperidinyl group, an azepanyl group, a diazepanyl group, a thiomorpholinyl group, a furyl group, an imidazolyl group, and a pyridinyl group, wherein each of these groups can be optionally substituted with 1-4-Y-R¹² groups, wherein Y and R¹² are as defined herein.

Compounds of the present teachings include the compounds presented in Table 1 below.

**Table 1**

| **Compound** | **Name** |
|---|---|
| 101 | 5-(3,4-dimethoxyphenyl)-4-indol-5-ylamino)nicotinonitrile |
| 102 | 4-(2,1,3-benzothiadiazol-4-ylamino)-5-(3,4-dimethoxyphenyl)nicotinonitrile |
| 103 | 5-(3,4-dimethoxyphenyl)-4-(isoquinolin-5-ylamino)nicotinonitrile |
| 104 | 5-(3,4-dimethoxyphenyl)-4-(quinolin-5-ylamino)nicotinonitrile |
| 105 | 5-(3,4-dimethoxyphenyl)-4-(5,6,7,8-tetrahydronaphthalen-1-ylamino)nicotinonitrile |
| 106 | 4-(2,3-dihydro-1,4-benzodioxin-6-ylamino)-5-(3,4-dimethoxyphenyl)nicotinonitrile |
| 107 | 4-(2,3-dihydro-1H-inden-5-ylamino)-5-(3,4-dimethoxyphenyl)nicotinonitrile |
| 108 | 5-(3,4-dimethoxyphenyl)-4-(1H-indol-6-ylamino)nicotinonitrile |
| 109 | 5-(3,4-dimethoxyphenyl)-4-(1H-indol-4-ylamino)nicotinonitrile |
| 110 | 5-(3,4-dimethoxyphenyl)-4-[(2-methyl-1H-indol-5-yl)amino]nicotinonitrile |
| 111 | 4-(1,3-benzodioxol-5-ylamino)-5-(3,4-dimethoxyphenyl)nicotinonitrile |
| 112 | 5-(3,4-dimethoxyphenyl)-4-(2-naphthylamino)nicotinonitrile |
| 113 | 5-(3-bromophenyl)-4-(1H-indol-5-ylamino)nicotinonitrile |
| 114 | 5-(3-bromophenyl)-4-(1H-indol-4-ylamino)nicotinonitrile |
| 115 | 5-(2-bromophenyl)-4-(1H-indol-5-ylamino)nicotinonitrile |
| 116 | 5-(4-bromophenyl)-4-(1H-indol-5-ylamino)nicotinonitrile |
| 117 | 5-(3'-aminobiphenyl-3-yl)-4-(1H-indol-5-ylamino)nicotinonitrile |
| 118 | 5-(4'-cyanobiphenyl-3-yl)-4-(1H-indol-5-ylamino)nicotinonitrile |
| 119 | 5-(4'-aminobiphenyl-3-yl)-4-(1H-indol-5-ylamino)nicotinonitrile |
| 120 | N-{3'-[5-cyano-4-(1H-indol-5-ylamino)pyridin-3-yl]biphenyl-4-yl}acetamide |
| 121 | 4-(1H-indol-5-ylamino)-5-(3-pyridin-4-ylphenyl)nicotinonitrile |
| 122 | 3'-[5-cyano-4-(1H-indol-5-ylamino)pyridin-3-yl]-N,N-dimethylbiphenyl-4-carboxamide |
| 123 | 3'-[5-cyano-4-(1H-indol-5-ylamino)pyridin-3-yl]N-cyclopentyibiphenyl-4-carboxamide |
| 124 | 4-(1H-indol-5-ylamino)-5-[3-(1H-pyrrof-3-yl)phenyl]nicotinonitrile |
| 125 | 5-(2-bromophenyl)-4-[(7-methyl-1H-indol-5-yl)amino]nicotinonitrile |
| 126 | 5-(2-bromophenyl)-4-(1H-indol-4-ylamino)nicotinonitrile |
| 127 | 4-(1H-indol-5-ylamino)-5-(3'-methylbiphenyl-3-yl)nicotinonitrile |
| 128 | 4-(1H-indol-5-ylamino)-5-(4'-methylbiphenyl-3-yl)nicotinonitrile |
| 129 | 5-(2'-chlorobiphenyl-3-yl)-4-(1H-indol-5-ylamino)nicotinonitrile |
| 130 | 5-(3'-chlorobiphenyl-3-yl)-4-(1H-indol-5-ylamino)nicotinonitrile |
| 131 | 5-(4'-chlorobiphenyl-3-yl)-4-(1H-indol-5-ylamino)nicotinonitrile |
| 132 | 5-(3'-cyanobiphenyl-3-yl)-4-(1H-indol-5-ylamino)nicotinonitrile |
| 133 | 3'-[5-cyano-4-(1H-indol-5-ylamino)pyridin-3-yl]biphenyl-3-carboxylic acid |
| 134 | 3'-[5-cyano-4-(1H-indol-5-ylamino)pyridin-3-yl]biphenyl-4-carboxylic acid |
| 135 | 3'-[5-cyano-4-(1H-indol-4-ylamino)pyridin-3-yl]biphenyl-4-carboxylic acid |
| 136 | 4-(1H-indol-5-ylamino)-5-[3-(2-thienyl)phenyl]nicotinonitrile |
| 137 | 4-(1H-indol-5-ylamino)-5-(3-pyridin-3-ylphenyl)nicotinonitrile |
| 138 | 4-(1H-indol-5-ylamino)-5-(3-pyrimidin-2-ylphenyl)nicotinonitrile |
| 139 | 4-(1H-indol-5-ylamino)-5-[3-(4-methyl-2-thienyl)phenyl]nicotinonitrile |
| 140 | 5-[3-(5-acetyl-2-thienyl)phenyl]-4-(1H-indol-5-ylamino)nicotinonitrile |
| 141 | 4-(1H-indol-5-ylamino)-5-[3-(3-thienyl)phenyl]nicotinonitrile |
| 142 | 5-[3-(3-furyl)phenyl]-4-(1H-indol-5-ylamino)nicotinonitrile |
| 143 | 5-(2'-chlorobiphenyl-2-yl)-4-(1H-indol-5-ylamino)nicotinonitrile |
| 144 | 5-(3'-chlorobiphenyl-2-yl)-4-(1H-indol-5-ylamino)nicotinonitrile |
| 145 | 5-(4'-chlorobiphenyl-2-yl)-4-(1H-indol-5-ylamino)nicotinonitrile |
| 146 | 4-(1H-indol-5-ylamino)-5-[2-(3-thienyl)phenyl]nicotinonitrile |
| 147 | 4-(1H-indol-4-ylamino)-5-[3-(2-thienyl)phenyl]nicotinonitrile |
| 148 | 3'-[5-cyano-4-(1H-indol-4-ylamino)pyridin-3-yl]-N-cyclopentylbiphenyl-4-carboxamide |
| 149 | 4-(1H-indol-4-ylamino)-5-[4'-(pyrrolidin-1-ylcarbonyl)biphenyl-3-yl]nicotinonitrile |
| 150 | 5-[3-(5-formyl-2-thienyl)phenyl]-4-(1H-indol-5-ylamino)nicotinonitrile |
| 151 | 4-(1H-indol-5-ylamino)-5-(3-nitrophenyl)nicotinonitrile |
| 152 | N-{3-[5-cyano-4-(1H-indol-5-ylamino)pyridin-3-yl]phenyl}acetamide |
| 153 | 4-(1H-indol-4-ylamino)-5-[4-methoxy-3-(2-methoxyethoxy)phenyl]nicotinonitrile |
| 154 | 4-(1H-indol-5-ylamino)-5-[4-methoxy-3-(2-methoxyethoxy)phenyl]nicotinonitrile |
| 155 | 4-(1H-indol-6-ylamino)-5-[4-methoxy-3-(2-methoxyethoxy)phenyl]nicotinonitrile |
| 156 | 4-(1,3-benzothiazol-6-ylamino)-5-[4-methoxy-3-(2-methoxyethoxy)phenyl]nicotinonitrile |
| 157 | 5-[4-methoxy-3-(2-methoxyethoxy)phenyl]-4-[(2-methyl-1H-indol-5-yl)amino]nicotinonitrile |
| 158 | 4-(1H-1,2,3-benzotriazol-5-ylamino)-5-[4-methoxy-3-(2-methoxyethoxy)phenyl]nicotinonitrile |
| 159 | 4-(1H-indol-4-ylamino)-5-[3-methoxy-4-(2-methoxyethoxy)phenyl]nicotinonitrile |
| 160 | 4-(1H-indol-5-ylamino)-5-[3-methoxy-4-(2-methoxyethoxy)phenyl]nicotinonitrile |
| 161 | 4-(1H-indol-6-ylamino)-5-[3-methoxy-4-(2-methoxyethoxy)phenyl]nicotinonitrile |
| 162 | 4-(1,3-benzothiazol-6-ylamino)-5-[3-methoxy-4-(2-methoxyethoxy)phenyl]nicotinonitrile |
| 163 | 5-[3-methoxy-4-(2-methoxyethoxy)phenyl]-4-[(2-methyl-1H-indol-5-yl)amino]nicotinonitrile |
| 164 | 4-(1H-indol-4-ylamino)-5-[3-(2-methoxyethoxy)phenyl]nicotinonitrile |
| 165 | 5-[3-(2-chloroethoxy)phenyl]-4-(1H-indol-4-ylamino)nicotinonitrile |
| 166 | 5-[3-(2-chloroethoxy)phenyl]-4-(1H-indol-6-ylamino)nicotinonitrile |
| 167 | 5-[3-(2-chloroethoxy)phenyl]-4-(1H-indol-5-ylamino)nicotinonitrile |
| 168 | 4-(1H-indol-4-ylamino)-5-{3-[2-(4-methylpiperazin-1-yl)ethoxy]phenyl}nicotinonitrile |
| 169 | 4-(1H-indol-4-ylamino)-5-[3-(2-pyrrolidin-1-ylethoxy)phenyl]nicotinonitrile |
| 170 | 4-(1H-indol-4-ylamino)-5-[3-(2-morpholin-4-ylethoxy)phenyl]nicotinonitrile |
| 171 | 4-(1H-indol-4-ylamino)-5-[3-(2-piperidin-1-ylethoxy)phenyl]nicotinonitrile |
| 172 | 4-(1H-indol-6-ylamino)-5-[3-(2-pyrrolidin-1-ylethoxy)phenyl]nicotinonitrile |
| 173 | 4-(1H-indol-6-ylamino)-5-[3-(2-morpholin-4-ylethoxy)phenyl]nicotinonitrile |
| 174 | 4-(1H-indol-5-ylamino)-5-[3-(2-pyrrolidin-1-ylethoxy)phenyl]nicotinonitrile |
| 175 | 4-(1H-indol-5-ylamino)-5-[3-(2-morpholin-4-ylethoxy)phenyl]nicotinonitrile |
| 176 | 5-(3-{2-[(2-hydroxyethyl)amino]ethoxy}phenyl)-4-(1H-indol-5-ylamino)nicotinonitrile |
| 177 | 4-(1H-indol-5-ylamino)-5-(3-{2-[(2-pyrrolidin-1-ylethyl)amino]ethoxy}phenyl)nicotinonitrile |
| 178 | 5-[3-(2-chloroethoxy)-4-methoxyphenyl]-4-(1H-indol-5-ylamino)nicotinonitrile |
| 179 | 5-{3-[2-(diethylamino)ethoxy]-4-methoxyphenyl}-4-(1H-indol-5-ylamino)nicotinonitrile |
| 180 | 5-{3-[2-(diisopropylamino)ethoxy]-4-methoxyphenyl}-4-(1H-indol-5-ylamino)nicotinonitrile |
| 181 | 5-{3-[2-(benzylamino)ethoxy]-4-methoxyphenyl}-4-(1H-indol-5-ylamino)nicotinonitrile |
| 182 | 4-(1H-indol-5-ylamino)-5-(4-methoxy-3-{2-[(2-methoxyethyl)amino]ethoxy}phenyl)nicotinonitrile |
| 183 | 4-(1H-indol-5-ylamino)-5-(4-methoxy-3-{2-[(5-methyl-1,3,4-thiadiazol-2-yl)amino]ethoxy}phenyl)nicotinonitrile |
| 184 | 4-(1H-indol-5-ylamino)-5-(3-{5-[(4-methylpiperazin-1-yl)methyl]-2-thienyl}phenyl)nicotinonitrile |
| 185 | 4-(1H-indol-5-ylamino)-5-{3-[5-(morpholin-4-ylmethyl)-2-thienyl]phenyl}nicotinonitrile |
| 186 | 4-(1H-indol-5-ylamino)-5-{3-[5-(piperidin-1-ylmethyl)-2-thienyl]phenyl}nicotinonitrile |
| 187 | 5-(3-{5-[(dimethylamino)methyl]-2-thienyl}phenyl)-4-(1H-indol-5-ylamino)nicotinonitrile |
| 188 | 5-(3-bromo-4-methoxyphenyl)-4-(1H-indol-5-ylamino)nicotinonitrile |
| 189 | 4-(1H-indol-5-ylamino)-5-[4-methoxy-3-(2-thienyl)phenyl]nicotinonitrile |
| 190 | 5-(4'-chloro-6-methoxybiphenyl-3-yl)-4-(1H-indol-5-ylamino)nicotinonitrile |
| 191 | 5-(3'-chloro-6-methoxybiphenyl-3-yl)-4-(1H-indol-5-ylamino)nicotinonitrile |
| 192 | 5'-[5-cyano-4-(1H-indol-5-ylamino)pyridin-3-yl]-N-cyclopentyl-2'-methoxybiphenyl-4-carboxamide |
| 193 | 5-(2'-chloro-6-methoxybiphenyl-3-yl)-4-(1H-indol-5-ylamino)nicotinonitrile |
| 194 | 5-[3-(benzyloxy)phenyl]-4-(1H-indol-5-ylamino)nicotinonitrile |
| 195 | 5-[4-(benzyloxy)phenyl]-4-(1H-indol-5-ylamino)nicotinonitrile |
| 196 | 3'-[5-cyano-4-(1H-indol-5-ylamino)pyridin-3-yl]-N-methylbiphenyl-4-carboxamide |
| 197 | N-butyl-3'-[5-cyano-4-(1H-indol-5-ylamino)pyridin-3-yl]biphenyl-4-carboxamide |
| 198 | 3'-[5-cyano-4-(1H-indol-5-ylamino)pyridin-3-yl]-N-(1-ethylpropyl)biphenyl-4-carboxamide |
| 199 | 3'-[5-cyano-4-(1H-indol-5-ylamino)pyridin-3-yl]-N-(2-hydroxyethyl)biphenyl-4-carboxamide |
| 200 | 3'-[5-cyano-4-(1H-indol-5-ylamino)pyridin-3-yl]-N-(2-methoxyethyl)biphenyl-4-carboxamide |
| 201 | 3'-[5-cyano-4-(1H-indol-5-ylamino)pyridin-3-yl]-N-cyclopropylbiphenyl-4-carboxamide |
| 202 | 3'-[5-cyano-4-(1H-indol-5-ylamino)pyridin-3-yl]-N-cyclohexylbiphenyl-4-carboxamide |
| 203 | 3'-[5-cyano-4-(1H-indol-5-ylamino)pyridin-3-yl]-N-(2-pyrrolidin-1-ylethyl)biphenyl-4-carboxamide |
| 204 | N-benzyl-3'-[5-cyano-4-(1H-indol-5-ylamino)pyridin-3-yl]biphenyl-4-carboxamide |
| 205 | 4-(1H-indol-5-ylamino)-5-[4'-(pyrrolidin-1-ylcarbonyl)biphenyl-3-yl]nicotinonitrile |
| 206 | 4-(1H-indol-5-ylamino)-5-[4'-(morpholin-4-ylcarbonyl)biphenyl-3-yl]nicotinonitrile |
| 207 | 4-(1H-indol-5-ylamino)-5-{4'-[(4-methylpiperazin-1-yl)carbonyl]biphenyl-3-yl}nicotinonitrile |
| 208 | 3'-[5-cyano-4-(1H-indol-5-ylamino)pyridin-3-yl]-N-cyclopentylbiphenyl-3-carboxamide |
| 209 | 3'-[5-cyano-4-(1H-indol-5-ylamino)pyridin-3-yl]-N-(4-hydroxybutyl)biphenyl-4-carboxamide |
| 210 | 3'-[5-cyano-4-(1H-indol-5-ylamino)pyridin-3-yl]-N-(3-hydroxypropyl)biphenyl-4-carboxamide |
| 211 | 3'-[5-cyano-4-(1H-indol-5-ylamino)pyridin-3-yl]-N-[2-(methylamino)ethyl]biphenyl-4-carboxamide |
| 212 | 3'-[5-cyano-4-(1H-indol-5-ylamino)pyridin-3-yl]-N-(pyridin-2-ylmethyl)biphenyl-4-carboxamide |
| 213 | 3'-[5-cyano-4-(1H-indol-5-ylamino)pyridin-3-yl]-N-(pyridin-3-ylmethyl)biphenyl-4-carboxamide |
| 214 | 3'-(5-cyano-4-(1H-indol-5-ylamino)pyridin-3-yl)-N-(pyridin-4-ylmethyl)biphenyl-4-carboxamide |
| 215 | N-butyl-3'-[5-cyano-4-(1H-indol-4-ylamino)pyridin-3-yl]biphenyl-4-carboxamide |
| 216 | 3'-[5-cyano-4-(1H-indol-4-ylamino)pyridin-3-yl]-N-(2-hydroxyethyl)biphenyl-4-carboxamide |
| 217 | 5-(3,4-dimethoxyphenyl)-4-[(7-methyl-1H-indol-5-yl)amino]nicotinonitrile |
| 218 | 5-(3,4-dimethoxyphenyl)-4-[(4'-methyl-1H-indol-5-yl)amino]nicotinonitrile |
| 219 | 5-(3,4-dimethoxyphenyl)-4-[1H-indol-5-yl(methyl)amino]nicotinonitrile |
| 220 | 5-(3,4-dimethoxyphenyl)-4-(1H-indol-5-yloxy)nicotinonitrile |
| 221 | 5-(3,4-dimethoxyphenyl)-4-(1H-indol-5-yl)nicotinonitrile |
| 222 | 5-(1-benzofuran-2-yl)-4-[(4-methyl-1*H-*indol-5-yl)amino]nicotinonitrile |
| 223 | 5-(5-formyl-1-benzothien-2-yl)-4-(1*H*-indol-4-ylamino)nicotinonitrile |
| 224 | 5-{5-[(dimethylamino)methyl]-1-benzothien-2-yl}-4-(1*H*-indol-4-ylamino)nicotinonitrile |
| 225 | 5-(4-hydroxyphenyl)-4-(1*H-*indol-4-ylamino)nicotinonitrile |
| 226 | 5-(4-{[(2*S*)-2-amino-3-phenylpropyl]oxy}phenyl)-4-(1*H*-indol-4-ylamino)nicotinonitrile |
| 227 | 5-(5-formyl-1-benzothien-2-yl)-4-[(4-methyl-1*H-*indol-5-yl)amino]nicotinonitrile |
| 228 | 4-[(4-methyl-1*H*-indol-5-yl)amino]-5-[5-(piperidin-1-ylmethyl)-1-benzothien-2-yl]nicotinonitrile |
| 229 | 5-[5-(hydroxymethyl)-1-benzothien-2-yl]-4-[(4-methyl-1*H*-indol-5-yl)amino]nicotinonitrile |
| 230 | 4-(1*H*-indol-5-ylamino)-5-[4-methoxy-3-(2-morpholin-4-ylethoxy)phenyl]nicotinonitrile |
| 231 | 4-(1*H-*indol-5-ylamino)-5-{4-methoxy-3-[2-(4-methylpiperazin-1-yl)ethoxy]phenyl} nicotinonitrile |
| 232 | 4-(1*H*-indol-5-ylamino)-5-[4-methoxy-3-(2-piperazin-1-ylethoxy)phenyl]nicotinonitrile |
| 233 | 4-(1*H*-indol-5-ylamino)-5-[4-methoxy-3-(2-thiomorpholin-4-ylethoxy)phenyl]nicotinonitrile |
| 234 | 5-{3-[2-(4-ethylpiperazin-1-yl)ethoxy]-4-methoxyphenyl}-4-(1*H*-indol-5-ylamino)nicotinonitrile |
| 235 | 4-(1*H*-indol-5-ylamino)-5-[4-methoxy-3-(2-piperidin-1-ylethoxy)phenyl]nicotinonitrile |
| 236 | 5-{3-[2-(dimethylamino)ethoxy]-4-methoxyphenyl}-4-(1*H*-indol-5-ylamino)nicotinonitrile |
| 237 | 5-(3-{2-[bis(2-hydroxyethyl)amino]ethoxy}-4-methoxyphenyl)-4-(1*H-*indol-5-ylamino)nicotinonitrile |
| 238 | 5-{3-[2-(4-hydroxypiperidin-1-yl)ethoxy]-4-methoxyphenyl}-4-(1*H*-indol-5-ylamino)nicotinonitrile |
| 239 | 4-(1*H*-indol-5-ylamino)-5-(4-methoxy-3-{2-[(pyridin-3-ylmethyl)amino]ethoxy}phenyl)nicotinonitrile |
| 240 | 4-(1*H-*indol-5-ylamino)-5-(4-methoxy-3-{2-[(pyridin-4-ylmethyl)amino]ethoxy}phenyl)nicotinonitrile |
| 241 | 4-(1*H*-indol-5-ylamino)-5-(4-methoxy-3-{2-[(pyridin-2-ylmethyl)amino]ethoxy}phenyl)nicotinonitrile |
| 242 | 4-(1*H*-indol-5-ylamino)-5-(4-methoxy-3-{2-[(2-phenylethyl)amino]ethoxy}phenyl)nicotinonitrile |
| 243 | 5-{3-[2-(cyclopentylamino)ethoxy]-4-methoxyphenyl}-4-(1*H*-indol-5-ylamino)nicotinonitrile |
| 244 | 5-{3-[2-(cyclohexylamino)ethoxy]-4-methoxyphenyl}-4-(1*H-*indol-5-ylamino)nicotinonitrile |
| 245 | 5-(3-{2-[(2-furylmethyl)amino]ethoxy}-4-methoxyphenyl)-4-(1*H*-indol-5-ylamino)nicotinonitrile |
| 246 | 5-(2-bromo-4,5-dimethoxyphenyl)-4-(1*H*-indol-5-ylamino)nicotinonitrile |
| 247 | 5-(2-bromo-4,5-dimethoxyphenyl)-4-[(4-methyl-1*H*-indol-5-yl)amino]nicotinonitrile |
| 248 | 4-(1*H*-indol-4-ylamino)-5-[4-methoxy-3-(2-piperidin-1-ylethoxy)phenyl]nicotinonitrile |
| 249 | 5-(3-{2-[(2-hydroxyethyl)amino]ethoxy}-4-methoxyphenyl)-4-(1*H*-indol-4-ylamino)nicotinonitrile |
| 250 | 4-(1*H*-indol-5-ylamino)-5-(4-methoxy-3-{2-[(3-phenylpropyl)amino]ethoxy}phenyl)nicotinonitrile |
| 251 | 4-(1*H*-indol-5-ylamino)-5-(4-methoxy-3-{2-[(2-pyridin-2-ylethyl)amino]ethoxy}phenyl)nicotinonitrile |
| 252 | 4-(1*H*-indol-5-ylamino)-5-(4-methoxy-3-{2-[(2-pyridin-3-ylethyl)amino]ethoxy}phenyl)nicotinonitrile |
| 253 | 4-(1*H*-indol-5-ylamino)-5-(4-methoxy-3-{2-[(2-pyridin-4-ylethyl)amino]ethoxy}phenyl)nicotinonitrile |
| 254 | 5-[3-(dimethylamino)phenyl]-4-(1*H*-indol-4-ylamino)nicotinonitrile |
| 255 | 4-(1*H*-indol-4-ylamino)-5-[3-(methylsulfonyl)phenyl]nicotinonitrile |
| 256 | N-{3-[5-cyano-4-(1*H*-indol-4-ylamino)pyridin-3-yl]phenyl}methanesulfonamide |
| 257 | 4-(1*H*-indol-5-ylamino)-5-phenylnicotinonitrile |
| 258 | 4-(1*H*-indol-5-ylamino)-5-(3-thienyl)nicotinonitrile |
| 259 | 4-(1*H*-indol-5-ylamino)-3,3'-bipyridine-5-carbonitrile |
| 260 | 5-(1,3-benzodioxol-5-yl)-4-(1*H*-indol-5-ylamino)nicotinonitrile |
| 261 | 4-(1*H-*indol-5-ylamino)-3,4'-bipyridine-5-carbonitrile |
| 262 | 5-(3-furyl)-4-(1*H-*indol-5-ylamino)nicotinonitrile |
| 263 | 5-(1*H*-indol-5-yl)-4-(1*H*-indol-5-ylamino)nicotinonitrile |
| 264 | 5-(2,3-dihydro-1,4-benzodioxin-6-yl)-4-(1*H*-indol-5-ylamino)nicotinonitrile |
| 265 | 4-(1*H*-indol-5-ylamino)-5-pyrimidin-5-ylnicotinonitrile |
| 266 | 4-(1*H*-indol-5-ylamino)-5-(2-methoxypyrimidin-5-yl)nicotinonitrile |
| 267 | 4-(1*H*-indol-5-ylamino)-5-(2-thienyl)nicotinonitrile |
| 268 | 5-(1-benzofuran-2-yl)-4-(1*H*-indol-5-ylamino)nicotinonitrile |
| 269 | 5-(3,5-dimethylisoxazol-4-yl)-4-(1*H*-indol-5-ylamino)nicotinonitrile |
| 270 | 5-[3-(hydroxymethyl)phenyl]-4-(1*H*-indol-4-ylamino)nicotinonitrile |
| 271 | 5-{3-[(dimethylamino)methyl]phenyl}-4-(1*H*-indol-4-ylamino)nicotinonitrile |
| 272 | 4-(1*H*-indol-4-ylamino)-5-{5-[(prop-2-yn-1-ylamino)methyl]-1-benzothien-2-yl}nicotinonitrile |
| 273 | 5-{5-[(butylamino)methyl]-1-benzothien-2-yl}-4-(1*H*-indol-4-ylamino)nicotinonitrile |
| 274 | 5-(5-{[(2-hydroxyethyl)amino]methyl}-1-benzothien-2-yl)-4-(1*H*-indol-4-ylamino)nicotinonitrile |
| 275 | 5-(5-{[(3-hydroxypropyl)amino]methyl}-1-benzothien-2-yl)-4-(1H-indol-4-ylamino)nicotinonitrile |
| 276 | 4-(1*H*-indol-4-ylamino)-5-(5-{[(3-methoxypropyl)amino]methyl}-1-benzothien-2-yl)nicotinonitrile |
| 277 | 5-(5-{[(4-hydroxybutyl)amino]methyl}-1-benzothien-2-yl)-4-(1*H*-indol-4-ylamino)nicotinonitrile |
| 278 | 5-{5-[(cyclopropylamino)methyl]-1-benzothien-2-yl}-4-(1*H*-indol-4-ylamino)nicotinonitrile |
| 279 | 5-(5-{[(cyclopropylmethyl)amino]methyl}-1-benzothien-2-yl)-4-(1*H*-indol-4-ylamino)nicotinonitrile |
| 280 | 4-(1*H*-indol-4-ylamino)-5-[5-(pyrrolidin-1-ylmethyl)-1-benzothien-2-yl]nicotinonitrile |
| 281 | 4-(1*H*-indol-4-ylamino)-5-[5-(morpholin-4-ylmethyl)-1-benzothien-2-yl]nicotinonitrile |
| 282 | 4-(1*H*-indol-4-ylamino)-5-(5-{[(2-morpholin-4-ylethyl)amino]methyl}-1-benzothien-2-yl)nicotinonitrile |
| 283 | 4-(1*H*-indol-4-ylamino)-5-[5-(piperidin-1-ylmethyl)-1-benzothien-2-yl]nicotinonitrile |
| 284 | 5-(5-{[4-(hydroxymethyl)piperidin-1-yl]methyl}-1-benzothien-2-yl)-4-(1*H-*indol-4-ylamino)nicotinonitrile |
| 285 | 5-[5-(hydroxymethyl)-1-benzothien-2-yl]-4-(1*H*-indol-4-ylamino)nicotinonitrile |
| 286 | 5-{5-[(benzylamino)methyl]-1-benzothien-2-yl}-4-(1*H*-indol-4-ylamino)riicotinonitrile |
| 287 | 4-(1*H*-indol-4-ylamino)-5-(5-{[(2-phenylethyl)amino]methyl}-1-benzothien-2-yl)nicotinonitrile |
| 288 | 4-(1*H*-indol-4-ylamino)-5-(5-{[(pyridin-2-ylmethyl)amino]methyl}-1-benzothien-2-yl)nicotinonitrile |
| 289 | 4-(1*H*-indol-4-ylamino)-5-(5-{[(pyridin-3-ylmethyl)amino]methyl}-1-benzothien-2-yl)nicotinonitrile |
| 290 | 4-(1*H*-indol-4-ylamino)-5-(5-{[(pyridin-4-ylmethyl)amino]methyl}-1-benzothien-2-yl)nicotinonitrile |
| 291 | 5-[4-(dimethylamino)phenyl]-4-(pyridin-3-ylamino)nicotinonitrile |
| 292 | 5-[4-(dimethylamino)phenyl]-4-(1*H*-indazol-5-ylamino)nicotinonitrile |
| 293 | 5-[4-(dimethylamino)phenyl]-4-(1*H*-indazol-6-ylamino)nicotinonitrile |
| 294 | 5-[4-(dimethylamino)phenyl]-4-[(5-hydroxy-1*H*-pyrazol-3-yl)amino]nicotinonitrile |
| 295 | 4-(1*H*-indazol-5-ylamino)-5-(3-methoxyphenyl)nicotinonitrile |
| 296 | 4-(1*H*-indazol-6-ylamino)-5-(3-methoxyphenyl)nicotinonitrile |
| 297 | 4-[(5-hydroxy-1*H*-pyrazol-3-yl)amino]-5-(3-methoxyphenyl)nicotinonitrile |
| 298 | 5-(3-bromophenyl)-4-[(4-methyl-1*H-*indol-5-yl)amino]nicotinonitrile |
| 299 | 4-(1H-indol-4-ylamino)-5-[4-methoxy-3-(2-morpholin-4-ylethoxy)phenyl]nicotinonitrile |
| 300 | 4-(1H-indol-4-ylamino)-5-{4-methoxy-3-[2-(4-methylpiperazin-1-yl)ethoxy]phenyl}nicotinonitrile |
| 301 | 5-{3-[2-(dimethylamino)ethoxy]-4-methoxyphenyl}-4-(1*H*-indol-4-ylamino)nicotinonitrile |
| 302 | 5-{3-[2-(4-hydroxypiperidin-1-yl)ethoxy]-4-methoxyphenyl}-4-(1*H*-indol-4-ylamino)nicotinonitrile |
| 303 | 5-[3-(2-chloroethoxy)-4-methoxyphenyl]-4-(1H-indol-4-ylamino)nicotinonitrile |
| 304 | 4-[(4-methyl-1*H-*indol-5-yl)amino]-5-[3-(2-thienyl)phenyl]nicotinonitrile |
| 305 | 5-(3,4-dimethoxyphenyl)-4-[(4-ethyl-1*H*-indol-5-yl)amino]nicotinonitrile |
| 306 | 5-[3-(5-formyl-2-thienyl)phenyl]-4-[(4-methyl-1*H*-indol-5-yl)amino]nicotinonitrile |
| 307 | 5-(3-{5-[(dimethylamino)methyl]-2-thienyl}phenyl)-4-[(4-methyl-1H-indol-5-yl)amino]nicotinonitrile |
| 308 | 3'-[5-cyano-4-(1*H*-indol-4-ylamino)pyridin-3-yl]-*N*-(4-hydroxybutyl)biphenyl-4-carboxamide |
| 309 | 3'-{5-cyano-4-[(4-methyl-1*H*-indol-5-yl)amino]pyridin-3-yl}-*N*-(4-hydroxybutyl)biphenyl-4-carboxamide |
| 310 | 4-(1*H*-indol-4-ylamino)-5-[3-(trifluoromethyl)phenyl]nicotinonitrile |
| 311 | 5-(3-cyanophenyl)-4-(1*H*-indol-4-ylamino)nicotinonitrile |
| 312 | 3-[5-cyano-4-(1*H*-indol-4-ylamino)pyridin-3-yl]-*N,N*-dimethylbenzamide |
| 313 | 3-[5-cyano-4-(1*H*-indol-4-ylamino)pyridin-3-yl]-*N*,*N-*dimethylbenzenesulfonamide |
| 314 | 3-[5-cyano-4-(1*H*-indol-4-ylamino)pyridin-3-yl]benzamide |
| 315 | 5-(3-{5-[(dimethylamino)methyl]-2-thienyl}phenyl)-4-(1*H-*indol-4-ylamino)nicotinonitrile |
| 316 | 2-[5-cyano-4-(1*H*-indol-4-ylamino)pyridin-3-yl]-*N*,*N-*dimethylbenzenesulfonamide |
| 317 | N-{4-[5-cyano-4-(1*H-*indol-4-ylamino)pyridin-3-yl]phenyl}methanesulfonamide |
| 318 | 5-(1-benzofuran-2-yl)-4-(1*H*-indol-4-ylamino)nicotinonitrile |
| 319 | 5-dibenzo[*b*,*d*]furan-4-yl-4-(1*H*-indol-4-ylamino)nicotinonitrile |
| 320 | 4-(1*H*-indol-4-ylamino)-5-{1-[(4-methylphenyl)sulfonyl]-1*H*-indol-3-yl}nicotinonitrile |
| 322 | 5-(1-benzothien-2-yl)-4-(1*H*-indol-4-ylamino)nicotinonitrile |
| 323 | 4-(1*H*-indol-4-ylamino)-5-(4-methoxyphenyl)nicotinonitrile |
| 324 | 4-(1*H-*indol-4-ylamino)-5-(2-methoxyphenyl)nicotinonitrile |
| 325 | 5-(1*H*-indol-2-yl)-4-(1*H*-indol-4-ylamino)nicotinonitrile |
| 326 | 4-[5-cyano-4-(1*H*-indol-4-ylamino)pyridin-3-yl]-*N*,*N-*dimethylbenzenesulfonamide |
| 327 | 3-[5-cyano-4-(1*H-*indol-4-ylamino)pyridin-3-yl]benzoic acid |
| 328 | 5-[3-(aminomethyl)phenyl]-4-(1*H*-indol-4-ylamino)nicotinonitrile |
| 329 | 5-(3,4-dimethoxyphenyl)-4-[(2-oxo-2,3-dihydro-1*H*-indol-4-yl)amino]nicotinonitrile |
| 330 | 4-[5-cyano-4-(1*H*-indol-4-ylamino)pyridin-3-yl]-*N*-(2-methoxyethyl)benzamide |
| 331 | 4-(1*H*-indol-4-ylamino)-5-(4-methoxy-3-{2-[(3-phenylpropyl)amino]ethoxy}phenyl)nicotinonitrile |
| 332 | 5-[4-(2-chloroethoxy)phenyl]-4-(1*H*-indol-4-ylamino)nicotinonitrile |
| 333 | 5-[3-(5-formyl-2-thienyl)phenyl]-4-(1H-indol-4-ylamino)nicotinonitrile |
| 334 | 4-(1*H*-indol-4-ylamino)-5-[4-(2-morpholin-4-ylethoxy)phenyl]nicotinonitrile |
| 335 | 4-(1*H*-indol-4-ylamino)-5-{4-[2-(4-methylpiperazin-1-yl)ethoxy]phenyl}nicotinonitrile |
| 336 | 5-(3,4-dimethoxyphenyl)-4-[(5-methyl-1*H*-indol-4-yl)amino]nicotinonitrile |
| 337 | 5-(2,4-dimethoxyphenyl)-4-(1*H*-indol-4-ylamino)nicotinonitrile |
| 338 | 4-(1*H*-indol-4-ylamino)-5-[4-methoxy-3-(2-{[3-(2-oxopyrrolidin-1-yl)propyl]amino}ethoxy)phenyl]nicotinonitrile |
| 339 | 5-[3-(2-{[2-(1*H*-imidazol-4-yl)ethyl]amino}ethoxy)-4-methoxyphenyl]-4-(1*H*-indol-4-ylamino)nicotinonitrile |
| 340 | 4-(1*H*-indol-4-ylamino)-5-(4-methoxy-3-{2-[(3-pyrrolidin-1-ylpropyl)amino]ethoxy}phenyl)nicotinonitrile |
| 341 | 4-(1*H*-indol-4-ylamino)-5-[4-methoxy-3-(2-{[2-(1-methylpyrrolidin-2-yl)ethyl]amino}ethoxy)phenyl]nicotinonitrile |
| 342 | 5-(4-methoxy-3-{2-[(3-phenylpropyl)amino]ethoxy}phenyl)-4-[(4-methyl-1*H*-indol-5-yl)amino]nicotinonitrile |
| 343 | 5-{4-methoxy-3-[2-(4-methylpiperazin-1-yl)ethoxy]phenyl}-4-[(4-methyl-1*H*-indol-5-yl)amino]nicotinonitrile |
| 344 | 5-(3-methoxy-4-{2-[(3-phenylpropyl)amino]ethoxy}phenyl)-4-[(4-methyl-1*H*-indol-5-yl)amino]nicotinonitrile |
| 345 | 5-{4-[2-(dimethylamino)ethoxy]-3-methoxyphenyl}-4-[(4-methyl-1*H-*indol-5-yl)amino]nicotinonitrile |
| 346 | 5-(3-bromophenyl)-4-[(2-oxo-2,3-dihydro-1*H*-indol-4-yl)amino]nicotinonitrile |
| 347 | 4-(1*H-*indol-4-ylamino)-5-(4-{2-[(3-phenylpropyl)amino]ethoxy}phenyl)nicotinonitrile |
| 348 | 5-[4-(2-chloroethoxy)phenyl]-4-[(4-methyl-1*H*-indol-5-yl)amino]nicotinonitrile |
| 349 | 5-(3,4-dimethoxyphenyl)-4-[(2-methyl-1*H*-indol-4-yl)amino]nicotinonitrile |
| 350 | 4-[(4-methyl-1*H*-indol-5-yl)amino]-5-{4-[2-(4-methylpiperazin-1-yl)ethoxy]phenyl}nicotinonitrile |
| 351 | 4-[(4-methyl-1*H*-indol-5-yl)amino]-5-[4-(2-morpholin-4-ylethoxy)phenyl]nicotinonitrile |
| 352 | 5-(1-benzofuran-3-yl)-4-(1*H*-indol-4-ylamino)nicotinonitrile |
| 353 | 4-(1*H*-indol-4-ylamino)-5-(4-methoxy-3-{2-[(2-phenylethyl)amino]ethoxy}phenyl)nicotinonitrile |
| 354 | 4-(1*H*-indol-4-ylamino)-5-(4-methoxy-3-{2-[(2-pyridin-3-ylethyl)amino]ethoxy}phenyl)nicotinonitrile |
| 355 | 4-[(4-methyl-1*H*-indol-5-yl)amino]-5-(4-{2-[(3-phenylpropyl)amino]ethoxy}phenyl)nicotinonitrile |
| 356 | 6'-[3-(dimethylamino)propoxy]-4-(1*H*-indol-4-ylamino)-3,3'-bipyridine-5-carbonitrile |
| 357 | 6'-[3-(dimethylamino)propoxy]-4-[(4-methyl-1*H-*indol-5-yl)amino]-3,3'-bipyridine-5-carbonitrile |
| 358 | 5-(3-hydroxyphenyl)-4-(1*H*-indol-4-ylamino)nicotinonitrile |
| 359 | 4-(1*H*-indol-4-ylamino)-5-[5-(piperazin-1-ylmethyl)-1-benzothien-2-yl]nicotinonitrile |
| 360 | N-({2-[5-cyano-4-(1*H*-indol-4-ylamino)pyridin-3-yl]-1-benzothien-5-yl}methyl)-b-alaninamide |
| 361 | 4-(1*H*-indol-4-ylamino)-6'-[(2-morpholin-4-ylethyl)amino]-3,3'-bipyridine-5-carbonitrile |
| 362 | 4-[(4-methyl-1*H*-indol-5-yl)amino]-6'-[(2-morpholin-4-ylethyl)amino]-3,3'-bipyridine-5-carbonitrile |
| 363 | 5-{2-chloro-4-[2-(dimethylamino)ethoxy]phenyl}-4-[(4-methyl-1*H*-indol-5-yl)amino]nicotinonitrile |
| 364 | 4-(1*H*-indol-4-ylamino)-5-(4-methoxy-3-{2-[(3-morpholin-4-ylpropyl)amino]ethoxy}phenyl)nicotinonitrile |
| 365 | 5-[3-(2-{[3-(1*H*-imidazol-1-yl)propyl]amino}ethoxy)-4-methoxyphenyl]-4-(1*H*-indol-4-ylamino)nicotinonitrile |
| 366 | 5-(3-{[(2*S*)-2-amino-3-phenylpropyl]oxy}phenyl)-4-(1*H*-indol-4-ylamino)nicotinonitrile |
| 367 | 5-{5-[(benzylamino)methyl]-1-benzothien-2-yl}-4-[(4-methyl-1*H*-indol-5-yl)amino]nicotinonitrile |
| 368 | 5-{4-[2-(4-butylpiperazin-1-yl)ethoxy]phenyl}-4-[(4-methyl-1*H*-indol-5-yl)amino]nicotinonitrile |
| 369 | 5-[5-(1,3-dioxan-2-yl)-1-benzofuran-2-yl]-4-(1H indol-4-ylamino)nicotinonitrile |
| 370 | 5-[5-(1,3-dioxan-2-yl)-1-benzofuran-2-yl]-4-[(4-methyl-1*H-*indol-5-yl)amino]nicotinonitrile |
| 371 | 5-(2-chloro-4-methoxyphenyl)-4-[(4-methyl-1*H-*indol-5-yl)amino]nicotinonitrile |
| 372 | 5-[4-(2-chloroethoxy)-3-methoxyphenyl]-4-[(4-methyl-1*H*-indol-5-yl)amino]nicotinonitrile |
| 373 | 5-(1-benzofuran-2-yl)-4-(1*H*-indazol-5-ylamino)nicotinonitrile |
| 374 | 5-(4-hydroxyphenyl)-4-[(4-methyl-1*H*-indol-5-yl)amino]nicotinonitrile |
| 375 | 5-(2-chloro-6-methoxyphenyl)-4-[(4-methyl-1*H-*indol-5-yl)amino]nicotinonitrile |
| 376 | 5-[3-methoxy-4-(2-piperidin-1-ylethoxy)phenyl]-4-[(4-methyl-1*H*-indol-5-yl)amino]nicotinonitrile |
| 377 | 5-{3-methoxy-4-[2-(4-methylpiperazin-1-yl)ethoxy]phenyl}-4-[(4-methyl-1*H*-indol-5-yl)amino]nicotinonitrile |
| 378 | 5-[3-methoxy-4-(2-morpholin-4-ylethoxy)phenyl]-4-[(4-methyl-1*H-*indol-5-yl)amino]nicotinonitrile |
| 379 | 5-(3,4-dimethoxyphenyl)-4-(1*H*-indazol-5-ylamino)nicotinonitrile |
| 380 | 5-(2,3-dichlorophenyl)-4-[(4-methyl-1*H*-indol-5-yl)amino]nicotinonitrile |
| 381 | 5-(4-bromo-2-fluorophenyl)-4-[(4-methyl-1*H*-indol-5-yl)amino]nicotinonitrile |
| 382 | 5-{5-[(dimethylamino)methyl]-1-benzofuran-2-yl}-4-[(4-methyl-1*H*-indol-5-yl)amino]nicotinonitrile |
| 383 | 5-(5-{[(2-hydroxyethyl)amino]methyl}-1-benzofuran-2-yl)-4-[(4-methyl-1*H*-indol-5-yl)amino]nicotinonitrile |
| 384 | 5-(5-{[(3-hydroxypropyl)amino]methyl}-1-benzofuran-2-yl)-4-[(4-methyl-1*H*-indol-5-yl)amino]nicotinonitrite |
| 385 | 5-(5-{[(2,3-dihydroxypropyl)amino]methyl}-1-benzofuran-2-yl)-4-[(4-methyl-1*H-*indol-5-yl)amino]nicotinonitrile |
| 386 | 5-(5-{[(2,3-dihydroxypropyl)(methyl)amino]methyl}-1-benzofuran-2-yl)-4-[(4-methyl-1*H*-indol-5-yl)amino]nicotinonitrile |
| 387 | 5-{5-[(cyclohexylamino)methyl]-1-benzofuran-2-yl}-4-[(4-methyl-1*H-*indol-5-yl)amino]nicotinonitrile |
| 388 | 4-[(4-methyl-1*H*-indol-5-yl)amino]-5-[5-(morpholin-4-ylmethyl)-1-benzofuran-2-yl]nicotinonitrile |
| 389 | 4-[(4-methyl-1*H*-indol-5-yl)amino]-5-[5-(pyrrolidin-1-ylmethyl)-1-benzofuran-2-yl]nicotinonitrile |
| 390 | 4-[(4-methyl-1*H*-indol-5-yl)amino]-5-(5-{[(2-pyrrolidin-1-ylethyl)amino]methyl}-1-benzofuran-2-yl)nicotinonitrile |
| 391 | 4-[(4-methyl-1*H*-indol-5-yl)amino]-5-[5-({[(1-methylpiperidin-4-yl)methyl]amino}methyl)-1-benzofuran-2-yl]nicotinonitrile |
| 392 | 5-(5-{[4-(hydroxymethyl)piperidin-1-yl]methyl}-1-benzofuran-2-yl)-4-[(4-methyl-1*H*-indol-5-yl)amino]nicotinonitrile |
| 393 | 4-[(4-methyl-1*H*-indol-5-yl)amino]-5-{5-[(4-pyrrolidin-1-ylpiperidin-1-yl)methyl]-1-benzofuran-2-yl}nicotinonitrile |
| 394 | 5-[5-(1,4'-bipiperidin-1-ylmethyl)-1-benzofuran-2-yl]-4-[(4-methyl-1*H-*indol-5-yl)amino]nicotinonitrile |
| 395 | 4-[(4-methyl-1*H*-indol-5-yl)amino]-5-{5-[(4-morpholin-4-ylpiperidin-1-yl)methyl]-1-benzofuran-2-yl}nicotinonitrile |
| 396 | 5-[5-({4-[2-(dimethylamino)ethyl]piperazin-1-yl}methyl)-1-benzofuran-2-yl]-4-[(4-methyl-1*H*-indol-5-yl)amino]nicotinonitrile |
| 397 | 4-[(4-methyl-1*H-*indol-5-yl)amino]-5-{5-[(4-pyridin-2-ylpiperazin-1-yl)methyl]-1-benzofuran-2-yl}nicotinonitrile |
| 398 | 4-[(4-methyl-1*H*-indol-5-yl)amino]-5-(5-{[(pyridin-2-ylmethyl)amino]methyl}-1-benzofuran-2-yl)nicotinonitrile |
| 399 | 4-[(4-methyl-1*H*-indol-5-yl)amino]-5-(5-{[(pyridin-3-ylmethyl)amino]methyl}-1-benzofuran-2-yl)nicotinonitrile |
| 400 | 4-[(4-methyl-1*H*-indol-5-yl)amino]-5-(5-{[(pyridin-4-ylmethyl)amino]methyl}-1-benzofuran-2-yl)nicotinonitrile |
| 401 | 4-[(4-methyl-1*H-*indol-5-yl)amino]-5-[5-(morpholin-4-ylmethyl)-2-furyl]nicotinonitrile |
| 402 | 4-[(4-methyl-1*H*-indol-5-yl)amino]-5-[5-(piperidin-1-ylmethyl)-2-furyl]nicotinonitrile |
| 403 | 5-[5-(1,4'-bipiperidin-1'-ylmethyl)-2-furyl]-4-[(4-methyl-1*H*-indol-5-yl)amino]nicotinonitrile |
| 404 | 4-[(4-methyl-1*H-*indol-5-yl)amino]-5-{5-[(4-pyrrolidin-1-ylpiperidin-1-yl)methyl]-2-furyl}nicotinonitrile |
| 405 | 4-[(4-methyl-1*H-*indol-5-yl)amino]-5-{5-[(4-morpholin-4-ylpiperidin-1-yl)methyl]-2-furyl}nicotinonitrile |
| 406 | 5-{5-[(diethylamino)methyl]-2-furyl}-4-[(4-methyl-1*H-*indol-5-yl)amino]nicotinonitrile |
| 407 | 5-{5-[(dibutylamino)methyl]-2-furyl}-4-[(4-methyl-1*H*-indol-5-yl)amino]nicotinonitrile |
| 408 | 5-{5-[(benzylamino)methyl]-2-furyl}-4-[(4-methyl-1*H*-indol-5-yl)amino]nicotinonitrile |
| 409 | 4-[(4-methyl-1*H*-indol-5-yl)amino]-5-(5-{[(3-phenylpropyl)amino]methyl}-2-furyl)nicotinonitrile |
| 410 | 4-[(4-methyl-1*H*-indol-5-yl)amino]-5-[5-(pyrrolidin-1-ylmethyl)-2-furyl]nicotinonitrile |
| 411 | 4-[(4-methyl-1*H-*indol-5-yl)amino]-5-[5-(thiomorpholin-4-ylmethyl)-2-furyl]nicotinonitrile |
| 412 | 5-(3,4-dimethoxyphenyl)-4-[(6-methyl-1*H*-indol-5-yl)amino]nicotinonitrile |
| 413 | 5-(1-benzofuran-2-yl)-4-[(6-methyl-1*H*-indol-5-yl)amino]nicotinonitrile |
| 414 | 5-(4-{2-[(2-hydroxyethyl)amino]ethoxy}phenyl)-4-[(4-methyl-1*H-*indol-5-yl)amino]nicotinonitrile |
| 415 | 5-(4-{2-[(3-hydroxypropyl)amino]ethoxy}phenyl)-4-[(4-methyl-1*H*-indol-5-yl)amino]nicotinonitrile |
| 416 | 5-(4-{2-[(2-ethoxyethyl)amino]ethoxy}phenyl)-4-[(4-methyl-1*H*-indol-5-yl)amino]nicotinonitrile |
| 417 | 5-[4-(2-{[2-(dimethylamino)ethyl]amino}ethoxy)phenyl]-4-[(4-methyl-1*H-*indol-5-yl)amino]nicotinonitrile |
| 418 | 4-[(4-methyl-1*H-*indol-5-yl)amino]-5-[4-(2-pyrrolidin-1-ylethoxy)phenyl]nicotinonitrile |
| 419 | 5-{4-[2-(benzylamino)ethoxy]phenyl}-4-[(4-methyl-1*H*-indol-5-yl)amino]nicotinonitrile |
| 420 | 4-[(4-methyl-1*H-*indol-5-yl)amino]-5-(4-{2-[(1-methylpiperidin-4-yl)amino]ethoxy}phenyl)nicotinonitrile |
| 421 | 4-[(4-methyl-1*H*-indol-5-yl)amino]-5-[4-(2-{[(1-methylpiperidin-4-yl)methyl]amino}ethoxy)phenyl]nicotinonitrile |
| 422 | 5-(4-{2-[4-(hydroxymethyl)piperidin-1-yl]ethoxy}phenyl)-4-[(4-methyl-1*H*-indol-5-yl)amino]nicotinonitrile |
| 423 | 4-[(4-methyl-1*H*-indol-5-yl)amino]-5-{4-[2-(4-pyrrolidin-1-ylpiperidin-1-yl)ethoxy]phenyl}nicotinonitrile |
| 424 | 4-[(4-methyl-1*H*-indol-5-yl)amino]-5-{4-[2-(4-morpholin-4-ylpiperidin-1-yl)ethoxy]phenyl}nicotinonitrile |
| 425 | 5-{4-[2-(4-ethylpiperazin-1-yl)ethoxy]phenyl}-4-[(4-methyl-1*H*-indol-5-yl)amino]nicotinonitrile |
| 426 | 5-{4-[2-(4-methyl-1,4-diazepan-1-yl)ethoxy]phenyl}-4-[(4-methyl-1*H-*indol-5-yl)amino]nicotinonitrile |
| 427 | 5-(4-{2-[4-(2-hydroxyethyl)piperazin-1-yl]ethoxy}phenyl)-4-[(4-methyl-1*H*-indol-5-yl)amino]nicotinonitrile |
| 428 | 5-[4-(2-{4-[2-(dimethylamino)ethyl]piperazin-1-yl}ethoxy)phenyl]-4-[(4-methyl-1*H-*indol-5-yl)amino]nicotinonitrile |
| 429 | 5-[4-(2-{[3-(1*H*-imidazol-1-yl)propyl]amino}ethoxy)phenyl]-4-[(4-methyl-1*H*-indol-5-yl)amino]nicotinonitrile |
| 430 | 4-[(4-methyl-1*H*-indol-5-yl)amino]-5-{4-[2-(4-pyridin-2-ylpiperazin-1-yl)ethoxy]phenyl}nicotinonitrile |
| 431 | 4-[(4-methyl-1*H*-indol-5-yl)amino]-5-{4-[2-(4-pyridin-4-ylpiperazin-1-yl)ethoxy]phenyl}nicotinonitrile |
| 432 | 4-[(4-methyl-1*H-*indol-5-yl)amino]-5-(4-{2-[(pyridin-2-ylmethyl)amino]ethoxy}phenyl)nicotinonitrile |
| 433 | 4-[(4-methyl-1*H*-indol-5-yl)amino]-5-(4-{2-[(pyridin-3-ylmethyl)amino]ethoxy}phenyl)nicotinonitrile |
| 434 | 4-[(4-methyl-1*H*-indol-5-yl)amino]-5-(4-{2-[(pyridin-4-ylmethyl)amino]ethoxy}phenyl)nicotinonitrile |
| 435 | 4-[(4-methyl-1*H*-indol-5-yl)amino]-5-{4-[2-(4-phenylpiperidin-1-yl)ethoxy]phenyl}nicotinonitrile |
| 436 | 5-(5-{[4-(dimethylamino)piperidin-1-yl]methyl}-2-furyl)-4-[(4-methyl-1*H-*indol-5-yl)amino]nicotinonitrile |
| 437 | 5-{5-[(4-isopropylpiperazin-1-yl)methyl]-2-furyl}-4-[(4-methyl-1*H*-indol-5-yl)amino]nicotinonitrile |
| 438 | 4-[(4-methyl-1*H*-indol-5-yl)amino]-5-{5-[2-(4-methylpiperazin-1-yl)ethoxy]-1-benzofuran-2-yl}nicotinonitrile |
| 439 | 5-(3,4-dimethoxyphenyl)-4-{[2-(4-methylpiperazin-1-yl)ethyl]amino}nicotinonitrile |
| 440 | 5-(3,4-dimethoxyphenyl)-4-{[3-(4-methylpiperazin-1-yl)propyl]amino}nicotinonitrile |
| 441 | 4-({[*trans*-4-(aminomethyl)cyclohexyl]methyl}amino)-5-(3,4-dimethoxyphenyl)nicotinonitrile |
| 442 | 4-{[(*trans*-4-aminocyclohexyl)methyl]amino}-5-(3,4-dimethoxyphenyl)nicotinonitrile |
| 443 | 4-({[*cis*-3-(aminomethyl)cyclohexyl]methyl}amino)-5-(3,4-dimethoxyphenyl)nicotinonitrile |
| 444 | 5-(3,4-dimethoxyphenyl)-4-[(2-piperidin-4-ylethyl)amino]nicotinonitrile |
| 445 | 5-(3,4-dimethoxyphenyl)-4-[(piperidin-4-ylmethyl)amino]nicotinonitrile |
| 446 | 4-[(*cis-*4-aminocyclohexyl)amino]-5-(3,4-dimethoxyphenyl)nicotinonitrile |
| 447 | 5-(3,4-dimethoxyphenyl)-4-{[2-(1-methylpiperidin-4-yl)ethyl]amino}nicotinonitrile |
| 448 | 5-(3,4-dimethoxyphenyl)-4-{[(1-methylpiperidin-4-yl)methyl]amino}nicotinonitrile |
| 449 | 4-[(4-methyl-1*H*-indol-5-yl)amino]-5-{4-[(4-methylpiperazin-1-yl)methyl]phenyl}nicotinonitrile |
| 450 | 4-[(4-methyl-1*H*-indol-5-yl)amino]-5-[4-(pyrrolidin-1-ylmethyl)phenyl]nicotinonitrile |
| 451 | 4-[(4-methyl-1*H*-indol-5-yl)amino]-5-[4-(morpholin-4-ylmethyl)phenyl]nicotinonitrile |
| 452 | 5-{4-[(dimethylamino)methyl]phenyl}-4-[(4-methyl-1*H-*indol-5-yl)amino]nicotinonitrile |
| 453 | 4-[(4-methyl-1*H*-indol-5-yl)amino]-2'-(morpholin-4-ylmethyl)-3,4'-bipyridine-5-carbonitrile |
| 454 | 4-[(4-methyl-1*H-*indol-5-yl)amino]-5-{3-[(4-methylpiperazin-1-yl)methyl]phenyl}nicotinonitrile |
| 455 | 4-[(4-methyl-1*H*-indol-5-yl)amino]-5-[3-(pyrrolidin-1-ylmethyl)phenyl]nicotinonitrile |
| 456 | 4-[(4-methyl-1*H*-indol-5-yl)aminol-5-[3-(morpholin-4-ylmethyl)phenyl]nicotinonitrile |
| 457 | 5-{3-[(dimethylamino)methyl]phenyl}-4-[(4-methyl-1*H*-indol-5-yl)amino]nicotinonitrile |
| 458 | 5-(4-{[(2R)-2-amino-3-phenylpropyl]oxy}phenyl)-4-(1H-indol-4-ylamino)nicotinonitrile |
| 459 | 5-{2-fluoro-4-[(4-methylpiperazin-1-yl)methyl]phenyl}-4-[(4-methyl-1*H-*indol-5-yl)amino]nicotinonitrile |
| 460 | 5-[4-(3-chloropropoxy)phenyl]-4-[(4-methyl-1*H*-indol-5-yl)amino]nicotinonitrile |
| 461 | 4-[(4-methyl-1*H-*indol-5-yl)amino]-5-[5-(piperidin-1-ylmethyl)-1-benzofuran-2-yl]nicotinonitrile |
| 462 | 4-[(4-methyl-1*H*-indol-5-yl)amino]-5-{5-[(4-methylpiperazin-1-yl)methyl]-1-benzofuran-2-yl}nicotinonitrile |
| 463 | 5-(5-formyl-2-thienyl)-4-[(4-methyl-1*H*-indol-5-yl)amino]nicotinonitrile |
| 464 | 4-[(4-methyl-1*H*-indol-5-yl)amino]-5-{5-[(4-methylpiperazin-1-yl)methyl]-2-thienyl}nicotinonitrile |
| 465 | 5-(5-formyl-1-benzofuran-2-yl)-4-[(4-methyl-1*H*-indol-5-yl)amino]nicotinonitrile |
| 466 | 5-(3-methyl-1-benzofuran-2-yl)-4-[(4-methyl-1*H*-indol-5-yl)amino]nicotinonitrile |
| 467 | 4-[(4-methyl-1*H*-indol-5-yl)amino]-3,4'-bipyridine-5-carbonitrile |
| 468 | 4-[(4-methyl-1*H*-indol-5-yl)amino]-5-{5-[(4-methylpiperazin-1-yl)methyl]-2-furyl}nicotinonitrile |
| 469 | 2'-chloro-4-[(4-methyl-1*H*-indol-5-yl)amino]-3,4'-bipyridine-5-carbonitrile |
| 470 | 5-{2-chloro-4-[2-(4-methylpiperazin-1-yl)ethoxy]phenyl}-4-[(4-methyl-1H-indol-5-yl)amino]nicotinonitrile |
| 471 | 2-chloro-4-[(4-methyl-1*H*-indol-5-yl)amino]-3,3'-bipyridine-5-carbonitrile |
| 472 | 4-[(4-methyl-1H-indol-5-yl)amino]-5-{4-[3-(4-methylpiperazin-1-yl)propoxy]phenyl}nicotinonitrile |
| 473 | 4-[(4-methyl-1H-indol-5-yl)amino]-5-[4-(3-morpholin-4-ylpropoxy)phenyl]nicotinonitrile |
| 474 | 4-[(4-methyl-1H-indol-5-yl)amino]-5-[4-(3-piperidin-1-ylpropoxy)phenyl]nicotinonitrile |
| 475 | 5-{4-[3-(dimethylamino)propoxy]phenyl}-4-[(4-methyl-1H-indol-5-yl)amino]nicotinonitrile |
| 477 | 5-[3,4-bis(2-methoxyethoxy)phenyl]-4-[(4-methyl-1*H*-indol-5-yl)amino]nicotinonitrile |
| 478 | 5-[3-methoxy-4-(2-methoxyethoxy)phenyl]-4-[(4-methyl-1*H*-indol-5-yl)amino]nicotinonitrile |
| 481 | 5-[5-(hydroxymethyl)-1-benzofuran-2-yl]-4-[(4-methyl-1*H*-indol-5-yl)amino]nicotinonitrile |
| 482 | 5-[4-(2-methoxyethoxy)phenyl]-4-[(4-methyl-1*H*-indol-5-yl)amino]nicotinonitrile |
| 483 | 5-[3-(2-methoxyethoxy)phenyl]-4-[(4-methyl-1*H*-indol-5-yl)amino]nicotinonitrile |
| 484 | 5-(5-formyl-1-benzofuran-^{.}2-yl)-4-(1*H*-indol-5-ylamino)nicotinonitrile |
| 485 | 4-(1H-indol-5-ylamino)-5-{5-[(4-methylpiperazin-1-yl)methyl]-1-benzofuran-2-yl}nicotinonitrile |
| 486 | 5-{5-[(4-cyclopentylpiperazin-1-yl)methyl]-2-furyl}-4-[(4-methyl-1H-indol-5-yl)amino]nicotinonitrile |
| 487 | 5-{5-[(1,1-dioxidothiomorpholin-4-yl)methyl]-2-furyl}-4-[(4-methyl-1*H-*indol-5-yl)amino]nicotinonitrile |
| 488 | 5-(3,4-dimethoxyphenyl)-4-(1H-pyrrolo[2,3-b]pyridin-5-ylamino)nicotinonitrile |
| 489 | 5-(5-formyl-2-furyl)-4-[(4-methyl-1*H*-indol-5-yl)amino]nicotinonitrile |
| 490 | 5-[4-(4-chlorobutoxy)phenyl]-4-[(4-methyl-1*H*-indol-5-yl)amino]nicotinonitrile |
| 491 | 4-[(4-methyl-1H-indol-5-yl)amino]-5-{4-[4-(4-methylpiperazin-1-yl)butoxy]phenyl}nicotinonitrile |
| 492 | 4-[(4-ch(oro-1*H*-pyrrolo[2,3-b]pyridin-5-yl)amino)-5-[3-methoxy-4-(2-methoxyethoxy)phenyl]nicotinonitrile |
| 494 | 5-[4-(2-chloroethoxy)phenyl]-4-[(6-methyl-1*H*-indol-5-yl)amino]nicotinonitrile |
| 495 | 5-(5-formyl-1-benzofuran-2-yl)-4-[(6-methyl-1*H-*indol-5-yl)amino]nicotinonitrile |
| 496 | 4-[(6-methyl-1H-indol-5-yl)amino]-5-{4-[2-(4-methylpiperazin-1-yl)ethoxy]phenyl}nicotinonitrile |
| 497 | 4-[(6-methyl-1H-indol-5-yl)amino]-5-{5-[(4-methylpiperazin-1-yl)methyl]-1-benzofuran-2-yl nicotinonitrile |
| 498 | 4-[(7-chloro-4-methyl-1*H*-indol-5-yl)amino]-5-(3,4-dimethoxyphenyl)nicotinonitrile |
| 499 | 5-[5-(hydroxymethyl)-1-benzofuran-2-yl]-4-[(6-methyl-1*H*-indol-5-yl)amino]nicotinonitrile |
| 500 | 5-{5-[(diethylamino)methyl]-1-benzofuran-2-yl}-4-[(6-methyl-1H-indol-5-yl)amino]nicotinonitrile |
| 501 | 5-[3-(4-chlorobutoxy)phenyl]-4-[(4-methyl-1*H*-indol-5-yl)amino]nicotinonitrile |
| 502 | 4-[(4-methyl-1H-indol-5-yl)amino]-5-{3-[4-(4-methylpiperazin-1-yl)butoxy]phenyl}nicotinonitrile |
| 503 | 4-[(4-methyl-1*H*-indol-5-yl)amino]-5-{5-[(4-methylpiperazin-1-yl)methyl]-3-furyl}nicotinonitrile |
| 504 | 4-[(6-methyl-1H-indol-5-yl)amino]-5-[4-(2-piperidin-1-ylethoxy)phenyl]nicotinonitrile |
| 505 | 5-{4-[2-(4-hydroxypiperidin-1-yl)ethoxy]phenyl}-4-[(6-methyl-1H-indol-5-yl)amino]nicotinonitrile |
| 506 | 4-[(6-methyl-1H-indol-5-yl)ammo]-5-{4-[2-(4-pyrrolidin-1-ylpiperidin-1-yl)ethoxy]phenyl}nicotinonitrile |
| 507 | 4-[(4-methyl-1*H*-indol-5-yl)amino]-6'-morpholin-4-yl-3,3'-bipyridine-5-carbonitrile |
| 508 | 4-[(4-methyl-1*H*-indol-5-yl)amino]-6'-piperidin-1-yl-3,3'-bipyridine-5-carbonitrile |
| 509 | 4-[(4-methyl-1*H*-indol-5-yl)amino]-5-pyrimidin-5-ylnicotinonitrile |
| 510 | 4-[(4-methyl-1*H*-indol-5-yl)amino]-5-(2-piperidin-1-ylpyrimidin-5-yl)nicotinonitrile |
| 511 | 4-[(4-methyl-1*H-*indol-5-yl)amino]-5-(2-morpholin-4-ylpyrimidin-5-yl)nicotinonitrile |
| 512 | 4-[(4-methyl-1*H*-indol-5-yl)amino]-5-(2-pyrrolidin-1-ylpyrimidin-5-yl)nicotinonitrile |
| 513 | 5-[2-(dimethylamino)pyrimidin-5-yl]-4-[(4-methyl-1*H*-indol-5-yl)amino]nicotinonitrile |
| 514 | 5-(1-benzothien-2-yl)-4-[(4-methyl-1*H*-indol-5-yl)amino]nicotinonitrile |
| 515 | 5-[4-(2-chloroethoxy)phenyl]-4-(1*H*-indol-5-ylamino)nicotinonitrile |
| 516 | 5-(5-formyl-3-thienyl)-4-[(4-methyl-1*H*-indol-5-yl)amino]nicotinonitrile |
| 517 | 5-(4-formyl-2-furyl)-4-[(4-methyl-1*H*-indol-5-yl)amino]nicotinonitrile |
| 518 | 4-[(4-methyl-1H-indol-5-yl)amino]-5-{5-[(4-methylpiperazin-1-yl)methyl]-3-thienyl}nicotinonitrile |
| 519 | 4-[(4-methyl-1*H*-indol-5-yl)amino]-3,3'-bipyridine-5-carbonitrile |
| 520 | 4-(1H-indol-5-ylamino)-5-{4-[2-(4-methylpiperazin-1-yl)ethoxy]phenyl}nicotinonitrile |
| 521 | 4-[(4-methyl-1H-indol-5-yl)amino]-5-{5-[(4-methylpiperazin-1-yl)methyl]-1-benzothien-2-yl}nicotinonitrile |
| 522 | 4-[(4-chloro-1H-pyrrolo[2,3-b]pyridin-5-yl)amino]-5-(3,4-dimethoxyphenyl)nicotinonitrile |
| 523 | 5-(3,4-dimethoxyphenyl)-4-(1*H*-indol-5-ylamino)nicotinonitrile 1-oxide |
| 524 | 4-[(*trans*-4-aminocyclohexyl)amino]-5-(3,4-dimethoxyphenyl)nicotinonitrile |
| 525 | 1-butyl-3-(4-{5-cyano-4-[(4-methyl-1*H*-indol-5-yl)amino]pyridin-3-yl}phenyl)urea |
| 526 | methyl (4-{5-cyano-4-[(4-methyl-1H-indol-5-yl)amino]pyridin-3-yl}phenyl)carbamate |
| 527 | benzyl(4-{5-cyano-4-[(4-methyl-1H-indol-5-yl)amino]pyridin-3-yl}-2-fluorophenyl)carbamate |
| 528 | 4-methoxybenzyl (4-{5-cyano-4-[(4-methyl-1*H*-indol-5-yl)amino]pyridin-3-yl}-2-fluorophenyl)carbamate |
| 529 | 4-[(7-chloro-4-methyl-1*H-*indol-5-yl)amino]-5-{5-[(4-methylpiperazin-1-yl)methyl]-1-benzofuran-2-yl}nicotinonitrile |
| 530 | 5-(3,4-dimethoxyphenyl)-4-[(4-methyl-1*H*-indol-7-yl)amino]nicotinonitrile |
| 531 | 5-(1-benzofuran-2-yl)-4-[(7-chloro-4-methyl-1*H*-indol-5-yl)amino]nicotinonitrile |
| 532 | 4-[(7-chloro-4-methyl-1*H*-indol-5-yl)amino}-5-(5-formyl-1-benzofuran-2-yl)nicotinonitrile |
| 533 | 5-[4-(2-chloroethoxy)phenyl]-4-[(7-chloro-4-methyl-1*H*-indol-5-yl)amino]nicotinonitrile |
| 534 | 4-[(7-chloro-4-methyl-1*H-*indol-5-yl)amino]-5-{4-[2-(dimethylamino)ethoxy]phenyl}nicotinonitrile |
| 535 | 4-[(7-chloro-4-methyl-1*H*-indol-5-yl)amino]-5-{4-[2-(4-methylpiperazin-1-yl)ethoxy]phenyl}nicotinonitrile |
| 536 | *tert*-butyl 4-[(2-{5-cyano-4-[(4-methyl-1*H*-indol-5-yl)amino]pyridin-3-yl}-1-benzofuran-5-yl)methyl]piperazine-1-carboxylate |
| 537 | 5-(2-formyl-1-methyl-1*H*-imidazol-5-yl)-4-[(4-methyl-1*H*-indol-5-yl)amino]nicotinonitrile |
| 538 | 4-[(4-methyl-1*H*-indol-5-yl)amino]-5-(5-{[4-(methylsulfonyl)piperazin-1-yl]methyl}-1-benzofuran-2-yl)nicotinonitrile |
| 539 | 4-[(4-methyl-1*H*-indol-5-yl)amino]-5-{1-methyl-2-[(4-methylpiperazin-1-yl)methyl]-1*H*-imidazol-5-yl}nicotinonitrile |
| 540 | 4-[(4-methyl-1*H*-indol-5-yl)amino]-5-[5-(piperazin-1-ylmethyl)-1-benzofuran-2-yl]nicotinonitrile |
| 541 | 4-[(4-methyl-1*H*-indol-5-yl)amino]-5-(1,3-thiazol-2-yl)nicotinonitrile |
| 542 | 5-(1-methyl-1*H*-imidazol-2-yl)-4-[(4-methyl-1*H*-indol-5-yl)amino]nicotinonitrile |
| 543 | 4-[(4-methyl-1*H-*indol-5-yl)amino]-5-(1,3-thiazol-4-yl)nicotinonitrile |
| 544 | 5-(3,4-dimethoxyphenyl)-4-(1*H*-indol-7-ylamino)nicotinonitrile |
| 545 | 5-(3,4-dimethoxyphenyl)-4-[(4-methoxy-1*H*-indol-5-yl)amino]nicotinonitrile |
| 546 | 5-(3,4-dimethoxyphenyl)-4-[(4-fluoro-1*H*-indol-5-yl)amino]nicotinonitrile |
| 547 | 4-[(7-chloro-4-methyl-1*H*-indol-5-yl)amino]-5-[5-(piperazin-1-ylmethyl)-1-benzofuran-2-yl]nicotinonitrile |
| 548 | *tert*-butyl 4-[(2-{4-[(7-chloro-4-methyl-1*H*-indol-5-yl)amino]-5-cyanopyridin-3-yl}-1-benzofuran-5-yl)methyl]piperazine-1-carboxylate |
| 549 | 5-(3,4-dimethoxyphenyl)-4-[(2,4-dimethyl-1*H-*indol-5-yl)amino]nicotinonitrile |
| 550 | 5-{2-[(dimethylamino)methyl]phenyl}-4-[(4-methyl-1*H*-indol-5-yl)amino]nicotinonitrile |
| 551 | 5-(5-formyl-2-methoxyphenyl)-4-[(4-methyl-1*H*-indol-5-yl)amino]nicotinonitrile |
| 552 | 5-{2-methoxy-5-[(4-methylpiperazin-1-yl)methyl]phenyl}-4-[(4-methyl-1*H*-indol-5-yl)amino]nicotinonitrile |
| 553 | 5- {5-[(4-ethylpiperazin-1-yl)methyl]-1-benzofuran-2-yl}-4-[(4-methyl-1H-indol-5-yl)amino]nicotinonitrile |
| 554 | 4-[(4-methyl-1H-indol-5-yl)amino]-5-{5-[(4-methyl-4-oxidopiperazin-1-yl)methyl]-1-benzofuran-2-yl}nicotinonitrile |
| 555 | 5-(3,4-dimethoxyphenyl)-4-[(1,4-dimethyl-1H-indol-5-yl)amino]nicotinonitrile |
| 556 | 3-{5-cyano-4-[(4-methyl-1H-indol-5-yl)amino]pyridin-3-yl}benzoic acid |
| 557 | 5-(2-methoxyphenyl)-4-[(4-methyl-1*H*-indol-5-yl)amino]nicotinonitrile |
| 558 | 5-(3-methoxyphenyl)-4-[(4-methyl-1*H*-indol-5-yl)amino]nicotinonitrile |
| 559 | 5-(4-methoxyphenyl)-4-[(4-methyl-1*H*-indol-5-yl)amino]nicotinonitrile |
| 560 | 4-[(4-methyl-1*H*-indol-5-yl)aminol-5-phenylnicotinonitrile |
| 561 | 4-[(4-methyl-1*H*-indol-5-yl)amino]-5-(2-thienyl)nicotinonitrile |
| 562 | 4-[(4-methyl-1*H*-indol-5-yl)amino]-5-(3-thienyl)nicotinonitrile |
| 563 | 5-(3-furyl)-4-[(4-methyl-1*H*-indol-5-yl)amino]nicotinonitrile |
| 564 | 5-(1-methyl-1H-imidazol-5-yl)-4-[(4-methyl-1H-indol-5-yl)amino]nicotinonitrile |
| 565 | 4'-[(4-methyl-1H-indol-5-yl)amino]-2,3'-bipyridine-5'-carbonitrile |
| 566 | 1-(4-{5-cyano-4-[(4-methyl-1H-indol-5-yl)amino]pyridin-3-yl}phenyl)-3-cyclopropylurea |
| 567 | 1-(4-{5-cyano-4-[(4-methyl-1H-indol-5-yl)amino]pyridin-3-yl}phenyl)-3-methylurea |
| 568 | 3-(4-{5-cyano-4-[(4-methyl-1H-indol-5-yl)amino]pyridin-3-yl}phenyl)-1,1-dimethylurea |
| 569 | N-(4-{5-cyano-4-[(4-methyl-1H-indol-5-yl)amino]pyridin-3-yl}phenyl)morpholine-4-carboxamide |
| 570 | 4-[(4-methyl-1H-indol-5-yl)amino]-5-(4-nitrophenyl)nicotinonilrile |
| 571 | 5-(4-aminophenyl)-4-[(4-methyl-1H-indol-5-yl)amino]nicotinonitrile |
| 572 | 5-(3-aminophenyl)-4-[(4-methyl-1H-indol-5-yl)amino]nicotinonitrile |
| 573 | 5-(2-aminophenyl)-4-[(4-methyl-1H-indol-5-yl)amino]nicotinonitrile |
| 574 | 5-[4-(dimethylamino)phenyl]-4-[(4-methyl-1H-indol-5-yl)amino]nicotinonitrile |
| 575 | 5-[3-(dimethylamino)phenyl]-4-[(4-methyl-1H-indol-5-yl)amino]nicotinonitrile |
| 576 | N-(4-{5-cyano-4-[(4-methyl-1H-indol-5-yl)amino]pyridin-3-yl}phenyl)acetamide |
| 577 | N-(2-{5-cyano-4-[(4-methyl-1H-indol-5-yl)amino]pyridin-3-yl}phenyl)acetamide |
| 578 | N-(3-{5-cyano-4-[(4-methyl-1H-indol-5-yl)amino]pyridin-3-yl}phenyl)acetamide |
| 579 | N-(4-{5-cyano-4-[(4-methyl-1H-indol-5-yl)amino]pyridin-3-yl}phenyl)-2-methylpropanamide |
| 580 | 4-{5-cyano-4-[(4-methyl-1H-indol-5-yl)amino]pyridin-3-yl}-N-methylbenzam ide |
| 581 | 4-[(4-methyl-1*H*-indol-5-yl)amino]-5-(1-naphthyl)nicotinonitrile |
| 582 | 4-[(4-methyl-1*H*-indol-5-yl)amino]-5-(2-naphthyl)nicotinonitrile |
| 583 | 4-[(4-methyl-1*H*-indol-5-yl)amino]-5-(1-methyl-1*H*-pyrazol-5-yl)nicotinonitrile |
| 584 | 4-[(4-methyl-1*H*-indol-5-yl)amino]-5-(1H-pyrazol-4-yl)nicotinonitrile |
| 585 | 5-(1-benzothiophen-3-yl)-4-[(4-methyl-1*H*-indol-5-yl)amino]nicotinonitrile |
| 586 | 5-(1-methyl-1*H*-indol-2-yl)-4-[(4-methyl-1*H*-indol-5-yl)amino]nicotinonitrile |
| 587 | 5-(1*H*-indol-5-yl)-4-[(4-methyl-1*H-*indol-5-yl)amino]nicotinonitrile |
| 588 | 5-(1*H*-indol-6-yl)-4-[(4-methyl-1*H*-indol-5-yl)amino]nicotinonitrile |
| 589 | 4-[(4-methyl-1*H*-indol-5-yl)amino]-5-quinolin-3-ylnicotinonitrile |
| 590 | 4-[(4-methyl-1*H*-indol-5-yl)amino]-5-quinolin-8-ylnicotinonitrile |
| 591 | 5-(1-benzofuran-5-yl)-4-[(4-methyl-1*H*-indol-5-yl)amino]nicotinonitrile |
| 592 | 4-(4-methyl-1H-indol-5-ylamino)-5-(quinolin-5-yl)nicotinonitrile |
| 593 | 5-(dibenzo[b,d]thiophen-3-yl)-4-(4-methyl-1H-indol-5-ylamino)nicotinonitrile |
| 594 | 5-(benzo[b]thiophen-5-yl)-4-(4-methyl-1H-indol-5-ylamino)nicotinonitrile |
| 595 | 5-(1H-indol-4-yl)-4-(4-methyl-1H-indol-5-ylamino)nicotinonitrile |
| 596 | 4-[(2,4-dimethyl-1H-indol-5-yl)amino]-5-{5-[(4-methylpiperazin-1-yl)methyl]-1-benzofuran-2-yl}nicotinonitrile |
| 597 | 4-[(2,4-dimethyl-1H-indol-5-yl)amino]-5-[5-(piperazin-1-ylmethyl)-1-benzofuran-2-yl]nicotinonitrile |
| 598 | 4-[(4-methyl-1H-indol-5-yl)amino]-5-{6-[(4-methylpiperazin-1-yl)methyl]-1-benzofuran-2-yl}nicotinonitrile |
| 599 | 4-[(4-methyl-1H-indol-5-yl)amino]-5-[6-(piperazin-1-ylmethyl)-1-benzofuran-2-yl]nicotinonitrile |
| 600 | 4-[(4-methyl-1H-indol-5-yl)amino]-5-{4-[(piperazin-1-yl)methyl]phenyl}nicotinonitrile |
| 601 | 4-[(2,4-dimethyl-1H-indol-5-yl)amino]-5-{4-[(4-methylpiperazin-1-yl)methyl]phenyl}nicotinonitrile |
| 602 | 4-(2,4-dimethyl-1H-indol-5-ylamino)-5-{3-[2-(4-methylpiperazin-1-yl)ethoxy]phenyl}nicotinonitrile |
| 603 | 5-{4-[2-(dimethylamino)ethoxy]-3-methoxyphenyl}-4-[(2,4-dimethyl-1H-indol-5-yl)amino]nicotinonitrile |
| 604 | 4-[(2,4-dimethyl-1H-indol-5-yl)amino]-5-{4-[2-(4-methylpiperazin-1-yl)ethoxy]phenyl}nicotinonitrile |
| 605 | 4-[(4-methyl-1H-indol-5-yl)amino]-5-{4-[2-(piperazin-1-yl)ethoxy]phenyl}nicotinonitrile |
| 606 | 4-(4-methyl-1H-indol-5-ylamino)-2'-((4-methylpiperazin-1-yl)methyl)-3,4'-bipyridine-5-carbonitrile |
| 607 | 4-(4-methyl-1H-indol-5-ylamino)-2'-((piperazin-1-yl)methyl)-3,4'-bipyridine-5-carbonitrile |
| 608 | 4'-(4-methyl-1H-indol-5-ylamino)-5-((4-methylpiperazin-1-yl)methyl)-2,3'-bipyridine-5'-carbonitrile |
| 609 | 4'-(4-methyl-1H-indol-5-ylamino)-5-(morpholinomethyl)-2,3'-bipyridine-5'-carbonitrile |
| 610 | 4'-(4-methyl-1H-indol-5-ylamino)-5-((piperazin-1-yl)methyl)-2,3'-bipyridine-5'-carbonitrile |
| 611 | 4'-(4-methyl-1H-indol-5-ylamino)-6-((4-methylpiperazin-1-yl)methyl)-2,3'-bipyridine-5'-carbonitrile |
| 612 | 4'-(4-methyl-1H-indol-5-ylamino)-6-(morpholinomethyl)-2,3'-bipyridine-5'-carbonitrile |
| 613 | 4'-(4-methyl-1H-indol-5-ylamino)-6-((piperazin-1-yl)methyl)-2,3'-bipyridine-5'-carbonitrile |
| 614 | 4'-(4-methyl-1H-indol-5-ylamino)-4-(morpholinomethyl)-2,3'-bipyridine-5'-carbonitrile |
| 615 | 4'-(4-methyl-1H-indol-5-ylamino)-4-((4-methylpiperazin-1-yl)methyl)-2,3'-bipyridine-5'-carbonitrile |
| 616 | 4'-(4-methyl-1H-indol-5-ylamino)-4-((piperazin-1-yl)methyl)-2,3'-bipyridine-5'-carbonitrile |
| 617 | 4-(4-methyl-1H-indol-5-ylamino)-5'-((4-methylpiperazin-1-yl)methyl)-3,3'-bipyridine-5-carbonitrile |
| 618 | 4-(4-methyl-1H-indol-5-ylamino)-5'-((piperazin-1-yl)methyl)-3,3'-bipyridine-5-carbonitrile |
| 619 | 4-(4-methyl-1H-indol-5-ylamino)-5'-(morpholinomethyl)-3,3'-bipyridine-5-carbonitrile |
| 620 | 4-(4-methyl-1H-indol-5-ylamino)-6'-((4-methylpiperazin-1-yl)methyl)-3,3'-bipyridine-5-carbonitrile |
| 621 | 4-(4-methyl-1H-indol-5-ylamino)-6'-((piperazin-1-yl)methyl)-3,3'-bipyridine-5-carbonitrile |
| 622 | 4-(4-methyl-1H-indol-5-ylamino)-6'-(morpholinomethyl)-3,3'-bipyridine-5-carbonitrile |
| 623 | 4-(4-methyl-1H-indol-5-ylamino)-5-(3-(piperazin-1-ylmethyl)phenyl)nicotinonitrile |
| 624 | 4-(4-methyl-1H-indol-5-ylamino)-5-(4-(piperazin-1-ylmethyl)phenyl)nicotinonitrile |
| 625 | 4-({[*cis*-4-(aminomethyl)cyclohexyl]methyl}amino)-5-(3,4-dimethoxyphenyl)nicotinonitrile |

Pharmaceutically acceptable salts of the compounds of formula I, which can have an acidic moiety, can be formed using organic and inorganic bases. Both mono and polyanionic salts are contemplated, depending on the number of acidic hydrogens available for deprotonation. Suitable salts formed with bases include metal salts, such as alkali metal or alkaline earth metal salts, for example sodium, potassium, or magnesium salts; ammonia salts and organic amine salts, such as those formed with morpholine, thiomorpholine, piperidine, pyrrolidine, a mono-, di- or tri-lower alkylamine (e.g., ethyl-tert-butyl-, diethyl-, diisopropyl-, triethyl-, tributyl- or dimethylpropylamine), or a mono-, di-, or trihydroxy lower alkylamine (e.g., mono-, di- or triethanolamine). Specific non-limiting examples of inorganic bases include NaHCO₃, Na₂CO₃, KHCO₃, K₂CO₃, Cs₂CO₃, LiOH, NaOH, KOH, NaH₂PO₄, Na₂HPO₄, and Na₃PO₄. Internal salts also can be formed. Similarly, when a compound disclosed herein contains a basic moiety, salts can be formed using organic and inorganic acids. For example, salts can be formed from the following acids: acetic, benzenesulfonic, benzoic, camphorsulfonic, citric, dichloroacetic, ethenesulfonic, formic, fumaric, gluconic, glutamic, hippuric, hydrobromic, hydrochloric, isethionic, lactic, maleic, malic, malonic, mandelic, methanesulfonic, mucic, naphthalenesulfonic, nitric, oxalic, pamoic, pantothenic, phosphoric, phthalic, propionic, succinic, sulfuric, tartaric, and toluenesulfonic, as well as other known pharmaceutically acceptable acids.

Esters of the compounds of formula I can include various pharmaceutically acceptable esters known in the art that can be metabolized into the free acid form (e.g., a free carboxylic acid form) in a mammal. Examples of such esters include alkyl esters (e.g., of 1 to 10 carbon atoms), cycloalkyl esters (e.g., of 3-10 carbon atoms), aryl esters (e.g., of 6-14 carbon atoms, including of 6-10 carbon atoms), and heterocyclic analogues thereof (e.g., of 3-14 ring atoms, 1-3 of which can be selected from oxygen, nitrogen, and sulfur heteroatoms), wherein the alcohol residue can include further substituents. In some embodiments, esters of the compounds disclosed herein can be C₁₋₁₀ alkyl esters, such as methyl esters, ethyl esters, propyl esters, isopropyl esters, butyl esters, isobutyl esters, t-butyl esters, pentyl esters, isopentyl esters, neopentyl esters, and hexyl esters; C₃₋₁₀ cycloalkyl esters, such as cyclopropyl esters, cyclopropylmethyl esters, cyclobutyl esters, cyclopentyl esters, and cyclohexyl esters; or aryl esters, such as phenyl esters, benzyl esters, and tolyl esters.

The compounds disclosed herein may be suitable for modification so as to provide prodrugs. As used herein, "prodrug" refers to a moiety that produces, generates or releases a compound of the present teachings when administered to a mammalian subject. Prodrugs can be prepared by modifying functional groups present in the compounds in such a way that the modifications are cleaved, either by routine manipulation or *in vivo,* from the parent compounds. Examples of prodrugs include compounds as described herein that contain one or more molecular moieties appended to a hydroxyl, amino, sulfhydryl, or carboxyl group of the compound, and that when administered to a mammalian subject, is cleaved *in vivo* to form the free hydroxyl, amino, sulfhydryl, or carboxyl group, respectively. Examples of prodrugs can include acetate, formate, and benzoate derivatives of alcohol and amine functional groups in the compounds of the present teachings. Preparation and use of prodrugs is discussed in T. Higuchi and V. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press,1987.

The present teachings also provide pharmaceutical compositions that include at least one compound described herein and one or more pharmaceutically acceptable carriers, excipients, or diluents. Examples of such carriers are well known to those skilled in the art and can be prepared in accordance with acceptable pharmaceutical procedures, such as, for example, those described in Remington: The Science and Practice of Pharmacy, 20th edition, ed. Alfonso R. Gennaro, Lippincott Williams & Wilkins, Baltimore, MD (2000). As used herein, "pharmaceutically acceptable" refers to a substance that is acceptable for use in pharmaceutical applications from a toxicological perspective and does not adversely interact with the active ingredient. Accordingly, pharmaceutically acceptable carriers are those that are compatible with the other ingredients in the formulation and are biologically acceptable. Supplementary active ingredients can also be incorporated into the pharmaceutical compositions.

Compounds of the present teachings can be useful for treating a pathological condition or disorder in a mammal, for example, a human. As used herein, "treating" refers to partially or completely alleviating and/or ameliorating the condition and/or symptoms thereof. The present teachings accordingly include a method of providing to a mammal a pharmaceutical composition that includes a compound of the present teachings in combination or association with a pharmaceutically acceptable carrier. Compounds of the present teachings can be administered alone or in combination with other therapeutically effective compounds or therapies for the treatment of a pathological condition or disorder. As used herein, "therapeutically effective" refers to a substance or an amount that elicits a desirable biological activity or effect.

The present teachings also include use of the compounds disclosed herein as active therapeutic substances for the treatment of a pathological condition or disorder mediated by a protein kinase such as protein kinase C (PKC) and its theta isoform (PKCθ). The pathological condition or disorder can include inflammatory diseases and autoimmune diseases such as asthma, colitis, multiple sclerosis, psoriasis, arthritis, rheumatoid arthritis, osteoarthritis, and joint inflammation. Accordingly, the present teachings further provide methods of treating these pathological conditions and disorders using the compounds described herein. In some embodiments, the methods include identifying a mammal having a pathological condition or disorder mediated by a protein kinase such as PKC and PKCθ, and providing to the mammal an effective amount of a compound as described herein. In some embodiments, the method includes administering to a mammal a pharmaceutical composition that includes a compound disclosed herein in combination or association with a pharmaceutically acceptable carrier.

The present teachings further include use of the compounds disclosed herein as active therapeutic substances for the prevention and/or inhibition of the pathological condition or disorder listed above. Accordingly, the present teachings further provide methods of preventing and/or inhibiting these pathological conditions and disorders using the compounds described herein. In some embodiments, the methods include identifying a mammal having a pathological condition or disorder mediated by a protein kinase such as PKC and PKCθ, and providing to the mammal an effective amount of a compound as described herein. In some embodiments, the method includes administering to a mammal a pharmaceutical composition that includes a compound disclosed herein in combination or association with a pharmaceutically acceptable carrier.

Compounds of the present teachings can be administered orally or parenterally, neat or in combination with conventional pharmaceutical carriers. Applicable solid carriers can include one or more substances which can also act as flavoring agents, lubricants, solubilizers, suspending agents, fillers, glidants, compression aids, binders or tablet-disintegrating agents, or encapsulating materials. The compounds can be formulated in conventional manner, for example, in a manner similar to that used for known antiinflammatory agents. Oral formulations containing an active compound disclosed herein can include any conventionally used oral form, including tablets, capsules, buccal forms, troches, lozenges and oral liquids, suspensions or solutions. In powders, the carrier can be a finely divided solid, which is an admixture with a finely divided active compound. In tablets, an active compound can be mixed with a carrier having the necessary compression properties in suitable proportions and compacted in the shape and size desired. The powders and tablets may contain up to 99% of the active compound.

Capsules can contain mixtures of active compound(s) with inert filler(s) and/or diluent(s) such as the pharmaceutically acceptable starches (e.g., corn, potato or tapioca starch), sugars, artificial sweetening agents, powdered celluloses (e.g., crystalline and microcrystalline celluloses), flours, gelatins, gums, and the like.

Useful tablet formulations can be made by conventional compression, wet granulation or dry granulation methods and utilize pharmaceutically acceptable diluents, binding agents, lubricants, disintegrants, surface modifying agents (including surfactants), suspending or stabilizing agents, including magnesium stearate, stearic acid, sodium lauryl sulfate, talc, sugars, lactose, dextrin, starch, gelatin, cellulose, methyl cellulose, microcrystalline cellulose, sodium carboxymethyl cellulose, carboxymethylcellulose calcium, polyvinylpyrrolidine, alginic acid, acacia gum, xanthan gum, sodium citrate, complex silicates, calcium carbonate, glycine, sucrose, sorbitol, dicalcium phosphate, calcium sulfate, lactose, kaolin, mannitol, sodium chloride, low melting waxes, and ion exchange resins. Preferred surface modifying agents include nonionic and anionic surface modifying agents. Representative examples of surface modifying agents include poloxamer 188, benzalkonium chloride, calcium stearate, cetostearl alcohol, cetomacrogol emulsifying wax, sorbitan esters, colloidal silicon dioxide, phosphates, sodium dodecylsulfate, magnesium aluminum silicate, and triethanolamine. Oral formulations herein can utilize standard delay or time-release formulations to alter the absorption of the active compound(s). The oral formulation can also comprise a compound as described herein in water or fruit juice, containing appropriate solubilizers or emulsifiers as needed.

Liquid carriers can be used in preparing solutions, suspensions, emulsions, syrups, elixirs, and for inhaled delivery. A compound described herein can be dissolved or suspended in a pharmaceutically acceptable liquid carrier such as water, an organic solvent, or a mixture of both, or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers, and osmo-regulators. Examples of liquid carriers for oral and parenteral administration include water (particularly containing additives as described above, e.g., cellulose derivatives such as a sodium carboxymethyl cellulose solution), alcohols (including monohydric alcohols and polyhydric alcohols, e.g., glycols) and their derivatives, and oils (e.g., fractionated coconut oil and arachis oil). For parenteral administration, the carrier can be an oily ester such as ethyl oleate and isopropyl myristate. Sterile liquid carriers are used in sterile liquid form compositions for parenteral administration. The liquid carrier for pressurized compositions can be halogenated hydrocarbon or other pharmaceutically acceptable propellants.

Liquid pharmaceutical compositions, which are sterile solutions or suspensions, can be utilized by, for example, intramuscular, intraperitoneal or subcutaneous injection. Sterile solutions can also be administered intravenously. Compositions for oral administration can be in either liquid or solid form.

Preferably the pharmaceutical composition is in unit dosage form, for example, as tablets, capsules, powders, solutions, suspensions, emulsions, granules, or suppositories. In such form, the pharmaceutical composition can be sub-divided in unit dose(s) containing appropriate quantities of the active compound. The unit dosage forms can be packaged compositions, for example, packeted powders, vials, ampoules, prefilled syringes or sachets containing liquids. Alternatively, the unit dosage form can be a capsule or tablet itself, or it can be the appropriate number of any such compositions in package form. Such unit dosage form may contain from about 1 mg/kg of active compound to about 500 mg/kg of active compound, and can be given in a single dose or in two or more doses. Such doses can be administered in any manner useful in directing the active compound(s) to the recipient's bloodstream, including orally, via implants, parenterally (including intravenous, intraperitoneal and subcutaneous injections), rectally, vaginally, and transdermally. Such administrations can be carried out using the compounds of the present teachings including pharmaceutically acceptable salts thereof, in lotions, creams, foams, patches, suspensions, solutions, and suppositories (rectal and vaginal).

When administered for the treatment or inhibition of a particular disease state or disorder, it is understood that an effective dosage can vary depending upon many factors such as the particular compound utilized, the mode of administration, and severity of the condition being treated, as well as the various physical factors related to the individual being treated. In therapeutic applications, a compound of the present teachings can be provided to a patient already suffering from a disease in an amount sufficient to cure or at least partially ameliorate the symptoms of the disease and its complications. The dosage to be used in the treatment of a specific individual typically must be subjectively determined by the attending physician. The variables involved include the specific condition and its state as well as the size, age and response pattern of the patient.

In some cases, for example those in which the lung is the targeted organ, it may be desirable to administer a compound directly to the airways of the patient, using devices such as metered dose inhalers, breath-operated inhalers, multidose dry-powder inhalers, pumps, squeeze-actuated nebulized spray dispensers, aerosol dispensers, and aerosol nebulizers. For administration by intranasal or intrabronchial inhalation, the compounds of the present teachings can be formulated into a liquid composition, a solid composition, or an aerosol composition. The liquid composition can include, by way of illustration, one or more compounds of the present teachings dissolved, partially dissolved, or suspended in one or more pharmaceutically acceptable solvents and can be administered by, for example, a pump or a squeeze-actuated nebulized spray dispenser. The solvents can be, for example, isotonic saline or bacteriostatic water. The solid composition can be, by way of illustration, a powder preparation including one or more compounds of the present teachings intermixed with lactose or other inert powders that are acceptable for intrabronchial use, and can be administered by, for example, an aerosol dispenser or a device that breaks or punctures a capsule encasing the solid composition and delivers the solid composition for inhalation. The aerosol composition can include, by way of illustration, one or more compounds of the present teachings, propellants, surfactants, and co-solvents, and can be administered by, for example, a metered device. The propellants can be a chlorofluorocarbon (CFC), a hydrofluoroalkane (HFA), or other propellants that are physiologically and environmentally acceptable.

Compounds described herein can be administered parenterally or intraperitoneally. Solutions or suspensions of these active compounds or pharmaceutically acceptable salts, hydrates, or esters thereof can be prepared in water suitably mixed with a surfactant such as hydroxyl-propylcellulose. Dispersions can also be prepared in glycerol, liquid polyethylene glycols, and mixtures thereof in oils. Under ordinary conditions of storage and use, these preparations typically contain a preservative to inhibit the growth of microorganisms.

The pharmaceutical forms suitable for injection can include sterile aqueous solutions or dispersions and sterile powders for the extemporaneous preparation of sterile injectable solutions or dispersions. In preferred embodiments, the form is sterile and its viscosity permits it to flow through a syringe. The form preferably is stable under the conditions of manufacture and storage and can be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

Compounds described herein can be administered transdermally, i.e., administered across the surface of the body and the inner linings of bodily passages including epithelial and mucosal tissues. Such administration can be carried out using the compounds of the present teachings including pharmaceutically acceptable salts, hydrates, and esters thereof, in lotions, creams, foams, patches, suspensions, solutions, and suppositories (rectal and vaginal). Topical formulations that deliver active compound(s) through the epidermis can be useful for localized treatment of inflammation and arthritis.

Transdermal administration can be accomplished through the use of a transdermal patch containing an active compound and a carrier that can be inert to the active compound, can be non-toxic to the skin, and can allow delivery of the active compound for systemic absorption into the blood stream via the skin. The carrier can take any number of forms such as creams and ointments, pastes, gels, and occlusive devices. The creams and ointments can be viscous liquid or semisolid emulsions of either the oil-in-water or water-in-oil type. Pastes comprised of absorptive powders dispersed in petroleum or hydrophilic petroleum containing the active compound can also be suitable. A variety of occlusive devices can be used to release the active compound into the blood stream, such as a semi-permeable membrane covering a reservoir containing the active compound with or without a carrier, or a matrix containing the active compound. Other occlusive devices are known in the literature.

Compounds described herein can be administered rectally or vaginally in the form of a conventional suppository. Suppository formulations can be made from traditional materials, including cocoa butter, with or without the addition of waxes to alter the suppository's melting point, and glycerin. Water-soluble suppository bases, such as polyethylene glycols of various molecular weights, can also be used.

Lipid formulations or nanocapsules can be used to introduce compounds of the present teachings into host cells either *in vitro* or *in vivo.* Lipid formulations and nanocapsules can be prepared by methods known in the art.

To increase the effectiveness of compounds of the present teachings, it can be desirable to combine a compound with other agents effective in the treatment of the target disease. For inflammatory diseases, other active compounds (i.e., other active ingredients or agents) effective in their treatment, and particularly in the treatment of asthma and arthritis, can be administered with active compounds of the present teachings. The other agents can be administered at the same time or at different times than the compounds disclosed herein.

Throughout the description, where compositions are described as having, including, or comprising specific components, or where processes are described as having, including, or comprising specific process steps, it is contemplated that compositions of the present teachings also consist essentially of, or consist of, the recited components, and that the processes of the present teachings also consist essentially of, or consist of, the recited processing steps.

In the application, where an element or component is said to be included in and/or selected from a list of recited elements or components, it should be understood that the element or component can be any one of the recited elements or components and can be selected from a group consisting of two or more of the recited elements or components. The use of the term "include" should be generally understood as open-ended and non-limiting unless specifically stated otherwise.

The use of the singular herein includes the plural (and vice versa) unless specifically stated otherwise. In addition, where the use of the term "about" is before a quantitative value, the present teachings also include the specific quantitative value itself, unless specifically stated otherwise.

It should be understood that the order of steps or order for performing certain actions is immaterial so long as the present teachings remain operable. Moreover, two or more steps or actions may be conducted simultaneously.

As used herein, a "compound" refers to the compound itself and its pharmaceutically acceptable salts, hydrates and esters, unless otherwise understood from the context of the description or expressly limited to one particular form of the compound, i.e., the compound itself, or a pharmaceutically acceptable salt, hydrate or ester thereof.

As used herein, "halo" or "halogen" refers to fluoro, chloro, bromo, and iodo.

As used herein, "oxo" refers to a double-bonded oxygen (i.e., =O).

As used herein, as a moiety or part of a moiety, "alkyl" refers to a straight-chain or branched saturated hydrocarbon group. In some embodiments, an alkyl group can have from 1 to 10 carbon atoms (e.g, from 1 to 6 carbon atoms). Examples of alkyl groups include methyl (Me), ethyl (Et), propyl (e.g., n-propyl and isopropyl), butyl (e.g., n-butyl, isobutyl, s-butyl, t-butyl), pentyl groups (e.g., n-pentyl, isopentyl, neopentyl), and the like. A lower alkyl group typically has up to 6 carbon atoms, i.e., one to six carbon atoms. Examples of lower alkyl groups include methyl, ethyl, propyl (e.g., n-propyl and isopropyl), and butyl groups (e.g., n-butyl, isobutyl, s-butyl, t-butyl).

As used herein, as a moiety or part of a moiety, "alkenyl" refers to a straight-chain or branched alkyl group having one or more carbon-carbon double bonds. In some embodiments, an alkenyl group can have from 2 to 10 carbon atoms (e.g., from 2 to 6 carbon atoms). Examples of alkenyl groups include ethenyl, propenyl, butenyl, pentenyl, hexenyl, butadienyl, pentadienyl, hexadienyl groups, and the like. The one or more carbon-carbon double bonds can be internal (such as in 2-butene) or terminal (such as in 1-butene).

As used herein, as a moiety or part of a moiety, "alkynyl" refers to a straight-chain or branched alkyl group having one or more carbon-carbon triple bonds. In some embodiments, an alkynyl group can have from 2 to 10 carbon atoms (e.g., from 2 to 6 carbon atoms). Examples of alkynyl groups include ethynyl, propynyl, butynyl, pentynyl, and the like. The one or more carbon-carbon triple bonds can be internal (such as in 2-butyne) or terminal (such as in 1-butyne).

As used herein, "alkoxy" refers to an -O-alkyl group. In some embodiments, an alkoxy group can have from 1 to 10 carbon atoms (e.g., from 1 to 6 carbon atoms). Examples of alkoxy groups include methoxy, ethoxy, propoxy (e.g., n-propoxy and isopropoxy), t-butoxy groups, and the like.

As used herein, "alkylthio" refers to an -S-alkyl group. Examples of alkylthio groups include methylthio, ethylthio, propylthio (e.g., n-propylthio and isopropylthio), t-butylthio groups, and the like.

As used herein, "haloalkyl" refers to an alkyl group having one or more halogen substituents. In some embodiments, a haloalkyl group can have from 1 to 10 carbon atoms (e.g., from 1 to 6 carbon atoms). Examples of haloalkyl groups include CF₃, C₂F₅, CHF₂, CH₂F, CCl₃, CHCl₂, CH₂Cl, C₂Cl₅, and the like. Perhaloalkyl groups, i.e., alkyl groups wherein all of the hydrogen atoms are replaced with halogen atoms (e.g., CF₃ and C₂F₅), are included within the definition of "haloalkyl."

As used herein, "cycloalkyl" refers to a non-aromatic carbocyclic group. A cycloalkyl group can be monocyclic (e.g., cyclohexyl) or polycyclic (e.g., containing fused, bridged, and/or spiro ring systems), wherein the carbon atoms are located inside or outside of the ring system. A cycloalkyl group, as a whole, can have from 3 to 14 ring atoms (e.g., from 3 to 8 carbon atoms for a monocyclic cycloalkyl group and from 7 to 14 carbon atoms for a polycyclic cycloalkyl group). Any suitable ring position of the cycloalkyl group can be covalently linked to the defined chemical structure. Examples of cycloalkyl groups include cyclopropyl, cyclobutyl, cyclopentyl, cyclohexyl, cycloheptyl, norbornyl, norpinyl, norcaryl, adamantyl, and spiro[4.5]decanyl groups, as well as their homologs, isomers, and the like.

As used herein, "heteroatom" refers to an atom of any element other than carbon or hydrogen and includes, for example, nitrogen, oxygen, sulfur, phosphorus, and selenium.

As used herein, "cycloheteroalkyl" refers to a non-aromatic cycloalkyl group that contains at least one ring heteroatom selected from O, N and S, which may be the same or different. A cycloheteroalkyl group, as a whole, can have, for example, from 3 to 14 ring atoms and contains from 1 to 5 ring heteroatoms (e.g., from 3-7 ring atoms for a monocyclic cycloheteroalkyl group and from 7 to 14 ring atoms for a polycyclic cycloheteroalkyl group). One or more N or S atoms in a cycloheteroalkyl ring may be oxidized (e.g., morpholine N-oxide, thiomorpholine S-oxide, thiomorpholine S,S-dioxide). Cycloheteroalkyl groups can also contain one or more oxo groups, such as piperidone, oxazolidinone, pyrimidine-2,4(1*H*,3*H*)-dione, pyridin-2(1H)-one, and the like. Examples of cycloheteroalkyl groups include, among others, morpholine, thiomorpholine, pyran, imidazolidine, oxazolidine, pyrazolidine, pyrrolidine, tetrahydrofuran, tetrahydrothiophene, piperidine, piperazine, and the like.

As used herein, "aryl" refers to an aromatic monocyclic hydrocarbon ring system or a polycyclic ring system in which two or more aromatic hydrocarbon rings are fused (i.e., having a bond in common with) together or. An aryl group can have from 6 to 14 carbon atoms in its ring system, which can include multiple fused rings. In some embodiments, a polycyclic aryl group can have from 8 to 14 carbon atoms. Any suitable ring position of the aryl group can be covalently linked to the defined chemical structure. Examples of aryl groups having only aromatic carbocyclic ring(s) include phenyl, 1-naphthyl (bicyclic), 2-naphthyl (bicyclic), anthracenyl (tricyclic), phenanthrenyl (tricyclic) and like groups.

As used herein, "heteroaryl" refers to an aromatic monocyclic ring system containing at least 1 ring heteroatom selected from oxygen (O), nitrogen (N) and sulfur (S) or a polycyclic ring system where at least one of the rings present in the ring system is aromatic and contains at least 1 ring heteroatom. When more than one ring heteroatoms are present they may be the same or different. Polycyclic heteroaryl groups include two or more heteroaryl rings fused together and monocyclic heteroaryl rings fused to one or more aromatic carbocyclic rings. A heteroaryl group, as a whole, can have, for example, from 5 to 14 ring atoms and contain 1-5 ring heteroatoms. The heteroaryl group can be attached to the defined chemical structure at any heteroatom or carbon atom that results in a stable structure. Generally, heteroaryl rings do not contain O-O, S-S, or S-O bonds. However, one or more N or S atoms in a heteroaryl group can be oxidized (e.g., pyridine N-oxide, thiophene S-oxide, thiophene S,S-dioxide). Examples of heteroaryl groups include, for example, the 5-membered monocyclic and 5-6 bicyclic ring systems shown below: 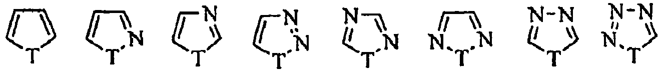 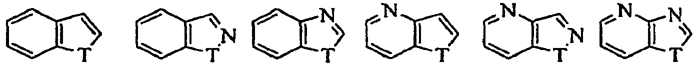 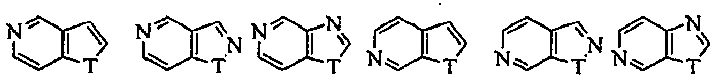 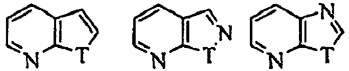 wherein T is O, S, or NH.
Examples of such heteroaryl rings include pyrrolyl, furyl, thienyl, pyridyl, pyrimidyl, pyridazinyl, pyrazinyl, triazolyl, tetrazolyl, pyrazolyl, imidazolyl, isothiazolyl, thiazolyl, thiadiazolyl, isoxazolyl, oxazolyl, oxadiazolyl, indolyl, isoindolyl, benzofuryl, benzothienyl, quinolyl, 2-methylquinolyl, isoquinolyl, quinoxalyl, quinazolyl, benzotriazolyl, benzimidazolyl, benzothiazolyl, benzisothiazolyl, benzisoxazolyl, benzoxadiazolyl, benzoxazolyl, cinnolinyl, 1H-indazolyl, 2H-indazolyl, indolizinyl, isobenzofuyl, naphthyridinyl, phthalazinyl, pteridinyl, purinyl, oxazolopyridinyl, thiazolopyridinyl, imidazopyridinyl, furopyridinyl, thienopyridinyl, pyridopyrimidinyl, pyridopyrazinyl, pyridopyridazinyl, thienothiazolyl, thienoxazolyl, thienoimidazolyl groups, and the like. Further examples of heteroaryl groups include benzofuropyridinyl groups, and the like.

The compounds of the present teachings can include a "divalent group" defined herein as a linking group capable of forming a covalent bond with two other moieties. For example, compounds described herein can include a divalent C₁₋₁₀ alkyl group, such as, for example, a methylene group.

At various places in the present specification, substituents of compounds are disclosed in groups or in ranges. It is specifically intended that the description include each and every individual subcombination of the members of such groups and ranges. For example, the term "C₁₋₁₀ alkyl" is specifically intended to individually disclose C₁, C₂, C₃, C₄, C₅, C₆, C₇, C₈, C₉, C₁₀, C₁-C₁₀, C₁-C₉, C₁-C₈, C₁-C₇, C₁-C₆, C₁-C₅, C₁-C₄, C₁-C₃, C₁-C₂, C₂-C₁₀, C₂-C₉, C₂-C₈, C₂-C₇, C₂-C₈, C₂-C₅, C₂-C₄, C₂-C₃, C₃-C₁₀, C₃-C₉, C₃-C₈, C₃-C₇, C₃-C₆, C₃-C₅, C₃-C₄, C₄-C₁₀, C₄-C₉, C₄-C₈, C₄-C₇, C₄-C₆, C₄-C₅, C₅-C₁₀, C₅-C₉, C₅-C₈, C₅-C₇, C₅-C₆, C₆-C₁₀, C₆-C₉, C₆-C₈, C₆-C₇. C₇-C₁₀, C₇-C₉, C₇-C₈, C₈-C₁₀, C₈-C₉, and C₉-C₁₀ alkyl. By way of other examples, the term "5-14 membered heteroaryl group" is specifically intended to individually disclose a heteroaryl group having 5, 6, 7, 8, 9, 10, 11, 12, 13, 14, 5-14, 5-13, 5-12, 5-11, 5-10, 5-9, 5-8, 5-7, 5-6, 6-14, 6-13, 6-12, 6-11, 6-10, 6-9, 6-8, 6-7, 7-14, 7-13, 7-12, 7-11, 7-10, 7-9, 7-8, 8-14, 8-13, 8-12, 8-11, 8-10, 8-9, 9-14, 9-13, 9-12, 9-11, 9-10, 10-14, 10-13, 10-12, 10-11, 11-14, 11-13, 11-12, 12-14, 12-13, and 13-14 ring atoms; and the phrase "optionally substituted with 1-4 substituents" is specifically intended to individually disclose a chemical group that can include 0, 1, 2, 3, 4, 0-4, 0-3, 0-2, 0-1, 1-4, 1-3, 1-2, 2-4, 2-3, and 3-4 substituents.

Compounds described herein can contain an asymmetric atom (also referred as a chiral center), and some of the compounds can contain one or more asymmetric atoms or centers, which can thus give rise to optical isomers (enantiomers) and diastereomers. The present teachings and compounds disclosed herein include such optical isomers (enantiomers) and diastereomers (geometric isomers), as well as the racemic and resolved, enantiomerically pure and stereoisomers, as well as other mixtures of the R and S stereoisomers and pharmaceutically acceptable salts thereof. Optical isomers can be obtained in pure form by standard procedures known to those skilled in the art, which include diastereomeric salt formation, kinetic resolution, and asymmetric synthesis. The present teachings also encompass cis and trans isomers of compounds containing alkenyl moieties (e.g., alkenes and imines). It is also understood that the present teachings encompass all possible regioisomers, and mixtures thereof, which can be obtained in pure form by standard separation procedures known to those skilled in the art, and include column chromatography, thin-layer chromatography, and high-performance liquid chromatography.

Throughout the specification, structures may or may not be presented with chemical names. Where any question arises as to nomenclature, the structure prevails.

An aspect of the present teachings relates to methods of preparing the compounds disclosed herein. The compounds of the present teachings can be prepared in accordance with the procedures outlined in the schemes below, from commercially available starting materials, compounds known in the literature, or readily prepared intermediates, by employing standard synthetic methods and procedures known to those skilled in the art. Standard synthetic methods and procedures for the preparation of organic molecules and functional group transformations and manipulations can be readily obtained from the relevant scientific literature or from standard textbooks in the field. It will be appreciated that where typical or preferred process conditions (i.e., reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures. Those skilled in the art of organic synthesis will recognize that the nature and order of the synthetic steps presented may be varied for the purpose of optimizing the formation of the compounds described herein.

The processes described herein can be monitored according to any suitable method known in the art. For example, product formation can be monitored by spectroscopic means, such as nuclear magnetic resonance spectroscopy (e.g., ¹H or ¹³C), infrared spectroscopy, spectrophotometry (e.g., UV-visible), or mass spectrometry, and/or by chromatography such as high performance liquid chromatograpy (HPLC) or thin layer chromatography.

Preparation of compounds can involve the protection and deprotection of various chemical groups. The need for protection and deprotection and the selection of appropriate protecting groups can be readily determined by one skilled in the art. The chemistry of protecting groups can be found, for example, in Greene, et al., Projective Groups in Organic Synthesis, 4th Ed., Wiley & Sons, 2006.

The reactions of the processes described herein can be carried out in suitable solvents which can be readily selected by one skilled in the art of organic synthesis. Suitable solvents typically are substantially nonreactive with the reactants, intermediates, and/or products at the temperatures at which the reactions are carried out, i.e., temperatures that can range from the solvent's freezing temperature to the solvent's boiling temperature. A given reaction can be carried out in one solvent or a mixture of more than one solvent. Depending on the particular reaction step, suitable solvents for a particular reaction step can be selected.

Scheme 1 below depicts an exemplary synthetic route for the preparation of an intermediate of compounds of formula **I**.

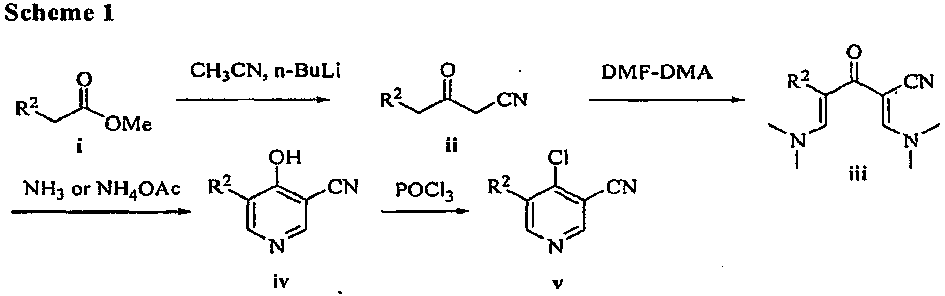

Acetic acid ester i is converted to 3-oxo-butyronitrile ii by reaction with the anion of acetonitrile prepared by reaction of acetonitrile (CH₃CN) with a strong base such as *n*-butyl lithium (n-BuLi) in a solvent such as THF. Reaction of oxo-butyronitrile **ii** with dimethylformamide-dimethyl acetal (DMF-DMA) in a solvent such as DMF at high temperature (e.g., 122°C) results in the formation of bisdimethylaminomethylene intermediate **iii** which is converted to 4-hydroxy-nicotinonitrile **iv** by reaction with ammonia (NH₃) or ammonium acetate (NH₄OAc) in a solvent such as ethanol at reflux. Reaction of the hydroxypyridine with refluxing phosphorous oxychloride (POCl₃) with or without catalytic DMF for 2 to 6 hours results in conversion to 4-chloro-nicotinonitrile **v**.

Scheme 2 below shows an alternative procedure for the preparation of 3-oxo-butyronitrile **ii**. This alternative procedure involves conversion of acetic acid **vi** to the corresponding acid chloride by reaction with a chlorinating agent such as thionyl chloride (SOCl₂) followed by reaction of the anion of *tert*-butylcyanoacetate prepared by reaction of *tert*-butylcyanoacetate with a base such as sodium hydride (NaH) in a solvent such as THF to give 2-cyano-3-oxo-butanoic acid tert-butyl ester **vii**, which undergoes deprotection of the ester and decarboxylation to give 3-oxo-butyronitrile **ii** by reaction with an acid such as trifluoroacetic acid (TFA).

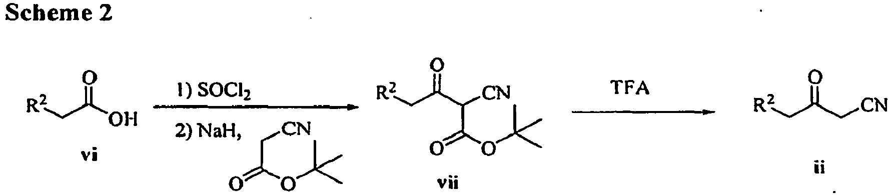

Alternatively, as shown in Scheme 3 below, the bisdimethylaminemethylene intermediate **iii** obtained by reaction of 3-oxo-butyronitrile **ii** with DMF-DMA can be reacted with 3,4-dimethoxybenzylamine at reflux in a solvent such as toluene to give 1-(3,4-dimethoxybenzyl)-4-oxo-1,4-dihydro-pyridine-3-carbonitrile **viii**. Reaction of **viii** with excess LiCl in refluxing POCl₃ results in removal of the dimethoxybenzyl group and conversion to the corresponding 4-chloro-nicotinonitrile **v**.

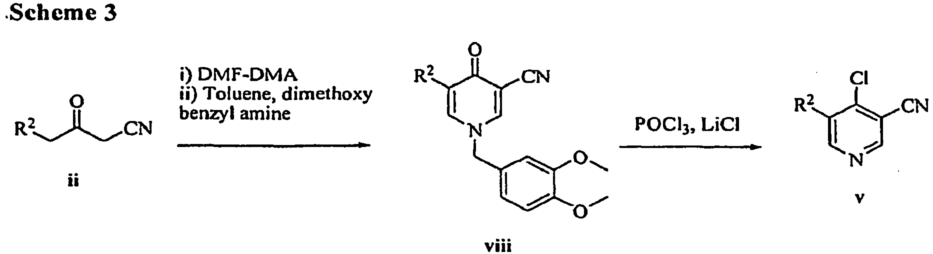

Scheme 4 below depicts an exemplary synthetic route for the preparation of compounds of formula **I.**

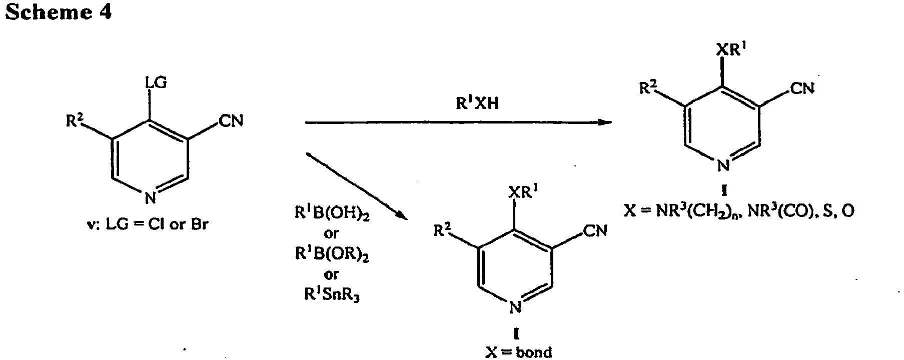

To prepare compounds of formula **I** where X is NR³-(CH₂)ₙ-, - NR³(CO)-, -O-, or -S-, where n = 0-10, a C-5 substituted 4-chloro-3-cyanopyridine **v** can be reacted with R¹XH under one of the following reaction conditions: **1**) in a solvent such as ethanol (EtOH), propanol, butanol, 2-ethoxyethanol (EtEtOH), 2-methoxyethanol, or 2-butoxyethanol at elevated temperature of 60-180°C, optionally in the presence of pyridine hydrochloride (Pyr.HCl); 2) using an alkali base such as sodium hydride (NaH) in a solvent such as tetrahydrofuran (THF) or dimethylformamide (DMF) at elevated temperatures of 60-120°C; 3) using a palladium catalyst such as tris(dibenzylidene)acetone dipalladium (Pd₂(dba)₃) and a phosphine ligand such as 2-dicyclohexylphosphino-2'-(N, N-dimethylamino)biphenyl (DavePhos) or tributylphosphine, in the presence of a base such as potassium phosphate (K₃PO₄) or potassium t-butoxide at elevated temperatures of 80-150°C; 4) using an organic base such as triethylamine (TEA), pyridine, or diisopropylethylamine (DIEA) in a solvent such as DMF, N-methyl-2-pyrrolidone (NMP) or EtEtOH at elevated temperatures of 80-150°C; 5) using an inorganic base such as cesium carbonate (Cs₂CO₃) in a solvent such as acetonitrile (CH₃CN) or DMF at elevated temperatures of 80-150°C.

When X is a covalent bond, compounds of formula **I** can be prepared by a coupling reaction of C-5 substituted 4-chloro-3-cyanopyridine **v** with a boronic acid of formula R¹B(OH)₂, or boronic ester of formula R¹B(OR)₂, where **R** is an alkyl group (e.g., a lower alkyl group), mediated by a palladium catalyst such as tetrakis(triphenylphosphine)-palladium (0) [(Ph₃P)₄Pd] or palladium (II) acetate (Pd(OAc)₂) in a solvent such as a mixture of dimethoxyethane(DME) and aqueous sodium bicarbonate (aq. NaHCO₃) or aqueous sodium carbonate (aq. Na₂CO₃), optionally in the presence of a phosphine ligand such as triphenyl phosphine (Ph₃P). Alternatively, 4-chloro-3-cyanopyridine **v** can be treated with a stannane R¹SnR₃, wherein R is an alkyl group (e.g., a lower alkyl group), to yield compounds of formula **I.**

Referring to Scheme 5 below, additional compounds of formula I where R² is substituted with an R⁴ group selected from an aryl group, a heteroaryl group, an alkenyl group and an alkynyl group (formula **Ib**) can be prepared from compounds of formula **I** where R² is substituted with a leaving group (LG) such as bromide (Br), iodide (I), chloride (CI) or trifluoromethane sulfonate (OTf) (formula **Ia)** as described in Scheme 5 below.

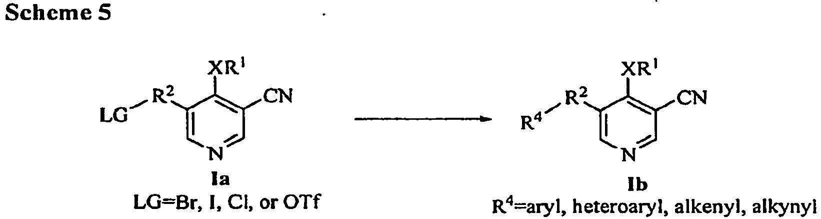

More specifically, compounds of formula **Ib** where R⁴ is an aryl group or a heteroaryl group can be prepared by treatment of compounds of formula **Ia** with a boronic acid (R⁴B(OH)₂), a boronic ester (R⁴B(OR)₂, where R is a lower alkyl group) or with an organic stannane reagent (e.g., R⁴SnBu₃) mediated by a palladium catalyst (e.g., (Ph₃P)₄Pd or Pd(OAc)₂) in a solvent such as a mixture of DME and aq. NaHCO₃ or aq. Na₂CO₃, optionally in the presence of a phosphine ligand such as Ph₃P.

Similarly, compounds of formula **Ib** where R⁴ is an alkenyl group or an alkynyl group can be prepared by treating compounds of formula **Ia** with an alkene or alkyne of formula R⁴-H or with a boronic acid or ester of or an organic stannane reagent in the presence of a palladium catalyst (e.g., (Ph₃P)₄Pd, dichlorobis(triphenylphosphine)palladium (II), or Pd(OAc)₂) in a solvent such as DMF, NMP, dioxane, or DME, in the presence of a ligand such as Ph₃P or tri-o-tolylphosphine and a base (e.g., potassium carbonate (K₂CO₃) or Nₐ2CO₃), optionally with the addition of an organic base such as TEA. A catalytic amount of copper(1) iodide can be optionally used for this coupling reaction.

Scheme 6 depicts a synthetic route for preparing additional compounds of formula I where both R² and R⁴ are aryl or heteroaryl groups and R⁴ is further substituted with an amide (formula **Id**).

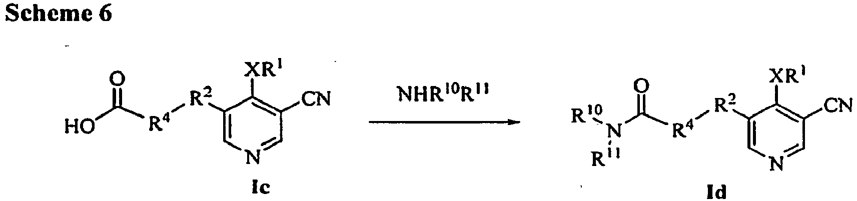

Compounds of formula **I** where R² is substituted by an aryl or heteroaryl group substituted by a carboxylic acid (formula **Ic**) can be treated with an amine of formula NHR¹⁰R¹¹ in the presence of a catalyst (e.g., benzotriazol-1-yloxytris(dimethyl amino)phosphonium hexafluorophosphate (BOP)) and an organic amine (e.g., TEA, DIEA, or pyridine) in a solvent such as MeOH or EtOH at ambient temperature to elevated temperatures of 50-80°C to provide compounds of formula **Id** as described.

Additional compounds of formula **I** where R² is substituted with -O-Y-NR⁶R⁷ (formula **If**) can be prepared as depicted in Scheme 7 below, by treating compounds of formula **I** where R² is substituted with -O-Y-LG (formula **Ic**), where LG is Cl, Br, methanesulfonyl (mesyl, OMs), or p-toluenesulfonyl (tosyl, OTs), with an amine of formula NHR⁶R⁷ in a solvent such as EtOH, DME or DMF optionally in the presence of NaI or a base such as K₂CO₃.

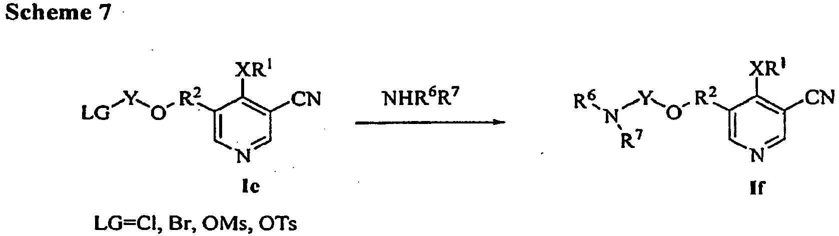

As depicted in Scheme 8, compounds of formula **I** wherein R² is substituted by -CH₂-NR⁶YR⁷ (formula **Ih**), can be prepared by treating compounds of formula **I** where R² contains an aldehyde functionality (formula **Ig**), with an amine of formula HNR⁶YR⁷ in the presence of a reducing agent (e.g., sodium triacetoxyborohydride (Na(OAc)₃BH) or sodium cyanoborohydride) in a solvent such as dichloromethane (CH₂Cl₂) or THF with the optional addition of DMF or NMP and preferably in the presence of acetic acid. Compounds of formula **I** wherein R² is substituted by - CH₂-OH (formula **Ii**) can be formed as a b-byproduct of this reductive amination reaction.

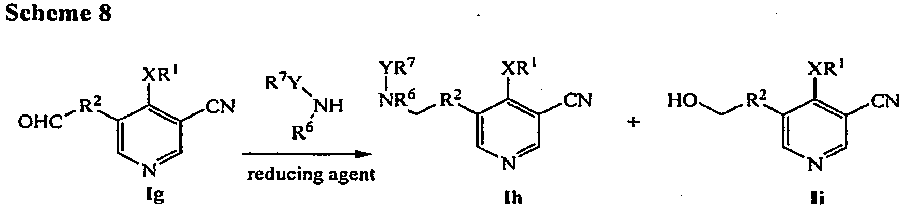

As depicted in Scheme 9, compounds of formula **I** where R² is substituted by -OYR⁵ (formula **Ik**) can be prepared by treating compounds of formula **I** where R² contains a hydroxyl functionality(formula **Ij**), with an alcohol of formula R⁵YOH under Mitsunobu conditions. This reaction can be conducted in a solvent such as THF in the presence of Ph₃P and either diethyl azodicarboxylate or di-t-butyl azodicarboxylate.

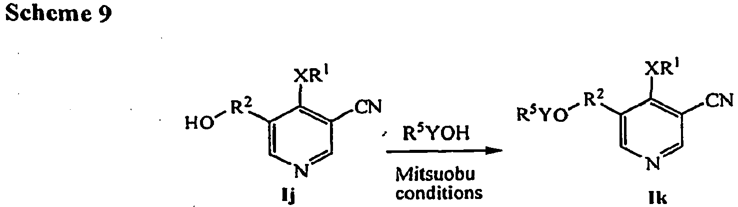

Additional compounds of formula **I** wherein X is not a bond can be prepared as shown in Scheme 10, Scheme 11, and Scheme 12 below.

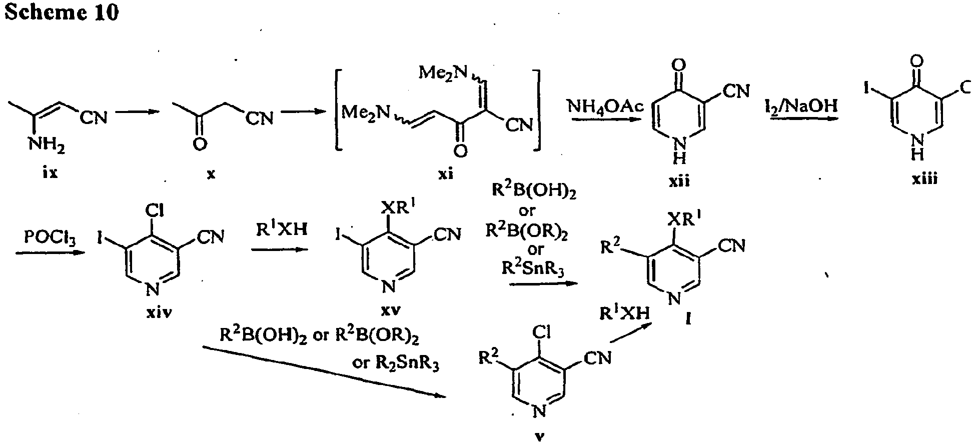

A mixture of 3-aminobut-2-enenitrile **ix** is heated in acid (e.g., aqueous HCl) to yield acetoacetonitrile **x**. Acetoacetonitrile **x** is treated with t-butoxybis(dimethyl amino)methane and DMF-DMA at an elevated temperature to yield 5-(dimethylamino)-2-[(dimethylamino)methylene]-3-oxopent-4-enenitrile **xi**, which is then treated with ammonium acetate in EtOH at reflux to produce 4-hydroxynicotinonitrile **xii**. (An alternate synthesis of 4-hydroxynicotinonitrile was reported in the literature: Broekman, F. W. et al., Recueil des Travaux Chimiques des Pays-Bas, 81: 792-796 (1962)). A mixture of 4-hydroxynicotinonitrile **xii**, iodine and NaOH in water is heated overnight to yield 4-hydroxy-5-iodonicotinonitrile **xiii**, which is then treated with POCl₃ at an elevated temperature to yield 4-chloro-5-iodonicotinonitrile **xiv**. Intermediate **xiv** can then be treated with R¹XH, wherein X is not a bond (e.g., R¹NH₂, R¹OH, R¹SH, etc.) to yield the 4-substituted 5-iodo-nicotinonitrile **xv**. Further treatment with a boronic acid R²B(OH)₂, boronic acid ester R²B(OR)₂ or stannane R²SnR₃ (where R, in each case, is a lower alkyl group) yields compounds of formula **I**. Alternatively, intermediate **xiv** can be treated with a boronic acid R²B(OH)₂, a boronic acid ester R²B(OR)₂ or a stannane R²SnR₃ (where R, in each case, is a lower alkyl group), followed by a reaction with R¹XH to provide compounds of formula **I**.

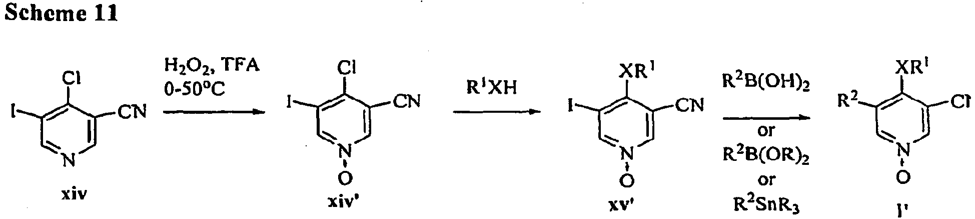

As depicted in Scheme 11, treatment of 4-chloro-5-iodonicotinonitrile **xiv** with an oxidizing agent, preferably hydrogen peroxide, in trifluoroacetic acid at temperatures of 0-50°C, provides 4-chloro-5-iodo-1-oxy-nicotinonitrile **xiv**'. Addition of R¹XH under the conditions noted previously provides compounds of formula **xv'** Addition of a boronic acid, ester, or an organostannane under the conditions noted previously provides compounds of formula **I'**.

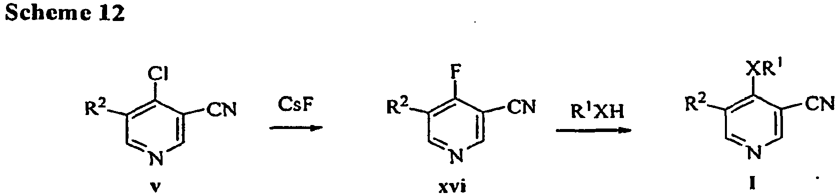

As shown in Scheme 12, treatment of compounds of formula **v** with CsF in a solvent such as DMF provides a 4-fluoro analog **xvi**. Subsequent displacement of the 4-fluoro group with R¹XH in a solvent such as DMSO provides compounds of formula **I**.

Aspects of the present teachings can be further understood in light of the following examples, which should not be construed as limiting the scope of the present teachings in any way.

More specifically, the following examples illustrate various synthetic routes which can be used to prepare compounds of formula **I**. HPLC conditions used in the examples are listed as the following:
(a): column: Prodigy ODS3, 4.6 x 150 mm, from Phenomenex (Torrance, CA); mobile phase A: 0.02% trifluoroacetic acid (TFA) in water; mobile phase B: 0.02% TFA in CH₃CN, 10-95% B in 20 minutes (min.); flow rate: 1.0 mL/min.; column temperature: 40 °C; detection wavelength: 215 nm;
(b): column: Prodigy ODS3, 4.6 x 150 mm, from Phenomenex (Torrance, CA); mobile phase A: 0.02% trifluoroacetic acid (TFA) in water; mobile phase B: CH₃CN, 10-90% B; flow rate: 1.0 mL/min.; detection wavelength: 215 nm;
(c): column: Prodigy ODS3, 4.6 × 150 mm, from Phenomenex (Torrance, CA); mobile phase A: 0.02% formic acid in water; mobile phase B: 0.02% formic acid in acetonitrile, 10-95% B in 3 min., 95-10% B in **I** min.; column temperature: 40 °C; detection wavelength: 215 nm;
(d): column: YMC C18, 4.6 × 50 mm, 5 microns, from YMC (Kyoto, Japan); mobile phase A: 90% water + 10% MeOH + 0.02% H₃PO₄; mobile phase B: 90% MeOH + 10% water + 0.02% H₃P0₄, 1-100% B in 2 min., up to 10 min. 100% B, then 100-1% B in 1 min.;
(e): column: Aquasil C18, 2.1 × 50 mm, from Thermo Fisher Scientific, Inc. (Waltham, MA); 5.5 min. gradient CH₃CN in water/formic acid; flow rate: 0.8 mL/min.; detection wavelength: 254 nm;
(f): column: Xterra MS C18, 3.5 µm, 2.1 × 30 mm, from Waters Corp. (Milford, MA); 5 min. gradient CH₃CN in water/formic acid; flow rate: 1.0 mL/min.; detection wavelength: 215 nm;
(g): column: Prodigy ODS3,4.6 x 150 mm, from Phenomenex (Torrance, CA); 20 min. gradient CH₃CN in water/TFA; flow rate: 1.0 mL/min.; detection wavelength: 215 nm;
(h): column: Prodigy ODS3, 4.6 × 150 mm, from Phenomenex (Torrance, CA); 20 min. gradient methanol (MeOH) in water/TFA; flow rate: 1.0 mL/min.; detection wavelength: 215 nm;
(i) column: XBridge C18, 4.6 × 50 mm, from Waters Corp. (Milford, MA); 5.5 min. gradient CH₃CN in water/formic acid; flow rate: 0.8 mL/min.; detection wavelength: 254 nm;
(j): column: Pursuit^{®} PFP, 4.6 × 150 mm, from Varian, Inc. (Palo Alto, CA); 20 min. gradient CH₃CN in water/TFA; flow rate: 1.0 mL/min.; detection wavelength: 215 nm; and
(k): column: Aquasil C18, 2.1 × 50 mm, from Thermo Fisher Scientific, Inc. (Waltham, MA); mobile phase A: 0.1 % formic acid in water; mobile phase B: 0.1 % formic acid in CH₃CN, 0 - 100 % B in 2.5 min; flow rate: 0.8 mL/min; column temperature: 40 °C; detection wavelength: 254 nm.

### Example 1: Preparation of 5-(3,4-dimethoxyphenyl)-4-(1H-indol-5-ylamino) nicotinonitrile 101

A solution of 3,4-dimethoxyphenyl acetic acid (50 mM) in MeOH (100 mL) with concentrated sulfuric acid (conc. H₂SO₄, 1 mL) or concentrated hydrochloric acid (conc. HCl) was heated at reflux overnight. Concentration to dryness on a rotary evaporator and high vacuum pump overnight gave the ester as an oil which was used directly in the next step.

To a 1.0 L three-necked round-bottomed flask was added 50 mL of THF and the reaction mixture was cooled to -78°C. Butyl lithium (BuLi, 1.6 M, 14.4 mL, 23 mmol) was added dropwise keeping the temperature below -70°C. Acetonitrile (1.3 mL, 25 mmol) in 30 mL of THF was added dropwise to the flask amidst stirring and cooling. After 2 hours (h) of stirring, (3,4-dimethoxyphenyl)acetic acid methyl ester (2.3 g, 11 mmol) was added to the resulting white colloidal mixture in the flask. The reaction mixture was stirred for a further 2 h, followed by the addition of saturated ammonium chloride solution (NH₄Cl, 75 mL) at -78°C. The organic layer was separated, dried with sodium sulfate (Na₂SO₄), filtered to remove the drying agent and evaporated to dryness to give the crude product. This crude product was purified by silica gel column chromatography, eluting with 30-70% ethyl acetate (EtOAc) in hexanes to yield 4-(3,4-dimethoxyphenyl)-3-oxo-butyronitrile in the form of a solidifying amber oil, 1.8 g (75%).

To a solution of 4-(3,4-dimethoxyphenyl)-3-oxo-butyronitrile (5.0 g, 23 mmol) in DMF (12 mL) was added dimethylformamide-dimethylacetal (DMF-DMA, 13.5 mL, 101 mmol) and the solution heated at 122°C overnight. Concentration on a rotary evaporator under high vacuum gave an orange-red solid. This solid was dissolved in EtOH (100 mL) and excess ammonium acetate was added and the reaction mixture was heated at 85°C for 1 h. The reaction mixture was allowed to cool to room temperature (r.t.) for 1 h then the solids were collected by filtration and washed with EtOH (cold) to give 5-(3,4-dimethoxyphenyl)-4-hydroxynicotinonitrile (4.1 g, 69%) as a brown solid. The filtrate was concentrated on a rotary evaporator and the residue purified on silica gel with 0-25% MeOH in CH₂Cl₂ to give an additional amount of 5-(3,4-dimethoxyphenyl)-4-hydroxynicotinonitrile.

A solution of 5-(3,4-dimethoxyphenyl)-4-hydroxynicotinonitrile (4 g, 15.7 mmol) in phosphorus oxychloride (POCl₃, 25 mL) was heated at 125°C for 1.5 h, then cooled to r.t. and poured into an ice/3 N sodium hydroxide (NaOH)/EtOAc mixture. The mixture was stirred and the layers separated. The organic layer was dried over magnesium sulfate (MgSO₄) filtered and concentrated to give 4-chloro-5-(3,4-dimethoxyphenyl)nicotinonitrile (3.9 g, 91%) as a brown solid.

A mixture of 4-chloro-5-(3,4-dimethoxyphenyl)nicotinonitrile (824 mg, 3 mmol), 5-aminoindole (396 mg, 3 mmol) and Pyr.HCl (345 mg, 3 mmol) in EtEtOH (25 mL) was heated at reflux for 8 h, cooled to r.t. and concentrated. The residue was purified by flash silica gel column chromatography eluting with 0-25% MeOH in CH₂Cl₂ to give 977 mg (88% yield) of a yellow-brown oil, that was triturated with MeOH/ethyl ether to give 525 mg (47%) of 5-(3,4-dimethoxyphenyl)-4-(1H-indol-5-ylamino)nicotinonitrile **101** as a yellow-brown solid. MS 371.2 (M+H), HPLC retention time: 1.70 min. ^{(a)}.

Following procedures analogous to those described for preparing compound **101** and using the appropriate amine in the last step, the compounds in Table 2 were prepared.

**Table 2**

| **Compound** | **Compound Name** | **HPLC Retention Time (min.)** | **Observed Ion m/e [M+H]** |
|---|---|---|---|
| 102 | 4-(2,1,3-benzothiadiazol-4-ylamino)-5-(3,4-dimethoxy phenyl)nicotinonitrile | 1.85 ^{(a)} | 390 |
| 103 | 5-(3,4-dimethoxyphenyl)-4-(isoquinolin-5-ylamino) nicotinonitrile | 1.50 ^{(a)} | 383 |
| 104 | 5-(3,4-dimethoxyphenyl)-4-(quinolin-5-ylamino) nicotinonitrile | 1.61 ^{(a)} | 383 |
| 105 | 5-(3,4-dimethoxyphenyl)-4-(5,6,7,8-tetrahydronaphthalen-1-ylamino)nicotinonitrile | 2.06 ^{(a)} | 386 |
| 106 | 4-(2,3-dihydro-1,4-benzodioxin-6-ylamino)-5-(3,4-dimethoxyphenyl)nicotinonitrile | 1.75 ^{(a)} | 390 |
| 107 | 4-(2,3-dihydro-1H-inden-5-ylamino)-5-(3,4-dimethoxy phenyl)nicotinonitrile | 2.03 ^{(a)} | 372 |
| 108 | 5-(3,4-dimethoxyphenyl)-4-(1H-indol-6-ylamino) nicotinonitrile | 1.65 ^{(a)} | 371 |
| 109 | 5-(3,4-dimethoxyphenyl)-4-(1H-indol-4-ylamino) nicotinonitrile | -1.62 ^{(a)} | 371 |
| 110 | 5-(3,4-dimethoxyphenyl)-4-[(2-methyl-11-1-indol-5-yl) amino]nicotinonitrile | 2.15 ^{(a)} | 385 |
| 111 | 4-(1,3-benzodioxol-5-ylamino)-5-(3,4-dimethoxy phenyl)nicotinonitrile | 2.01 ^{(a)} | 376.1 |
| 112 | 5-(3,4-dimethoxyphenyl)-4-(2-naphthylamino) nicotinonitrile | 2.22 ^{(a)} | 382.1 |

### Example 2: Preparation of 5-(3-bromophenyl)-4-(1H-indol-5-ylamino) nicotinonitrile 113

Following procedures analogous to those described for preparing 4-chloro-5-(3,4-dimethoxyphenyl) nicotinonitrile (Example 1), 5-(3-bromophenyl)-4-chloronicotinonitrile was prepared from 3-bromophenylacetic acid. 5-(3-Bromophenyl)-4-(1H-indol-5-ylamino)nicotinonitrile **113** was then prepared using two methods.

Method A: Following procedures analogous to those described for preparing compound **101** (Example 1), the title compound was prepared from 5-(3-bromophenyl)-4-chloronicotinonitrile and purified by flash silica gel column chromatography eluting with 0-25% MeOH in CH₂Cl₂. MS: 389.0 (M+H), HPLC retention time: 1.92 min. ^{(a)}

Method B: A solution of 5-(3-bromophenyl)-4-chloronicotinonitrile (4.42 g, 15 mmol), 5-aminoindole (1.99 g, 15 mmol) in EtEtOH (44 mL) was heated at reflux for 12 h, then cooled to r. t. The reaction mixture was poured into saturated aq. NaHCO₃ solution, whereupon the crude product precipitated. The latter was filtered and the crude solid was dissolved in CH₂Cl₂, dried over Na₂SO₄, concentrated and purified by flash chromatography on silica gel eluting with 1:1 EtOAc/hexane to give 4.2 g (72% yield) of the title compound as a foamy yellow solid. MS: 389.0 (M+H), HPLC retention time: 1.92 min. ^{(a)}.

### Example 3: Preparation of 5-(3-bromophenyl)-4-(1H-indol-4-ylamino) nicotinonitrile 114

Following procedures analogous to those described in Example 2, Method B, 5-(3-bromophenyl)-4-(1H-indol-4-ylamino) nicotinonitrile was prepared from 5-(3-bromophenyl)-4-chloronicotinonitrile and 4-aminoindole. MS: 389.2 (M+H).

### Example 4: Preparation of 5-(2-bromophenyl)-4-(1H-indol-5-ylamino) nicotinonitrile 115

Following procedures analogous to those described for preparing 4-chloro-5-(3,4-dimethoxyphenyl) nicotinonitrile (Example 1), 5-(2-bromophenyl)-4-chloronicotinonitrile was prepared from 2-bromophenylacetic acid. Following procedures analogous to those described for preparing compound **113** (Example 2, Method B), 5-(2-bromophenyl)-4-(1H-indol-5-ylamino)nicotinonitrile **115** was prepared from 5-(2-bromophenyl)-4-chloronicotinonitrile. MS: 389.0 (M+H).

### Example 5: Preparation of 5-(4-bromophenyl)-4-(1H-indol-5-ylamino) nicotinonitrile 116

Following procedures analogous to those described for preparing 4-chloro-5-(3,4-dimethoxyphenyl) nicotinonitrile (Example 1), 5-(4-bromophenyl)-4-chloronicotinonitrile was prepared from 4-bromophenylacetic acid. Following procedures analogous to those described for preparing compound **113** (Example 2, Method B), 5-(4-bromophenyl)-4-(1H-indol-5-ylamino)nicotinonitrile **116** was prepared from 5-(4-bromophenyl)-4-chloronicotinonitrile. MS: 389.0 (M+H).

### Example 6: Preparation of 5-(3-methoxy-4-(2-methoxyethoxy)phenyl]-4-[(4-mcthyl-1H-indol-5-yl)amino]nicotinonitrile 478

Following procedures analogous to those described for preparing compound **113** (Method B), 5-[3-methoxy-4-(2-methoxyethoxy)phenyl]-4-[(4-methyl-1H-indol-5-yl)amino]nicotinonitrile was prepared. HPLC retention time: 8.0 mien. ^{(g)}; melting range: 208-210 °C; and HRMS: 429.19278.

### Example 7: Preparation of 5-(3'-aminobiphenyl-3-yl)-4-(1H-indol-5-ylamino)nicotinonitrile 117

A mixture of 5-(3-bromophenyl)-4-(1H-indol-5-ylamino)nicotinonitrile **113** (39 mg, 0.1 mmol), (Ph₃P)₄Pd (2.3 mg, 0.002 mmol) and 2M aq. Na₂CO₃ (0.1 mL) in DME (2 mL) was degassed by bubbling nitrogen for 5 min., then 3-aminophenylboronic acid (17 mg, 0.11 mmol) was added and the mixture was heated at reflux for 12 h. After cooling to r.t., the mixture was filtered and the filtrate was purified by preparative HPLC to give 5-(3'-aminobiphenyl-3-yl)-4-(1H-indol-5-ylamino)nicotinonitrile **117**. MS: 402 (M+H), HPLC retention time: 1.72 min. ^{(a)}.

Following procedures analogous to those described for preparing compound **117**, compounds **118-150** in Table 3 were synthesized from 5-(3-bromophenyl)-4-(1H-indol-5-ylamino)nicotinonitrile **113**, 5-(3-bromophenyl)-4-(1H-indol-4-ylamino) nicotinonitrile **114**, 5-(2-bromophenyl)-4-(1H-indol-5-ylamino)nicotinonitrile **115** or 5-(4-bromophenyl)-4-(1H-indol-5-ylamino)nicotinonitrile **116** and the appropriate boronic acid.

**Table 3**

| **Compound** | **Compound Name** | **HPLC Retention Time (min.)** | **Observed Ion m/e [M+H]** | **Melting Range (°C)** |
|---|---|---|---|---|
| 118 | 5-(4'-cyanobiphenyl-3-yl)-4-(1H-indol-5-ylamino)nicotinonitrile | 2.01 ^{(a)} | 412 | N/A |
| 119 | 5-(4'-aminobiphenyl-3-yl)-4-(1H-indol-5-ylamino)nicotinonitrile | 1.71 ^{(a)} | 402 | N/A |
| 120 | N-{3'-[5-cyano-4-(1H-indol-5-ylamino) pyridin-3-yl]biphenyl-4-yl}acetamide | 1.87 ^{(a)} | 444 | N/A |
| 121 | 4-(1H-indol-5-ylamino)-5-(3-pyridin-4-ylphenyl)nicotinonitrile | 1.50 ^{(a)} | 388 | N/A |
| 122 | 3'-[5-cyano-4-(1H-indol-5-ylamino)pyridin-3-yl]-N,N-dimethylbiphenyl-4-carboxamide | 1.88 ^{(a)} | 458 | N/A |
| 123 | 3'-[5-cyano-4-(1H-indol-5-ylamino) pyridin-3-yl]-N-cyclopentylbiphenyl-4-carboxamide | 2.04 ^{(a)} | 498 | N/A |
| 124 | 4-(1H-indol-5-ylamino)-5-[3-(1H-pyrrol-3-yl)phenyl]nicotinonitrile | 1.84 ^{(a)} | 374 | N/A |
| 125 | 5-(2-bromophenyl)-4-[(7-methyl-1H-indol-5-yl)amino]nicotinonitrile | N/A | 403.0 | N/A |
| 126 | 5-(2-bromophenyl)-4-(1H-indol-4-ylamino)nicotinonitrile | 9.3 ^{(a)} | 389.0 | N/A |
| 127 | 4-(1H-indol-5-ylamino)-5-(3'-methyl biphenyl-3-yl)nicotinonitrile | 2.20 ^{(a)} | 401.5 | N/A |
| 128 | 4-(1H-indol-5-ylamino)-5-(4'-methyl biphenyl-3-yl)nicotinonitrile | 2.20 ^{(a)} | 401.5 | N/A |
| 129 | 5-(2'-chlorobiphenyl-3-yl)-4-(1H-indol-5-ylamino)nicotinonitrile | 2.18 ^{(a)} | 421.9 | N/A |
| 130 | 5-(3'-chlorobiphenyl-3-yl)-4-(1H-indol-5-ylamino)nicotinonitrile | 2.23 ^{(a)} | 421.9 | N/A |
| 131 | 5-(4'-chlorobiphenyl-3-yl)-4-(1H-indol-5-ylamino)nicotinonitrile | 2.24 ^{(a)} | 421.9 | N/A |
| 132 | 5-(3'-cyanobiphenyl-3-yl)-4-(1 H-indol-5-ylamino)nicotinonitrile | 2.04 ^{(a)} | 412.5 | N/A |
| 133 | 3'-[5-cyano-4-(1H-indol-5-ylamino) pyridin-3-yl]biphenyl-3-carboxylic acid | 2.80 ^{(**a**)} | 431.5 | N/A |
| 134 | 3'-[5-cyano-4-(1H-indol-5-ylamino) pyridin-3-yl]biphenyl-4-carboxylic acid | 1.91 ^{(a)} | 431.5 | N/A |
| 135 | 3'-[5-cyano-4-(1H-indol-4-ylamino) pyridin-3-yl]biphenyl-4-carboxylic acid | N/A | 431.1 | N/A |
| 136 | 4-(1H-indol-5-ylamino)-5-[3-(2-thienyl) phenyl]nicotinonitrile | 2.08 ^{(a)} | 393.5 | N/A |
| 137 | 4-(1H-indol-5-ylamino)-5-(3-pyridin-3-ylphenyl)nicotinonitrile | 1.59 ^{(a)} | 388.5 | N/A |
| 138 | 4-(1H-indol-5-y(amino)-5-(3-pyrimidin-2-ylphenyl)nicotinonitrile | 1.80 ^{(a)} | 389.4 | N/A |
| 139 | 4-(1H-indol-5-ylamino)-5-[3-(4-methyl-2-thienyl)phenyl]nicotinonitrile | 2.50 ^{(a)} | 405.1 | N/A |
| 140 | 5-[3-(5-acetyl-2-thienyl)phenyl]-4-(1H-indol-5-ylamino)nicotinonitrile | 2.31 ^{(a)} | 433.1 | N/A |
| 141 | 4-(1H-indol-5-ylamino)-5-[3-(3-thienyl)phenyl]nicotinonitrile | 2.39 ^{(a)} | 391.1 | N/A |
| 142 | 5-[3-(3-furyl)phenyl]-4-(1H-indol-5-ylamino)nicotinonitrile | 2.30 ^{(a)} | 375.1 | N/A |
| 143 | 5-(2'-chlorobiphenyl-2-yl)-4-(1H-indol-5-ylamino)nicotinonitrile | 2.37 ^{(a)} | 419.1 | N/A |
| 144 | 5-(3'-chlorobiphenyl-2-yl)-4-(1H-indol-5-ylamino)nicotinonitrile | 2.38 ^{(a)} | 419.1 | N/A |
| 145 | 5-(4'-chlorobiphenyl-2-yl)-4-(1H-indol-5-ylamino)nicotinonitrile | 2.40 ^{(a)} | 419.1 | N/A |
| 146 | 4-(1H-indol-5-ylamino)-5-[2-(3-thienyl) phenyl]nicotinonitrile | 2.28 ^{(a)} | 391.1 | N/A |
| 147 | 4-(1H-indol-4-ylamino)-5-[3-(2-thienyl) phenyl]nicotinonitrile | N/A | 393.2 | N/A |
| 148 | 3'-[5-cyano-4-(1H-indol-4-ylamino) pyridin-3-yl]-N-cyclopentylbiphenyl-4-carboxamide | N/A | 498.3 | N/A |
| 149 | 4-(1H-indol-4-ylamino)-5-[4'-(pyrrolidin-1-ylcarbonyl)biphenyl-3-yl]nicotinonitrile | N/A | 484.3 | N/A |
| 150 | 5-[3-(5-formyl-2-thienyl)phenyl]-4-(1H-indol-5-ylamino)nicotinonitrile | N/A | 421.1 | 196-199 (decom.) |

### Example 8: Preparation of 4-(1H-indol-5-ylamino)-5-(3-nitrophenyl) nicotinonitrile 151

3-Nitrophenyl acetic acid (9.5 g, 52 mmol) and thionyl chloride (20 mL) were stirred overnight at r.t., then evaporated to dryness. In a separate flask NaH (60% dispersion in oil, 5.5 g, 1.4 mmol) was suspended in THF (100 mL) and the mixture was cooled to 0°C and tert-butylcyanoacetate (8.8 g, 62 mmol) was added. After 15 min., a solution of 3-nitrophenylacetyl chloride from above in THF was added dropwise. The reaction mixture was allowed to warm to r.t. and stirred for 4 h, quenched by the addition of brine, and extracted with EtOAc (2 x 200 mL). The combined organic extracts were dried over MgSO₄ and concentrated. The residue was used in the next step without further purification.

To a solution of 2-cyano-4-(3-nitrophenyl)-3-oxo-butyric acid tert-butyl ester (9.5 g, 31 mmol) in toluene (40 mL) was added TFA (4 mL) and the solution was heated at reflux for 2 h, then the solvent evaporated in vacuo. The residue was purified by flash chromatography on silica gel to give 4.0 g of 4-(3-nitrophenyl)-3-oxo-butyronitrile (37% over 2 steps).

Following procedures analogous to those described for preparing compound 101 (Example 1), 4-(3-nitrophenyl)-3-oxo-butyronitrile was converted to 4-(1H-indol-5-ylamino)-5-(3-nitrophenyl)-nicotinonitrile 151. MS: 356 (M+H), HPLC retention time: 2.50 min. ^{(a)}.

### Example 9: Preparation of N-{3-[5-cyano-4-(1H-indol-5-ylamino)pyridin-3-yl]phenyl}acetamide 152

To a solution of 4-(1H-indol-5-ylamino)-5-(3-nitrophenyl)nicotinonitrile 151 (9 mg, 0.025 mmol) in MeOH (1 mL) were added hydrazine (20 uL) and Raney-Nickel (2-5 mg). The mixture was stirred for 2 h then filtered through celite and the filtrate was concentrated to give the crude reduced product which was then dissolved in CH₂Cl₂ (1 mL) and pyridine (20 uL), and acetyl chloride (20 uL) was added. After stirring for 1 h, the reaction mixture was evaporated and the residue was purified by preparative HPLC to give the title compound (4 mg). MS: 366.0 (M+H), HPLC retention time: 1.61 min. ^{(a)}.

### Example 10: Preparation of 4-(1H-indol-4-ylamino)-5-[4-methoxy-3-(2-methoxyethoxy)phenyl]nicotinonitrile 153

To a stirred solution of 3-hydroxy-4-methoxyphenyl acetic acid (24.8 g, 136 mmol) in 200 mL of MeOH was added 1 mL of H₂SO₄ and the reaction mixture was heated at reflux overnight. The methanol was removed by evaporation in vacuo and the residue was poured into saturated NaHCO₃ solution and extracted with EtOAc (3 x 150 mL). Combined organic extracts were then washed with brine, dried over anhydrous MgSO₄, filtered, and concentrated in vacuo to yield 23.9 g (90% yield) of 3-hydroxy-4-methoxyphenyl acetic acid methyl ester as a yellow oil.

To a stirred solution of 3-hydroxy-4-methoxyphenyl acetic acid methyl ester (5.0 g, 25.5 mmol), tetrabutylammonium iodide (TBAI, 0.941 g, 2.5 mmol), and 2-bromoethylmethyl ether (4.6 mL, 50.9 mmol) in 150 mL of acetone was added Cs₂CO₃ (17.4 g, 53.4 mmol). The mixture was stirred for 21.5 h at reflux. The mixture was concentrated and the residue was partitioned between water and EtOAc. The combined organic extracts were dried over anhydrous Na₂SO₄, filtered, and concentrated in vacuo to yield 8.15 g of an orange oil, which was purified by flash chromatography on silica gel eluting with 10-50% EtOAc in hexane to give 5.33 g (82% yield) of 4-methoxy-3-(2-methoxyethoxy)phenylacetic acid methyl ester as a light yellow oil.

To a 250 mL three-necked round-bottomed flask was added 10 mL of anhydrous THF and cooled to -78°C. *n*-Butyl lithium (2.5 M in hexane, 8.06 mL, 12.9 mmol) was added and the mixture was stirred for 5 min. Anhydrous CH₃CN (0.696 mL, 13.3 mmol) in 5 mL of anhydrous THF was added dropwise at -78°C. After 1 h of stirring, 4-methoxy-3-(2-methoxyethoxy)phenylacetic acid methyl ester (1.10 g, 4.3 mmol) in 10 mL of anhydrous THF was added dropwise to the resulting white colloidal mixture. The reaction mixture was stirred for an additional 2 h, followed by the addition of saturated NH₄Cl solution at -78°C. The solution was warmed to r.t., diluted with 100 mL water and extracted with EtOAc (3 x 100 mL). The combined organics were washed with brine, dried with anhydrous MgSO₄, filtered, and concentrated in vacuo. The crude product was purified by flash chromatography on silica gel eluting with gradient 30-60% EtOAc in hexanes to yield 769 mg (68% yield) of 4-[4-methoxy-3-(2-methoxyethoxy)phenyl]-3-oxo-butyronitrile as a colorless oil.

To a stirred solution of 4-[4-methoxy-3-(2-methoxyethoxy)phenyl]-3-oxo-butyronitrile (9.91 g, 34.5 mmol) in 20 mL of anhydrous DMF was added DMF-DMA (20.2 mL, 152 mmol). The resulting mixture was heated at 100° C for 15 h and was concentrated in vacuo. The crude material was mixed with 3,4-dimethoxybenzylamine (0.687 mL, 41.4 mmol) in 20 mL of anhydrous toluene and the mixture was heated at reflux for 2 h. The reaction mixture was cooled, concentrated in vacuo, and purified by flash chromatography on silica gel eluting with gradient 50-100% EtOAc/hexane to yield 8.5 g (55% yield) of 1-(3,4-dimethoxybenzyl)-5-[4-methoxy-3-(2-methoxyethoxy)phenyl]-4-oxo-1,4-dihydro-pyridine-3-carbonitrile as a yellow/orange foam.

A solution of 1-(3,4-dimethoxybenzyl)-5-[4-methoxy-3-(2-methoxyethoxy)phenyl]-4-oxo-1,4-dihydro-pyridine-3-carbonitrile (300 mg, 0.666 mmol) and lithium chloride (LiCl, 254 mg, 6 mmol) in 2.5 mL of POCl₃ was heated at reflux for 2.5 h. The excess POCl₃ was removed by evaporating in vacuo and then was co-evaporated with toluene. The residue was dissolved in 100 mL ethyl acetate and washed with ice-cold 1 N aqueous sodium hydroxide. The organic layer was separated, dried over anhydrous MgSO₄, filtered, and concentrated in vacuo, and the resulting solid was triturated with isopropyl alcohol to yield 166 mg (78% yield) of 4-chloro-5-[4-methoxy-3-(2-methoxyethoxy)phenyl]-nicotinonitrile as an off-white solid.

To a stirred solution of 4-chloro-5-[4-methoxy-3-(2-methoxyethoxy)phenyl]-nicotinonitrile (100 mg, 0.313 mmol), 4-aminoindole (62 mg, 0.47 mmol,), DavePhos (37 mg, 0.094 mmol), and K₃PO₄ (99.8 mg, 0.47 mmol) in 4 mL of anhydrous ethylene glycol dimethyl ether was added Pd₂(dba)₃ (28.7 mg, 0.031 mmol). The mixture was heated to 90°C for 2 h, then cooled, filtered through celite, concentrated in vacuo, and crystallized by tritration with ether/hexane to yield 42.5 mg (33% yield) of the title compound 153 as a tan solid. MS: 415.1 (M+H), HPLC retention time: 7.70 min ^{(b)}.

Following procedures analogous to those described for preparing compound 153 and using the appropriate amine, the compounds in Table 4 were prepared.

**Table 4**

| **Compound** | **Compound Name** | **HPLC Retention Time (min.)** | **Observed Ion m/e [M+H]** |
|---|---|---|---|
| 154 | 4-(1H-indol-5-ylamino)-5-[4-methoxy-3-(2-methoxyethoxy)phenyl]nicotinonitrile | 7.70 ^{(b)} | 415.2 |
| 155 | 4-(1H-indol-6-ylamino)-5-[4-methoxy-3-(2-methoxyethoxy)phenyl]nicotinonitrile | 8.50 ^{(b)} | 415.2 |
| 156 | 4-(1,3-benzothiazol-6-ylamino)-5-[4-methoxy-3-(2-methoxyethoxy)phenyl]nicotinonitrile | 7.85 ^{(b)} | 433.1 |
| 157 | 5-[4-methoxy-3-(2-methoxyethoxy)phenyl]-4-[(2-methyl-1H-indol-5-yl)amino]nicotinonitrile | 8.50 ^{(b)} | 429.1 |
| 158 | 4-(1H-1,2,3-benzotriazol-5-ylamino)-5-(4-methoxy-3-(2-methoxyethoxy)phenyl] nicotinonitrile | 10.7 ^{(b)} | 417.0 |

### Example 11: Preparation of 4-(1H-indol-4-ylamino)-5-[3-methoxy-4-(2-methoxyethoxy)phenyl]nicotinonitrile 159

Following procedures analogous to those described for preparing 4-methoxy-3-(2-methoxyethoxy)phenylacetic acid methyl ester in Example 9, ethyl [3-methoxy-4-(2-methoxyethoxy)phenyl]acetate was prepared from 4-hydroxy-3-methoxyphenylacetic acid ethyl ester. 4-(1H-Indol-4-ylamino)-5-[3-methoxy-4-(2-methoxyethoxy)phenyl] nicotinonitrile 159 was then prepared following procedures analogous to those described for preparing 4-(1H-indol-4-ylamino)-5-[4-methoxy-3-(2-methoxyethoxy)phenyl]-nicotinonitrile 153 (Example 9). MS: 415.2 (M+H), HPLC retention time: 7.9 min ^{(b)}.

Compounds in Table 5 were prepared following procedures described for preparing compound **153** from 4-chloro-5-[3-methoxy-4-(2-methoxyethoxy)phenyl] nicotinonitrile.

**Table 5**

| **Compound** | **Compound Name** | **HPLC Retention Time (min.)** | **Observed Ion m/e [M+H]** |
|---|---|---|---|
| 160 | 4-(1H-indol-5-ylamino)-5-[3-methoxy-4-(2-methoxyethoxy)phenyl]nicotinonitrile | 7.8 ^{(b)} | 415.2 |
| 161 | 4-(1H-indol-6-ylamino)-5-[3-methoxy-4-(2-methoxyethoxy)phenyl]nicotinonitrile | 8.6 ^{(b)} | 415.2 |
| 162 | 4-(1,3-benzothiazol-6-ylamino)-5-[3-methoxy-4-(2-methoxyethoxy)phenyl)nicotinonitrile | 7.8 ^{(b)} | 433.0 |
| 163 | 5-[3-methoxy-4-(2-methoxyethoxy)phenyl]-4-[(2-methyl-1H-indol-5-yl)amino]nicotinonitrile | 8.7 ^{(b)} | 429.1 |

### Example 12: Preparation of 4-(1H-indol-4-ylamino)-5-[3-(2-methoxyethoxy)phenyl]nicotinonitrile 164

Following procedures analogous to those described in Example 9, 4-(1H-indol-4-ylamino)-5-[3-(2-methoxyethoxy)phenyl]nicotinonitrile 164 was prepared from 3-hydroxyphenylacetic acid. MS: 385.2 (M+H), HPLC retention time: 7.10 min. ^{(b)}.

### Example 13: Preparation of 5-[3-(2-chloroethoxy)phenyl]-4-(1H-indol-4-ylamino)-nicotinonitrile 165

To a stirred solution of 3-hydroxyphenylacetic acid methyl ester (22.6 g, 136 mmol) and 2-chloroethyl p-toluenesulfonate (40 g, 171 mmol) in 900 mL of acetone was added Cs₂CO₃ (88.8 g, 272 mmol) and the mixture was headed at reflux for 3 h. The reaction mixture was then cooled, filtered, and concentrated in vacuo. The residue was purified by flash chromatography on silica gel eluting with a gradient of 0-7% EtOAc in hexanes to yield 28.9 g (90% yield) of [3-(2-chloroethoxy)phenyl]-acetic acid methyl ester as a colorless oil.

To a 1.0 L three-necked round-bottomed flask was added 150 mL of anhydrous THF and cooled to -78°C. *n*-Butyl lithium (2.5 M in hexane, 52.5 mL, 131 mmol) was added dropwise. Anhydrous CH₃CN (7.2 mL, 138 mmol) in 150 mL of anhydrous THF was subsequently added dropwise to the flask amidst stirring and cooling. After 1 h of stirring, [3-(2-chloroethoxy)phenyl]-acetic acid methyl ester (15 g, 66 mmol) in 20 mL of anhydrous THF was added dropwise to the resulting white colloidal mixture in the flask. The reaction mixture was stirred for an additional 2 h, followed by the addition of 4:1 mixture of MeOH: acetic acid (AcOH) at -78°C. The solution was diluted with 500 mL water and extracted with EtOAc (4 x 150 mL). The organic layer was separated, dried with anhydrous MgSO₄, filtered, and concentrated in vacuo. Residual AcOH was removed by concentrating in vacuo with toluene. The residue was passed through silica gel eluting with CH₂Cl₂ to yield 4-[3-(2-chloroethoxy)phenyl]-3-oxo-butyronitrile as an off-white solid, 16.0 g (99% yield).

To a stirred solution of 4-[3-(2-chloroethoxy)phenyl]-3-oxo-butyronitrile (16.0 g, 67.0 mmol) in 100 mL anhydrous DMF was added DMF-DMA (19.7 mL, 148 mmol) and TEA (9.4 mL, 67.0 mmol), and the solution was heated at 100°C for 2.5 h. The reaction mixture was concentrated in vacuo and the residue was dissolved in CH₂Cl₂, passed through Magnesol^{®} and concentrated. The crude material was then stirred with 3,4-dimethoxybenzylamine (11 mL, 74 mmol) in 100 mL of anhydrous toluene at reflux for 2 h. The reaction was cooled, concentrated in vacuo, and purified by flash chromatography on silica gel eluting with EtOAc to yield 11.8 g (41% yield) of 5-[3-(2-chloroethoxy)phenyl]-1-(3,4-dimethoxybenzyl)-4-oxo-1,4-dihydro-pyridine-3-carbonitrile as an off-white solid.

A solution of 5-[3-(2-chloroethoxy)-phenyl]-1-(3,4-dimethoxybenzyl)-4-oxo-1,4-dihydro-pyridine-3-carbonitrile (2.5 g, 5.9 mmol) and LiCl (2.3 g, 53 mmol) in 22 mL of POCl₃ was heated at reflux for 2.5 h. The excess POCl₃ was removed by concentrating in vacuo. The residue was dissolved in 100 mL CH₂Cl₂ and washed with ice-cold 3 N NaOH. The organic layer was separated, dried over anhydrous MgSO₄, filtered, concentrated in vacuo, and purified by flash chromatography on silica gel eluting with 30% EtOAc in hexanes to yield 1.3 g

(75% yield) of 4-chloro-5-[3-(2-chloroethoxy)phenyl]nicotinonitrile as an off-white solid.

To a stirred solution of 4-chloro-5-[3-(2-chloroethoxy)phenyl]-nicotinonitrile (200 mg, 0.68 mmol), 4-aminoindole (135 mg, 1 mmol), DavePhos (80 mg, 0.20 mmol), and K₃PO₄ (216 mg, 1 mmol) in 4 mL anhydrous ethylene glycol dimethyl ether was added Pd₂(dba)₃ (62 mg, 0.07 mmol). The mixture was heated at 90°C for 2 h then cooled, filtered through celite, concentrated in vacuo, and purified by flash chromatography on silica gel eluting with a gradient of 5-50% MeOH in CH₂Cl₂ to yield 155 mg (59% yield) of 5-[3-(2-chloroethoxy)phenyl]-4-(1H-indol-4-ylamino)-nicotinonitrile **165** as a tan solid. MS: 389.1 (M+H), HPLC retention time: 9.60 min. ^{(a)}.

Following procedures analogous to those described for preparing compound 165, compounds **166** and **167** in Table 6 were prepared from 4-chloro-5-[3-(2-chloroethoxy)phenyl]-nicotinonitrile.

**Table 6**

| **Compound** | **Compound Name** | **HPLC Retention Time (min)** | **Observed Ion m/e [M+H]** |
|---|---|---|---|
| 166 | 5-[3-(2-chloroethoxy)phenyl]-4-(1H-indol-6-ylamino)nicotinonitrile | 9.91 ^{(a)} | 389.1 |
| 167 | 5-[3-(2-chloroethoxy)phenyl]-4-(1H-indol-5-ylamino)nicotinonitrile | 9.45 ^{(a)} | 389.1 |

### Example 14: Preparation of 4-(1H-indol-4-ylamino)-5-{3-[2-(4-methyl-piperazin-1-yl)-ethoxy]-phenyl}-nicotinonitrile 168

Method A: A stirred solution of 5-[3-(2-chloroethoxy)phenyl]-4-(1H-indol-4-ylamino)-nicotinonitrile **165** (100 mg, 0.26 mmol) and N-methylpiperazine (262 mg, 2.6 mmol) in 2.5 mL of EtOH was heated to 105°C for 7 h. The reaction was cooled, poured into 25 mL of water, and chilled to 0°C. The solid was filtered and dried in vacuo at 50°C overnight to yield 40 mg (34% yield) of the title compound as a brown solid.

Method B: A mixture of 5-[3-(2-chloroethoxy)phenyl]-4-(1H-indol-4-ylamino)-nicotinonitrile **165** (150 mg, 0.39 mmol), N-methylpiperazine (390 mg, 3.9 mmol) and NaI (catalytic amount) in DME (2.0 mL) was heated at reflux for overnight. The reaction mixture was partitioned between saturated NaHCO₃ and CH₂Cl₂. The combined organics were dried over Na₂SO₄, concentrated and purified by flash chromatography on silica gel eluting with a gradient of 2-15% MeOH in CH₂Cl₂ to give 141 mg (79% yield) of the title compound as an off-white solid.

Following procedures analogous to those described in Method A for preparing compound **168**, compounds **169-171** in Table 7 were prepared from 5-[3-(2-chloroethoxy)phenyl]-4-(1H-indol-4-ylamino)nicotinonitrile **165**; compounds **172** and **173** were prepared from 5-[3-(2-chloroethoxy)phenyl]-4-(1H-indol-6-ylamino)nicotinonitrile **166**; and compounds **174** and **175** were prepared from 5-[3-(2-chloroethoxy)phenyl]-4-(1H-indol-5-ylamino)nicotinonitrile **167**. Following procedures analogous to those described in Method B for preparing compound **168**, compounds **176** to **177** in Table 7 were prepared from 5-[3-(2-chloroethoxy)phenyl]-4-(1H-indol-5-ylamino)nicotinonitrile **167**.

**Table 7**

| **Compound** | **Compound Name** | **HPLC Retention Time (min.)** | **Observed Ion m/c [M+H]** |
|---|---|---|---|
| 168 | 4-(1H-indol-4-ylamino)-5-{3-[2-(4-methylpiperazin-1-yl)ethoxy]phenyl}nicotinonitrile | 5.01^{(a)} | 453.2 |
| 169 | 4-(1H-indol-4-ylamino)-5-[3-(2-pyrrolidin-1-yl ethoxy)phenyl]nicotinonitrile | 5.40^{(a)} | 424.2 |
| 170 | 4-(1H-indol-4-ylamino)-5-[3-(2-morpholin-4-yl ethoxy)phenyl]nicotinonitrile | 5.20^{(a)} | 440.2 |
| 171 | 4-(1H-indol-4-ylamino)-5-[3-(2-piperidin-1-yl ethoxy)phenyl]nicotinonitrile | 3.91^{(a)} | 438.2 |
| 172 | 4-(1H-indol-6-ylamino)-5-[3-(2-pyrrolidin-1-yl ethoxy)phenyl]nicotinonitrile | 5.97^{(a)} | 424.2 |
| 173 | 4-(1H-indol-6-ylamino)-5-[3-(2-morpholin-4-yl ethoxy)phenyl]nicotinonitrile | 5.52^{(a)} | 440.2 |
| 174 | 4-(1H-indol-5-ylamino)-5-[3-(2-pyrrolidin-1-yl ethoxy)phenyl]nicotinonitrile | 5.14^{(a)} | 424.2 |
| 175 | 4-(1H-indol-5-ylamino)-5-[3-(2-morpholin-4-yl ethoxy)phenyl]nicotinonitrile | 5.33^{(a)} | 440.2 |
| 176 | 5-(3-{2-[(2-hydroxyethyl)amino]ethoxy}phenyl)-4-(1*H-*indol-5-ylamino)nicotinonitrile | 1.35^{(c)} | 414.4 |
| 177 | 4-(1*H*-indol-5-ylamino)-5-(3-{2-[(2-pyrrolidin-1-ylethyl)amino]ethoxy}phenyl)nicotinonitrile | 0.67^{(c)} | 467.5 |

### Example 15: Preparation of 5-[3-(2-chloroethoxy)-4-methoxyphenyl]-4-(1H-indol-5-ylamino)nicotinonitrile 178

Following procedures analogous to those described in Example 12, 4-chloro-5-[3-(2-chloroethoxy)-4-methoxyphenyl] nicotinonitrile was prepared from methyl (3-hydroxy-4-methoxyphenyl)acetate. Following procedures analogous to those described for preparing compound **113** (Example 2, Method B), 5-[3-(2-chloroethoxy)-4-methoxyphenyl]-4-(1H-indol-5-ylamino)nicotinonitrile **178** was prepared from 4-chloro-5-[3-(2-chloroethoxy)-4-methoxyphenyl]-nicotinonitrile. MS: 419.1 (M+H); melting range: 153-155 °C.

Following procedures for preparing compound **168** (Example 13, Method A), compounds **179-183** in Table 8 were prepared from 5-[3-(2-chloroethoxy)-4-methoxyphenyl]-4-(1H-indol-5-ylamino)nicotinonitrile **178**.

**Table 8**

| **Compound** | **Compound Name** | **HPLC Retention Time (min.)** | **Observed Ion m/e [M+H]** |
|---|---|---|---|
| 179 | 5-{3-[2-(diethylamino)ethoxy]-4-methoxyphenyl}-4-(1*H*-indol-5-ylamino)nicotinonitrile | 0.613^{(c)} | 456.2 |
| 180 | 5-{3-[2-(diisopropylamino)ethoxy]4-methoxy phenyl}-4-(1*H*-indol-5-ylamino)nicotinonitrile | 0.597^{(c)} | 484.2 |
| 181 | 5-{3-[2-(benzylamino)ethoxy]-4-methoxyphenyl}-4-(1*H*-indol-5-ylamino)nicotinonitrile | 1.038^{(c)} | 490.2 |
| 182 | 4-(1*H*-indol-5-ylamino)-5-(4-methoxy-3-{2-[(2-methoxyethyl)amino]ethoxy}phenyl)nico tinonitrile | 0.456^{(c)} | 458.2 |
| 183 | 4-(1*H*-indol-5-ylamino)-5-(4-methoxy-3-{2-[(5-methyl-1,3,4-thiadiazol-2-yl)amino]ethoxy}phenyl)nicotinonitrile | 0.533^{(c)} | 498.1 |

Following procedures analogous to those described for the preparation of compound **168** (Example 13, Method B), compounds in Table 9 were prepared.

**Table 9**

| **Compound** | **Compound Name** | **HPLC Retention Time (min.)** | **Observed Ion m/e [M+H]** | **Observed HRMS [M+H]** | **Melting Range (°C)** |
|---|---|---|---|---|---|
| 345 | 5-{4-[2-(dimethylamino)ethoxy]-3-methoxyphenyl}-4-[(4-methyl-1*H*-indol-5-yl)amino]nicotinonitrile | 7.7^{(g)} | 442.2 | 442.22426 | 186-188 |
| 414 | 5-(4-{2-[(2-hydroxyethyl)amino]ethoxy} phenyl)-4-[(4-methyl-1*H*-indol-5-yl)amino]nicotinonitrile | 1.41^{(e)} | 428.2 | N/A | N/A |
| 415 | 5-(4-{2-[(3-hydroxypropyl)amino]ethoxy} phenyl)-4-[(4-methyl-1*H*-indol-5-yl)amino]nicotinonitrile | 1.41^{(e)} | 442.2 | N/A | N/A |
| 416 | 5-(4-{2-[(2-ethoxyethyl)amino]ethoxy}phenyl )-4-[(4-methyl-1*H*-indol-5-yl)amino]nicotinonitrile | 1.51^{(e)} | 456.2 | N/A | N/A |
| 417 | 5-[4-(2-{[2-(dimethylamino)ethyl] amino}ethoxy)phenyl]-4-[(4-methyl-1*H*-indol-5-yl)amino]nicotinonitrile | 1.32^{(e)} | 455.2 | N/A | N/A |
| 418 | 4-[(4-methyl-1*H*-indol-5-yl)amino]-5-(4-(2-pyrrolidin-1-ylethoxy)phenyl]nicotinonitrile | 1.48^{(e)} | 438.2 | N/A | N/A |
| 419 | 5-{4-[2-(benzylamino)ethoxy]phenyl}-4-[(4-methyl-1*H*-indol-5-yl)amino]nicotinonitrile | 1.62^{(e)} | 474.2 | N/A | N/A |
| 420 | 4-[(4-methyl-1*H*-indol-5-yl)amino]-5-(4-{2-[(1-methylpiperidin-4-yl)amino]ethoxy} phenyl)nicotinonitrile | 1.31^{(e)} | 481.3 | N/A | N/A |
| 421 | 4-[(4-methyl-1*H*-indol-5-yl)amino]-5-[4-(2-{[(1-methylpiperidin-4-yl)methyl]amino} ethoxy)phenyl]nicotinonitrile | 1.35^{(e)} | 493.3 | N/A | N/A |
| 422 | 5-(4-{2-[4-(hydroxymethyl)piperidin-1-yl]ethoxy}phenyl)-4-[(4-methyl-1*H*-indol-5-yl)amino]nicotinonitrile | 1.47^{(e)} | 482.2 | N/A | N/A |
| 423 | 4-[(4-methyl-1*H*-indol-5-yl)amino]-5-{4-[2-(4-pyrrolidin-1-ylpiperidin-1-yl)ethoxy]phenyl}nicotinonitrile | 1.36^{(e)} | 521.3 | N/A | N/A |
| 424 | 4-[(4-methyl-1*H*-indol-5-yl)amino]-5-{4-[2-(4-morpholin-4-ylpiperidin-1-yl)ethoxy]phenyl}nicotinon itrile | 1.36^{(e)} | 535.3 | N/A | N/A |
| 425 | 5-{4-[2-(4-ethylpiperazin-1-yl)ethoxy]phenyl}-4-[(4-methyl-1*H*-indol-5-yl)amino] nicotinonitrile | 1.50^{(e)} | 481.3 | N/A | N/A |
| 426 | 5-{4-[2-(4-methyl-1,4-diazepan-1-yl)ethoxy]phenyl}-4-[(4-methyl-1*H*-indol-5-yl)amino]nicotinonitrile | 1.36^{(e)} | 481.3 | N/A | N/A |
| 427 | 5-(4-{2-[4-(2-hydroxyethyl)piperazin-1-yl]ethoxy}phenyl)-4-[(4-methyl-1*H*-indol-5-yl)amino]nicotinonitrile | 1.36^{(e)} | 497.3 | N/A | N/A |
| 428 | 5-[4-(2-{4-[2-(dimethylamino)ethyl]piperazin-1-yl}ethoxy)phenyl]-4-[(4-methyl-1*H*-indol-5-yl)amino]nicotinonitrile | 1.36^{(e)} | 524.3 | N/A | N/A |
| 429 | 5-[4-(2-{[3-(1*H*-imidazol-1-yl)propyl]amino}ethoxy)phenyl]-4-[(4-methyl-1*H*-indol-5-yl)amino]nicotinonitrile | 1.34^{(e)} | 492.2 | N/A | N/A |
| 430 | 4-[(4-methyl-1*H*-indol-5-yl)amino]-5-{4-[2-(4-pyridin-2-ylpiperazin-1-yl)ethoxy]phenyl}nicotinonitrile | 1.53^{(e)} | 530.3 | N/A | N/A |
| 431 | 4-[(4-methyl-1*H*-indol-5-yl)amino]-5-{4-[2-(4-pyridin-4-ylpiperazin-1-yl)ethoxy]phenyl}nicotinonitrile | 1.40^{(e)} | 530.3 | N/A | N/A |
| 432 | 4-[(4-methyl-1*H*-indol-5-yl)amino]-5-(4-{2-[(pyridin-2-ylmethyl)amino]ethoxy}phenyl) nicotinonitrile | 1.55^{(e)} | 475.2 | N/A | N/A |
| 433 | 4-[(4-methyl-1*H*-indol-5-yl)amino]-5-(4-{2-[(pyridin-3-ylmethyl)amino]ethoxy}phenyl) nicotinonitrile | 1.47 ^{(e)} | 475.2 | N/A | N/A |
| 434 | 4-[(4-methyl-1*H*-indol-5-yl)amino]-5-(4-{2-[(pyridin-4-ylmethyl)amino]ethoxy}phenyl) nicotinonitrile | 1.44^{(e)} | 475.2 | N/A | N/A |
| 435 | 4-[(4-methyl-1*H*-indol-5-yl)amino]-5-{4-[2-(4-phenylpiperidin-1-yl)ethoxy]phenyl}nicotinonitrile | 1.76^{(e)} | 528.3 | N/A | N/A |
| 438 | 4-[(4-methyl-1*H*-indol-5-yl)amino]-5-{5-[2-(4-methylpiperazin-1-yl)ethoxy]-1-benzofuran-2-yl}nicotinonitrile | 1.312^{(f)} | 507.1 | N/A | N/A |
| 472 | 4-[(4-methyl-1H-indol-5-yl)aminol-5-{4-[3-(4-methylpiperazin-1-yl)propoxy] phenyl}nicotinonitrile | 4.6^{(g)} | 481.3 | 481.2715 | 168-169 |
| 473 | 4-[(4-methyl-1H-indol-5-yl)amino]-5-[4-(3-morpholin-4-ylpropoxy)phenyl]nicotinonitrile | 5.1^{(g)} | 468.3 | N/A | 176-177 |
| 474 | 4-[(4-methyl-1H-indol-5-yl)amino]-5-[4-(3-piperidin-1-ylpropoxy)phenyl]nicotinonitrile | 5.7^{(g)} | 466.3 | 466.2608 | 169-170 |
| 475 | 5-{4-[3-(dimethylamino)propoxy]phenyl}-4-[(4-methyl-1H-indol-5-yl)amino]nicotinonitrile | 5.0^{(g)} | 426.3 | 426.22905 | 180-181 |
| 491 | 4-[(4-methyl-1H-indol-5-yl)amino]-5-{4-[4-(4-methylpiperazin-1-yl)butoxy]phenyl}nicotinonitrile | 4.8^{(g)} | 495.4 | 495.28638 | 149-151 |
| 496 | 4-[(6-methyl-1H-indol-5-yl)amino]-5-{4-[2-(4-methylpiperazin-1-yl)ethoxy]phenyl}nicotinonitrile | 4.4^{(g)} | 467.4 | 467.25576 | 167-168 |
| 502 | 4-[(4-methyl-1H-indol-5-yl)amino]-5-{3-[4-(4-methylpiperazin-1-yl)butoxy]phenyl}nicotinonitrile | 0.7^{(g)} | 495.4 | 495.28504 | 204-207 |
| 504 | 4-[(6-methyl-1H-indol-5-yl)amino]-5-[4-(2-piperidin-1-ylethoxy)phenyl]nicotinonitrile | 7.1^{(h)} | 452.2 | 452.246 | 96.9-98.6 |
| 505 | 5-{4-[2-(4-hydroxypiperidin-1-yl)ethoxy]phenyl}-4-[(6-methyl-1H-indol-5-yl)amino]nicotinonitrile | 5.9^{(h)} | 468.2 | 468.23924 | 96.0-97.2 |
| 506 | 4-[(6-methyl-1H-indol-5-yl)amino]-5-{4-[2-(4-pyrrolidin-1-ylpiperidin-1-yl)ethoxy]phenyl}nicotinonitrile | 4.7^{(h)} | 521.3 | 521.30196 | 187-190 |
| 520 | 4-(1H-indol-5-ylamino)-5-{4-[2-(4-methylpiperazin-1-yl)ethoxy]phenyl}nicotinonitrile | 6.5^{(h)} | N/A | 453.24093 | 125-128 (decom. ) |

### Example 16: Preparation of 4-(1H-indol-5-ylamino)-5-(3-{5-[(4-methyl piperazin-1-yl)methyl]-2-thienyl}phenyl)nicotinonitrile 184

To 5-[3-(5-formyl-2-thienyl)phenyl]-4-(1H-indol-5-ylamino)nicotinonitrile **150** (100 mg, 0.238 mmol) in CH₂Cl₂ (5 mL) and NMP (0.5 mL), cooled with ice-water bath, was added Na(OAc)₃BH (264 mg, 1.2 mmol) and N-methylpiperazine (133 µL, 1.2 mmol). The resulting mixture was stirred at r.t. overnight and partitioned between CH₂Cl₂ and aq. NaHCO₃. The combined organics were dried over Na₂SO₄, concentrated and purified by preparative TLC eluting with 10% MeOH/CH₂Cl₂ to give 63 mg (53% yield) of the title compound as a light yellow solid. MS: 505.2 (M+H); melting range: 120-123 °C.

Following procedures analogous to those described for preparing compound **184**, compounds **185-187** in Table 10 were prepared from 5-[3-(5-formyl-2-thienyl)phenyl]-4-(1H-indol-5-ylamino)nicotinonitrile **150**.

**Table 10**

| **Compound** | **Compound Name** | **Observed Ion m/e [M+H]** | **Melting Range (°C)** |
|---|---|---|---|
| 185 | 4-(1*H*-indol-5-ylamino)-5-{3-[5-(morpholin-4-yl methyl)-2-thienyl]phenyl}nicotinonitrile | 492.1 | 103-107 (decom.) |
| 186 | 4-(1*H*-indol-5-ylamino)-5-{3-[5-(piperidin-1-ylmethyl)-2-thienyl]phenyl}nicotinonitrile | 490.2 | 135-137 |
| 187 | 5-(3-{5-[(dimethylamino)methyl]-2-thienyl} phenyl)-4-(1*H*-indol-5-ylamino)nicotinonitrile | 450.1 | 102-107 (decom.) |

### Example 17: Preparation of 5-(3-bromo-4-methoxyphenyl)-4-(1H-indol-5-ylamino)nicotinonitrile 188

Following procedures analogous to those described in Example 1, 5-(3-bromo-4-methoxyphenyl)-4-chloronicotinonitrile was prepared from methyl (3-bromo-4-methoxyphenyl)acetate. Following procedures analogous to those described above for preparing compound **101** (Example 1), 5-(3-bromo-4-methoxyphenyl)-4-chloronicotinonitrile was treated with 5-aminoindole to produce 5-(3-bromo-4-methoxyphenyl)-4-(1H-indol-5-ylamino) nicotinonitrile **188**. MS: 419.2 (M+H); melting range: 148-150 °C.

Compounds **189-193** in Table 11 were prepared from 5-(3-bromo-4-methoxyphenyl)-4-(1H-indol-5-ylamino) nicotinonitrile **188** and the appropriate boronic acid following procedures analogous to those described for preparing compound **114** (Example 6).

**Table 11**

| **Compound** | **Compound Name** | **Observed Ion m/e [M+H]** | **Melting Range (°C)** |
|---|---|---|---|
| 189 | 4-(1*H*-indol-5-ylamino)-5-[4-methoxy-3-(2-thienyl)phenyl]nicotinonitrile | 423.1 | 122-124 |
| 190 | 5-(4'-chloro-6-methoxybiphenyl-3-yl)-4-(1*H*-indol-5-ylamino)nicotinonitrile | 451.1 | 140-142 |
| 191 | 5-(3'-chloro-6-methoxybiphenyl-3-yl)-4-(1H-indol-5-ylamino)nicotinonitrile | 451.1 | 134-136 |
| 192 | 5'-[5-cyano-4-(1*H*-indol-5-ylamino)pyridin-3-yl]-*N*-cyclopentyl-2'-methoxybiphenyl-4-carboxamide | 528.2 | 155-157 |
| 193 | 5-(2'-chloro-6-methoxybiphenyl-3-yl)-4-(1H-indol-5-ylamino)nicotinonitrile | 451.1 | 158-160 |

### Example 18: Preparation of 5-[3-benzyloxyphenyl]-4-(1H-indol-5-ylamino)nicotinonitrile 194

Following procedures analogous to those described in Example 1, 5-(3-benzyloxyphenyl)-4-chloronicotinonitrile was prepared from methyl (3-benzyloxyphenyl)acetate. Following procedures analogous to those described for preparing compound **113** (Example 2, Method B), 5-[3-benzyloxyphenyl]-4-(1H-indol-5-ylamino)nicotinonitrile **194** was prepared from 5-(3-(benzyloxy)phenyl)-4-chloronicotinonitrile. MS: 417.3 (M+H); melting range: 165-166 °C.

### Example 19: Preparation of 5-[4-benzyloxyphenyl]-4-(1H-indol-5-ylamino)nicotinonitrile 195

Following procedures analogous to those described in Example 1, 5-(4-benzyloxyphenyl)-4-chloronicotinonitrile was prepared from methyl (4-benzyloxyphenyl)acetate. Following procedures analogous to those described for preparing compound **113** (Example 2, Method B), 5-[4-benzyloxyphenyl]-4-(1H-indol-5-ylamino)nicotinonitrile **195** was prepared from 5-(4-benzyloxyphenyl)-4-chloronicotinonitrile. MS: 417.1; melting range: 163-165 °C.

### Example 20: Preparation of 3'-[5-cyano-4-(1H-indol-5-ylamino)pyridin-3-yl]-N-methylbiphenyl-4-carboxamide 196

To a solution of 3'-[5-cyano-4-(1H-indol-5-ylamino)pyridin-3-yl]biphenyl-4-carboxylic acid **134** (40mg, 0.09 mmol) and BOP (41mg, 0.09 mmol) in DMF (1mL) was added methylamine solution in MeOH (2M, 47uL, 0.09 mmol) followed by TEA (19 uL, 0.14 mmol). The reaction mixture was stirred for 12 h at r.t. The crude reaction mixture was then dissolved in dimethylsulfoxide (DMSO, 1 mL) and purified by reversed phase HPLC to give 16 mg 3'-[5-cyano-4-(1H-indol-5-ylamino)pyridin-3-yl]-N-methylbiphenyl-4-carboxamide **196** (39%). MS: 444.2 (M+H); HPLC retention time: 2.10 min. ^{(a)}.

Compounds **197-214** in Table 12 were prepared from 3'-[5-cyano-4-(1H-indol-5-ylamino)pyridin-3-yl]biphenyl-4-carboxylic acid **134** using a procedure analogous to the one described above. Similarly, compounds **215** and **216 in** Table 12 were prepared from 3'-[5-cyano-4-(1H-indol-4-ylamino)pyridin-3-yl]biphenyl-4-carboxylic acid **135** using a procedure analogous to the one described above.

**Table 12**

| **Compound** | **Compound Name** | **HPLC Retention Time (min.)** | **Observe d Ion m/e [M+H]** |
|---|---|---|---|
| 197 | N-butyl-3'-[5-cyano-4-(1H-indol-5-ylamino) pyridin-3-yl] biphenyl-4-carboxamide | 2.28 ^{(a)} | 486.2 |
| 198 | 3'-[5-cyano-4-(1H-indol-5-ylamino)pyridin-3-yl]-N-(1-ethyl propyl) biphenyl-4-carboxamide | 2.30^{(a)} | 500.2 |
| 199 | 3'-[5-cyano-4-(1H-indol-5-ylamino)pyridin-3-yl]-N-(2-hydroxyethyl)biphenyl-4-carboxamide | 1.99^{(a)} | 474.2 |
| 200 | 3'-[5-cyano-4-(1H-indol-5-ylamino)pyridin-3-yl]-N-(2-methoxyethyl)biphenyl-4-carboxamide | 2.11^{(a)} | 488.2 |
| 201 | 3'-[5-cyano-4-(1H-indol-5-ylamino)pyridin-3-yl]-N-cyclopropylbiphenyl-4-carboxamide | 2.14^{(a)} | 470.2 |
| 202 | 3'-[5-cyano-4-(1H-indol-5-ylamino)pyridin-3-yl]-N-cyclohexylbiphenyl-4-carboxamide | 2.35^{(a)} | 512.2 |
| 203 | 3'-[5-cyano-4-(1H-indol-5-ylamino)pyridin-3-yl]-N-(2-pyrrolidin-1-ylethyl)biphenyl-4-carboxamide | 2.04^{(a)} | 527.2 |
| 204 | N-benzyl-3'-[5-cyano-4-(1H-indol-5-ylamino)pyridin-3-yl]biphenyl-4-carboxamide | 2.29^{(a)} | 520.2 |
| 205 | 4-(1H-indol-5-ylamino)-5-[4'-(pyrrolidin-1-ylcarbonyl) biphenyl-3-yl]nicotinonitrile | 2.23^{(a)} | 484.2 |
| 206 | 4-(1H-indol-5-ylamino)-5-[4'-(morpholin-4-ylcarbonyl)biphenyl-3-yl]nicotinonitrile | 2.12^{(a)} | 500.2 |
| 207 | 4-(1H-indol-5-ylamino)-5-{4'-[(4-methylpiperazin-1-yl)carbonyl]biphenyl-3-yl}nicotinonitrile | 1.97^{(a)} | 513.2 |
| 208 | 3'-[5-cyano-4-(1H-indol-5-ylamino)pyridin-3-yl]-N-cyclopentylbiphenyl-3-carboxamide | N/A | 498.4 |
| 209 | 3'-[5-cyano-4-(1H-indol-5-ylamino)pyridin-3-yl]-N-(4-hydroxybutyl)biphenyl-4-carboxamide | 2.05^{(a)} | 502.2 |
| 210 | 3'-[5-cyano-4-(1H-indol-5-ylamino)pyridin-3-yl]-N-(3-hydroxypropyl)biphenyl-4-carboxamide | 2.03^{(a)} | 488.2 |
| 211 | 3'-[5-cyano-4-(1H-indol-5-ylamino)pyridin-3-yl]-N-[2-(methylamino)ethyl]biphenyl-4-carboxamide | 1.94^{(a)} | 487.2 |
| 212 | 3'-[5-cyano-4-(1H-indol-5-ylamino)pyridin-3-yl]-N-(pyridin-2-ylmethyl)biphenyl-4-carboxamide | 2.17^{(a)} | 521.2 |
| 213 | 3'-[5-cyano-4-(1H-indol-5-yiamino)pyridin-3-yl]-N-(pyridin-3-ylmethyl)biphenyl-4-carboxamide | 2.15^{(a)} | 521.2 |
| 214 | 3'-[5-cyano-4-(1H-indol-5-ylamino)pyridin-3-yl]-N-(pyridin-4-ylmethyl)biphenyl-4-carboxamide | 2.12^{(a)} | 521.1 |
| 215 | N-butyl-3'-[5-cyano-4-(1H-indol-4-ylamino)pyridin-3-yl]biphenyl-4-carboxamide | 2.26^{(a)} | 486.2 |
| 216 | 3'-[5-cyano-4-(1H-indol-4-ylamino)pyridin-3-yl]-N-(2-hydroxyethyl)biphenyl-4-carboxamide | 1 99^{(a)} | 474.2 |

### Example 21: Preparation of 5-(3,4-dimethoxyphenyl)-4-[(7-methyl-1H-indol-5-yl)amino]nicotinonitrile 217

Following procedures analogous to those described for preparing compound 113 (Example 2, Method B), the title compound was prepared from 4-chloro-5-(3,4-dimethoxyphenyl)nicotinonitrile and 5-amino-7-methylindole. MS: 385.3 (M+H).

### Example 22: Preparation of 5-(3,4-dimethoxyphenyl)-4-[(4-methyl-1H-indol-5-yl)amino]nicotinonitrile 218

Following procedures analogous to those described for preparing compound **113** (Example 2, Method B), 5-(3,4-dimethoxyphenyl)-4-[(4-methyl-1H-indol-5-yl)amino]nicotinonitrile **218** was prepared from 4-chloro-5-(3,4-dimethoxyphenyl)nicotinonitrile and 5-amino-4-methylindole. MS: 385.2 (M+H); melting range: 130-133 °C.

### Example 23: Preparation of 5-(3,4-dimethoxyphenyl)-4-[1H-indol-5-yl (methyl)amino]nicotinonitrile 219

Following procedures analogous to those described for preparing compound **113** (Example 2, Method B), 5-(3,4-dimethoxypbenyl)4-[1H-indol-5-yl(methyl)amino] nicotinonitrile **219** was prepared from 4-chloro-5-(3,4-dimethoxyphenyl) nicotinonitrile and (1H-indol-5-yl)-methylamine. MS: 385.1; melting range: 224-226 °C (decom.).

### Example 24: Preparation of 5-(3,4-dimethoxyphenyl)-4-(1H-indol-5-yloxy)nicotinonitrile 220

A mixture of 4-chloro-5-(3,4-dimethoxyphenyl)nicotinonitrile (120 mg, 0.44 mmol), 5-hydroxyindole (71 mg, 0.53 mmol) and K₂CO₃ (91 mg, 0.66 mmol) in CH₃CN (4.0 mL) was heated at 80°C for 4 h. After cooling to r.t., the reaction mixture was diluted with water (20 mL) and the precipitate was collected by filtration and washed with ether containing MeOH to give 121 mg (74%) of 5-(3,4-dimethoxyphenyl)-4-(1H-indol-5-yloxy)nicotinonitrile **220** as a tan solid. MS: 372.2; melting range: 192-194 °C.

### Example 25: Preparation of 5-(3,4-dimethoxyphenyl)-4-(1H-indol-5-yl)nicotinonitrile 221

A mixture of 4-chloro-5-(3,4-dimethoxyphenyl)nicotinonitrile (120 mg, 0.44 mmol), indole-5-boronic acid (77 mg, 0.48 mmol), Pd(PPh₃)₄ (25 mg, 0.022 mmol) and aqueous saturated NaHCO₃ (3 mL) in DME (4.0 mL) was heated at reflux for 5 h. The reaction mixture was partitioned between water and EtOAc. The combined organics were dried over Na₂SO₄ and concentrated to give a brown syrup, which was purified by preparative thin-layer chromatography eluting with 3% MeOH/CH₂Cl₂ to give 94 mg (61%) of 5-(3,4-dimethoxyphenyl)-4-(1H-indol-5-yl)nicotinonitrile **221** as an off-white solid. MS: 356.2 (M+H); melting range: 215-217 °C.

### Example 26: Preparation of 5-(1-benzofuran-2-yl)-4-[(4-methyl-1H-indol-5-yl)amino]nicotinonitrile 222

A mixture of 3-aminobut-3-enenitrile (100 g, 1.22 mol) and conc. HCl (125 mL) in water (125 mL) was heated at 80°C for 2 h, cooled to r.t. and filtered to remove the solid. The filtrate was extracted with EtOAc and the combined extracts were dried over Na₂SO₄ and concentrated to give a semi-solid residue which was distilled under vacuum to give 77.4 g (76%) of acetoacetonitrile (73-77°C/3-5 mmHg).

A mixture of acetoacetonitrile (41 g, 493 mmol), t-butoxybis(dimethyl amino)methane (86 g, 493 mmol) and DMF-DMA (263 mL, 1.97 mol) was heated at 100°C overnight and evaporated to remove the volatiles. The residue was triturated with hexanes/ether (1:1) and the solids were collected by filtration and washed with hexanes/ether (1:1) and a minimum amount of EtOAc to give 64.3 g (67%) of 5-(dimethylamino)-2-[(dimethylamino)methylene]-3-oxopent-4-enenitrile as a light yellow solid, which was used for the next step without further purification.

A mixture of 5-(dimethylamino)-2-[(dimethylamino)methylene]-3-oxopent-4-enenitrile (64.3 g, 333 mmol) and ammonium acetate (126 g, 1.66 mol) in EtOH (1.8 L) was heated at reflux for 60 h and concentrated to remove solvent. The resultant semi-solid residue was diluted with EtOAc, filtered and washed with EtOAc followed by CH₂Cl₂. The filtrate was evaporated to a reduced volume. The precipitated solids were collected by filtration, washed with EtOAc and a minimum amount of EtOH. The process of evaporation and crystallization was repeated to obtain more solid product from the mother liquor. The combined off-white solids provided 20.9 g of 4-hydroxynicotinonitrile (53%); melting range: 234-236°C.

Alternatively, 4-hydroxynicotinonitrile may be synthesized according to the methods described in Broekman, F. W. et al., Recueil des Travaux Chimiques des Pays-Bas, 81: 792-796 (1962).

A mixture of 4-hydroxynicotinonitrile (45.7 g, 381 mmol), iodine (96.6 g, 381 mmol) and NaOH (19.8 g, 825 mmol) in water (600 mL) was heated at 85°C overnight, cooled to r.t. and diluted with water. The precipitate was collected by filtration and washed with water to give 57.5 g (61%) of 4-hydroxy-5-iodonicotinonitrile as a tan solid. Mp >245°C.

A mixture of 4-hydroxy-5-iodonicotinonitrile (57.5 g, 234 mmol) and POCl₃ (200 mL) was heated at 100°C for 2 hours, cooled to room temperature and evaporated to remove excess POCl₃. The residue was cooled in an ice-water bath, adjusted to pH 8-9 with aqueous 10 N NaOH and extracted with ethyl acetate. The combined organics were washed with water and brine, dried over magnesium sulfate and concentrated. The resulting solid residue was washed with a minimum amount of methanol and methylene chloride to give 46.5 g (75%) of 4-chloro-5-iodonicotinonitrile as a tan solid. Melting range: 120-122°C.

A mixture of 4-chloro-5-iodonicotinonitrile (5.0 g, 18.9 mmol) and 5-amino-4-methylindole (3.0 g, 20.8 mmol) in EtOH (100 mL) was heated at reflux for 3 days, cooled to r.t. and diluted with saturated aq. Na₂SO₄ (300 mL). The precipitated solids were collected by filtration, washed with water and dried to give 5.3 g (75%) of 5-iodo-4-[(4-methyl-1H-indol-5-yl)amino]nicotinonitrile as a grey solid. Melting range: 192-194°C; MS (M+H⁺): 375.1.

To a mixture of 5-iodo-4-[(4-methyl-1H-indol-5-yl)amino]nicotinonitrile (200 mg, 0.53 mmol), 2-benzofuranboronic acid (173 mg, 1.07 mmol) and Pd(PPh₃)₄ (31 mg, 0.027 mmol) in DME (4.0 mL) was added 2.0 M aqueous Na₂CO₃ (0.8 mL). The resulting mixture was heated at 80°C for 2 h, cooled to r.t. and filtered. The filtrate was concentrated and purified by HPLC to give 35 mg of 5-(1-benzofuran-2-yl)-4-[(4-methyl-1H-indol-5-yl)amino]nicotinonitrile 222 as a yellow solid. MS: 365.2 (M+H); HPLC retention time: 11.4 min.^{(a)}.

Following procedures analogous to those described for the preparation of 5-(1-benzofuran-2-yl)-4-[(4-methyl-1H-indol-5-yl)amino]nicotinonitrile 222, compounds in Table 13 were prepared.

**Table 13**

| **Compd.** | **Compound Name** | **Melting range (°C)** | **HPLC Retention Time (min.)** | **Observed ion m/e [M+H]⁺** | **Observed HRMS [M+H]⁺** |
|---|---|---|---|---|---|
| 412 | 5-(3,4-dimethoxyphenyl)-4-[(6-methyl-1*H*-indol-5-yl)amino]nicotinonitrile | N/A | 1.72^{(e)} | 385.2 | N/A |
| 413 | 5-(1-benzofuran-2-yl)-4-[(6-methyl-1*H*-indol-5-yl)amino]nicotinonitrile | N/A | 3.7⁽ⁱ⁾ | 365.4 | N/A |
| 453 | 4-[(4-methyl-1*H*-indol-5-yl)amino]-2'-(morpholin-4-ylmethyl)-3,4'-bipyridine-5-carbonitrile | N/A | 0.339⁽¹⁾ | 425.0 | N/A |
| 460 | 5-[4-(3-chloropropoxy)phenyl]-4-[(4-methyl-1*H*-indol-5-yl)amino]nicotinonitrile | 151-153 | 10.3^{(g)} | N/A | 417.14818 |
| 463 | 5-(5-formyl-2-thienyl)-4-[(4-methyl-1*H*-indol-5-yl)amino]nicotinonitrile | 190-192 | 8.3^{(g)} | 359.2 | 359.09612 |
| 466 | 5-(3-methyl-1-benzofuran-2-yl)-4-[(4-methyl-1*H-*indol-5-yl)amino]nicotinonitrile | 224-226 | 11.4^{(g)} | 379.3 | 379.15464 |
| 467 | 4-[(4-methyl-1*H*-indol-5-yl)amino]-3,4'-bipyridine-5-carbonitrile | 250 (decom.) | 4.9^{(g)} | 326.2 | 326.13969 |
| 469 | 2'-chloro-4-((4-methyl-1*H-*indol-5-yl)amino]-3,4'-bipyridine-5-carbonitrile | 134-135 | 8.3^{(g)} | 360.2 | 360.10099 |
| 470 | 5-{2-chloro-4-[2-(4-methylpiperazin-1-yl)ethoxy]phenyl}-4-[(4-methyl-1H-indol-5-yl)amino]nicotinonitrile | N/A | N/A | 501.4 | 501.21649 |
| 471 | 2'-chloro-4-[(4-methyl-1*H-*indol-5-yl)amino]-3,3'-bipyridine-5-carbonitrile | N/A | 7.2^{(g)} | 360.2 | 360.10091 |
| 477 | 5-[3,4-bis(2-methoxyethoxy)phenyl]-4-[(4-methyl-1*H*-indol-5-yl)amino]nicotinonitrile | N/A | 8.6^{(g)} | N/A | 529.18902 |
| 488 | 5-(3,4-dimethoxyphenyl)-4-(1H-pyrrolo[2,3-b]pyridin-5-ylamino)nicotinonitrile | 235-236 | 6.2^{(g)} | 372.3 | 372.14654 |
| 489 | 5-(5-formyl-2-furyl)-4-[(4-methyl-1H-indol-5-yl)amino]nicotinonitrile | 225-227 (decom.) | N/A | 343.2 | 343.11964 |
| 494 | 5-[4-(2-chloroethoxy)phenyl]-4-[(6-methyl-1*H*-indol-5-yl)amino]nicotinonitrile | N/A | 9.6^{(g)} | 403.2 | N/A |
| 498 | 4-[(7-chloro-4-methyl-1*H-*indol-5-yl)amino]-5-(3,4-dimethoxyphenyl)nicotino nitrile | N/A | 8.8^{(g)} | 419.2 | 419.12665 |
| 507 | 4-[(4-methyl-1*H*-indol-5-yl)amino]-6'-morpholin-4-yl-3,3'-bipyrid ine-5-carbonitrile | > 245 | 5.6^{(g)} | N/A | 411.19329 |
| 508 | 4-[(4-methyl-1*H*-indol-5-yl)amino]-6'-piperidin-1-yl-3,3'-bipyridine-5-carbonitrile | > 245 | 6.0^{(g)} | N/A | 409.21414 |
| 509 | 4-[(4-methyl-1*H*-indol-5-yl)amino]-5-pyrimidin-5-ylnicotinonitrile | > 245 | 5.5^{(g)} | N/A | 327.13563 |
| 510 | 4-[(4-methyl-1*H*-indol-5-yl)amino]-5-(2-piperidin-1-ylpyrimidin-5-yl)nicotinonitrile | > 245 | 9.3^{(g)} | N/A | 410.20928 |
| 511 | 4-[(4-methyl-1*H*-indol-5-yl)amino]-5-(2-morpholin-4-ylpyrimidin-5-yl)nicotinonitrile | > 245 | 7.1^{(g)} | 412.3 | 412.18835 |
| 512 | 4-[(4-methyl-1*H*-indol-5-yl)amino]-5-(2-pyrrolidin-1-ylpyrimidin-5-yl)nicotinonitrile | >245 | 6.9^{(g)} | 396.3 | 396.19427 |
| 513 | 5-[2-(dimethylamino)pyrimidin-5-yl]-4-[(4-methyl-1*H-*indol-5-yl)amino]nicotinonitrile | > 245 | 6.7^{(g)} | 370.3 | 370.17834 |
| 514 | 5-(1-benzothien-2-yl)-4-[(4-methyl-1*H*-indol-5-yl)amino]nicotinonitrile | > 245 | 11.3^{(g)} | N/A | 381.11811 |
| 515 | 5-[4-(2-chloroethoxy)phenyl]-4-(1*H*-indol-5-ylamino)nicotinonitrile | N/A | N/A | N/A | 389.11768 |
| 516 | 5-(5-formyl-3-thienyl)-4-[(4-methyl-1*H*-indol-5-yl)amino]nicotinonitrile | 241-244 (decom.) | 7.4^{(g)} | 359.3 | N/A |
| 517 | 5-(4-formyl-2-furyl)-4-[(4-methyl-1*H*-indol-5-yl)amino]nicotinonitrile | 240-242 (decom.) | 8.0^{(g)} | 343.3 | N/A |
| 519 | 4-[(4-methyl-1*H*-indol-5-yl)amino]-3,3'-bipyridine-5-carbonitrile | > 245 | 5.1^{(g)} | 326.2 | 326.14049 |
| 523 | 5-(3,4-dimethoxyphenyl)-4-(1*H*-indol-5-ylamino)nicotinonitrile 1-oxide | N/A | 8.5^{(g)} | 387.3 | 387.14545 |
| 525 | 1-butyl-3-(4-{5-cyano-4-[(4-methyl-1*H*-indol-5-yl)amino]pyridin-3-yl}phenyl)urea | 142.7-147.2 | 8.9^{(g)} | 439.1 | 439.2241 |
| 526 | methyl (4-{S-cyano-4-[(4-methyl-1H-indol-5-yl)amino]pyridin-3-yl}phenyl)carbamate | 216.4-219.2 | 7.8^{(g)} | 398.3 | 398.1611 |
| 527 | benzyl (4-{5-cyano-4-[(4-methyl-1H-indol-5-yl)amino]pyridin-3-yl}-2-fluorophenyl)carbamate | 200.9-203 | 10.9^{(g)} 10.9 | 492.3 | 492.1830 |
| 528 | 4-methoxybenzyl (4-{5-cyano-4-[(4-methyl-1*H-*indol-5-yl)amino]pyridin-3-yl}-2-fluorophenyl)carbamate | 115-116 | 11.2^{(g)} | 522.3 | 522.1946 |
| 545 | 5-(3,4-dimethoxyphenyl)-4-[(4-methoxy-1*H*-indol-5-yl)amino]nicotinonitrile | N/A | 7.5^{(g)} | 401.2 | 401.1613 |
| 546 | 5-(3,4-dimethoxyphenyl)-4-[(4-fluoro-1*H*-indol-5-yl)amino]nicotinonitrile | N/A | 8.2^{(g)} | 389.2 | 389.1410 |
| 549 | 5-(3,4-dimethoxyphenyl)-4-[(2,4-dimethyl-1*H*-indol-5-yl)amino]nicotinonitrile | N/A | 8.8^{(g)} | 399.2 | 399.1819 |
| 550 | 5-{2-[(dimethylamino)methyl]p henyl}-4-[(4-methyl-1*H-*indol-5-yl)amino]nicotinonitrile | 200-202 (decom.) | N/A | 382.3 | N/A |
| 551 | 5-(5-formyl-2-methoxyphenyl)-4-[(4-methyl-1*H*-indol-5-yl)amino]nicotinonitrile | N/A | 7.3^{(g)} | 383.3 | N/A |
| 555 | 5-(3,4-dimethoxyphenyl)-4-[(1,4-dimethyl-1H-indol-5-yl)amino]nicotinonitrile | 233-234 (decom.) | 8.9^{(g)} | 399.3 | N/A |
| 556 | 3-{5-cyano-4-[(4-methyl-1H-indol-5-yl)amino]pyridin-3-yl}benzoic acid | > 250 | 7.0^{(g)} | 369.3 | N/A |
| 557 | 5-(2-methoxyphenyl)-4-[(4-methyl-1*H*-indol-5-yl)amino]nicotinonitrile | N/A | 6.7^{(g)} | 355.3 | N/A |
| 558 | 5-(3-methoxyphenyl)-4-[(4-methyl-1*H*-indol-5-yl)amino]nicotinonitrile | N/A | 8.4^{(g)} | 355.3 | N/A |
| 559 | 5-(4-methoxyphenyl)-4-[(4-methyl-1*H*-indol-5-yl)amino]nicotinonitrile | N/A | 8.3^{(g)} | 355.3 | N/A |
| 560 | 4-[(4-methyl-1*H*-indol-5-yl)amino]-5-phenylnicotinonitrile | N/A | 8.0^{(g)} | 325.3 | N/A |
| 561 | 4-[(4-methyl-1*H*-indol-5-yl)amino]-5-(2-thienyl)nicotinonitrile | N/A | 8.2^{(g)} | 331.2 | N/A |
| 562 | 4-[(4-methyl-1*H*-indol-5-yl)amino]-5-(3-thienyl)nicotinonitrile | N/A | 7.7^{(g)} | 331.2 | N/A |
| 563 | 5-(3-furyl)-4-[(4-methyl-1*H*-indol-5-yl)amino]nicotinonitrile | N/A | 7.0^{(g)} | 315.3 | N/A |
| 566 | 1-(4-{5-cyano-4-[(4-methyl-1*H*-indol-5-yl)amino]pyridin-3-yl}phenyl)-3-cyclopropylurea | N/A | 7.2^{(g)} | 423.4 | N/A |
| 567 | 1-(4-{5-cyano-4-[(4-methyl-1H-indol-5-yl)amino]pyridin-3-yl}phenyl)-3-methylurea | N/A | 6.4^{(g)} | 397.4 | N/A |
| 568 | 3-(4-{5-cyano-4-[(4-methyl-1H-indol-5-yl)amino]pyridin-3-yl}phenyl)-1,1-dimethylurea | N/A | 6.9^{(g)} | 411.4 | N/A |
| 569 | N-(4-{5-cyano-4-[(4-methyl-1*H*-indol-5-yl)amino]pyridin-3-yl}phenyl)morpholine-4-carboxamide | N/A | 6.8^{(g)} | 453.4 | N/A |
| 570 | 4-[(4-methyl-1H-indol-5-yl)amino]-5-(4-nitrophenyl)nicotinonitrile | N/A | 9.0^{(g)} | 370.2 | N/A |
| 571 | 5-(4-aminophenyl)-4-[(4-methyl-1H-indol-5-yl)amino]nicotinonitrile | N/A | 5.8^{(g)} | 340.2 | N/A |
| 572 | 5-(3-aminophenyl)-4-[(4-methyl-1H-indol-5-yl)amino]nicotinonitrile | N/A | 6.1^{(g)} | 340.2 | N/A |
| 573 | 5-(2-aminophenyl)-4-[(4-methyl-1H-indol-5-yl)amino]nicotinonitrile | N/A | 7.7^{(g)} | 340.2 | N/A |
| 574 | 5-[4-(dimethylamino)phenyl]-4-[(4-methyl-1H-indol-5-yl)amino]nicotinonitrile | N/A | 8.1^{(g)} | 368.3 | N/A |
| 575 | 5-[3-(dimethylamino)phenyl]-4-[(4-methyl-1H-indol-5-yl)amino]nicotinonitrile | N/A | 8.0^{(g)} | 368.3 | N/A |
| 576 | N-(4-{5-cyano-4-[(4-methyl-1H-indol-5-yl)amino]pyridin-3-yl}phenyl)acetamide | N/A | 6.6^{(g)} | 382.3 | N/A |
| 577 | N-(2-{5-cyano-4-[(4-methyl-1H-indol-5-yl)amino]pyridin-3-yl}phenyl)acetamide | N/A | 9.3^{(g)} | 382.3 | N/A |
| 578 | N-(3-{5-cyano-4-[(4-methyl-1H-indol-5-yl)amino]pyridin-3-yl}phenyl)acetamide | N/A | 6.6^{(g)} | 382.3 | N/A |
| 579 | N-(4-{5-cyano-4-[(4-methyl-1H-indol-5-yl)amino]pyridin-3-yl}phenyl)-2-methylpropanamide | N/A | 8.2^{(g)} | 410.4 | N/A |
| 580 | 4-{5-cyano-4-[(4-methyl-1H-indol-5-yl)amino]pyridin-3-yl}-N-methylbenzamide | N/A | 6.1^{(g)} | 382.3 | N/A |

### Example 27: Preparation of 5-{5-[(dimethylamino)methyl]-1-benzothien-2-yl}-4-(1H-indol-4-ylamino)nicotinonitrile 224

Following procedures analogous to those described in Example 25, 5-(5-formyl-1-benzothien-2-yl)-4-(1H-indol-4-ylamino)nicotinonitrile **223** was prepared from 5-iodo-4-[(1H-indol-4-yl)amino] nicotinonitrile, MS: 395.1 (M+H); melting range: 206-208 °C (decom.).

To 5-(5-formyl-1-benzothien-2-yl)-4-(1H-indol-4-ylamino)nicotinonitrile **223** (166 mg, 0.38 mmol) in THF (7 mL) was added dimethylamine in THF (2.0 M, 0.58 mL, 1.15 mmol) followed by acetic acid (126 mg, 2.09 mmol). The resulting mixture was stirred at r.t. for 1 h and additional dimethylamine (1.2 mL, 2.30 mmol) was added. After stirring at r.t. for an additional hour, Na(OAc)₃BH (242 mg, 1.15 mmol) was added. The reaction mixture was stirred at r.t. for 2 h and was partitioned between diluted HCl and EtOAc. The combined aqueous extracts were basified with aqueous NaHCO₃ and extracted with EtOAc. The combined organics were dried over Na₂SO₄, concentrated and purified by flash column chromatography to give 115 mg (65%) of 5-{5-[(dimethylamino)methyl]-1-benzothien-2-yl}-4-(1H-indol-4-ylamino)nicotinonitrile **224** as a pale yellow solid. MS: 424.2 (M+H); HPLC retention time: 2.1 min.⁽²⁾.

Following procedures analogous to those described for the preparation of 5-(1-benzofuran-2-yl)-4-[(4-methyl-1H-indol-5-yl)amino]nicotinonitrile **224**, compounds in Table 14 were prepared.

**Table 14**

| **Compd.** | **Compound Name** | **Melting range (°C)** | **HPLC retention time (min.)** | **Observed ion m/e [M+H]⁺** | **Observed HRMS (M+H]⁺** |
|---|---|---|---|---|---|
| 382 | 5-{5-[(dimethylamino)methyl]-1-benzofuran-2-yl}-4-[(4-methyl-1*H-*indol-5-yl)amino]nicotinonitrile | N/A | 1.75^{(e)} | 422.2 | N/A |
| 383 | 5-(5-{[(2-hydroxyethyl)amino]methyl}-1-benzofuran-2-yl)-4-[(4-methyl-1*H-*indol-5-yl)amino]nicotinonitrile | N/A | 1.69^{(e)} | 438.2 | N/A |
| 384 | 5-(5-{[(3-hydroxypropyl)amino]methyl}-1-benzofuran-2-yl)-4-[(4-methyl-1*H-*indol-5-yl)amino]nicotinonitrile | N/A | 1.71^{(e)} | 452.2 | N/A |
| 385 | 5-(5-{[(2,3-dihydroxypropyl)amino]methyl}-1-benzofuran-2-yl)-4-[(4-methyl-1*H-*indol-5-yl)amino]nicotinonitrile | N/A | 1.71^{(e)} | 468.2 | N/A |
| 386 | 5-(5-{[(2,3-dihydroxypropyl)(methyl)amino]met hyl}-1-benzofuran-2-yl)-4-[(4-methyl-1*H*-indol-5-yl)amino]nicotinonitrile | N/A | 1.69^{(e)} | 482.2 | N/A |
| 387 | 5-{5-[(cyclohexylamino)methyl]-1-benzofuran-2-yl}-4-[(4-methyl-1*H-*indol-5-yl)amino]nicotinonitrile | N/A | 2.04^{(e)} | 478.6 | N/A |
| 388 | 4-[(4-methyl-1*H*-indol-5-yl)amino]-5-(5-(morpholin-4-ylmethyl)-1-benzofuran-2-yl]nicotinonitrile | N/A | 1.76^{(e)} | 464.2 | N/A |
| 389 | 4-[(4-methyl-1*H*-indol-5-yl)amino]-5-[5-(pyrrolidin-1-ylmethyl)-1-benzofuran-2-yl]nicotinonitrile | N/A | 1.81^{(e)} | 448.2 | N/A |
| 391 | 4-[(4-methyl-1*H*-indol-5-yl)amino]-5-[5-({[(1-methylpiperidin-4-yl)methyl]amino}methyl)-1-benzofuran-2-yl]nicotinonitrile | 196-199 | 1.58^{(e)} | 505.3 | N/A |
| 392 | 5-(5-{[4-(hydroxymethyl)piperidin-1-yl]methyl}-1-benzofuran-2-yl)-4-[(4-methyl-1*H*-indol-5-yl)amino]nicotinonitrile | N/A | 1.77^{(e)} | 492.2 | N/A |

| **Compd.** | **Compound Name** | **Melting range (°C)** | **HPLC retention time (min.)** | **Observed ion m/e [M+H]⁺** | **Observed HRMS [M+H]⁺** |
|---|---|---|---|---|---|
| 393 | 4-[(4-methyl-1*H*-indol-5-yl)amino]-5-{5-[(4-pyrrolidin-1-ylpiperidin-1-yl)methyl]-1-benzofuran-2-yl}nicotinonitrile | N/A | 1.59^{(e)} | 531.3 | N/A |
| 394 | 5-[5-(1,4'-bipiperidin-1'-ylmethyl)-1-benzofuran-2-yl]-4-[(4-methyl-1*H-*indol-5-yl)amino]nicotinonitrile | N/A | 1.61^{(e)} | 545.3 | N/A |
| 395 | 4-[(4-methyl-1*H*-indol-5-yl)amino]-5-{5-[(4-morpholin-4-ylpiperidin-1-yl)methyl]-1-benzofuran-2-yl}nicotinonitrile | N/A | 1.58^{(e)} | 547.3 | N/A |
| 396 | 5-[5-({4-[2-(dimethylamino)ethyl]piperazin-1-yl}methyl)-1-benzofuran-2-yl]-4-[(4-methyl-1*H*-indol-5-yl)amino]nicotinonitrile | N/A | 1.58 ^{(e)} | 534.3 | N/A |
| 397 | 4-[(4-methyl-1*H*-indol-5-yl)amino]-5-{5-[(4-pyridin-2-ylpiperazin-1-yl)methyl]-1-benzofuran-2-yl}nicotinonitrile | N/A | 1.83 ^{(e)} | 540.2 | N/A |
| 398 | 4-[(4-methyl-1*H*-indol-5-yl)amino]-5-(5-{[(pyridin-2-ylmethyl)amino]methyl}-1-benzofuran-2-yl)nicotinonitrile | N/A | 1.85^{(e)} | 485.2 | N/A |
| 399 | 4-[(4-methyl-1*H*-indol-5-yl)amino]-5-(5-{[(pyridin-3-ylmethyl)amino]methyl}-1-benzofuran-2-yl)nicotinonitrile | N/A | 1.75 ^{(e)} | 485.2 | N/A |
| 400 | 4-[(4-methyl-1*H*-indol-5-yl)amino]-5-(5-{[(pyridin-4-ylmethyl)amino]methyl}-1-benzofuran-2-yl)nicotinonitrile | N/A | 1.71 ^{(e)} | 485.2 | N/A |
| 401 | 4-[(4-methyl-1*H*-indol-5-yl)amino]-5-[5-(morpholin-4-ylmethyl)-2-furyl]nicotinonitrile | N/A | 0.914 ^{(f)} | 414.0 | N/A |
| 402 | 4-[(4-methyl-1*H*-indol-5-yl)amino]-5-[5-(piperidin-1-ylmethyl)-2-furyl]nicotinonitrile | N/A | 0.388 ^{(f)} | 412.0 | N/A |
| 403 | 5-[5-(1,4'-bipiperidin-1'-ylmethyl)-2-furyl]-4-[(4-methyl-1*H*-indol-5-yl)amino]nicotinonitrile | N/A | 0.322 ^{(f)} | 495.1 | N/A |
| 404 | 4-[(4-methyl-1*H*-indol-5-yl)amino]-5-{5-[(4-pyrrolidin-1-ylpiperidin-1-yl)methyl]-2-furyl}nicotinonitrile | N/A | 0.288 ^{(f)} | 481.1 | N/A |
| 405 | 4-[(4-methyl-1*H*-indol-5-yl)amino]-5-{5-[(4-morpholin-4-ylpiperidin-1-yl)methyl]-2-furyl}nicotinonitrile | N/A | 0.324^{(f)} | 497.1 | N/A |
| 406 | 5-{5-[(diethylamino)methyl]-2-furyl}-4-[(4-methyl-1*H*-indol-5-yl)amino]nicotinonitrile | N/A | 0.356^{(f)} | 400.0 | N/A |
| 407 | 5-{5-[(dibutylamino)methyl]-2-furyl}-4-[(4-methyl-1*H*-indol-5-yl)amino]nicotinonitrile | N/A | 1.497 ^{(f)} | 456.1 | N/A |
| 408 | 5-{5-[(benzylamino)methyl]-2-furyl}-4-[(4-methyl-1*H*-indol-5-yl)amino]nicotinonitrile | N/A | 0.819 ^{(f)} | 433.9 | N/A |
| 409 | 4-[(4-methyl-1*H*-indol-5-yl)amino]-5-(5-{[(3-phenylpropyl)amino]methyl}-2-furyl)nicotinonitrile | N/A | 0.316 ^{(f)} | 398.0 | N/A |
| 410 | 4-[(4-methyl-1*H*-indol-5-yl)amino]-5-[5-(pyrrolidin-1-ylmethyl)-2-furyl]nicotinonitrile | N/A | 0.873 ^{(f)} | 430.0 | N/A |
| 411 | 4-[(4-methyl-1*H*-indol-5-yl)amino]-5-[5-(thiomorpholin-4-ylmethyl)-2-furyl]nicotinonitrile | N/A | 1.381 ^{(f)} | 462.0 | N/A |
| 436 | 5-(5-{[4-(dimethylamino)piperidin-1-yl]methyl}-2-furyl)-4-[(4-methyl-1*H*-indol-5-yl)amino]nicotinonitrile | N/A | 0.280 ^{(f)} | 455.0 | N/A |
| 437 | 5-{5-[(4-isopropylpiperazin-1-yl)methyl]-2-fury}-4-[(4-methyl-1*H*-indol-5-yl)amino]nicotinonitrile | N/A | 0.407 ^{(f)} | 455.0 | N/A |
| 449 | 4-[(4-methyl-1*H*-indol-5-yl)amino]-5-{4-[(4-methylpiperazin-1-yl)methyl]phenyl}nicotinonitrile | N/A | 0.314 ^{(f)} | 437.0 | N/A |
| 450 | 4-[(4-methyl-1*H*-indol-5-yl)amino]-5-[4-(pyrrolidin-1-ylmethyl)phenyl]nicotinonitrile | N/A | 0.298 ^{(f)} | 408.0 | N/A |
| 451 | 4-[(4-methyl-1*H*-indol-5-yl)amino]-5-[4-(morpholin-4-ylmethyl)phenyl]nicotinonitrile | N/A | 0.381 ^{(f)} | 424.0 | N/A |
| 452 | 5-{4-[(dimethylamino)methyl]phenyl}-4-[(4-methyl-1*H*-indol-5-yl)amino]nicotinonitrile | N/A | 0.263^{(f)} | 381.9 | N/A |
| 454 | 4-[(4-methyl-1*H*-indol-5-yl)amino]-5-{3-[(4-methylpiperazin-1-yl)methyl]phenyl}nicotinonitrile | N/A | 0.355 ^{(f)} | 437.0 | N/A |
| 455 | 4-[(4-methyl-1*H*-indol-5-yl)amino]-5-[3-(pyrrolidin-1-ylmethyl)phenyl]nicotinonitrile | N/A | 0.320 ^{(f)} | 408.0 | N/A |
| 456 | 4-[(4-methyl-1*H*-indol-5-yl)amino]-5-[3-(morpholin-4-ylmethyl)phenyl]nicotinonitrile | N/A | 0.425 ^{(f)} | 424.0 | N/A |
| 457 | 5-{3-[(dimethylamino)methyl]phenyl}-4-[(4-methyl-1*H*-indol-5-yl)amino]nicotinonitrile | N/A | 0.678^{(f)} | 382.0 | N/A |
| 459 | 5-{2-fluoro-4-[(4-methylpiperazin-1-yl)methyl]phenyl}-4-[(4-methyl-1*H-*indol-5-yl)amino]nicotinonitrile | N/A | 5.0 ^{(g)} | 455.4 | 455.23501 |
| 461 | 4-[(4-methyl-1*H*-indol-5-yl)amino]-5-[5-(piperidin-1-ylmethyl)-1-benzofuran-2-yl]nicotinonitrile | 212-214 | 6.9 ^{(g)} | N/A | 462.22967 |
| 486 | 5-{5-[(4-cyclopentylpiperazin-1-yl)methyl]-2-furyl}-4-[(4-methyl-1H-indol-5-yl)amino]nicotinonitrile | 174-176 (decom.) | 5.9 ^{(g)} | 481.4 | 481.26968 |
| 487 | 5-{5-[(1,1-dioxidothiomorpholin-4-yl)methyl]-2-furyl}-4-[(4-methyl-1*H*-indol-5-yl)amino]nicotinonitrile | 170-172 (decom.) | 7.2 ^{(g)} | 462.3 | 462.15913 |
| 521 | 4-[(4-methyl-1H-indol-5-yl)amino]-5-{5-[(4-methylpiperazin-1-yl)methyl]-1-benzothien-2-yl}nicotinonitrile | 171-172 | 10.7 ^{(h)} | N/A | 493.21682 |

### Example 28: Preparation of 5-(4-{[(2S)-2-amino-3-phenylpropyl]oxy}phenyl)-4-(1H-indol-4-ylamino)nicotinonitrile 226

Following procedures analogous to those described in Example 25, 5-(4-hydroxyphenyl)-4-(1H-indol-4-ylamino)nicotinonitrile **225** was prepared from 5-iodo-4-[(1H-indol-4-yl)amino]nicotinonitrile. MS: 327.1 (M+H); melting range: 235-237 °C.

To a suspension of 5-(4-hydroxyphenyl)-4-(1H-indol-4-ylamino)nicotinonitrile **225** (100 mg, 0.31 mmol), tert-butyl (1S)-1-benzyl-2-hydroxyethylcarbamate (77 mg, 0.31 mmol), and Ph₃P (97 mg, 0.37 mmol) in THF (2.0 mL) was added diethyl azodicarboxylate (64 mg, 0.37 mmol) via a syringe over 6 min. The resulting mixture was stirred at r.t. for 21 h and treated with concentrated HCl (-200 mg). After heating at 60°C for one h, the reaction mixture was partitioned between saturated NaHCO₃ and CH₂Cl₂. The combined organics were dried over Na₂SO₄, concentrated and purified by silica gel flash column chromatography (eluting with 4% MeOH/CH₂Cl₂) to give 42 mg (30%) of 5-(4-{[(2S)-2-amino-3-phenylpropyl]oxy}phenyl)-4-(1H-indol-4-ylamino)nicotinonitrile **226** as a white solid. MS: 460.4 (M+H); melting range: 83-85 °C.

Following procedures analogous to those described for the preparation of 5-(4-{[(2S)-2-amino-3-phenylpropyl]oxy}phenyl)-4-(1H-indol-4-ylamino)nicotinonitrile **226**, 5-(4-{[(2R)-2-amino-3-phenylpropyl]oxy}phenyl)-4-(1H-indol-4-ylamino)nicotinonitrile **458** was prepared. HPLC retention time: 6.2 min. ^{(g)}; MS: 460.2 (M+H); melting point: 95-96 °C; and HRMS: 460.21519.

### Example 29: Preparation of 4-[(4-methyl-1H-indol-5-yl)amino]-5-[5-(piperidin-1-ylmethyl)-1-benzothien-2-yl]nicotinonitrile 228 and 5-[5-(hydroxymethyl)-1-benzothien-2-yl]-4-[(4-methyl-1H-indol-5-yl)amino]nicotinonitrile 229

Following procedures analogous to those described in Example 25, 5-(5-formyl-1-benzothien-2-yl)-4-[(4-methyl-1H-indol-5-yl)amino]nicotinonitrile **227** was prepared from 5-iodo-4-[(4-methyl-1H-indol-5-yl)amino]nicotinonitrile. MS: 409.2 (M+H); melting range: 185-187 °C.

Sodium cyanoborohydride (8.0 mg, 0.13 mmol) was added in portions to a stirred mixture of 5-(5-formyl-1-benzothien-2-yl)-4-[(4-methyl-1H-indol-5-yl)amino]nicotinonitrile **227** (40 mg, 0.098 mmol), piperidine (10 mg, 0.12 mmol) and acetic acid (7.0 mg, 0.12 mmol) in EtOH (3.0 mL). The resulting mixture was stirred at r.t. overnight and diluted with CH₂Cl₂ (30 mL). Silica gel and potassium bicarbonate (20 mg) were added and the mixture was concentrated to give a solid residue which was purified by silica gel flash column chromatography (eluting with 3% MeOH in CH₂Cl₂) to give 26 mg (57%) of 4-[(4-methyl-1H-indol-5-yl)amino]-5-[5-(piperidin-1-ylmethyl)-1-benzothien-2-yl]nicotinonitrile **228** as an off-white solid (MS: 478.3 (M+H); melting range: 180-182 °C; HPLC retention time: 7.1 min.), and 7 mg (18%) of 5-[5-(hydroxymethyl)-1-benzothien-2-yl]-4-[(4-methyl-1H-indol-5-yl)amino]nicotinonitrile **229** as an off-white solid (MS: 411.3 (M+H); melting range: 174-175 °C, HPLC retention time: 8.6 min. ^{(a)}).

Following procedures analogous to those described for the preparation of 4-[(4-methyl-1H-indol-5-yl)amino]-5-[5-(piperidin-1-ylmethyl)-1-benzothien-2-yl]nicotinonitrile **228**, the following compounds were prepared:

4-[(4-Methyl-1H-indol-5-yl)amino]-5-{5-[(4-methylpiperazin-1-yl)methyl]-2-thienyl}nicotinonitrile **464**. HPLC retention time: 4.9 min. ^{(g)}; MS: 443.4 (M+H); melting point: 255 °C (decom.); and HRMS: 443.20223; and

4-[(4-Methyl-1H-indol-5-yl)amino]-5-{5-[(4-methylpiperazin-1-yl)methyl]-2-furyl}nicotinonitrile **468**. HPLC retention time: 5.2 min. ^{(g)}; MS: 427.3 (M+H); melting point: 245 °C (decom.); and HRMS: 427.22449.

### Example 30: Preparation of 4-chloro-5-iodo-1-oxy-nicotinonitrile

To a solution of 4-chloro-5-iodo-nicotinonitrile (529 mg, 2.0 mmol) in TFA (5 mL) was added H₂O₂ (30wt% in H₂O, 5 mL). The reaction mixture was stirred at room temperature overnight, heated to 50 °C for 8 h, and concentrated. To the residue was added saturated aqueous NaHCO₃ (10 mL) followed by extraction with EtOAc/THF. The organic layers were combined, washed with H₂O, dried over Na₂SO₄, filtered, and concentrated. The residue was purified by a flash chromatography (CH₂Cl₂-THF = 10:1) to give 202 mg (36%) of 4-chloro-5-iodo-1-oxy-nicotinonitrile as a pale-yellow solid.

### Example 31: Preparation of 4-fluoro-5-[3-methoxy-4-(2-methoxyethoxy)phenyl] nicotinonitrile

4-Chloro-5-(3,4-dimethoxyphenyl)nicotinonitrile (2.0 g, 7.3 mmol) was dissolved in DMF (70 mL) and treated with CsF (2.2 g, 14.6 mmol). After the resulting solution was heated at 80 °C for 2 h, additional CsF (1 g, 7 mmol) was added and the heating was continued overnight. The crude product was purified by chromatography (EtOAc/Hex) to give 300 mg of 4-fluoro-5-[3-methoxy-4-(2-methoxyethoxy)phenyl]nicotinonitrile.

### Example 32: Preparation of 5-amino-6-methylindole

3-Methyl-4-nitroaniline was treated with iodine and silver sulfate to give 2-iodo-5-methyl-4-nitroaniline. Treatment of the nitroaniline with 2-(trimethylsilyl)acetylene under the typical Sonogashira conditions gave 5-methyl-4-nitro-2-[(trimethylsilyl)ethynyl]aniline, which was treated with a base in an aqueous methanolic solution to give 2-ethynyl-5-methyl-4-nitroaniline. A subsequent base-induced cyclization at an elevated temperature gave 5-nitro-6-methylindole, which was treated under a high pressure hydrogenation condition to provide 5-amino-6-methylindole.

### Example 33: Preparation of 5-amino-4-fluoroindole

Treatment of 1-(4-fluoro-2,3-dihydro-indol-1-yl)-ethanone, prepared from 4-fluoroindole (See e.g., EP 0645385A1), with concentrated H₂SO₄ and fuming HNO₃ in glacial acetic acid gave 1-(4-fluoro-5-nitro-2,3-dihydro-indol-1-yl)-ethanone. Deprotection of the acetyl group with Na₂S in aqueous ethanol provided 4-fluoro-5-nitro-2,3-dihydro-1*H*-indole, which was treated with 2,3-dicyano-5,6-dichloro-parabenzoquinone (DDQ) to provide 4-fluoro-5-nitroindole. A subsequent hydrogenation over Pd/C gave 5-amino-4-fluoroindole.

### Example 34: Preparation of 5-amino-4-methoxyindole

Treatment of 1-(4-fluoro-5-nitro-2,3-dihydro-indol-1-yl)-ethanone with Claisen's alkali in CH₃OH gave 4-methoxy-5-nitro-2,3-dihydro-1*H*-indole, which was treated with DDQ followed by a hydrogenation over Pd/C to produce 5-amino-4-methoxyindole.

### Example 35: Preparation of 5-amino-2,4-dimethylindole

Addition of MeMgBr to 2-methyl-5-nitroindole followed by hydrogenation over Pd/C provided 5-amino-2,4-dimethylindole.

### Example 36: Preparation of 5-amino-4-ethylindole

Addition of EtMgBr to 5-nitroindole followed by hydrogenation over Pd/C provided 5-amino-4-ethylindole.

### Example 37: Preparation of 5-amino-7-chloro-4-methylindole

5-Chloro-2-methyl-4-nitroaniline was treated with acetic anhydride in the presence of 4-dimethylaminopyridine (DMAP) in CH₂Cl₂ to provide N-(5-chloro-2-methyl-4-nitrophenyl)acetamide, which was reacted with vinyl magnesium bromide at -40 °C to room temperature to give N-(7-chloro-4-methyl-1H-indol-5-yl)acetamide. Deprotection of the acetyl group by heating in an aqueous HCl solution at the reflux temperature provided 5-amino-7-chloro-4-methylindole.

### Example 38: Preparation of diisopropyl 2-chloro-4-(2-(4-methylpiperazin-1-yl)ethoxy)phenylboronate

4-Bromo-3-chlorophenol was reacted with 1-methyl-4-(2-hydroxyethyl)piperazine under the Mitsunobu conditions to provide 1-[2-(4-bromo-3-chlorophenoxy)ethyl]-4-methyl-piperazine.

1-[2-(4-Bromo-3-chlorophenoxy)ethyl]-4-methyl-piperazine was reacted with n-BuLi at 0 °C in the presence of triisopropyl borate to give diisopropyl 2-chloro-4-(2-(4-methylpiperazin-1-yl)ethoxy)phenylboronate upon warming to room temperature.

### Example 39: Preparation of tributyl (5-(2-chloroethoxy)benzofuran-2-yl) stannane

5-Methoxybenzofuran was treated with 2,4,5-collidine and LiI at 170 °C to provide benzofuran-5-ol, which was reacted with 2-chloroethyl-p-toluenesulfonate and Cs₂CO₃ to give 5-(2-chloroethoxy)-1-benzofuran.

Treatment of 5-(2-chloroethoxy)-1-benzofuran with n-BuLi for 16 h followed by the addition of tributyltin chloride at -50 °C gave tributyl (5-(2-chloroethoxy)benzofuran-2-yl) stannane upon warming to room temperature.

### Example 40: Preparation of diisopropyl 4-(3-chloropropoxy)phenylboronate

4-Bromophenol was reacted with 2-chloropropyl-p-toluenesulfonate and Cs₂CO₃ to give 1-bromo-4-(3-chloropropoxy)benzene.

Treatment of 1-bromo-4-(3-chloropropoxy)benzene with n-BuLi at 0 °C in the presence of triisopropyl borate gave diisopropyl 4-(3-chloropropoxy)phenylboronate upon warming to room temperature.

### Example 41: Preparation of diisopropyl 4-(2-chloroethoxy)-3-methoxyphenylboronate

4-Bromo-2-methoxyphenol was reacted with 2-chloroethyl-p-toluenesulfonate and Cs₂CO₃ at 50 °C for 4 h to give 1-bromo-4-(2-chloroethoxy)-3-methoxybenzene.

Treatment of 1-bromo-4-(2-chloroethoxy)-3-methoxybenzene with n-BuLi at 0 °C in the presence of triisopropyl borate gave diisopropyl 4-(2-chloroethoxy)-3-methoxyphenylboronate upon warming to room temperature.

### Example 42: Preparation of 4-((4-(tributylstannyl)pyridin-2-yl)methyl)morpholine

4-Chloro-2-picolene (10.00 g, 78.74 mmol) in 2-butanone (100 ml) was treated with sodium iodide (50.00 g, 335.82 mmol) and hydriodic acid (12 ml, 57%) and the resulting mixture was heated at the reflux temperature for 18 h. The reaction was cooled to room temperature and the solvent was removed under reduced pressure. The residue was partitioned between ethyl acetate and saturated aqueous sodium bicarbonate. The organic layer was dried over MgSO₄ and concentrated to give a dark brown syrup, which was purified by column chromatography (25% ethyl acetate in hexanes) to give 4-iodo-2-picolene as an orange syrup which later crystallized to a red solid (12.3 g, 71% yield).

4-Iodo-2-picolene (10.00 g, 45.66 mmol) in dry carbon tetrachloride (200 ml) was treated with NBS (9.75 g, 54.78 mmol) and benzoyl peroxide (550 mg, 2.27 mmol) under nitrogen and the resulting mixture was heated at reflux for 24 h. After cooled to room temperature, the reaction mixture was filtered and the solid was washed with dichloromethane, which was combined with the filtrate. The combined organic solution was concentrated to give the crude product, which was purified by column chromatography (20% ethyl acetate in hexanes) to give 2-(bromomethyl)-4-iodopyridine as a yellow solid (4.00 g).

2-(Bromomethyl)-4-iodopyridine (500 mg, 1.68 mmol) in 1,2-dimethoxyethane (15 ml) was treated with morpholine (1.00 g, 11.48 mmol) and sodium iodide (13 mg, 0.09 mmol) and the resulting solution was heated at 90 °C for 5 h. After cooled to room temperature, the reaction mixture was partitioned between dichloromethane and saturated aqueous sodium bicarbonate. The organic layer was dried over MgSO₄ and concentrated to give 4-[(4-iodo-2-pyridinyl)methyl]morpholine as a brown solid (315 mg, 62% yield).

Treatment of 4-[(4-iodo-2-pyridinyl)methyl]morpholine with n-BuLi at 0°C and tributyltin chloride provided 4-((4-(tributylstannyl)pyridin-2-yl)methyl)morpholine.

### Example 43: Preparation of 2-(3,4-bis(2-methoxyethoxy)phenyl)-4,4,5,5-tetramethyl-1,3,2-dioxaborolane

### Step 1: Preparation of 1,2-bis(2-methoxyethoxy)benzene

Ortho-catechol (4.75 g, 43 mmol), 2-bromo-1-methoxyethane (15 g, 108 mmol), and potassium carbonate (18 g, 129 mmol) were stirred in 200 mL DMF at 80 °C overnight. The suspension was partitioned between water and EtOAc. The organic layer was washed with water 4 times, dried over magnesium sulfate, and concentrated to give 9.9 g of 1,2-bis(2-methoxyethoxy)benzene.

### Step 2: Preparation of 4-bromo-1,2-bis(2-methoxyethoxy)benzene

1,2-Bis(2-methoxyethoxy)benzene (8.1 g, 31.9 mmol) and N-bromosuccinimide (6.2 g, 35 mmol) were stirred in 50 mL DMF at room temperature overnight. The reaction was diluted with 200 mL EtOAc and washed with 1N NaOH and water. The organic layer was dried over magnesium sulfate and concentrated to give 9.7 g (92%) of 4-bromo-1,2-bis(2-methoxyethoxy)benzene.

### Step 3: 2-(3,4-bis(2-methoxyethoxy)phenyl)-4,4,5,5-tetramethyl-1,3,2-dioxaborolane

4-Bromo-1,2-bis(2-methoxyethoxy)benzene (4 g, 12 mmol), bis(pinacolato)diboron (3.5 g, 13.8 mmol), KOAc (3.5 g, 36 mmol), and Pd(dppf)₂Cl₂ (490 mg, 0.6 mmol) were mixed in 50 mL DMSO and the resulting suspension was heated at 80 °C overnight. The reaction was diluted with EtOAc and washed 4 times with water. The organic layer was concentrated to provide the crude product, which was purified by silica gel chromatography (EtOAc/Hex) to give 4.8 g of 2-(3,4-bis(2-methoxyethoxy)phenyl)-4,4,5,5-tetramethyl-1,3,2-dioxaborolane.

### Example 44: Preparation of 2-[4-(2-methoxyethoxy)phenyl]4,4,5,5-tetramethyl-1,3,2-dioxaborolane

To a solution of 4-(4,4,5,5-tetramethyl-1,3,2-dioxaboralan-2-yl)phenol (1 g, 4.5 mmol), 2-methoxyethanol (450 mg , 5.9 mmol), and triphenylphosphine (1.6 g, 5.9 mmol) in THF (35 mL) was added dropwise diethyl azodicarboxylate (2.7 ml, 5.9 mmol, 40% in toluene) at 0-5 °C. The resulting mixture was stirred at room temperature for 18 h and partitioned between ethyl acetate and water. The organic phase was dried over Na₂SO₄, filtered, and concentrated in vacuo. The residue was purified by flash column chromatography (ethyl acetate /hexane, 5% to 20%) to provide 989 mg (79%) of 2-[4-(2-methoxyethoxy)phenyl]-4,4,5,5-tetramethyl-1,3,2-dioxaborolane as a white solid.

Following procedures analogous to those described above, the following boronic esters were prepared: 2-[3-(2-methoxyethoxy)phenyl]-4,4,5,5-tetramethyl-1,3,2-dioxaborolane, 2-[4-(4-chlorobutoxy)phenyl]-4,4,5,5-tetramethyl-1,3,2-dioxaborolane, and 2-[3-(4-chlorobutoxy)phenyl]-4,4,5,5-tetramethyl-1,3,2-dioxaborolane.

### Example 45: Preparation of 1-benzofuran-5-carbaldehyde

To a solution of 1-benzofuran-5-carbonitrile (5.0 g, 34.9 mmol) in CH₂Cl₂ under nitrogen at -15 to -20 °C was added DIBAL-H (41.9 mL, 41.9 mmol, 1M/heptane), maintaining the reaction temperature below -15 °C, and the reaction mixture was stirred at -15 to -20 °C for additional 10 min. The reaction mixture was then quenched via dropwise addition of aqueous 2N HCl, maintaining the temperature below room temperature. The organic layer was separated, washed with water, dried over sodium sulfate, and concentrated to give 4.0 g (78%) of 1-benzofuran-5-carbaldehyde as a yellow oil.

### Example 46: Preparation of 2-(tributylstannyl)-1-benzofuran-5-carbaldehyde

To a solution of N-methylpiperazine (0.75 g, 7.5 mmol) in hexane (15 mL) at 0 °C under nitrogen was added dropwise a solution of n-BuLi (3 mL, 7.43 mmol, 2.5M/hexanes), and the reaction mixture was stirred at 0 °C for 40 min. 1-Benzofuran-5-carbaldehyde (1.0 g, 6.8 mmol) was added dropwise to the reaction mixture at 0 °C and the resulting mixture was stirred at 0 °C.for 15 min. After addition of tetramethylethylenediamine (TMEDA) (1.7 g, 14.96 mmol), a solution of n-BuLi (6.0 mL, 14.86 mmol, 2.5M/hexanes) was added dropwise to the reaction mixture at 0°C and the reaction mixture was allowed to warm up to room temperature and stirred for a total of 18 h. After the reaction mixture was cooled to 0 °C and THF (30 mL) was added, the reaction mixture was cooled to -50 °C, tributyltin chloride (4.87 g, 14.96 mmol) was added dropwise, and the reaction mixture was stirred at -50 °C for 15 min. and at room temperature for 5-6 h. The reaction mixture was quenched with saturated aqueous NaHCO₃ and extracted into diethyl ether. The organic layer was dried over sodium sulfate and concentrated and the crude product was purified by column chromatography (ethyl acetate/hexane, 2 %) to give 1.0 g (34%) of 2-(tributylstannyl)-1-benzofuran-5-carbaldehyde as a yellow oil.

### Example 47: Preparation of dimethyl 5-(piperidin-1-ylmethyl)benzofuran-2-ylboronate

1-Benzofuran-5-carbaldehyde was treated with piperidine and sodium triacetoxyborohydride under the standard reductive amination protocol to provide 1-(5-benzofuranylmethyl)piperidine.

Treatment of 1-(5-benzofuranylmethyl)piperidine with butyl lithium and trimethylborate at low temperature provided dimethyl 5-(piperidin-1-ylmethyl)benzofuran-2-ylboronate.

### Example 48: Preparation of 5-(5-formyl-1-benzofuran-2-yl)-4-[(4-methyl-1H-indol-5-yl)amino]nicotinonitrile 465

A mixture of 5-iodo-4[(4-methyl-1H-indol-5-yl)amino]nicotinonitrile (2.5 g, 6.7 mmol), 2-(tributylstannyl)-1-benzofuran-5-carbaldehyde (4.35 g, 10 mmol), and tetrakis(triphenylphosphine)palladium(0) (0.38 g, 0.34 mmol) in DMF (25 mL) was heated at 120 °C for 1 h. The reaction mixture was cooled to room temperature and partitioned between ethyl acetate arid water, resulting in a suspension. The insoluble material was removed by filtration and the residue was washed thoroughly with ethyl acetate. The organic layer (from filtrate) was washed with water twice and brine, dried over sodium sulfate, and concentrated and the residue was purified by column chromatography (ethyl acetate/hexane, 1:1) to give 2.0 g (76%) of 5-(5-formyl-1-benzofuran-2-yl)-4-[(4-methyl-1H-indol-5-yl)amino]nicotinonitrile as a yellow solid. HPLC retention time: 10.7 min. ^{(h)}; MS: 393.2 (M + H); melting point: 235-237 °C; and HRMS: 393.13484.

Following procedures analogous to those described for the preparation of 5-(5-formyl-1-benzofuran-2-yl)-4-[(4-methyl-1H-indol-5-yl)amino]nicotinonitrile 465, compounds in Table 15 were prepared.

**Table 15**

| **Compound** | **Compound Name** | **HPLC retention time (min.)** | **Observed Ion m/e [M+H]** |
|---|---|---|---|
| 484 | 5-(5-formyl-1-benzofuran-2-yl)-4-(1*H*-indol-5-ylamino)nicotinonitrile | 10.5^{(g)} | 379.2 |
| 495 | 5-(5-formyl-1-benzofuran-2-yl)-4-[(6-methyl-1*H*-indol-5-yl)amino]nicotinonitrile | N/A | 393.2 |

### Example 49: Preparation of 4-[(4-methyl-1H-indol-5-yl)amino]-5-{5-[(4-methylpiperazin-1-yl)methyl]-1-benzofuran-2-yl}nicotinonitrile 462

To a solution of 5-(5-formyl-1-benzofuran-2-yl)-4-[(4-methyl-1H-indol-5-yl)amino]nicotinonitrile (800 mg, 2.04 mmol) in a mixture of CH₂Cl₂ (40 mL) and NMP (5 mL) at room temperature under nitrogen was added N-methylpiperazine (613 mg, 6.12 mmol) followed by glacial acetic acid (674 mg, 11.22 mmol) and the reaction mixture was stirred at room temperature for 1 h. Sodium triacetoxyborohydride (2.38 g, 11.22 mmol) was added to the reaction mixture and the reaction was stirred at room temperature for 5 h. The reaction mixture was partitioned between CH₂Cl₂ and aqueous 1N HCl. The aqueous layer was washed with CH₂Cl₂ and treated with aqueous 1N NaOH whereupon solids precipitated. The solids were collected and dissolved in CH₂Cl₂. The solution was washed with water thrice and brine, dried over sodium sulfate, and concentrated and the residue was purified by column chromatography (MeOH/CH₂Cl₂, 6% to 7.5%) to give 560 mg (58%) of 4-[(4-methyl-1H-indol-5-yl)amino]-5-{5-[(4-methylpiperazin-1-yl)methyl]-1-benzofuran-2-yl}nicotinonitrile as a yellow solid. HPLC retention time: 5.9 min. ^{(g)}; melting range: 162-165 °C (decom.); and HRMS: 477.24093.

### Example 50: Preparation of 5-(5-{[4-(hydroxymethyl)piperidin-1-yl]methyl}-1-benzofuran-2-yl)-4-[(4-methyl-1H-indol-5-yl)amino]nicotinonitrile 392 and 5-[5-(hydroxymethyl)-1-benzofuran-2-yl]-4-[(4-methyl-1H-indol-5-yl)amino]nicotinonitrile 481

An alternative procedure for preparing 5-(5-{[4-(hydroxymethyl)piperidin-1-yl]methyl}-1-benzofuran-2-yl)-4-[(4-methyl-1H-indol-5-yl)amino]nicotinonitrile **392** is described below (compare Example 27).

To a suspension of 5-(5-formyl-1-benzofuran-2-yl)-4-[(4-methyl-1H-indol-5-yl)amino]nicotinonitrile (200 mg, 0.51 mmol) in a mixture of CH₂Cl₂ (10 mL) and NMP (1 mL) at room temperature under nitrogen was added 4-(hydroxymethyl)piperidine (176 mg, 1.53 mmol) followed by glacial acetic acid (153 mg, 2.55 mmol) and the reaction mixture was stirred at room temperature for 0.5 h. Sodium triacetoxyborohydride (540 mg, 2.55 mmol) was added in portions at 0 °C and the resulting mixture was stirred at room temperature overnight. The reaction mixture was partitioned between CH₂Cl₂ and saturated aqueous sodium bicarbonate, and the aqueous layer was extracted with CH₂Cl₂. The combined organic phases were washed with water and brine, dried over sodium sulfate, filtered, and concentrated in vacuo. The residue was purified by flash column chromatography (MeOH/CH₂Cl₂, 3% to 20%) to give 128 mg (51%) of 5-(5-{[4-(hydroxymethyl)piperidin-1-yl]methyl}-1-benzofuran-2-yl)-4-[(4-methyl-1H-indol-5-yl)amino]nicotinonitrile **392** as a yellow solid and 23.8 mg of 5-[5-(hydroxymethyl)-1-benzofuran-2-yl]-4-[(4-methyl-1H-indol-5-yl)amino]nicotinonitrile **481** as a yellow solid. Compound 392: HPLC retention time: 6.4 min. ^{(g)}; melting range: 196-199 °C; and HRMS: 395.15023. Compound **481**: HPLC retention time: 8.9 min. ^{(g)}; MS: 395.3 (M + H); melting range: 232-234 °C; and HRMS: 395.15023.

Following procedures analogous to those described for the preparation of 5-(5-{[4-(hydroxymethyl)piperidin-1-yl]methyl}-1-benzofuran-2-yl)-4-[(4-methyl-1H-indol-5-yl)amino]nicotinonitrile **392** and 5-[5-(hydroxymethyl)-1-benzofuran-2-yl]-4-[(4-methyl-1H-indol-5-yl)amino]nicotinonitrile **481**, the compounds in Table 16 were prepared.

**Table 16**

| **Compd.** | **Compound Name** | **Melting range (°C)** | **HPLC retention time (min.)** | **Observed ion m/e [M+H]** | **Observed HRMS [M+H]** |
|---|---|---|---|---|---|
| 390 | 4-[(4-methyl-1*H*-indol-5-yl)amino]-5-(5-{[(2-pyrrolidin-1-ylethyl)amino]methyl}-1-benzofuran-2-yl)nicotinonitrile | 125 (decom.) | 5.3 ^{(g)} | 491.4 | 491.25451 |
| 485 | 4-(1H-indol-5-ylamino)-5-{5-[(4-methylpiperazin-1-yl)methyl]-1-benzofuran-2-yl}nicotinonitrile | 192-194 | 5.8 ^{(g)} | 463.4 | 463.22532 |
| 497 | 4-[(6-methyl-1H-indol-5-yl)amino]-5-{5-[(4-methylpiperazin-1-yl)methyl]-1-benzofuran-2-yl}nicotinonitrile | 202.4-205.2 | 6.0 ^{(g)} | 477.4 | 477.2395 |
| 499 | 5-[5-(hydroxymethyl)-1-benzofuran-2-yl]-4-[(6-methyl-1*H*-indol-5-yl)amino]nicotinonitrile | N/A | 9.0 ^{(g)} | 395.3 | 395.14956 |
| 500 | 5-{5-[(diethylamino)methyl]-1-benzofuran-2-yl}-4-[(6-methyl-1H-indol-5-yl)amino]nicotinonitrile | N/A | 6.9 ^{(g)} | 450.3 | 450.2296 |
| 518 | 4-[(4-methyl-1H-indol-5-yl)amino]-5-{5-[(4-methylpiperazin-1-yl)methyl]-3-thienyl}nicotinonitrile | 185-187 | N/A | 443.4 | 443.20262 |
| 536 | *tert*-butyl 4-[(2-{5-cyano-4-[(4-methyl-1*H*-indol-5-yl)amino]pyridin-3-yl}-1-benzofuran-5-yl)methyl]piperazine-1-carboxylate | 183-185 | N/A | . 563.4 | 563.2768 |
| 538 | 4-[(4-methyl-1*H*-indol-5-yl)amino]-5-(5-{[4-(methylsulfonyl)piperazin-1-yl]methyl}-1-benzofuran-2-yl)nicotinonitrile | 239-241 | N/A | 541.3 | 541.2011 |
| 539 | 4-[(4-methyl-1*H*-indol-5-yl)amino]-5-{1-methyl-2-[(4-methylpiperazin-1-yl)methyl]-1*H*-imidazol-5-yl}nicotinonitrile | 165-168 | 3.4 ^{(g)} | 441.3 | 441.2510 |
| 552 | 5-{2-methoxy-5-[(4-methylpiperazin-1-yl)methyl]phenyl}-4-[(4-methyl-1*H*-indol-5-yl)amino]nicotinonitrile | N/A | 4.8 ^{(g)} | 467.4 | N/A |
| 553 | 5-{5-[(4-ethylpiperazin-1-yl)methyl]-1-benzofuran-2-yl}-4-[(4-methyl-1H-indol-5-yl)amino]nicotinonitrile | 129-132 | 7.8 ^{(j)} | N/A | N/A |
| 554 | 4-[(4-methyl-1H-indol-5-yl)amino]-5-{5-[(4-methyl-4-oxidopiperazin-1-yl)methyl]-1-benzofuran-2-yl}nicotinonitrile | 120 (decom.) | 6.7 ^{(g)} | 493.5 | N/A |

### Example 51: Preparation of the trifluoroacetic salt of 4-[(4-methyl-1H-indol-5-yl)amino]-5-[5-(piperazin-1-ylmethyl)-1-benzofuran-2-yl]nicotinonitrile 540

A mixture of tert-butyl 4-[(2-{5-cyano-4-[(4-methyl-1H-indol-5-yl)amino]pyridin-3-yl}-1-benzofuran-5-yl)methyl]piperazine-1-carboxylate (63 mg, 0.11 mmol) in 10% trifluoroacetic acid/CH₂Cl₂ was stirred at room temperature for 4 h, concentrated in vacuo, and the residue was purified by preparative HPLC (column: Prodigy ODS3, 4.6 x 150 mm, from Phenomenex (Torrance, CA); mobile phase A: 0.02% trifluoroacetic acid (TFA) in water; mobile phase B: 0.02% TFA in CH₃CN, 10-95% B in 25 minutes (min.); flow rate: 1.0 mL/min; column temperature: 40°C; detection wavelength: 254 and 215 nm.) to provide 46 mg (50%) of the trifluoroacetic salt of 4-[(4-methyl-1H-indol-5-yl)amino]-5-[5-(piperazin-1-ylmethyl)-1-benzofuran-2-yl]nicotinonitrile **540** as an orange solid. HPLC retention time: 5.8 min. ^{(g)}; MS: 463.3 (M + H); melting point: 113-115 °C; and HRMS: 463.2234.

### Example 52: Preparation of 5-(3,4-dimethoxyphenyl)-4-[(4-methyl-1H-indol-7-yl)amino]nicotinonitrile 530

A solution of 5-(3,4-dimethoxyphenyl)-4-chloronicotinonitrile (120 mg, 0.44 mmol) and 4-methyl-7-aminoindole (77.8 mg, 0.52 mmol) in ethanol (2.5 mL) was heated at the reflux temperature for 24 h and cooled to room temperature. The reaction mixture was treated with aqueous NH₄OH and concentrated in vacuo, and the residue was purified by flash column chromatography (ethyl acetate/CH₂Cl₂ 5% to 25%) to provide a 48 mg (29%) of 5-(3,4-dimethoxyphenyl)-4-[(4-methyl-1H-indol-7-yl)amino]nicotinonitrile **530** as an off white solid. HPLC retention time: 9.0 min. ^{(g)}; MS: 385.3 (M + H); and HRMS: 385.1659.

Following procedures analogous to those described for the preparation of 5-(3,4-dimethoxyphenyl)-4-[(4-methyl-1H-indol-7-yl)amino]nicotinonitrile **530**, 5-(3,4-dimethoxyphenyl)-4-(1H-indol-7-ylamino)nicotinonitrile **544** was prepared. HPLC retention time: 8.6 min. ^{(g)}; HRMS: 371.1498; melting range: 205-207 °C.

### Example 53: Preparation of 5-(2-formyl-1-methyl-1H-imidazol-5-yl)-4-[(4-methyl-1H-indol-5-yl)amino]nicotinonitrile 537

A mixture of 5-iodo-4[(4-methyl-1H-indol-5-yl)amino]nicotinonitrile (250 mg, 0.67 mmol), 1-methyl-5-(tributylstannyl)-1H-imidazole-2-carbaldehyde (see e.g., U.S. Patent No. 6,521,618) (401 mg, 1.0 mmol), dichlorobis(triphenylphosphine)palladium(II) (24 mg, 0.034 mmol), and triethylamine (74.9 mg, 0.74 mmol) in dioxane (6 mL) was heated at the reflux temperature for 22 h, cooled to room temperature, and concentrated in vacuo. The residue was suspended in CH₂Cl₂ and filtered and the solid was washed with CH₂Cl₂. The combined filtrate and washings were concentrated in vacuo and the residue was purified by column chromatography (ethyl acetate /CH₂Cl₂, 20% to 90%; MeOH/ ethyl acetate, 5%) to provide 103 mg (43%) of 5-(2-formyl-1-methyl-1H-imidazol-5-yl)-4-[(4-methyl-1H-indol-5-yl)amino]nicotinonitrile **537** as a dark yellow solid. MS: 355.2 (M + H); melting range: 200-202 °C; and HRMS: 357.1458.

Following procedures analogous to those described for the preparation of 5-(2-formyl-1-methyl-1H-imidazol-5-yl)-4-[(4-methyl-1H-indol-5-yl)amino]nicotinonitrile **537**, compounds in Table 17 were prepared.

**Table 17**

| **Compound** | **Compound Name** | **Melting range (°C)** | **HPLC retention time (min.)** | **Observed ion m/e [M + H]** | **Observed HRMS [M + H]** |
|---|---|---|---|---|---|
| 542 | 5-(1-methyl-1*H-*imidazol-2-yl)-4-[(4-methyl-1*H*-indol-5-yl)amino]nicotinonitrile | 239-241 | 4.2 ^{(g)} | 329.2 | 329.1510; |
| 564 | 5-(1-methyl-1H-imidazol-5-yl)-4-[(4-methyl-1H-indol-5-yl)amino]nicotinonitrile | N/A | 4.4 ^{(g)} | 329.3 | N/A |
| 565 | 4'-[(4-methyl-1H-indol-5-yl)amino]-2,3'-bipyridine-5'-carbonitrile | N/A | 8.1 ^{(g)} | 326.3 | N/A |

### Example 54: Preparation of 4-[(4-methyl-1H-indol-5-yl)amino]-5-(1,3-thiazol-2-yl)nicotinonitrile 541

A mixture of 5-iodo-4[(4-methyl-1H-indol-5-yl)amino]nicotinonitrile (120 mg, 0.32 mmol), 2-(tributylstannyl)-1,3-thiazole (340 mg, 0.91 mmol), dichlorobis(triphenylphosphine)palladium(II) (28 mg, 0.04 mmol), and triethylamine (36 mg, 0.35 mmol) in dioxane (4 mL) was heated at reflux for 24 h. 4-[(4-Methyl-1H-indol-5-yl)amino]-5-(1,3-thiazol-2-yl)nicotinonitrile was obtained following the work-up and purification procedures as described for the preparation of 5-(2-formyl-1-methyl-1H-imidazol-5-yl)-4-[(4-methyl-1H-indol-5-yl)amino]nicotinonitrile 541. HPLC retention time: 10.1 min. ^{(g)}; MS: 332.2 (M + H); melting range: 245-247 °C; and HRMS: 332.0964.

Following procedures analogous to those described for the preparation of 5-(2-formyl-1-methyl-1H-imidazol-5-yl)-4-[(4-methyl-1H-indol-5-yl)amino]nicotinonitrile **541**, 4-[(4-methyl-1H-indol-5-yl)amino]-5-(1,3-thiazol-4-yl)nicotinonitrile **543** was prepared. HPLC retention time: 7.7 min. ^{(g)}; MS: 332.2 (M + H); melting range: 245-247 °C; and HRMS: 332.0966.

### Example 55: Preparation of 5-[4-(2-methoxyethoxy)phenyl]-4-[(4-methyl-1H-indol-5-yl)amino]nicotinonitrile 482

A mixture of 5-iodo-4[(4-methyl-1H-indol-5-yl)amino]nicotinonitrile (100 mg, 0.27 mmol), 2-[4-(2-methoxyethoxy)phenyl]-4,4,5,5-tetramethyl-1,3,2-dioxaborolane (98 mg, 0.35 mmol), and tetrakis(triphenylphosphine)palladium(0) (16 mg, 0.014 mmol) in DME (6 mL) and saturated aqueous NaHCO₃ (4 mL) was heated at 95 °C for 3 h, cooled to room temperature, and treated with water. The precipitate was filtered, washed with water, dried in vacuo, and purified by flash column chromatography (MeOH/ CH₂Cl₂, 2% to 4%) to provide 75 mg (70%) of 5-[4-(2-methoxyethoxy)phenyl]-4-[(4-methyl-1H-indol-5-yl)amino]nicotinonitrile as a tan solid. HPLC retention time: 8.2 min. ^{(g)}; MS: 399.3 (M + H); melting range: 208-210 °C; and HRMS: 399.18056.

### Example 56: Preparation of 5-[3-(2-methoxyethoxy)phenyl]-4-[(4-methyl-1H-indol-5-yl)amino]nicotinonitrile 483

A mixture of 5-iodo-4[(4-methyl-1H-indol-5-yl)amino]nicotinonitrile (100 mg, 0.27 mmol), 2-[3-(2-methoxyethoxy)phenyl]-4,4,5,5-tetramethyl-1,3,2-dioxaborolane (98 mg, 0.35 mmol) and tetrakis(triphenylphosphine)palladium(0) (16 mg, 0.014 mmol) in DME (6 mL) and saturated aqueous NaHCO₃ (4 mL) was heated at 95 °C for 2 h, cooled to room temperature and partitioned between dichloromethane and water. The organic phase was dried over Na₂SO₄, filtered, and concentrated in vacuo, and the residue was purified by flash column chromatography (MeOH/ CH₂Cl₂, 1% to 5%) to provide 71 mg (66%) of 5-[3-(2-methoxyethoxy)phenyl]-4-[(4-methyl-1H-indol-5-yl)amino]nicotinonitrile as a tan solid. HPLC retention time: 8.3 min. ^{(g)}; MS: 399.3 (M + H); melting range: 158-160 °C; and HRMS: 399.18291.

Following procedures analogous to those described for the preparation of 5-[3-(2-methoxyethoxy)phenyl]-4-[(4-methyl-1H-indol-5-yl)amino]nicotinonitrile **483**, 5-[4-(4-chlorobutoxy)phenyl]-4-[(4-methyl-1H-indol-5-yl)amino]nicotinonitrile **490** and 5-[3-(4-chlorobutoxy)phenyl]-4-[(4-methyl-1H-indol-5-yl)amino]nicotinonitrile **501** were prepared. Compound **490**: HPLC retention time: 11.1 min. ^{(g)}; MS: 431.3 (M+H); melting point: 157-159 °C; and HRMS: 431.16279; and compound **501**: HPLC retention time: 11.2 min. ^{(g)} ; MS: 431.3 (M+H); melting point: 151-153 °C; and HRMS: 431.16345.

### Example 57: Preparation of 4-[(4-methyl-1H-indol-5-yl)amino]-5-{5-[(4-methylpiperazin-1-yl)methyl]-3-furyl}nicotinonitrile 503

To a solution of 1-[4-bromo-2-furyl)methyl]-4-methylpiperazine (see *e.g.*, J. Med Chem., 49, 7868, (2006)) (145 mg, 0.56 mmol), and triisopropylborate (132 mg, 0.16 mL, 0.7 mmol) in THF was added dropwise n-BuLi (0.3 ml, 0.76 mmol, 2.5M/hexane) at -78 °C and the reaction mixture was stirred at -78 °C for 3 h and warmed to room temperature in 1 h. A few drops of water were added and the mixture was concentrated in vacuo. To the residue was added 5-iodo-4[(4-methyl-1H-indol-5-yl)amino]nicotinonitrile (100 mg, 0.27 mmol), [1,1'-bis(diphenylphosphino)ferrocene]dichloropalladium(II)-CH₂Cl₂ ((dppf)₂PdCl₂, 12 mg, 0.014 mmol), DME (6 mL) and saturated aqueous Na₂CO₃ (4 mL) and the resulting mixture was heated at 85 °C for 1.5 h and cooled to room temperature. The mixture was partitioned between dichloromethane and water, and the organic phase was dried over Na₂SO₄, filtered and concentrated in vacuo. The residue was purified by flash column chromatography (MeOH/ CH₂Cl₂, 5% to 20%) to provide 68 mg (57%) of 4-[(4-methyl-1H-indol-5-yl)amino]-5-{5-[(4-methylpiperazin-1-yl)methyl]-3-furyl}nicotinonitrile as a light tan solid. HPLC retention time: 4.8 min. ^{(g)}; Melting point: 129 °C (decom.); and HRMS: 427.22597.

### Example 58: Preparation of 4-[(7-chloro-4-methyl-1H-indol-5-yl)amino]-5-(5-formyl-1-benzofuran-2-yl)nicotinonitrile 532

To a stirred solution of 4-[(7-chloro-4-methyl-1H-indol-5-yl)amino]-5-iodonicotinonitrile (405 mg, 1 mmol) in DMF (7 mL) was added 2-(tributylstannyl)-1-benzofuran-5-carbaldehyde (609 mg, 1.4 mmol) and tetrakis(triphenylphosphine)palladium(0) (110 mg, 0.1 mmol) under nitrogen atmosphere, the reaction mixture was heated at 110 °C for 1.5 h and cooled to room temperature and ethyl acetate was added to the resulting mixture. The organic phase was washed with water, saturated aqueous sodium chloride, dried over magnesium sulfate and concentrated and the residue was purified by flash column chromatography (ethyl acetate/hexane) to give 4-[(7-chloro-4-methyl-1H-indol-5-yl)amino]-5-(5-formyl-1-benzofuran-2-yl)nicotinonitrile as a yellow solid (351 mg, 82%). HPLC retention time: 12.4 min. ^{(g)}; and MS: 427.3 (M + H).

### Example 59: Preparation of 4-[(7-chloro-4-methyl-1H-indol-5-yl)amino]-5-{5-[(4-methylpiperazin-1-yl)methyl]-1-benzofuran-2-yl}nicotinonitrile 529

To a stirred solution of 4-[(7-chloro-4-methyl-1H-indol-5-yl)amino]-5-(5-formyl-1-benzofuran-2-yl)nicotinonitrile (106 mg, 0.25 mmol) in THF (3 mL) and EtOH (1 mL) was added 1-methylpiperazine (75 mg, 0.75 mmol) and acetic acid (75 mg, 1.2 mmol) and the reaction mixture was stirred at room temperature for 1 h. Sodium triacetoxyborohydride (159 mg, 0.75 mmol) was added to the reaction mixture and the solution was stirred at room temperature overnight, and concentrated. Aqueous 1N HCl was added and the aqueous phase was extracted with ethyl acetate, basified to pH of 10 by adding sodium carbonate, and extracted with ethyl acetate. The combined organic phases were washed with water and brine, dried over magnesium sulfate, and concentrated to give 4-[(7-chloro-4-methyl-1H-indol-5-yl)amino]-5-{5-[(4-methylpiperazin-1-yl)methyl]-1-benzofuran-2-yl}nicotinonitrile as a yellow solid (68 mg, 53%). HPLC retention time: 7.0 min. ^{(g)}; and MS: 511.5 (M+H).

### Example 60: Preparation of tert-butyl 4-[(2-{4-[(7-chloro-4-methyl-1H-indol-5-yl)amino]-5-cyanopyridin-3-yl}-1-benzofuran-5-yl)methyl]piperazine-1-carboxylate 548

To a stirred solution of 4-[(7-chloro-4-methyl-1H-indol-5-yl)amino]-5-(5-formyl-1-benzofuran-2-yl)nicotinonitrile (200 mg, 0.47 mmol) in THF (5 mL) and EtOH (2 mL) was added *tert*-butyl 1-piperazinecarboxylate (262 mg, 1.41 mmol) and acetic acid (54 mg, 0.9 mmol) and the reaction mixture was stirred at room temperature for 1 h. Sodium triacetoxyborohydride (398 mg, 1.88 mmol) was added to the reaction mixture and the resulting solution was stirred at room temperature overnight. After the reaction was concentrated, ethyl acetate was added; and the organic phase was washed with saturated aqueous sodium bicarbonate solution and saturated aqueous sodium chloride solution, dried over magnesium sulfate, and concentrated. The residue was purified by flash column chromatography (ethyl acetate/hexane) to give tert-butyl 4-[(2-{4-[(7-chloro-4-methyl-1H-indol-5-yl)amino]-5-cyanopyridin-3-yl}-1-benzofuran-5-yl)methyl]piperazine-1-carboxylate as a yellow solid (203 mg, 73%). HPLC retention time: 9.5 min. ^{(g)}; MS: 597.4 (M + H); and HRMS: 597.2378.

### Example 61: Preparation of 4-[(7-chloro-4-methyl-1H-indol-5-yl)aminol-5-[5-(piperazin-1-ylmethyl)-1-benzofuran-2-yl]nicotinonitrile 547

A solution of *tert*-butyl 4-[(2-{4-[(7-chloro-4-methyl-1H-indol-5-yl)amino]-5-cyanopyridin-3-yl}-1-benzofuran-5-yl)methyl]piperazine-1-carboxylate (176 mg, 0.29 mmol) in CH₂Cl₂ (4 mL) and trifluoroacetic acid (2 mL) was stirred at room temperature for 4 h and concentrated to provide 4-[(7-chloro-4-methyl-1H-indol-5-yl)amino]-5-[5-(piperazin-1-ylmethyl)-1-benzofuran-2-yl]nicotinonitrile as a yellow solid in quantitative yield (182 mg). HPLC retention time: 6.5 min. ^{(g)}; MS: 497.3 (M + H); and HRMS: 497.1851.

### Example 62: Preparation of 5-(1-benzofuran-2-yl)-4-[(7-chloro-4-methyl-1H-indol-5-yl)amino]nicotinonitrile 531

To a stirred solution of 4-[(7-chloro-4-methyl-1H-indol-5-yl)amino]-5-iodonicotinonitrile (122 mg, 0.3 mmol) in DMF (4 mL) was added 2-benzofuranboronic acid (162 mg, 0.4 mmol) and tetrakis(triphenylphosphine)palladium(0) (34 mg, 0.03 mmol) under nitrogen atmosphere and the reaction mixture was heated at 60 °C overnight. After the solution was cooled to room temperature, ethyl acetate was added, and the organic phase was washed with water and saturated aqueous sodium chloride, dried over magnesium sulfate and concentrated. The residue was purified by flash column chromatography (ethyl acetate/hexane) to give 5-(1-benzofuran-2-yl)-4-[(7-chloro-4-methyl-1H-indol-5-yl)amino]nicotinonitrile (18 mg, 15%). HPLC retention time: 13.4 min. ^{(g)}; MS: 399.3 (M + H); and HRMS: 399.1009.

### Example 63: Preparation of 5-[4-(2-chloroethoxy)phenyl]-4-[(7-chloro-4-methyl-1H-indol-5-yl)amino]nicotinonitrile 533

To a stirred solution of 4-[(7-chloro-4-methyl-1H-indol-5-yl)amino]-5-iodonicotinonitrile (534 mg, 1.3 mmol) in DME (30 mL) was added diisopropyl 4-(2-chloroethoxy)phenylboronate (754 mg, 2.6 mmol), sodium carbonate solution (2.7 mL of 2M solution, 5.4 mmol) and tetrakis(triphenylphosphine)palladium(0) (150 mg, 0.15mmol) under nitrogen atmosphere and the reaction mixture was heated at 80 °C for 6 h and cooled to room temperature. Ethyl acetate was added and the organic phase was washed with saturated sodium bicarbonate solution and saturated sodium chloride solution, dried over magnesium sulfate, and concentrated. The residue was purified by flash column chromatography (ethyl acetate/hexane) to give 5-[4-(2-chloroethoxy)phenyl]-4-[(7-chloro-4-methyl-1H-indol-5-yl)amino] nicotinonitrile as a yellow solid (482 mg, 84%). HPLC retention time: 10.7 min. ^{(g)} and MS: 437.2 (M + H).

### Example 64: Preparation of 4-[(7-chloro-4-methyl-1H-indol-5-yl)amino]-5-{4-[2-(4-methylpiperazin-1-yl)ethoxy]phenyl}nicotinonitrile 535

A solution of 5-[4-(2-chloroethoxy)phenyl]-4-[(7-chloro-4-methyl-1H-indol-5-yl)amino]nicotinonitrile (144 mg, 0.3 mmol) and 1-methylpiperazine (330 mg, 3.3 mmol) in DME (3 mL) was stirred at 110 °C for 48 h. After the solution was concentrated, 1 N HCl was added and the aqueous phase was extracted with ethyl acetate, adjusted to pH of 10 by adding sodium carbonate, and extracted with ethyl acetate. The combined organic phases were washed with water and brine, dried over magnesium sulfate, and concentrated to give 4-[(7-chloro-4-methyl-1H-indol-5-yl)amino]-5-{4-[2-(4-methylpiperazin-1-yl)ethoxy]phenyl}nicotinonitrile as a yellow solid (140 mg, 84% yield). HPLC retention time: 5.1 min. ^{(g)} ; MS: 501.3 (M + H); and HRMS: 501.2159.

### Example 65: Preparation of 4-[(7-chloro-4-methyl-1H-indol-5-yl)amino]-5-{4-[2-(dimethylamino)ethoxy]phenyl}nicotinonitrile 534

A solution of 5-[4-(2-chloroethoxy)phenyl]-4-[(7-chloro-4-methyl-1H-indol-5-yl)amino]nicotinonitrile (144 mg, 0.3 mmol) and dimethyl amine (1.7 mL of a 2.0 M solution in THF, 3.4 mmol) in DME (3 mL) in a sealed tube was stirred at 110 °C for 48 h and cooled to room temperature. After the resulting solution was concentrated, 1 N aqueous HCl was added and the aqueous phase was extracted with ethyl acetate and the aqueous solution was adjusted to pH of 10 by adding sodium carbonate and extracted with ethyl acetate. The combined organic phases were washed with water and brine, dried over magnesium sulfate, and concentrated to give 4-[(7-chloro-4-methyl-1H-indol-5-yl)amino]-5-{4-[2-(dimethylamino)ethoxy]phenyl} nicotinonitrile as a yellow solid (109 mg, 74%). HPLC retention time: 5.1 min. ^{(g)}; MS: 446.3 (M + H); and HRMS: 446.1743.

### Example 66: Preparation of 5-(3,4-dimethoxyphenyl)4-{[2-(4-methylpiperazin-1-yl)ethyl]amino}nicotinonitrile 439

A mixture of 4-chloro-5-(3,4-dimethoxyphenyl)nicotinonitrile (74 mg, 0.27 mmol), 2-(4-methylpiperazin-1-yl)ethanamine (58 mg, 0.40 mmol) and triethylamine (40 mg, 0.40 mmol) in 3 mL DMF was heated at 60 °C overnight, cooled to room temperature, and concentrated to dryness. The residue was dissolved in 3 mL DMSO, filtered, and purified by a preparative HPLC to give 5-(3,4-dimethoxyphenyl)-4-{[2-(4-methylpiperazin-1-yl)ethyl]amino}nicotinonitrile as a TFA salt (67 mg). HPLC retention time: 1.24 min. ^{(k)}; MS: 382.2 (M + H).

Following procedures analogous to those described for the preparation of 5-(3,4-dimethoxyphenyl)-4-{[2-(4-methylpiperazin-1-yl)ethyl]amino}nicotinonitrile **439**, the compounds in Table 18 were prepared.

**Table 18**

| **Compound** | **Compound Name** | **HPLC retention time (min.)** | **Observed Ion m/e [M+H]** |
|---|---|---|---|
| 440 | 5-(3,4-dimethoxyphenyl)-4-{[3-(4-methylpiperazin-1-yl)propyl]amino} nicotinonitrile | 1.24 ^{(k)} | 396.2 |
| 446 | 4-[(*cis*-4-aminocyclohexyl)amino]-5-(3,4-dimethoxyphenyl)nicotinonitrile | 1.27 ^{(k)} | 353.2 |
| 447 | 5-(3,4-dimethoxyphenyl)-4-{[2-(1-methylpiperidin-4-yl)ethyl]amino}nicotinonitrile | 1.3 ^{(k)} | 381.2 |
| 448 | 5-(3,4-dimethoxyphenyl)-4-{[(1-methylpiperidin-4-yl)methyl]amino}nicotinonitrile | 1.25 ^{(k)} | 367.2 |
| 524 | 4-[(*trans*-4-aminocyclohexyl)amino]-5-(3,4-dimethoxyphenyl)nicotinonitrile | 5.4 ^{(h)} | 353.2; |

### Example 67: Preparation of 4-({[trans-4-(aminomethyl)cyclohexyl]methyl}amino)-5-(3,4-dimethoxyphenyl)nicotinonitrile 441

A solution of 4-chloro-5-(3,4-dimethoxyphenyl)nicotinonitrile (0.27 mmol, 74 mg), trans-tert-butyl (4-(aminomethyl)cyclohexyl)methylcarbamate (0.40 mmol, 97 mg), and triethylamine (0.40 mmol, 40 mg) in 3 mL DMF was heated at 60 °C overnight, cooled to room temperature, and concentrated to dryness. A mixture of dichloromethane (2 mL) and trifluoroacetic acid (2 mL) were added to the residue, and, after standing for 1h at r.t., the solution was concentrated to dryness, redissolved in 3 mL DMSO, filtered, and purified by a preparative HPLC to give 4-({[trans-4-(aminomethyl)cyclohexyl]methyl}amino)-5-(3,4-dimethoxyphenyl)nicotinonitrile as a TFA salt (92 mg). HPLC retention time: 1.33 min. ^{(k)} and MS: 381.2 (M + H).

Following procedures analogous to those described for the preparation of 4-({[trans-4-(aminomethyl)cyclohexyl]methyl}amino)-5-(3,4-dimethoxyphenyl)nicotinonitrile **441**, compounds in Table 19 were prepared.

**Table 19**

| **Compound** | **Compound Name** | **HPLC retention time (min.)** | **Observed Ion m/c [M+H]** |
|---|---|---|---|
| 442 | 4-{[(*trans*-4-aminocyclohexyl)methyl]amino}-5-(3,4-dimethoxyphenyl)nicotinonitrile | 1.27 ^{(k)} | 367.2 |
| 443 | 4-({[*cis*-3-(aminomethyl)cyclohexyl]methyl}amino)-5-(3,4-dimethoxyphenyl)nicotinonitrile | 1.37 ^{(k)} | 381.2 |
| 444 | 5-(3,4-dimethoxyphenyl)-4-[(2-piperidin-4-ylethyl)amino]nicotinonitrile | 1.28 ^{(k)} | 367.2 |
| 445 | 5-(3,4-dimethoxyphenyl)-4-[(piperidin-4-ylmethyl)amino]nicotinonitrile | 1.22 ^{(k)} | 353.2 |

### Example 68: Preparation of 4-[(4-chloro-1H-pyrrolo[2,3-b]pyridin-5-yl)amino]-5-(3,4-dimethoxyphenyl)nicotinonitrile 522

A mixture of 4-chloro-1H-pyrrolo[2,3-b]pyridin-5-amine (see e.g., Tetrahedron Lett., 45(11), 2317-2319 (2004)) (0.30 mmol, 51 mg) and 4-fluoro-5-[3-methoxy-4-(2-methoxyethoxy)phenyl] nicotinonitrile (0.23 mmol, 60 mg) in 2 mL DMSO was heated to 100 °C overnight. The product was purified by a preparative HPLC followed by silica gel chromatography (CH₂Cl₂/MeOH) to give 4-[(4-chloro-1H-pyrrolo[2,3-b]pyridin-5-yl)amino]-5-(3,4-dimethoxyphenyl)nicotinonitrile (23 mg). HPLC retention time: 7.9 min. ^{(g)} and MS: 406.2 (M + H) and HRMS: 406.10747.

Following procedures analogous to those described for the preparation of 4-[(4-chloro-1H-pyrrolo[2,3-b]pyridin-5-yl)amino]-5-(3,4-dimethoxyphenyl)nicotinonitrile **522**, 4-[(4-chloro-1H-pyrrolo[2,3-b]pyridin-5-yl)amino]-5-[3-methoxy-4-(2-methoxyethoxy)phenyl]nicotinonitrile **492** was prepared. HPLC retention time: 8.5 min. ^{(g)} and HRMS: 450.13144.

Additional compounds in Table 20 were prepared in accordance with the procedures outlined in the schemes above and/or by standard synthetic methods and procedures known to those skilled in the art.

**Table 20**

| **Compd.** | **Compound Name** | **HPLC Retention Time (min.)** | **Observed Ion m/e [M+H]** | **Melting Range (°C)** |
|---|---|---|---|---|
| 230 | 4-(1H-indol-5-ylamino)-5-[4-methoxy-3-(2-morpholin-4-ylethoxy)phenyl]nicotinonitrile | 4.1 ^{(c)} | 469.2 | N/A |
| 231 | 4-(1H-indol-5-ylamino)-5-{4-methoxy-3-[2-(4-methylpiperazin-1-yl)ethoxy]phenyl} nicotinonitrile | 4.0 ^{(c)} | 482.3 | N/A |
| 232 | 4-(1H-indol-5-ylamino)-5-[4-methoxy-3-(2-piperazin-1-ylethoxy)phenyl]nicotinonitrile | 3.8 ^{(c)} | 468.2 | N/A |
| 233 | 4-(1H-indol-5-ylamino)-5-[4-methoxy-3-(2-thiomorpholin-4-ylethoxy)phenyl]nicotinonitrile | 4.4 ^{(c)} | 485.2 | N/A |
| 234 | 5-{3-[2-(4-ethylpiperazin-1-yl)ethoxy]-4-methoxyphenyl}-4-(1H-indol-5-ylamino) nicotinonitrile | 4.0 ^{(c)} | 496.3 | N/A |
| 235 | 4-(1H-indol-5-ylamino)-5-[4-methoxy-3-(2-piperidin-1-ylethoxy)phenyl]nicotinonitrile | 4.5 ^{(c)} | 467.2 | N/A |
| 236 | 5-{3-[2-(dimethylamino)ethoxy]-4-methoxy phenyl}-4-(1H-indol-5-ylamino)nicotinonitrile | 4.0 ^{(c)} | 427.2 | N/A |
| 237 | 5-(3-{2-[bis(2-hydroxyethyl)amino]ethoxy}-4-methoxyphenyl)-4-(1H-indol-5-ylamino) nicotinonitrile | 3.8 ^{(c)} | 487.2 | N/A |
| 238 | 5-{3-[2-(4-hydroxypiperidin-1-yl)ethoxy]-4-methoxyphenyl}-4-(1H-indol-5-ylamino) nicotinonitrile | 4.1 ^{(c)} | 483.2 | N/A |
| 239 | 4-(1H-indol-5-ylamino)-5-(4-methoxy-3-{2-[(pyridin-3-ylmethyl)amino]ethoxy}phenyl) nicotinonitrile | 4.0^{(c)} | 490.2 | N/A |
| 240 | 4-(1H-indol-5-ylamino)-5-(4-methoxy-3-{2-[(pyridin-4-ylmethyl)amino]ethoxy}phenyl) nicotinonitrile | 3.9^{(c)} | 490.2 | N/A |
| 241 | 4-(1H-indol-5-ylamino)-5-(4-methoxy-3-{2-[(pyridin-2-ylmethyl)amino]ethoxy}phenyl) nicotinonitrile | 4.5^{(c)} | 490.2 | N/A |
| 242 | 4-(1H-indol-5-ylamino)-5-(4-methoxy-3-{2-[(2-phenylethyl)amino]ethoxy}phenyl)nicotinoni trile | 5.2^{(c)} | 503.2 | N/A |
| 243 | 5-{3-[2-(cyclopentylamino)ethoxy]-4-methoxyphenyl}-4-(1H-indol-5-ylamino) nicotinonitrile | 4.7^{(c)} | 467.3 | N/A |
| 244 | 5-{3-[2-(cyclohexylamino)ethoxy]-4-methoxyphenyl}-4-(1H-indol-5-ylamino) nicotinonitrile | 5.0^{(c)} | 481.3 | N/A |
| 245 | 5-(3-{2-[(2-furylmethyl)amino]ethoxy}-4-methoxyphenyl)-4-(1H-indol-5-ylamino) nicotinonitrile | 4.6^{(c)} | 479.2 | N/A |
| 246 | 5-(2-bromo-4,5-dimethoxyphenyl)-4-(1H-indol-5-ylamino)nicotinonitrile | 2.5^{(c)} | 449.1 | N/A |
| 247 | 5-(2-bromo-4,5-dimethoxyphenyl)-4-[(4-methyl-1H-indol-5-yl)amino]nicotinonitrile | 2.6^{(c)} | 462.2 | N/A |
| 248 | 4-(1H-indol-4-ylamino)-5-[4-methoxy-3-(2-piperidin-1-ylethoxy)phenyl**]**nicotinonitrile | 2.5^{(c)} | 467.2 | N/A |
| 249 | 5-(3-{2-[(2-hydroxyethyl)amino]ethoxy}-4-methoxyphenyl)-4-(1H-indol-4-ylamino) nicotinonitrile | 1.9^{(c)} | 443.2 | N/A |
| 250 | 4-(1H-indol-5-ylamino)-5-(4-methoxy-3-{2-[(3-phenylpropyl)amino]ethoxy}phenyl)nicotino nitrile | 2.8^{(c)} | 517.2 | N/A |
| 251 | 4-(1H-indol-5-ylamino)-5-(4-methoxy-3-{2-[(2-pyridin-2-ylethyl)amino]ethoxy}phenyl) nicotinonitrile | 2.3^{(c)} | 50.42 | N/A |
| 252 | 4-(1H-indol-5-ylamino)-5-(4-methoxy-3-{2-[(2-pyridin-3-ylethyl)amino]ethoxy}phenyl) nicotinonitrile | 2.3^{(c)} | 504.2 | N/A |
| 253 | 4-(1H-indol-5-ylamino)-5-(4-methoxy-3-{2-[(2-pyridin-4-ylethyl)amino]ethoxy}phenyl) nicotinonitrile | 2.2^{(c)} | 504.2 | N/A |
| 254 | 5-[3-(dimethylamino)phenyl]-4-(1*H*-indol-4-yl amino)nicotinonitrile | 1.8^{(c)} | 354.2 | N/A |
| 255 | 4-(1*H*-indol-4-ylamino)-5-[3-(methylsulfonyl) phenyl]nicotinonitrile | 1.7^{(c)} | 389.7 | N/A |
| 256 | N-{3-[5-cyano-4-(1H-indol-4-ylamino)pyridin-3-yl]phenyl}methanesulfonamide | 1.7^{(c)} | 404.1 | N/A |
| 257 | 4-(1H-indol-5-ylamino)-5-phenylnicotinonitrile | 2.4^{(c)} | 310.1 | N/A |
| 258 | 4-(1H-indol-5-ylamino)-5-(3-thienyl) nicotinonitrile | 2.3^{(c)} | 316.1 | N/A |
| 259 | 4-(1H-indol-5-ylamino)-3,3'-bipyridine-5-carbonitrile | 1.8^{(c)} | 311.1 | N/A |
| 260 | 5-(1,3-benzodioxol-5-yl)-4-(1H-indol-5-ylamino) nicotinonitrile | 2.4^{(c)} | 354.1 | N/A |
| 261 | 4-(1H-indol-5-ylamino)-3,4'-bipyridine-5-carbonitrile | 1.8^{(c)} | 311.1 | N/A |
| 262 | 5-(3-furyl)-4-(1H-indol-5-ylamino)nicotinonitrile | 2.2^{(c)} | 300.1 | N/A |
| 263 | 5-(1H-indol-5-yl)-4-(1H-indol-5-ylamino) nicotinonitrile | 2.3^{(c)} | 349.1 | N/A |
| 264 | 5-(2,3-dihydro-1,4-benzodioxin-6-yl)-4-(1H-indol-5-ylamino)nicotinonitrile | 2.4^{(c)} | 368.1 | N/A |
| 265 | 4-(1H-indol-5-ylamino)-5-pyrimidin-5-yl nicotinonitrile | 1.4^{(c)} | 312.1 | N/A |
| 266 | 4-(1H-indol-5-ylamino)-5-(2-methoxypyrimidin-5-yl)nicotinonitrile | 1.8^{(c)} | 342.1 | N/A |
| 267 | 4-(1H-indol-5-ylamino)-5-(2-thienyl) nicotinonitrile | 2.3^{(c)} | 316.1 | N/A |
| 268 | 5-(1-benzofuran-2-yl)-4-(1H-indol-5-ylamino)nicotinonitrile | 2.7^{(c)} | 350.1 | N/A |
| 269 | 5-(3,5-dimethylisoxazol-4-yl)-4-(1H-indol-5-yl amino)nicotinonitrile | 2.3^{(c)} | 329.2 | N/A |
| 270 | 5-[3-(hydroxymethyl)phenyl]-4-(1*H*-indol-4-yl amino)nicotinonitrile | 1.6^{(c)} | 341.1 | N/A |
| 271 | 5-{3-[(dimethylamino)methyl]phenyl}-4-(1*H*-indol-4-ylamino)nicotinonitrile | 1.4^{(c)} | 368.2 | N/A |
| 272 | 4-(1H-indol-4-ylamino)-5-{5-[(prop-2-yn-1-yl amino)methyl]-1-benzothien-2-yl}nicotinonitrile | 2.2^{(c)} | 434.2 | N/A |
| 273 | 5-{5-[(butylamino)methyl]-1-benzothien-2-yl}-4-(1*H*-indol-4-ylamino)nicotinonitrile | 2.3^{(c)} | 452.3 | N/A |
| 274 | 5-(5-{[(2-hydroxyethyl)amino]methyl}-1-benzothien-2-yl)-4-(1*H*-indol-4-ylamino) nicotinonitrile | 2.1^{(c)} | 440.2 | N/A |
| 275 | 5-(5-{[(3-hydroxypropyl)amino]methyl}-1-benzothien-2-yl)-4-(1*H*-indol-4-ylamino) nicotinonitrile | 2.1^{(c)} | 454.3 | N/A |
| 276 | 4-(1*H*-indol-4-ylamino)-5-(5-{[(3-methoxy propyl)amino]methyl}-1-benzothien-2-yl) nicotinonitrile | 2.2^{(c)} | 468.3 | N/A |
| 277 | 5-(5-{[(4-hydroxybutyl)amino]methyl}-1-benzothien-2-yl)-4-(1*H*-indol-4-ylamino) nicotinonitrile | 2.2^{(c)} | 468.3 | N/A |
| 278 | 5-{5-[(cyclopropylamino)methyl]-1-benzothien-2-yl}-4-(1*H*-indol-4-ylamino) nicotinonitrile | 2.2^{(c)} | 436.2 | N/A |
| 279 | 5-(5-{[(cyclopropylmethyl)amino]methyl}-1-benzothien-2-yl)-4-(1*H*-indol-4-ylamino) nicotinonitrile | 2.3^{(c)} | 450.3 | N/A |
| 280 | 4-(1*H*-indol-4-ylamino)-5-[5-(pyrrolidin-1-ylmethyl)-1-benzothien-2-yl]nicotinonitrile | 2.2^{(c)} | 450.3 | N/A |
| 281 | 4-(1*H*-indol-4-ylamino)-5-[5-(morpholin-4-ylmethyl)-1-benzothien-2-yl]nicotinonitrile | 2.2^{(c)} | 466.3 | N/A |
| 282 | 4-(1*H*-indol-4-ylamino)-5-(5-{[(2-morpholin-4-yl ethyl)amino]methyl}-1-benzothien-2-yl)nicotinonitrile | 2.1^{(c)} | 509.3 | N/A |
| 283 | 4-(1*H*-indol-4-ylamino)-5-[5-(piperidin-1-yl methyl)-1-benxothien-2-yl]nicotinonitrile | 2.3^{(c)} | 464.3 | N/A |
| 284 | 5-(5-{[4-(hydroxymethyl)piperidin-1-yl]methyl}-1-benzothien-2-yl)-4-(1*H*-indol-4-ylamino)nicotinonitrile | 2.2^{(c)} | 494.3 | N/A |
| 285 | 5-[5-(hydroxymethyl)-1-benzothien-2-yl]-4-(1*H*-indol-4-ylamino)nicotinonitrile | 3.0^{(c)} | 397.2 | N/A |
| 286 | 5-{5-[(benzylamino)methyl]-1-benzothien-2-yl}-4-(1*H*-indol-4-ylamino)nicotinonitrile | 2.4^{(c)} | 486.3 | N/A |
| 287 | 4-(1*H*-indol-4-ylamino)-5-(5-{[(2-phenylethyl)amino]methyl}-1-benzothien-2-yl)nicotinonitrile | 2.5^{(c)} | 500.3 | N/A |
| 288 | 4-(1*H*-indol-4-ylamino)-5-(5-{[(pyridin-2-yl methyl)amino]methyl}-1-benzothien-2-yl) nicotinonitrile | 2.3^{(c)} | 487.3 | N/A |
| 289 | 4-(1*H*-indol-4-ylamino)-5-(5-{[(pyridin-3-yl methyl)amino]methyl}-1-benzothien-2-yl) nicotinonitrile | 2.2^{(c)} | 487.3 | N/A |
| 290 | 4-(1*H*-indol-4-ylamino)-5-(5-{[(pyridin-4-yl methyl)amino]methyl}-1-benzothien-2-yl) nicotinonitrile | 2.2^{(c)} | 487.3 | N/A |
| 291 | 5-[4-(dimethylamino)phenyl]-4-(pyridin-3-yl amino)nicotinonitrile | 2.1^{(d)} | 316.1 | N/A |
| 292 | 5-[4-(dimethylamino)phenyl]-4-(1H-indazol-5-ylamino)nicotinonitrile | 2.4^{(d)} | 355.4 | N/A |
| 293 | 5-[4-(dimethylamino)phenyl]-4-(1H-indazol-6-ylamino)nicotinonitrile | 2.7^{(d)} | 355.4 | N/A |
| 294 | 5-[4-(dimethylamino)phenyl]-4-[(5-hydroxy-1H-pyrazol-3-yl)amino]nicotinonitrile | 2.4^{(d)} | 320.8 | N/A |
| 295 | 4-(1H-indazol-5-ylamino)-5-(3-methoxyphenyl) nicotinonitrile | 2.7^{(d)} | 342.4 | N/A |
| 296 | 4-(1H-indazol-6-ylamino)-5-(3-methoxyphenyl) nicotinonitrile | 2.8^{(d)} | 341.7 | N/A |
| 297 | 4-[(5-hydroxy-1H-pyrazol-3-yl)amino]-5-(3-methoxyphenyl)nicotinonitrile | 2.5^{(d)} | 413.7 | N/A |
| 298 | 5-(3-bromophenyl)4-[(4-methyl-1H-indol-5-yl)amino]nicotinonitrile | N/A | 403.2 | N/A |
| 299 | 4-(1H-indol-4-ylamino)-5-[4-methoxy-3-(2-morpholin-4-ylethoxy)phenyl]nicotinonitrile | 5.1^{(a)} | 470.3 | 172-174 |
| 300 | 4-(1H-indol-4-ylamino)-5-{4-methoxy-3-[2-(4-methylpiperazin-1-yl)ethoxy]phenyl} nicotinonitrile | N/A | 483.2 | 99-102 (decom. ) |
| 301 | 5-{3-[2-(dimethylamino)ethoxy]-4-methoxy phenyl}-4-(1H-indol-4-ylamino)nicotinonitrile | N/A | 428.2 | 179-181 (decom. ) |
| 302 | 5-{3-[2-(4-hydroxypiperidin-1-yl)ethoxy]-4-methoxyphenyl}-4-(1H-indol-4-ylamino) nicotinonitrile | N/A | 484.2 | 107-110 (decom. ) |
| 303 | 5-[3-(2-chloroethoxy)-4-methoxyphenyl]-4-(1H-indol-4-ylamino)nicotinonitrile | N/A | 419.2 | 171-173 |
| 304 | 4-[(4-methyl-1H-indol-5-yl)amino]-5-[3-(2-thienyl)phenyl]nicotinonitrile | 3.6^{(c)} | 407.2 | N/A |
| 305 | 5-(3,4-dimethoxyphenyl)-4-[(4-ethyl-1H-indol-5-yl)amino]nicotinonitrile | N/A | 399.2 | 190-192 |
| 306 | 5-[3-(5-formyl-2-thienyl)phenyl]-4-[(4-methyl-1H-indol-5-yl)amino]nicotinonitrile | 9.4^{(a)} | 435.2 | N/A |
| 307 | 5-(3-{5-[(dimethylamino)methyl]-2-thienyl} phenyl)-4-[(4-methyl-1H-indol-5-yl)amino) nicotinonitrile | 6.9^{(a)} | 464.2 | N/A |
| 308 | 3'-[5-cyano-4-(1*H*-indol-4-ylamino)pyridin-3-yl]-*N*-(4-hydroxybutyl)biphenyl-4-carboxamide | 7.9^{(a)} | 502.2 | N/A |
| 309 | 3'-{5-cyano-4-[(4-methyl-1*H*-indol-5-yl)amino]pyridin-3-yl}-N-(4-hydroxybutyl)biphenyl-4-carboxamide | 8.0^{(a)} | 516.3 | N/A |
| 310 | 4-(1H-indol-4-ylamino)-5-[3-(trifluoromethyl)phenyl]nicotinonitrile | 10.8^{(a)} | 379.1 | N/A |
| 311 | 5-(3-cyanophenyl)-4-(1H-indol-4-ylamino) nicotinonitrile | 8.5^{(a)} | 336.1 | N/A |
| 312 | 3-[5-cyano-4-(1*H*-indol-4-ylamino) pyridin-3-yl]-*N,N*-dimethylbenzamide | 6.9^{(a)} | 382.2 | N/A |
| 313 | 3-[5-cyano-4-(1H-indol-4-ylamino)pyridin-3-yl]-N,N-dimethylbenzenesulfonamide | 9.0^{(a)} | 418.2 | N/A |
| 314 | 3-[5-cyano-4-(1*H-*indol-4-ylamino) pyridin-3-yl] benzamide | 5.8^{(a)} | 354.2 | N/A |
| 315 | 5-(3-{5-[(dimethylamino)methyl]-2-thienyl} phenyl)-4-(1*H-*indol-4-ylamino)nicotinonitrile | 6.1^{(a)} | 450.3 | N/A |
| 316 | 2-[5-cyano-4-(1H-indol-4-ylamino)pyridin-3-yl]-N,N-dimethylbenzenesulfonamide | 6.6^{(a)} | 418.2 | N/A |
| 317 | N-{4-[5-cyano-4-(1H-indol-4-ylamino)pyridin-3-yl]phenyl}methanesulfonamide | 5.9^{(a)} | 404.2 | N/A |
| 318 | 5-(1-benzofuran-2-yl)-4-(1H-indol-4-ylamino)nicotinonitrile | 11.5^{(a)} | 351.2 | N/A |
| 319 | 5-dibenzo[b,d]furan-4-yl-4-(1H-indol-4-ylamino)nicotinonitrile | 10.7^{(a)} | 401.3 | N/A |
| 320 | 4-(1H-indol-4-ylamino)-5-{1-[(4-methylphenyl)sulfonyl]-1H-indol-3-yl}nicotinonitrile | 12.3^{(a)} | 504.3 | N/A |
| 322 | 5-(1-benzothien-2-yl)-4-(1H-indol-4-ylamino)nicotinonitrile | N/A | 367.1 | > 245 |
| 323 | 4-(1H-indol-4-ylamino)-5-(4-methoxyphenyl)nicotinonitrile | N/A | 341.2 | 204-206 (decom. ) |
| 324 | 4-(1H-indol-4-ylamino)-5-(2-methoxyphenyl) nicotinonitrile | N/A | 341.2 | >245 |
| 325 | 5-(1H-indol-2-yl)-4-(1H-indol-4-ylamino) nicotinonitrile | N/A | 350.2 | 242-244 (decom. ) |
| 326 | 4-[5-cyano-4-(1H-indol-4-ylamino)pyridin-3-yl]-N,N-dimethylbenzenesulfonamide | 8.4^{(a)} | 418.3 | N/A |
| 327 | 3-[5-cyano-4-(1*H*-indol-4-ylamino)pyridin-3-yl]benzoic acid | 6.4^{(a)} | 355.2 | N/A |
| 328 | 5-[3-(aminomethyl)phenyl]-4-(1H-indol-4-yl amino)nicotinonitrile | 4.0^{(a)} | 340.2 | N/A |
| 329 | 5-(3,4-dimethoxyphenyl)-4-[(2-oxo-2,3-dihydro-1H-indol-4-yl)amino]nicotinonitrile | 6.1^{(a)} | 387.2 | N/A |
| 330 | 4-[5-cyano-4-(1*H*-indol-4-ylamino)pyridin-3-yl]-N-(2-methoxyethyl)benzamide | 6.6^{(a)} | 412.3 | N/A |
| 331 | 4-(1H-indol-4-ylamino)-5-(4-methoxy-3-{2-[(3-phenylpropyl)amino]ethoxy}phenyl) nicotinonitrile | N/A | 518.3 | 202-204 |
| 332 | 5-[4-(2-chloroethoxy)phenyl]-4-(1H-indol-4-ylamino)nicotinonitrile | N/A | 389.2 | 190-191 |
| 333 | 5-[3-(5-formyl-2-thienyl)phenyl]-4-(1H-indol-4-ylamino)nicotinonitrile | 3.2^{(a)} | 421.2 | N/A |
| 334 | 4-(1H-indol-4-ylamino)-5-[4-(2-morpholin-4-ylethoxy)phenyl]nicotinonitrile | N/A | 440.3 | 130-132 |
| 335 | 4-(1H-indol-4-ylamino)-5-{4-[2-(4-methyl piperazin-1-yl)ethoxy]phenyl}nicotinonitrile | N/A | 453.3 | 114-115 |
| 336 | 5-(3,4-dimethoxyphenyl)-4-[(5-methyl-1H-indol-4-yl)amino]nicotinonitrile | N/A | 385.2 | 204-205 |
| 337 | 5-(2,4-dimethoxyphenyl)-4-(1H-indol-4-yl amino)nicotinonitrile | N/A | 374.9 | 192-193 |
| 338 | 4-(1H-indol-4-ylamino)-5-[4-methoxy-3-(2-{[3-(2-oxopyrrolidin-1-yl)propyl]amino}ethoxy) phenyl]nicotinonitrile | 0.7^{(c)} | 525.1 | N/A |
| 339 | 5-[3-(2-{[2-(1H-imidazol-4-yl)ethyl]amino} ethoxy)-4-methoxyphenyl]-4-(1H-indol-4-yl amino)nicotinonitrile | 0.45^{(c)} | 494.0 | N/A |
| 340 | 4-(1H-indol-4-ylamino)-5-(4-methoxy-3-{2-[(3-pyrrolidin-1-ylpropyl)amino]ethoxy}phenyl) nicotinonitrile | 0.43^{(c)} | 571.1 | N/A |
| 341 | 4-(1H-indol-4-ylamino)-5-[4-methoxy-3-(2-{[2-(1-methylpyrrolidin-2-yl)ethyl]amino}ethoxy)phenyl] nicotinonitrile | 0.24^{(c)} | 571.1 | N/A |
| 342 | 5-(4-methoxy-3-{2-[(3-phenylpropyl)amino] ethoxy}phenyl)-4-[(4-methyl-1H-indol-5-yl)amino]nicotinonitrile | 3.0^{(c)} | 531.1 | N/A |
| 343 | 5-{4-methoxy-3-[2-(4-methylpiperazin-1-yl) ethoxy]phenyl}-4-[(4-methyl-1H-indol-5-yl) amino]nicotinonitrile | 2.4^{(c)} | 495.1 | N/A |
| 344 | 5-(3-methoxy-4-{2-[(3-phenylpropyl)amino] ethoxy}phenyl)-4-[(4-methyl-1H-indol-5-yl)amino]nicotinonitrile | 1.6^{(c)} | 532.1 | N/A |
| 346 | 5-(3-bromophenyl)-4-[(2-oxo-2,3-dihydro-1H-indol-4-yl)amino]nicotinonitrile | 8.4^{(a)} | 405 | N/A |
| 347 | 4-(1H-indol-4-ylamino)-5-(4-{2-[(3-phenylpropyl) amino}ethoxy}phenyl)nicotinonitrile | N/A | 448.2 | 105-107 |
| 348 | 5-[4-(2-chloroethoxy)phenyl]-4-[(4-methyl-1H-indol-5-yl)amino]nicotinonitrile | N/A | 403.1 | 200-202 |
| 349 | 5-(3,4-dimethoxyphenyl)-4-[(2-methyl-1H-indol-4-yl)amino]nicotinonitrile | N/A | 385.1 | 112-115 |
| 350 | 4-[(4-methyl-1H-indol-5-yl)amino]-5-{4-[2-(4-methylpiperazin-1-yl)ethoxy]phenyl} nicotinonitrile | 4.60^{(a)} | 467.2 | 215-217 (decom. ) |
| 351 | 4-[(4-methyl-1H-indol-5-yl)amino]-5-[4-(2-morpholin-4-ylethoxy)phenyl]nicotinonitrile | N/A | 454.2 | 154-156 |
| 352 | 5-(1-benzofuran-3-yl)-4-(1H-indol-4-ylamino)nicotinonitrile | N/A | 357.2 | 238-240 |
| 353 | 4-(1H-indol-4-ylamino)-5-(4-methoxy-3-{2-[(2-phenylethyl)amino]ethoxy}phenyl)nicotinoni trile | N/A | 504.3 | 180-182 |
| 354 | 4-(1H-indol-4-ylamino)-5-(4-methoxy-3-{2-[(2-pyridin-3-ylethyl)amino]ethoxy}phenyl) nicotinonitrile | N/A | 505.4 | 170-172 |
| 355. | 4-[(4-methyl-1H-indol-5-yl)amino]-5-(4-{2-[(3-phenylpropyl)amino]ethoxy}phenyl)nicotino nitrile | N/A | 502.3 | 115-117 |
| 356 | 6'-[3-(dimethylamino)propoxy]-4-(1H-indol-4-ylamino)-3,3'-bipyridine-5-carbonitrile | N/A | 413.2 | 120-122 |
| 357 | 6'-[3-(dimethylamino)propoxy]-4-[(4-methyl-1H-indol-5-yl)amino]-3,3'-bipyridine-5-carbonitrile | N/A | 427.2 | 125-127 |
| 358 | 5-(3-hydroxyphenyl)-4-(1H-indol-4-ylamino) nicotinonitrile | N/A | 325.2 [M-H] | 268-270 |
| 359 | 4-(1H-indol-4-ylamino)-5-[5-(piperazin-1-yl methyl)-1-benzothien-2-yl]nicotinonitrile | 5.4^{(a)} | 465.1 | N/A |
| 360 | N-({2-[5-cyano-4-(1H-indol-4-ylamino) pyridin-3-yl]-1-benzothien-5-yl}methyl)-b-alaninamide | 5.6^{(a)} | 467.3 | N/A |
| 361 | 4-(1H-indol-4-ylamino)-6'-[(2-morpholin-4-yl ethyl)amino]-3,3'-bipyridine-5-carbonitrile | N/A | 440.4 | 148-150 |
| 362 | 4-[(4-methyl-1H-indol-5-yl)amino]-6'-[(2-morpholin-4-ylethyl)amino]-3,3'-bipyridine-5-carbonitrile | N/A | 445.4 | 173-175 |
| 363 | 5-{2-chloro-4-[2-(dimethylamino)ethoxy]phenyl}-4-[(4-methyl-1H-indol-5-yl)amino]nicotinonitrile | 5.2^{(a)} | 446.2 | N/A |
| 364 | 4-(1H-indol-4-ylamino)-5-(4-methoxy-3-{2-[(3-morpholin-4-ylpropyl)amino]ethoxy} phenyl)nicotinonitrile | N/A | 527.4 | 71-74 |
| 365 | 5-[3-(2-{[3-(1H-imidazol-1-yl)propyl]amino}ethoxy)-4-methoxyphenyl]-4-(1H-indol-4-ylamino)nicotinonitrile | N/A | 508.4 | 100-104 |
| 366 | 5-(3-{[(2S)-2-amino-3-phenylpropyl]oxy} phenyl)-4-(1H-indol-4-ylamino)nicotinonitrile | N/A | 460.2 | 90-92 |
| 367 | 5-{5-[(benzylamino)methyl]-1-benzothien-2-yl}-4-[(4-methyl-1H-indol-5-yl)amino]nicotinonitrile | 7.9^{(a)} | N/A | 145-147 |
| 368 | 5- {4-[2-(4-butylpiperazin-1-yl)ethoxy]phenyl}-4-[(4-methyl-1H-indol-5-yl)amino]nicotinonitrile | 5.70^{(a)} | N/A | 158-160 |
| 369 | 5-[5-(1,3-dioxan-2-yl)-1-benzofuran-2-yl]-4-(1H-indol-4-ylamino)nicotinonitrile | N/A | 437.2 | 225-227 (decom. ) |
| 370 | 5-[5-(1,3-dioxan-2-yl)-1-benzofuran-2-yl]-4-[(4-methyl-1H-indol-5-yl)amino]nicotinonitrile | N/A | 451.2 | 231-233 (decom. ) |
| 371 | 5-(2-chloro-4-methoxyphenyl)-4-[(4-methyl-1H-indol-5-yl)amino]nicotinonitrile | 14.3^{(a)} | 389.2 | N/A |
| 372 | 5-[4-(2-chloroethoxy)-3-methoxyphenyl]-4-[(4-methyl-1H-indol-5-yl)amino]nicotinonitrile | N/A | 433.3 | > 245 |
| 373 | 5-(1-benzofuran-2-yl)-4-(1H-indazol-5-ylamino)nicotinonitrile | 10.0^{(a)} | 352.2 | 190-192 |
| 374 | 5-(4-hydroxyphenyl)-4-[(4-methyl-1H-indol-5-yl)amino]nicotinonitrile | 6.5^{(a)} | 341.2 | 240 (decom. ) |
| 375 | 5-(2-chloro-6-methoxyphenyl)-4-[(4-methyl-1H-indol-5-yl)amino]nicotinonitrile | 8.8^{(a)} | 389.2 | N/A |
| 376 | 5-[3-methoxy-4-(2-piperidin-1-ylethoxy) phenyl]-4-[(4-methyl-1H-indol-5-yl)amino] nicotinonitrile | N/A | 482.4 | 114-116 |
| 377 | 5-{3-methoxy-4-[2-(4-methylpiperazin-1-yl) ethoxy]phenyl}-4-[(4-methyl-1H-indol-5-yl) amino]nicotinonitrile | N/A | 497.5 | 107-109 |
| 378 | 5-[3-methoxy-4-(2-morpholin-4-ylethoxy)phenyl]-4-[(4-methyl-1H-indol-5-yl)amino]nicotinonitrile | N/A | 484.4 | . 113-115 |
| 379 | 5-(3,4-dimethoxyphenyl)-4-(1H-indazol-5-yl amino)nicotinonitrile | N/A | 372.2 | 191-193 |
| 380 | 5-(2,3-dichlorophenyl)-4-[(4-methyl-1H-indol-5-yl)amino]nicotinonitrile | 10.1^{(a)} | 393.2 | N/A |
| 381 | 5-(4-bromo-2-fluorophenyl)-4-[(4-methyl-1H-indol-5-yl)amino]nicotinonitrile | 10.2^{(a)} | 421.1 | N/A |

### Example 69: Pharmacological testing

Evaluation of representative compounds of the present teachings in several standard pharmacological test procedures indicated that the compounds are inhibitors of PKCθ. Based on the activity shown in the standard pharmacological test procedures, the compounds of the present teachings are therefore useful as antiinflammatory agents.

### A radioactive kinase assay for inhibition of the active kinase domain (KD) of PKC

This assay is based on the phosphorylation of a biotinylated substrate by a kinase utilizing radiolabeled ATP (ATP γ P33). The substrate was a biotinylated peptide with a sequence of biotin-FARKGSLRQ-C(O)NH₂. The enzyme was purified recombinant active kinase domain of full length PKC theta (amino acids 362-706). The assay buffer was composed of 100mM Hepes, pH7.5, 2mM MgCl₂, 20mM β-glycerophosphate and 0.008% TritonX 100. A reaction mixture of ATP, ATP γ P33 (PerkinElmer), DTT, and the enzyme was prepared in the assay buffer and added to a 96-well polypropylene plate. The compound (diluted in DMSO in a separate 96-well polypropylene plate) was added to the reaction mixture and incubated at room temperature. Following the incubation, the peptide substrate was added to the reaction mixture to initiate the enzymatic reaction. The reaction was terminated with the addition of a stop solution (100mM EDTA, 0.2% TritonX100, and 20mM NaHPO₄) and transferred from the assay plate to a washed streptavidin-coated 96-well scintiplate (PerkinElmer). The scintiplate was incubated at room temperature, washed in PBS with 0.1% TritonX 100, and counted in the 1450 Microbeta Trilux (Wallac, Version 2.60). Counts were recorded for each well as corrected counts per minute (CCPM). The counts were considered corrected because they were adjusted according to a P33 normalization protocol, which corrects for efficiency and background differences between the instrument detectors (software version 4.40.01).

### A radioactive kinase assay for inhibition of full length (FL) PKCθ inhibitors

This assay differs from what was described above in that the enzyme used was purified recombinant full length PKC theta (Panvera, P2996).

### PKCθ IMAP Assay

The materials used include the following: human PKCθ full length enzyme (Panvera Cat# P2996); substrate peptide: 5FAM-RFARKGSLRQKNV-OH (Molecular Devices, RP7032); ATP (Sigma Cat # A2383); DTT (Pierce, 20291); 5x kinase reaction buffer (Molecular Devices, R7209); 5x binding buffer A (Molecular Devices, R7282), 5x binding buffer B (Molecular Devices, R7209); IMAP Beads (Molecular Devices, R7284); and 384-well plates (Coming Costar, 3710).

The reaction buffer was prepared by diluting the 5x stock reaction buffer and adding DTT to obtain a concentration of 3.0 mM. The binding buffer was prepared by diluting the 5x binding buffer A. A master mix solution was prepared using a 90% dilution of the reaction buffer containing 2x ATP (12 uM) and 2x peptide (200 nm). Compounds were diluted in DMSO to 20x of the maximum concentration for the IC50 measurement. 27 µl of the master mix solution for each IC50 curve was added to the first column in a 384-well plate and 3 µl of 20x compound in DMSO was added to each well. The final concentration of compound was 2x and 10% DMSO. DMSO was added to the rest of the master mix to increase the concentration to 10%. 10 µl of the master mix containing 10% DMSO was added to the rest of the wells on the plate except the 2nd column. 20 µl was transferred from the first column to the 2nd column. The compounds were serially diluted in 2:1 ratio starting from the 2nd column. A 2x (2 nM) PKCθ solution was made in the reaction buffer. 10 µl of the PKCθ solution was added to every well to achieve these final concentrations: PKCθ - 1 nM; ATP - 6 µM; peptide - 100 nM; DMSO - 5%. Samples were incubated for 25 minutes at room temperature. The binding reagent was prepared by diluting the beads in 1x binding buffer to 800:1. 50 µl of the binding reagent was added to every well and incubated for 20 minutes. FP was measured using Envision2100 (PerkinElmer Life Sciences). Wells with no ATPs and wells with no enzymes were used as controls.

The results obtained are summarized in Table 21 below. Data presented represent the average value when one or more samples were tested.

**Table 21**

| **Compound** | **IC₅₀ PKCθ IMAP (µM)** | **IC₅₀ PKCθ FL (µM)** | **IC₅₀ PKCθKD (µM)** |
|---|---|---|---|
| 101 | 0.14 | 0.083 | 0.14 |
| 102 | N/A | N/A | 0.96 |
| 103 | N/A | N/A | 5.68 |
| 104 | N/A | N/A | 6.56 |
| 105 | N/A | N/A | 2.66 |
| 106 | N/A | N/A | 11.4 |
| 107 | 9.32 | N/A | N/A |
| 108 | 0.45 | 2.25 | 1.01 |
| 109 | 0.013 | 0.04 | 0.15 |
| 110 | 0.065 | N/A | N/A |
| 111 | 0.58 | N/A | N/A |
| 112 | 1.13 | N/A | N/A |
| 113 | 0.05 | N/A | N/A |
| 114 | 0.002 | N/A | N/A |
| 115 | 0.31 | N/A | N/A |
| 116 | 0.07 | N/A | N/A |
| 117 | N/A | 0.14 | N/A |
| 118 | N/A | 0.09 | N/A |
| 119 | 0.3 | N/A | N/A |
| 120 | 0.23 | 0.12 | N/A |
| 121 | N/A | 0.17 | N/A |
| 122 | N/A | 0.34 | N/A |
| 123 | 0.18 | N/A | N/A |
| 124 | 1.1 | N/A | N/A |
| 127 | N/A | 0.29 | N/A |
| 128 | 0.13 | N/A | N/A |
| 129 | 0.24 | 0.06 | N/A |
| 130 | 0.16 | 0.08 | N/A |
| 131 | 0.12 | 0.05 | N/A |
| 132 | 0.11 | N/A | N/A |
| 133 | N/A | 0.37 | N/A |
| 134 | N/A | 0.39 | N/A |
| 136 | 0.13 | N/A | N/A |
| 137 | N/A | 0.07 | N/A |
| 138 | 0.19 | 0.1 | N/A |
| 139 | 0.25 | N/A | N/A |
| 140 | 0.11 | N/A | N/A |
| 141 | N/A | 0.09 | N/A |
| 142 | 0.12 | N/A | N/A |
| 143 | N/A | 0.91 | N/A |
| 144 | N/A | 0.91 | N/A |
| 145 | N/A | 1.92 | N/A |
| 146 | N/A | 1.33 | N/A |
| 147 | 0.02 | N/A | N/A |
| 148 | 0.04 | N/A | N/A |
| 149 | 0.07 | N/A | N/A |
| 150 | 0.12 | N/A | N/A |
| 152 | N/A | 1.94 | N/A |
| 153 | N/A | 0.04 | N/A |
| 154 | 0.32 | 0.36 | N/A |
| 155 | N/A | 7.8 | N/A |
| 156 | N/A | >20 | N/A |
| 157 | N/A | 0.55 | N/A |
| 158 | N/A | 37.4 | N/A |
| 159 | 0.016 | 0.06 | N/A |
| 160 | N/A | 0.14 | N/A |
| 161 | N/A | 5.5 | N/A |
| 162 | N/A | 15 | N/A |
| 163 | N/A | 0.32 | N/A |
| 164 | 0.03 | N/A | N/A |
| 165 | N/A | 0.02 | N/A |
| 166 | N/A | 3.83 | N/A |
| 167 | N/A | 0.11 | N/A |
| 168 | 0.02 | N/A | N/A |
| 169 | N/A | 0.03 | N/A |
| 170 | N/A | 0.02 | N/A |
| 171 | 0.05 | N/A | N/A |
| 172 | N/A | 2.59 | N/A |
| 173 | N/A | 2.63 | N/A |
| 174 | N/A | 0.07 | N/A |
| 175 | N/A | 0.1 | N/A |
| 176 | 0.03 | N/A | N/A |
| 177 | 0.02 | N/A | N/A |
| 178 | 0.11 | N/A | N/A |
| 179 | 0.41 | N/A | N/A |
| 180 | 0.69 | N/A | N/A |
| 181 | 0.039 | N/A | N/A |
| 182 | 0.12 | N/A | N/A |
| 183 | 0.07 | N/A | N/A |
| 184 | 0.26 | N/A | N/A |
| 185 | 0.5 | N/A | N/A |
| 186 | 0.14 | N/A | N/A |
| 187 | 0.11 | N/A | N/A |
| 188 | 0.01 | N/A | N/A |
| 189 | 0.03 | N/A | N/A |
| 190 | 0.18 | N/A | N/A |
| 191 | 0.32 | N/A | N/A |
| 192 | 0.28 | N/A | N/A |
| 193 | 0.25 | N/A | N/A |
| 194 | N/A | 0.05 | N/A |
| 195 | 0.25 | N/A | N/A |
| 196 | 0.46 | N/A | N/A |
| 197 | 0.19 | N/A | N/A |
| 198 | 0.33 | N/A | N/A |
| 199 | 1.3 | N/A | N/A |
| 200 | 0.19 | N/A | N/A |
| 201 | 0.3 | N/A | N/A |
| 202 | 0.34 | N/A | N/A |
| 203 | 0.29 | N/A | N/A |
| 204 | N/A | 0.19 | N/A |
| 205 | 0.51 | N/A | N/A |
| 206 | N/A | 1.14 | N/A |
| 207 | N/A | 0.53 | N/A |
| 208 | N/A | 0.39 | N/A |
| 209 | 0.28 | N/A | N/A |
| 210 | 2.58 | N/A | N/A |
| 211 | 0.33 | N/A | N/A |
| 212 | 0.59 | N/A | N/A |
| 213 | 0.27 | N/A | N/A |
| 214 | 0.25 | N/A | N/A |
| 215 | 0.06 | N/A | N/A |
| 216 | 0.05 | N/A | N/A |
| 217 | N/A | 0.36 | N/A |
| 218 | 0.006 | N/A | N/A |
| 219 | 0.27 | N/A | N/A |
| 220 | N/A | >20 | N/A |
| 221 | 1.75 | N/A | N/A |
| 222 | 0.001 | N/A | N/A |
| 223 | 0.006 | N/A | N/A |
| 224 | 0.004 | N/A | N/A |
| 225 | 0.005 | N/A | N/A |
| 226 | 0.003 | N/A | N/A |
| 227 | 0.001 | N/A | N/A |
| 228 | 0.002 | N/A | N/A |
| 229 | 0.008 | N/A | N/A |
| 230 | 0.33 | N/A | N/A |
| 231 | 0.25 | N/A | N/A |
| 232 | 0.2 | N/A | N/A |
| 233 | 0.18 | N/A | N/A |
| 234 | 0.19 | N/A | N/A |
| 235 | 0.24 | N/A | N/A |
| 236 | 0.2 | N/A | N/A |
| 237 | 4.32 | N/A | N/A |
| 238 | 0.21 | N/A | N/A |
| 239 | 0.08 | N/A | N/A |
| 240 | 0.08 | N/A | N/A |
| 241 | 0.08 | N/A | N/A |
| 242 | 0.02 | N/A | N/A |
| 243 | 0.23 | N/A | N/A |
| 244 | 0.17 | N/A | N/A |
| 245 | 0.27 | N/A | N/A |
| 246 | 0.16 | N/A | N/A |
| 247 | 0.1 | N/A | N/A |
| 248 | 0.05 | N/A | N/A |
| 249 | 0.03 | N/A | N/A |
| 250 | 0.02 | N/A | N/A |
| 251 | 0.04 | N/A | N/A |
| 252 | 0.03 | N/A | N/A |
| 253 | 0.05 | N/A | N/A |
| 254 | 0.08 | N/A | N/A |
| 255 | 0.08 | N/A | N/A |
| 256 | 0.02 | N/A | N/A |
| 257 | 0.1 | N/A | N/A |
| 258 | 0.1 | N/A | N/A |
| 259 | 1.37 | N/A | N/A |
| 260 | 0.07 | N/A | N/A |
| 261 | 0.08 | N/A | N/A |
| 262 | 0.12 | N/A | N/A |
| 263 | 0.05 | N/A | N/A |
| 264 | 0.06 | N/A | N/A |
| 265 | 3.86 | N/A | N/A |
| 266 | 1.45 | N/A | N/A |
| 267 | 0.1 | N/A | N/A |
| 268 | 0.01 | N/A | N/A |
| 269 | 10.9 | N/A | N/A |
| 270 | 0.02 | N/A | N/A |
| 271 | 0.1 | N/A | N/A |
| 272 | 0.004 | N/A | N/A |
| 273 | 0.002 | N/A | N/A |
| 274 | 0.002 | N/A | N/A |
| 275 | 0.002 | N/A | N/A |
| 276 | 0.002 | N/A | N/A |
| 277 | 0.003 | N/A | N/A |
| 278 | 0.002 | N/A | N/A |
| 279 | 0.001 | N/A | N/A |
| 280 | 0.004 | N/A | N/A |
| 281 | 0.01 | N/A | N/A |
| 282 | 0.003 | N/A | N/A |
| 283 | 0.002 | N/A | N/A |
| 284 | 0.004 | N/A | N/A |
| 285 | 0.004 | N/A | N/A |
| 286 | 0.001 | N/A | N/A |
| 287 | 0.003 | N/A | N/A |
| 288 | 0.003 | N/A | N/A |
| 289 | 0.004 | N/A | N/A |
| 290 | 0.004 | N/A | N/A |
| 291 | 4.86 | N/A | N/A |
| 292 | 13.2 | N/A | N/A |
| 293 | 36 | N/A | N/A |
| 294 | 33.4 | N/A | N/A |
| 295 | 2.95 | N/A | N/A |
| 296 | 16.2 | N/A | N/A |
| 298 | 0.004 | N/A | N/A |
| 299 | 0.03 | N/A | N/A |
| 300 | 0.02 | N/A | N/A |
| 301 | 0.05 | N/A | N/A |
| 302 | 0.03 | N/A | N/A |
| 303 | 0.04 | N/A | N/A |
| 304 | 0.11 | N/A | N/A |
| 305 | 0.06 | N/A | N/A |
| 306 | 0.06 | N/A | N/A |
| 308 | 0.03 | N/A | N/A |
| 309 | 0.12 | N/A | N/A |
| 310 | 0.02 | N/A | N/A |
| 311 | 0.02 | N/A | N/A |
| 312 | 0.6 | N/A | N/A |
| 313 | 0.02 | N/A | N/A |
| 314 | 0.1 | N/A | N/A |
| 315 | 0.01 | N/A | N/A |
| 316 | 1.35 | N/A | N/A |
| 317 | 0.02 | N/A | N/A |
| 318 | 0.01 | N/A | N/A |
| 319 | 0.06 | N/A | N/A |
| 320 | 0.03 | N/A | N/A |
| 322 | 0.02 | N/A | N/A |
| 323 | 0.02 | N/A | N/A |
| 324 | 0.02 | N/A | N/A |
| 325 | 0.06 | N/A | N/A |
| 326 | 0.19 | N/A | N/A |
| 327 | 0.12 | N/A | N/A |
| 328 | 0.02 | N/A | N/A |
| 329 | 8.21 | N/A | N/A |
| 330 | 0.13 | N/A | N/A |
| 332 | 0.008 | N/A | N/A |
| 334 | 0.03 | N/A | N/A |
| 335 | 0.01 | N/A | N/A |
| 336 | 0.2 | N/A | N/A |
| 337 | 0.1 | N/A | N/A |
| 338 | 0.008 | N/A | N/A |
| 339 | 0.01 | N/A | N/A |
| 340 | 0.008 | N/A | N/A |
| 341 | 0.003 | N/A | N/A |
| 342 | 0.001 | N/A | N/A |
| 343 | 0.01 | N/A | N/A |
| 344 | 0.008 | N/A | N/A |
| 345 | 0.007 | N/A | N/A |
| 346 | 0.54 | N/A | N/A |
| 347 | 0.004 | N/A | N/A |
| 348 | 0.004 | N/A | N/A |
| 349 | 0.13 | N/A | N/A |
| 350 | 0.008 | N/A | N/A |
| 351 | 0.02 | N/A | N/A |
| 352 | 0.02 | N/A | N/A |
| 353 | 0.001 | N/A | N/A |
| 354 | 0.004 | N/A | N/A |
| 355 | 0.003 | N/A | N/A |
| 356 | 0.04 | N/A | N/A |
| 357 | 0.03 | N/A | N/A |
| 358 | 0.01 | N/A | N/A |
| 359 | 0.02 | N/A | N/A |
| 360 | 0.002 | N/A | N/A |
| 361 | 0.04 | N/A | N/A |
| 362 | 0.05 | N/A | N/A |
| 363 | 0.02 | N/A | N/A |
| 364 | 0.01 | N/A | N/A |
| 365 | 0.004 | N/A | N/A |
| 366 | 0.02 | N/A | N/A |
| 367 | 0.003 | N/A | N/A |
| 368 | 0.01 | N/A | N/A |
| 369 | 0.04 | N/A | N/A |
| 370 | 0.004 | N/A | N/A |
| 371 | 0.03 | N/A | N/A |
| 372 | 0.007 | N/A | N/A |
| 373 | 1 | N/A | N/A |
| 374 | 0.02 | N/A | N/A |
| 375 | 0.15 | N/A | N/A |
| 376 | 0.02 | N/A | N/A |
| 377 | 0.01 | N/A | N/A |
| 378 | 0.03 | N/A | N/A |
| 379 | 2.53 | N/A | N/A |
| 380 | 0.05 | N/A | N/A |
| 381 | 0.02 | N/A | N/A |
| 382 | 0.006 | N/A | N/A |
| 383 | 0.002 | N/A | N/A |
| 384 | 0.002 | N/A | N/A |
| 385 | 0.002 | N/A | N/A |
| 386 | 0.005 | N/A | N/A |
| 387 | 0.001 | N/A | N/A |
| 388 | 0.013 | N/A | N/A |
| 389 | 0.003 | N/A | N/A |
| 390 | 0.001 | N/A | N/A |
| 391 | 0.001 | N/A | N/A |
| 392 | 0.005 | N/A | N/A |
| 393 | 0.001 | N/A | N/A |
| 394 | 0.001 | N/A | N/A |
| 395 | 0.004 | N/A | N/A |
| 396 | 0.002 | N/A | N/A |
| 397 | 0.013 | N/A | N/A |
| 398 | 0.003 | N/A | N/A |
| 399 | 0.002 | N/A | N/A |
| 400 | 0.002 | N/A | N/A |
| 401 | 0.008 | N/A | N/A |
| 402 | 0.041 | N/A | N/A |
| 403 | 0.005 | N/A | N/A |
| 404 | 0.004 | N/A | N/A |
| 405 | 0.009 | N/A | N/A |
| 406 | 0.042 | N/A | N/A |
| 407 | 0.020 | N/A | N/A |
| 408 | 0.011 | N/A | N/A |
| 409 | 0.027 | N/A | N/A |
| 410 | 0.035 | N/A | N/A |
| 411 | 0.008 | N/A | N/A |
| 412 | 0.010 | N/A | N/A |
| 413 | 0.055 | N/A | N/A |
| 414 | 0.004 | N/A | N/A |
| 415 | 0.005 | N/A | N/A |
| 416 | 0.006 | N/A | N/A |
| 417 | 0.006 | N/A | N/A |
| 418 | 0.004 | N/A | N/A |
| 419 | 0.004 | N/A | N/A |
| 420 | 0.003 | N/A | N/A |
| 421 | 0.003 | N/A | N/A |
| 422 | 0.008 | N/A | N/A |
| 423 | 0.011 | N/A | N/A |
| 424 | 0.004 | N/A | N/A |
| 425 | 0.009 | N/A | N/A |
| 426 | 0.013 | N/A | N/A |
| 427 | 0.014 | N/A | N/A |
| 428 | 0.015 | N/A | N/A |
| 429 | 0.005 | N/A | N/A |
| 430 | 0.017 | N/A | N/A |
| 431 | 0.005 | N/A | N/A |
| 432 | 0.009 | N/A | N/A |
| 433 | 0.010 | N/A | N/A |
| 434 | 0.014 | N/A | N/A |
| 435 | 0.049 | N/A | N/A |
| 436 | 0.001 | N/A | N/A |
| 437 | 0.002 | N/A | N/A |
| 438 | 0.004 | N/A | N/A |
| 439 | 5.845 | N/A | N/A |
| 440 | 14.321 | N/A | N/A |
| 441 | 0.529 | N/A | N/A |
| 442 | 1.765 | N/A | N/A |
| 443 | 1.125 | N/A | N/A |
| 444 | 2.458 | N/A | N/A |
| 445 | 20.423 | N/A | N/A |
| 446 | 4.880 | N/A | N/A |
| 447 | 1.934 | N/A | N/A |
| 448 | 14.064 | N/A | N/A |
| 449 | 0.005 | N/A | N/A |
| 450 | 0.016 | N/A | N/A |
| 451 | 0.009 | N/A | N/A |
| 452 | 0.008 | N/A | N/A |
| 453 | 0.060 | N/A | N/A |
| 454 | 0.003 | N/A | N/A |
| 455 | 0.007 | N/A | N/A |
| 456 | 0.023 | N/A | N/A |
| 457 | 0.007 | N/A | N/A |
| 458 | 0.014 | N/A | N/A |
| 459 | 0.006 | N/A | N/A |
| 461 | 0.001 | N/A | N/A |
| 462 | 0.0002 | N/A | N/A |
| 464 | 0.006 | N/A | N/A |
| 465 | 0.001 | N/A | N/A |
| 466 | 0.017 | N/A | N/A |
| 467 | 0.036 | N/A | N/A |
| 468 | 0.004 | N/A | N/A |
| 469 | 0.031 | N/A | N/A |
| 470 | 0.107 | N/A | N/A |
| 471 | 0.156 | N/A | N/A |
| 472 | 0.019 | N/A | N/A |
| 473 | 0.024. | N/A | N/A |
| 474 | 0.013 | N/A | N/A |
| 475 | 0.009 | N/A | N/A |
| 477 | 0.072 | N/A | N/A |
| 478 | 0.011 | N/A | N/A |
| 481 | 0.00021 | N/A | N/A |
| 482 | 0.011 | N/A | N/A |
| 483 | 0.036 | N/A | N/A |
| 485 | 0.005 | N/A | N/A |
| 486 | 0.004 | N/A | N/A |
| 487 | 0.016 | N/A | N/A |
| 488 | 2.023 | N/A | N/A |
| 490 | 0.051 | N/A | N/A |
| 491 | 0.015 | N/A | N/A |
| 492 | 0.061 | N/A | N/A |
| 496 | 0.033 | N/A | N/A |
| 497 | 0.007 | N/A | N/A |
| 498 | 0.016 | N/A | N/A |
| 499 | 0.003 | N/A | N/A |
| 500 | 0.005 | N/A | N/A |
| 501 | 0.089 | N/A | N/A |
| 502 | 0.021 | N/A | N/A |
| 503 | 0.012 | N/A | N/A |
| 504 | 0.084 | N/A | N/A |
| 505 | 0.076 | N/A | N/A |
| 506 | 0.083 | N/A | N/A |
| 507 | 0.030 | N/A | N/A |
| 508 | 0.078 | N/A | N/A |
| 509 | 0.261 | N/A | N/A |
| 510 | 0.300 | N/A | N/A |
| 511 | 0.083 | N/A | N/A |
| 512 | 0.215 | N/A | N/A |
| 513 | 0.125 | N/A | N/A |
| 514 | 0.023 | N/A | N/A |
| 516 | 0.005 | N/A | N/A |
| 517 | 0.008 | N/A | N/A |
| 518 | 0.005 | N/A | N/A |
| 519 | 0.036 | N/A | N/A |
| 520 | 0.073 | N/A | N/A |
| 521 | 0.001 | N/A | N/A |
| 522 | 0.020 | N/A | N/A |
| 523 | 0.737 | N/A | N/A |
| 524 | 4.095 | N/A | N/A |
| 525 | 0.007 | N/A | N/A |
| 526 | 0.010 | N/A | N/A |
| 527 | 0.027 | N/A | N/A |
| 528 | 0.040 | N/A | N/A |
| 529 | 0.002 | N/A | N/A |
| 530 | 0.179 | N/A | N/A |
| 531 | 0.020 | N/A | N/A |
| 533 | 0.051 | N/A | N/A |
| 534 | 0.017 | N/A | N/A |
| 535 | 0.038 | N/A | N/A |
| 536 | 0.058 | N/A | N/A |
| 537 | 0.023 | N/A | N/A |
| 538 | 0.015 | N/A | N/A |
| 539 | 0.036 | N/A | N/A |
| 540 | 0.005 | N/A | N/A |
| 541 | 0.007 | N/A | N/A |
| 542 | 0.042 | N/A | N/A |
| 543 | 0.008 | N/A | N/A |
| 544 | 0.737 | N/A | N/A |
| 545 | 0.011 | N/A | N/A |
| 546 | 0.005 | N/A | N/A |
| 547 | 0.004 | N/A | N/A |
| 548 | 0.406 | N/A | N/A |
| 549 | 0.010 | N/A | N/A |
| 550 | 0.205 | N/A | N/A |
| 551 | 0.028 | N/A | N/A |
| 552 | 0.084 | N/A | N/A |
| 553 | 0.00048 | N/A | N/A |
| 554 | 0.006 | N/A | N/A |
| 555 | 0.226 | N/A | N/A |
| 556 | 0.110 | | |
| 557 | 0.082 | N/A | N/A |
| 558 | 0.036 | N/A | N/A |
| 559 | 0.006 | N/A | N/A |
| 560 | 0.027 | N/A | N/A |
| 561 | 0.021 | N/A | N/A |
| 562 | 0.012 | N/A | N/A |
| 563 | 0.022 | N/A | N/A |
| 564 | 0.098 | N/A | N/A |
| 565 | 0.022 | N/A | N/A |
| 566 | 0.023 | N/A | N/A |
| 567 | 0.021 | N/A | N/A |
| 568 | 0.056 | N/A | N/A |
| 569 | 0.059 | N/A | N/A |
| 570 | 0.016 | N/A | N/A |
| 571 | 0.016 | N/A | N/A |
| 572 | 0.050 | N/A | N/A |
| 573 | 0.057 | N/A | N/A |
| 574 | 0.015 | N/A | N/A |
| 575 | 0.036 | N/A | N/A |
| 576 | 0.073 | N/A | N/A |
| 577 | 0.036 | N/A | N/A |
| 578 | 0.097 | N/A | N/A |
| 579 | 4.728 | N/A | N/A |
| 580 | 0.016 | N/A | N/A |
| 581 | 0.023 | N/A | N/A |
| 582 | 0.092 | N/A | N/A |
| 583 | 0.061 | N/A | N/A |
| 584 | 0.043 | N/A | N/A |
| 585 | 0.088 | N/A | N/A |
| 586 | 0.0087 | N/A | N/A |
| 587 | 0.036 | N/A | N/A |
| 588 | 0.057 | N/A | N/A |
| 589 | 0.1913 | N/A | N/A |
| 590 | 0.972 | N/A | N/A |
| 591 | 0.0197 | N/A | N/A |
| 592 | 0.0069 | N/A | N/A |
| 593 | 0.1205 | N/A | N/A |
| 594 | 0.038 | N/A | N/A |
| 595 | 0.046 | N/A | N/A |

## Claims

1. A compound of formula **I** or formula **I**': 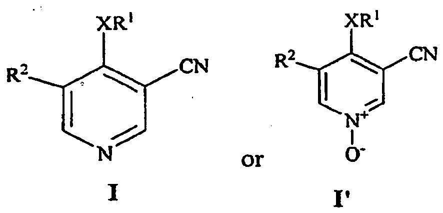 or a pharmaceutically acceptable salt, hydrate, or ester thereof, wherein:
X is selected from a) -NR³-Y- b) -O-Y-, c) -S(O)ₘ-Y-, d) -S(O)ₘNR³-Y-, e) -NR³S(O)ₘ-Y-, f) -CC(O)R³-Y-, g) -C(S)NR³-Y-, h) -NR³C(O)-Y-, i) NR³C(S)-Y-, j) -C(O)O-Y-, k) -OC(O)-Y-, and l) a covalent bond;
Y, at each occurrence, independently is selected from a) a divalent C₁₋₁₀ alkyl group, b) a divalent C₂₋₁₀ alkenyl group, c) a divalent C₂₋₁₀ alkynyl group, d) a divalent C₁₋₁₀ haloalkyl group, and e) a covalent bond;
R¹ is selected from a) a C₁₋₁₀ alkyl group, b) a C₃₋₁₄ cycloalkyl group, c) a 3-14 membered cycloheteroalkyl group, d) a C₈₋₁₄ polycyclic aryl group and e) a 5-14 membered heteroaryl group, wherein each group optionally is substituted with 1-4 -Y-R⁴;
R² is a C₆₋₁₄ aryl group or a 5-14 membered heteroaryl group, wherein each group optionally is substituted with 1-4 groups independently selected from -Y-R⁴ or -O-Y-R⁴;
R³ is selected from a) H, b) a C₁₋₁₀ alkyl group, c) a C₂₋₁₀ alkenyl group, d) a C₂₋₁₀ alkynyl group, and e) a C₁₋₁₀ haloalkyl group;
R⁴, at each occurrence, independently is selected from a) halogen, b) -CN, c) -NO₂, d) oxo, e) -O-Y-R⁵, f) -NR⁶-Y-R⁷, g) -N(O)R⁶-Y-R⁷, h) -S(O)ₘ-Y-R⁵, i) -S(O)ₘO-Y-R⁵, j) -S(O)ₘNR⁶-Y-R⁷, k) -C(O)-Y-R⁵, 1) -C(O)O-Y-R⁵, m) -C(O)NR⁶-Y-R⁷, n) -C(S)NR⁶-Y-R⁷, o) a C₁₋₁₀ alkyl group, p) a C₂₋₁₀ alkenyl group, q) a C₂₋₁₀ alkynyl group, r) a C₁₋₁₀ haloalkyl group, s) a C₃₋₁₄ cycloalkyl group, t) a C₆₋₁₄ aryl group, u) a 3-14 membered cycloheteroalkyl group, and v) a 5-14 membered heteroaryl group, wherein each of o) - v) optionally is substituted with 1-4 -Y-R⁸ groups;
R⁵, at each occurrence, independently is selected from a) H, b) -C(O)R⁹, c)-C(O)OR⁹, d) a C₁₋₁₀ alkyl group, e) a C₂₋₁₀ alkenyl group, f) a C₂₋₁₀ alkynyl group, g) a C₁₋₁₀ haloalkyl group, h) a C₃₋₁₄ cycloalkyl group, i) a C₆₋₁₄ aryl group, j) a 3-14 membered cycloheteroalkyl group, and k) a 5-14 membered heteroaryl group, wherein each of d) - k) optionally is substituted with 1-4-Y-R⁸ groups;
R⁶ and R⁷, at each occurrence, independently are selected from a) H, b) -O-Y-R⁹, c) -S(O)ₘ-Y-R⁹, d) -S(O)ₘO-Y-R⁹, e) -C(O)-Y-R⁹, f) -C(O)O-Y-R⁹, g) -C(O)NR¹⁰-Y-R¹¹, h) -C(S)NR¹⁰-Y-R¹¹, i) a C₁₋₁₀ alkyl group, j) a C₂₋₁₀ alkenyl group, k) a C₂₋₁₀ alkynyl group, 1) a C₁₋₁₀ haloalkyl group, m) a C₃₋₁₄ cycloalkyl group, n) a C₆₋₁₄ aryl group, o) a 3-14 membered cycloheteroalkyl group, and p) a 5-14 membered heteroaryl group; wherein each of i) - p) optionally is substituted with 1-4 -Y-R⁸ groups;
R⁸, at each occurrence, independently is selected from a) halogen, b) -CN, c) NO₂, d) oxo, e) -O-Y-R⁹, f) -NR¹⁰-Y-R¹¹, g) -N(O)R¹⁰-Y-R¹¹, h) - S(O)ₘ-Y-R⁹, i) -S(O)ₘO-Y-R⁹,j) -S(O)ₘNR¹⁰-Y-R¹¹, k) -C(O)-Y-R⁹, l) -C(O)O-Y-R⁹, m) -C(O)NR¹⁰-Y-R¹¹, n) -C(S)NR¹⁰-Y-R¹¹, o) a C₁₋₁₀ alkyl group, p) a C₂₋₁₀ alkenyl group, q) a C₂₋₁₀ alkynyl group, r) a C₁₋₁₀ haloalkyl group, s) a C₃₋₁₄ cycloalkyl group, t) a C₆₋₁₄ aryl group, u) a 3-14 membered cycloheteroalkyl group, and v) a 5-14 membered heteroaryl group, wherein each of o) - v) optionally is substituted with 1-4-Y-R¹² groups;
R⁹, at each occurrence, independently is selected from a) H, b) -C(O)-C₁₋₁₀ alkyl, c) -C(O)OH, d) -C(O)O-C₁₋₁₀alkyl, e) a C₁₋₁₀ alkyl group, f) a C₂₋₁₀ alkenyl group, g) a C₂₋₁₀ alkynyl group, h) a C₁₋₁₀ haloalkyl group, i) a C₃₋₁₄ cycloalkyl group, j) a C₆₋₁₄ aryl group, k) a 3-14 membered cycloheteroalkyl group, and l) a 5-14 membered heteroaryl group, wherein each of the C₁₋₁₀ alkyl group, the C₂₋₁₀ alkenyl group, the C₂₋₁₀ alkynyl group, the C₁₋₁₀ haloalkyl group, the C₃₋₁₄ cycloalkyl group, the C₆₋₁₄ aryl group, the 3-14 membered cycloheteroalkyl group, and the 5-14 membered heteroaryl group optionally is substituted with 1-4 -YR¹² groups;
R¹⁰ and R¹¹, at each occurrence, independently are selected from a) H, b)-OH, c) -SH, d) NH₂, e) -NH-C₁₋₁₀ alkyl, f) -N(C₁₋₁₀ alkyl)₂, g) -S(O)ₘ-C₁₋₁₀ alkyl, h) -S(O)₂OH, i) -S(O)ₘ-OC₁₋₁₀ alkyl, j) -C(O)-C₁₋₁₀ alkyl, k)-C(O)OH, l) -C(O)-OC₁₋₁₀ alkyl, m) -C(O)NH₂, n) -C(O)NH-C₁₋₁₀ alkyl, o) -C(O)N(C₁₋₁₀ alkyl)₂, p) -C(S)NH₂, q) -C(S)NH-C₁₋₁₀ alkyl, r) -C(S)N(C₁₋₁₀ alkyl)₂, s) a C₁₋₁₀ alkyl group, t) a C₂₋₁₀ alkenyl group, u) a C₂₋₁₀ alkynyl group, v) a C₁₋₁₀ alkoxy group, w) a C₁₋₁₀ haloalkyl group, x) a C₃₋₁₄ cycloalkyl group, y) a C₆₋₁₄ aryl group, z) a 3-14 membered cycloheteroalkyl group, and aa) a 5-14 membered heteroaryl group, wherein each of the C₁₋₁₀ alkyl group, the C₂₋₁₀ alkenyl group, the C₂₋₁₀ alkynyl group, the C₁₋₁₀ alkoxy group, the C₁₋₁₀ haloalkyl group, the C₃₋₁₄ cycloalkyl group, the C₆₋₁₄ aryl group, the 3-14 membered cycloheteroalkyl group, and the 5-14 membered heteroaryl group optionally is substituted with 1-4 -Y-R¹² groups;
R¹², at each occurrence, independently is selected from a) halogen, b) -CN, c) -NO₂, d) oxo, e) -OH, f) -NH₂, g) -NH(C₁₋₁₀ alkyl), h) -N(C₁₋₁₀ alkyl)₂, i) -SH, j) -S(O)ₘ-C₁₋₁₀ alkyl, k) -S(O)₂OH, 1) -S(O)ₘ-OC₁₋₁₀ alkyl, m) -C(O)-C₁₋₁₀ alkyl, n) -C(O)OH, o) -C(O)-OC₁₋₁₀ alkyl, p) -C(O)NH₂, q) - C(O)NH-C₁₋₁₀ alkyl, r) -C(O)N(C₁₋₁₀ alkyl)₂, s) -C(S)NH₂, t) -C(S)NH-C₁₋₁₀ alkyl, u) -C(S)N(C₁₋₁₀ alkyl)₂, v) a C₁₋₁₀ alkyl group, w) a C₂₋₁₀ alkenyl group, x) a C₂₋₁₀ alkynyl group, y) a C₁₋₁₀ alkoxy group, z) a C₁₋₁₀ haloalkyl group, aa) a C₃₋₁₄ cycloalkyl group, ab) a C₆₋₁₄ aryl group, ac) a 3-14 membered cycloheteroalkyl group, and ad) a 5-14 membered heteroaryl group; and m is 0, 1, or 2.

2. The compound of claim 1 or a pharmaceutically acceptable salt, hydrate, or ester thereof, wherein X is selected from NH-, -N(CH₃)-, -NH-CH₂-,-NH-CH₂CH₂-, NH-CH₂CH₂CH₂-, -O-, and a covalent bond.

3. The compound of claim 1 or 2 or a pharmaceutically acceptable salt, hydrate, or ester thereof, wherein R¹ is a C₈₋₁₄ polycyclic aryl group or a 5-14 membered heteroaryl group, wherein each group optionally is substituted with 1-4-Y-R⁴ groups.

4. The compound of any one of claims 1-3 or a pharmaceutically acceptable salt, hydrate, or ester thereof, wherein R¹ is selected from a benzimidazolyl group, a benzodioxolyl group, a benzodioxinyl group, a benzodioxanyl group, a benzofuranyl group, a benzothienyl group, a benzoxadiazolyl group, a benzoxazolyl group, a benzisoxazolyl group, a benzothiadiazolyl group, a benzothiazolyl group, a benzisothiazolyl group, a benzo[c]isothiazolyl group, a benzo[c]thienyl group, a benzotriazolyl group, an indazolyl group, an indenyl group, an indanyl group, an indolyl group, an isobenzofuranyl group, an isoindolyl group, an isoquinolinyl group, a naphthyl group, an indolinyl group, a pyrazolyl group, a pyridinyl group, a pyrrolopyridinyl group, a pyrrolyl group, a quinolinyl group, and a tetrahydronaphthalenyl group, wherein each group optionally is substituted with 1-4 -Y-R⁴ groups.

5. The compound of any one of claims 1-4 or a pharmaceutically acceptable salt, hydrate, or ester thereof, wherein R¹ is an indolyl group or a pyrrolopyridinyl group, each of which optionally is substituted with 1-4-Y-R⁴ groups.

6. The compound of any one of claims 1-5 or a pharmaceutically acceptable salt, hydrate, or ester thereof, wherein R¹ is a 1*H-*indol-4-yl group, a 1*H-*indol-5-yl group, a 1*H*-indol-6-yl group, or a 1*H*-indol-7-yl group, wherein each group optionally is substituted with 1-4 groups independently selected from a halogen, a C₁₋₄ alkyl group, and a C₁₋₄ alkoxy group.

7. The compound of claim 1 or 2 or a pharmaceutically acceptable salt, hydrate, or ester thereof, wherein R¹ is a C₃₋₁₄ cycloalkyl group or a 3-14 membered cycloheteroalkyl group, wherein each group optionally is substituted with 1-4 -Y-R⁴ groups.

8. The compound of claim 7 or a pharmaceutically acceptable salt, hydrate, or ester thereof, wherein R¹ is selected from a cyclobutyl group, a cyclopentyl group, a cyclohexyl group, a cycloheptyl group, a pyrrolidinyl group, a piperidinyl group, a piperazinyl group, a morpholinyl group, and a thiomorpholinyl group, wherein each group optionally is substituted with 1-4 -Y-R⁴ groups.

9. The compound of any one of claims 1-8 or a pharmaceutically acceptable salt, hydrate, or ester thereof, wherein R² is selected from a phenyl group, a C₈₋₁₄ aryl group and a 5-14 membered heteroaryl group, wherein each group optionally is substituted with 1-4 groups independently selected from -Y-R⁴ and -O-Y-R⁴.

10. The compound of any one of claims 1-9 or a pharmaceutically acceptable salt, hydrate, or ester thereof, wherein R² is: 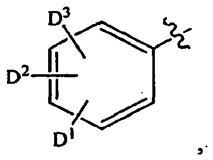 wherein D¹, D², and D³ independently are H, a -Y-R⁴ group, or an -O-Y-R⁴ group.

11. The compound of claim 10 or a pharmaceutically acceptable salt, hydrate, or ester thereof, wherein at least one of D¹, D², and D³ is a -Y-R⁴ group or an-O-Y-R⁴ group, wherein Y, at each occurrence, independently is a divalent C₁₋₄ alkyl group or a covalent bond, and R⁴, at each occurrence, independently is selected from a halogen, -CN, NO₂, -O-Y-R⁵, NR⁶-Y-R⁷, -S(O)₂-Y-R⁵, -S(O)₂NR⁶-Y-R⁷, -C(O)-Y-R⁵, -C(O)O-Y-R⁵,-C(O)NR⁶-Y-R⁷, a C₁₋₁₀ alkyl group, a C₁₋₁₀ haloalkyl group, a C₃₋₁₄ cycloalkyl group, a C₆₋₁₄ aryl group, a 3-14 membered cycloheteroalkyl group, and a 5-14 membered heteroaryl group, wherein each of the C₁₋₁₀ alkyl group, the C₁₋₁₀ haloalkyl group, the C₃₋₁₄ cycloalkyl group, the C₆₋₁₄ aryl group, the 3-14 membered cycloheteroalkyl group, and the 5-14 membered heteroaryl group is optionally substituted with 1-4 -Y-R⁸ groups.

12. The compound of claim 11 or a pharmaceutically acceptable salt, hydrate, or ester thereof, wherein the -Y-R⁴ group and the -O-Y-R⁴ group are selected from -O-(CH₂)ₙNR⁶-Y-R⁷, -(CH₂)ₙNR⁶-Y-R⁷, an -O-(CH₂)ₙ-3-14 membered cycloheteroalkyl group, and a -(CH₂)ₙ-3-14 membered cycloheteroalkyl group, wherein each of the 3-14 membered cycloheteroalkyl group optionally is substituted with 1-4 -Y-R⁸ groups, and n, at each occurrence, independently is 0, 1, 2, 3, or 4.

13. The compound of claim 12 or a pharmaceutically acceptable salt, hydrate, or ester thereof, wherein the 3-14 membered cycloheteroalkyl group of the -O-(CH₂)ₙ-3-14 membered cycloheteroalkyl group and the -(CH₂)ₙ-3-14 membered cycloheteroalkyl group is selected from a pyrrolidinyl group, a morpholinyl group, a piperazinyl group, a piperidinyl group, an azepanyl group, a diazepanyl group, and a thiomorpholinyl group.

14. The compound of claim 11 or a pharmaceutically acceptable salt, hydrate, or ester thereof, wherein the Y-R⁴ group and the -O-Y-R⁴ group are 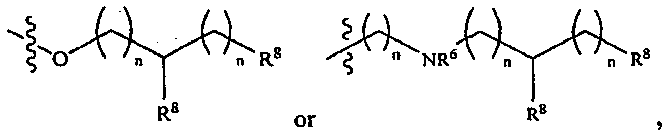 wherein R⁸, at each occurrence, independently is selected from -O-Y-R⁹,-NR¹⁰-Y-R¹¹, a C₆₋₁₄ aryl group, and a 5-14 membered heteroaryl group, wherein the C₆₋₁₄ aryl group and the 5-14 membered heteroaryl group optionally are substituted with 1-4-Y-R¹² groups, and n, at each occurrence, independently is 0, 1, 2, 3, or 4.

15. The compound of any one of claims 11-14 or a pharmaceutically acceptable salt, hydrate, or ester thereof, wherein at least one of D¹, D², and D³ is selected from a halogen, -CN, NO₂, -S(O)₂-Y-R⁵, -S(O)₂NR⁶-Y-R⁷, - C(O)O-Y-R⁵, -C(O)NR⁶-Y-R⁷, a C₁₋₁₀ alkyl group, and a C₁₋₁₀ haloalkyl group.

16. The compound of any one of claims 11-15 or a pharmaceutically acceptable salt, hydrate, or ester thereof, wherein at least one of D¹, D², and D³ is a C₆₋₁₄ aryl group or a 5-14 membered heteroaryl group, wherein each group optionally is substituted with 1-4 -Y-R⁸ groups.

17. The compound of claim 16 or a pharmaceutically acceptable salt, hydrate, or ester thereof, wherein at least one of D¹, D², and D³ is selected from a benzothienyl group, a benzofuryl group, a furyl group, a pyridyl group, a pyrimidinyl group, a pyrrolyl group, and a thienyl group, wherein each group optionally is substituted with 1-4 -Y-R⁸ groups, Y, at each occurrence, is independently a C₁₋₄ alkyl group or a covalent bond, and R⁸, at each occurrence, is independently selected from halogen, -CN, -NO₂, -O-Y-R⁹, -NR¹⁰-Y-R¹¹, -C(O)-Y-R⁹, -C(O)NR¹⁰₋Y-R¹¹, -S(O)₂-Y-R⁹, - S(O)₂NR¹⁰-Y-R¹¹, and a 3-14 membered cycloheteroalkyl group optionally substituted with a C₁₋₄ alkyl group.

18. The compound of any one of claims 1-9 or a pharmaceutically acceptable salt, hydrate, or ester thereof, wherein R² is a C₈₋₁₄ bicyclic aryl group or a 5-14 membered heteroaryl group, wherein each group optionally is substituted with 1-4 groups independently selected from -Y-R⁴ and -O-Y-R⁴.

19. The compound of claim 18 or a pharmaceutically acceptable salt, hydrate, or ester thereof, wherein R² is selected from a pyridyl group, a pyrimidyl group, a pyrazinyl group, a furyl group, a thienyl group, a thiazolyl group, an oxazolyl group, a benzofuranyl group, a benzothienyl group, an indolyl group, a benzodioxinyl group, a benzodioxolyl group, a benzodioxanyl group, a dibenzofuranyl group, a dibenzothienyl group, a benzoindolyl group, an indanyl group, an indenyl group, an isothiazolyl group, a pyridazinyl group, a pyrazolyl group, a tetrahydronaphthyl group, an isoxazolyl group, a quinolinyl group, a naphthyl group, an imidazolyl group, and a pyrrolyl group, wherein each group optionally is substituted with 1-4 groups independently selected from -(CH₂)ₙ-R⁴ and -O-(CH₂)ₙ-R⁴, wherein n, at each occurrence, independently is 0, 1, 2, 3, or 4, and R⁴, at each occurrence, independently is NR⁶-Y-R⁷ or a 3-14 membered cycloheteroalkyl group optionally substituted with a -Y-R⁸ group.

20. A compound of claim 1 selected from the following compounds:
5-(3,4-dimethoxyphenyl)-4-(1*H-*indol-5-ylamino)nicotinonitrile,
4-(2,1,3-benzothiadiazol-4-ylamino)-5-(3,4-dimethoxyphenyl)nicotinonitrile,
5-(3,4-dimethoxyphenyl)-4-(isoquinolin-5-ylamino)nicotinonitrile,
5-(3,4-dimethoxyphenyl)-4-(quinolin-5-ylamino)nicotinonitrile,
5-(3,4-dimethoxyphenyl)-4-(5,6,7,8-tetrahydronaphthalen-1-ylamino)nicotinonitrile,
4-(2,3-dihydro-1,4-benzodioxin-6-ylamino)-5-(3,4-dimethoxyphenyl)nicotinonitrile,
4-(2,3-dihydro-1*H*-inden-5-ylamino)-5-(3,4-dimethoxyphenyl)nicotinonitrile,
5-(3,4-dimethoxyphenyl)-4-(1*H*-indol-6-ylamino)nicotinonitrile,
5-(3,4-dimethoxyphenyl)-4-(1*H-*indol-4-ylamino)nicotinonitrile,
5-(3,4-dimethoxyphenyl)-4-[(2-methyl-1*H-*indol-5-yl)amino]nicotinonitrile,
4-(1,3-benzodioxol-5-ylamino)-5-(3,4-dimethoxyphenyl)nicotinonitrile,
5-(3,4-dimethoxyphenyl)-4-(2-naphthylamino)nicotinonitrile,
5-(3-bromophenyl)-4-(1*H*-indol-5-ylamino)nicotinonitrile.
5-(3-bromophenyl)-4-(1*H*-indol-4-ylamino)nicotinonitrile,
5-(2-bromophenyl)-4-(1*H*-indol-5-ylamino)nicotinonitriie,
5-(4-bromophenyl)-4-(1*H*-indol-5-ylamino)nicotinonitrile,
5-(3'-aminobiphenyl-3-yl)-4-(1*H*-indol-5-ylamino)nicotinonitrile,
5-(4'-cyanobiphenyl-3-yl)-4-(1*H-*indol-5-ylamino)nicotinonitrile,
5-(4'-aminobiphenyl-3-yl)-4-(1*H*-indol-5-ylamino)nicotinonitrile,
N-{3'-[5-cyano-4-(1*H*-indol-5-ylamino)pyridin-3-yl]biphenyl-4-yl}acetamide,
4-(1*H-*indol-5-ylamino)-5-(3-pyridin-4-ylphenyl)nicotinonitrile,
3'-[5-cyano-4-(1*H-*indol-5-ylamino)pyridin-3-yl]-N,N-dimethylbiphenyl-4-carboxamide,
3'-[5-cyano-4-(1*H*-indol-5-ylamino)pyridin-3-yl]-N-cyclopentylbiphenyl-4-carboxamide,
4-(1*H*-indol-5-ylamino)-5-[3-(1*H*-pyrrol-3-yl)phenyl]nicotinonitrile,
5-(2-bromophenyl)-4-[(7-methyl-1*H*-indol-5-yl)amino]nicotinonitrile,
5-(2-bromophenyl)-4-(1*H-*indol-4-ylamino)nicotinonitrile,
4-(1*H-*indol-5-ylamino)-5-(3'-methylbiphenyl-3-yl)nicotinonitrile,
4-(1*H*-indol-5-ylamino)-5-(4'-methylbiphenyl-3-yl)nicotinonitrile,
5-(2'-chlorobiphenyl-3-yl)-4-(1*H-*indol-5-ylamino)nicotinonitrile,
5-(3'-chlorobiphenyl-3-yl)-4-(1*H*-indol-5-ylamino)nicotinonitrile,
5-(4'-chlorobiphenyl-3-yl)-4-(1*H*-indol-5-ylamino)nicotinonitrile,
5-(3'-cyanobiphenyl-3-yl)-4-(1*H*-indol-5-ylamino)nicotinonitrile,
3'-[5-cyano-4-(1*H-*indol-5-ylamino)pyridin-3-yl]biphenyl-3-carboxylic acid,
3'-[5-cyano-4-(1*H*-indol-5-ylamino)pyridin-3-yl]biphenyl-4-carboxylic acid,
3'-[5-cyano-4-(1*H-*indol-4-ylamino)pyridin-3-yl]biphenyl-4-carboxylic acid,
4-(1*H*-indol-5-ylamino)-5-[3-(2-thienyl)phenyl]nicotinonitrile,
4-(1*H-*indol-5-ylamino)-5-(3-pyridin-3-ylphenyl)nicotinonitrile,
4-(1*H-*indol-5-ylamino)-5-(3-pyrimidin-2-ylphenyl)nicotinonitrile,
4-(1*H*-indol-5-ylamino)-5-[3-(4-methyl-2-thienyl)phenyl]nicotinonitrile,
5-[3-(5-acetyl-2-thienyl)phenyl]-4-(1*H-*indol-5-ylamino)nicotinonitrile,
4-(1*H-*indol-5-ylamino)-5-[3-(3-thienyl)phenyl]nicotinonitrile,
5-[3-(3-furyl)phenyl]-4-(1*H-*indol-5-ylamino)nicotinonitrile,
5-(2'-chlorobiphenyl-2-yl)-4-(1*H-*indol-5-ylamino)nicotinonitrile,
5-(3'-chlorobiphenyl-2-yl)-4-(1*H-*indol-5-ylamino)nicotinonitrile,
5-(4'-chlorobiphenyl-2-yl)-4-(1*H-*indol-5-ylamino)nicotinonitrile,
4-(1*H*-indol-5-ylamino)-5-[2-(3-thienyl)phenyl]nicotinonitrile,
4-(1*H*-indol-4-ylamino)-5-[3-(2-thienyl)phenyl]nicotinonitrile,
3'-[5-cyano-4-(1*H-*indol-4-ylamino)pyridin-3-yl]-N-cyclopentylbiphenyl-4-carboxamide,
4-(1*H*-indol-4-ylamino)-5-[4'-(pyrrolidin-1-ylcarbonyl)biphenyl-3-yl]nicotinonitrile,
5-[3-(5-formyl-2-thienyl)phenyl]-4-(1*H-*indol-5-ylamino)nicotinonitrile,
4-(1*H-*indol-5-ylamino)-5-(3-nitrophenyl)nicotinonitrile,
N-{3-[5-cyano-4-(1*H-*indol-5-ylamino)pyridin-3-yl]phenyl}acetamide,.
4-(1*H-*indol-4-ylamino)-5-[4-methoxy-3-(2-methoxyethoxy)phenyl]nicotinonitrile,
4-(1*H*-indol-5-ylamino)-5-[4-methoxy-3-(2-methoxyethoxy)phenyl]nicotinonitrile,
4-(1*H-*indol-6-ylamino)-5-[4-methoxy-3-(2-methoxyethoxy)phenyl]nicotinonitrile,
4-(1,3-benzothiazol-6-ylamino)-5-[4-methoxy-3-(2-methoxyethoxy)phenyl]nicotinonitrile,
5-[4-methoxy-3-(2-methoxyethoxy)phenyl]-4-[(2-methyl-1*H*-indol-5-yl)amino]nicotinonitrile,
4-(1*H*-1,2,3-benzotriazol-5-ylamino)-5-[4-methoxy-3-(2-methoxyethoxy)phenyl]nicotinonitrile,
4-(1*H*-indol-4-ylamino)-5-[3-methoxy-4-(2-methoxyethoxy)phenyl]nicotinonitrile,
4-(1*H-*indol-5-ylamino)-5-[3-methoxy-4-(2-methoxyethoxy)phenyl]nicotinonitrile,
4-(1*H-*indol-6-ylamino)-5-[3-methoxy-4-(2-methoxyethoxy)phenyl]nicotinonitrile,
4-(1,3-benzothiazol-6-ylamino)-5-[3-methoxy-4-(2-methoxyethoxy)phenyl]nicotinonitrile,
5-[3-methoxy-4-(2-methoxyethoxy)phenyl]-4-[(2-methyl-1*H*-indol-5-yl)amino]nicotinonitrile,
4-(1*H-*indol-4-ylamino)-5-[3-(2-methoxyethoxy)phenyl]nicotinonitrile,
5-[3-(2-chloroethoxy)phenyl]-4-(1*H*-indol-4-ylamino)nicotinonitrile.
5-[3-(2-chloroethoxy)phenyl]-4-(1*H-*indol-6-ylamino)nicotinonitrile, 5-[3-(2-chloroethoxy)phenyl]-4-(1*H*-indol-5-ylamino)nicotinonitrile,
4-(1*H*-indo1-4-ylamino)-5-{3-[2-(4-methylpiperazin-1-yl)ethoxy]phenyl}nicotinonitrile,
4-(1*H*-indol-4-ylamino)-5-[3-(2-pyrrolidin-1-ylethoxy)phenyl]nicotinonitrile,
4-(1*H*-indol-4-ylamino)-5-[3-(2-morpholin-4-ylethoxy)phenyl]nicotinonitrile,
4-(1*H*-indol-4-ylamino)-5-[3-(2-piperidin-1-ylethoxy)phenyl]nicotinonitrile,
4-(1*H*-indol-6-ylamino)-5-[3-(2-pyrrolidin-1-ylethoxy)phenyl]nicotinonitrile,
4-(1*H*-indol-6-ylamino)-5-[3-(2-morpholin-4-ylethoxy)phenyl]nicotinonitrile,
4-(1*H*-indol-5-ylamino)-5-[3-(2-pyrrolidin-1-ylethoxy)phenyl]nicotinonitrile,
4-(1*H-*indol-5-ylamino)-5-[3-(2-morpholin-4-ylethoxy)phenyl]nicotinonitrile,
5-(3-{2-[(2-hydroxyethyl)amino]ethoxy}phenyl)-4-(1*H-*indol-5-ylamino)nicotinonitrile,
4-(1*H-*indol-5-ylamino)-5-(3-{2-[(2-pyrrolidin-1-ylethyl)amino]ethoxy}phenyl)nicotinonitrile,
5-[3-(2-chloroethoxy)-4-methoxyphenyl]-4-(1*H*-indol-5-ylamino)nicotinonitrile,
5-{3-[2-(diethylamino)ethoxy]-4-methoxyphenyl}-4-(1*H*-indol-5-ylamino)nicotinonitrile,
5-{3-[2-(diisopropylamino)ethoxy]-4-methoxyphenyl}-4-(1*H*-indol-5-ylamino)nicotinonitrile,
5-{3-[2-(benzylamino)ethoxy]-4-methoxyphenyl}-4-(1*H*-indol-5-ylamino)nicotinonitrile,
4-(1*H-*indol-5-ylamino)-5-(4-methoxy-3-{2-[(2-methoxyethyl)amino]ethoxy}phenyl)nicotinonitrile,
4-(1*H-*indol-5-ylamino)-5-(4-methoxy-3-{2-[(5-methyl-1,3,4-thiadiazol-2-yl)amino]ethoxy}phenyl)nicotinonitrile,
4-(1*H*-indol-5-ylamino)-5-(3-{5-[(4-methylpiperazin-1-yl)methyl]-2-thienyl}phenyl)nicotinonitrile,
4-(1*H*-indol-5-ylamino)-5-{3-[5-(morpholin-4-ylmethyl)-2-thienyl]phenyl}nicotinonitrile,
4-(1*H*-indol-5-ylamino)-5-{3-[5-(piperidin-1-ylmethyl)-2-thienyl]phenyl}nicotinonitrile,
5-(3-{5-[(dimethylamino)methyl]-2-thienyl}phenyl)-4-(1*H*-indol-5-ylamino)nicotinonitrile,
5-(3-bromo-4-methoxyphenyl)-4-(1*H*-indol-5-ylamino)nicotinonitrile,
4-(1*H-*indol-5-ylamino)-5-[4-methoxy-3-(2-thienyl)phenyl]nicotinonitrile,
5-(4'-chloro-6-methoxybiphenyl-3-yl)-4-(1*H-*indol-5-ylamino)nicotinonitrile,
5-(3'-chloro-6-methoxybiphenyl-3-yl)-4-(1*H-*indol-5-ylamino)nicotinonitrile,
5-[5-cyano-4-(1*H*-indol-5-ylamino)pyridin-3-yl]-N-cyclopentyl-2'-methoxybiphenyl-4-carboxamide,
5-(2'-chloro-6-methoxybiphenyl-3-yl)-4-(1*H*-indol-5-ylamino)nicotinonitrile,
5-[3-(benzyloxy)phenyl]-4-(1*H*-indol-5-ylamino)nicotinonitrile,
5-[4-(benzyloxy)phenyl]-4-(1*H*-indol-5-ylamino)nicotinonitrile,
3'-[5-cyano-4-(1*H-*indol-5-ylamino)pyridin-3-yl]-N-methylbiphenyl-4-carboxamide,
N-butyl-3'-[5-cyano-4-(1*H*-indol-5-ylamino)pyridin-3-yl]biphenyl-4-carboxamide,
3'-[5-cyano-4-(1*H*-indol-5-ylamino)pyridin-3-yl]-N-(1-ethylpropyl)biphenyl-4-carboxamide,
3'-[5-cyano-4-(1*H*-indol-5-ylamino)pyridin-3-yl]-N-(2-hydroxyethyl)biphenyl-4-carboxamide,
3'-[5-cyano-4-(1*H*-indol-5-ylamino)pyridin-3-yl]-N-(2-methoxyethyl)biphenyl-4-carboxamide,
3'-[5-cyano-4-(1*H-*indol-5-ylamino)pyridin-3-yl]-N-cyclopropylbiphenyl-4-carboxamide,
3'-[5-cyano-4-(1*H*-indol-5-ylamino)pyridin-3-yl]-N-cyclohexylbiphenyl-4-carboxamide,
3'-[5-cyano-4-(1*H*-indol-5-ylamino)pyridin-3-yl]-N-(2-pyrrolidin-1-ylethyl)biphenyl-4-carboxamide,
N-benzyl-3'-[5-cyano-4-(1*H-*indol-5-ylamino)pyridin-3-yl]biphenyl-4-carboxamide,
4-(1*H*-indol-5-ylamino)-5-[4'-(pyrrolidin-1-ylcarbonyl)biphenyl-3-yl]nicotinonitrile,
4-(1*H*-indol-5-ylamino)-5-[4'-(morpholin-4-ylcarbonyl)bipbenyl-3-yl]nicotinonitrile,
4-(1*H*indol-5-ylamino)-5-{4'-[(4-methylpiperazin-1-yl)carbonyl]biphenyl-3-yl]nicotinonitrile,
3'-[5-eyano-4-(1*H*-indol-5-ylamino)pyridin-3-yl]-N-cyclopentylbiphenyl-3-carboxamide,
3'-[5-cyano-4-(1*H*-indol-5-ylamino)pyridin-3-yl]-N-(4-hydroxybutyl)biphenyl-4-carboxamide,
3'-[5-cyano-4-(1*H*-indol-5-ylamino)pyridin-3-yl]-N-(3-hydroxypropyl)biphenyl-4-carboxamide,
3'-[5-cyano-4-(1*H-*indol-5-ylamino)pyridin-3-yl]-N-[2-(methylamino)ethyl]biphenyl-4-carboxamide,
3'-[5-cyano-4-(1*H-*indol-5-ylamino)pyridin-3-yl]-N-(pyridin-2-ylmethyl)biphenyl-4-carboxamide,
3'-[5-cyano-4-(1*H*-indol-5-ylamino)pyridin-3-yl]-N-(pyridin-3-ylmethyl)biphenyl-4-carboxamide,
3'-[5-cyano-4-(1*H*-indol-5-ylamino)pyridin-3-yl]-N-(pyridin-4-ylmethyl)biphenyl-4-carboxamide,
N-butyl-3'-[5-cyano-4-(1*H-*indol-4-ylamino)pyridin-3-yl]biphenyl-4-carboxamide,
3'-[5-cyano-4-(1*H-*indol-4-ylarnino)pyridin-3-yl]-N-(2-hydroxyethyl)biphenyl-4-carboxamide,
5-(3,4-dimethoxyphenyl)-4-[(7-methyl-1*H*-indol-5-yl)amino]nicotinonitrile,
5-(3,4-dimethoxyphenyl)-4-[(4-methyl-1*H*-indol-5-yl)amino]nicotinonitrile,
5-(3,4-dimethoxyphenyl)-4-[1*H*-indol-5-yl(methyl)amino]nicotinonitrile,
5-(3,4-dimethoxyphenyl)-4-(1*H-*indol-5-yloxy)nicotinonitrile,
5-(3,4-dimethoxyphenyl)-4-(1*H*-indol-5-yl)nicotinonitrile,
5-(1-benzofuran-2-yl)-4-[(4-methyl-1*H-*indol-5-yl)amino]nicotinonitrile,
5-(5-formyl-1-benzothien-2-yl)-4-(1*H*-indol-4-ylamino)nicotinonitrile,
5-{5-[(dimethylamino)methyl]-1-benzothien-2-yl}-4-(1*H*-indol-4-ylamino)nicotinonitrile,
5-(4-hydroxyphenyl)-4-(1*H*-indol-4-ylamino)nicotinonitrile,
5-(4-{[(2*S*)-2-amino-3-phenylpropyl]oxy}phenyl)-4-(1*H*-indol-4-ylamino)nicotinonitrile,
5-(5-formyl-1-benzothien-2-yl)-4-[(4-methyl-1*H*-indol-5-yl)amino]nicotinonitrile,
4-[(4-methyl-1*H*-indol-5-yl)amino]-5-[5-(piperidin-1-ylmethyl)-1-benzothien-2-yl]nicotinonitrile,
5-[5-(hydroxymethyl)-1-benzothien-2-yl]-4-[(4-methyl-1*H*-indol-5-yl)amino]nicotinonitrile,
4-(1*H*-indol-5-ylamino)-5-[4-methoxy-3-(2-morpholin-4-ylethoxy)phenyl]nicotinonitrile,
4-(1*H*-indol-5-ylamino)-5-{4-methoxy-3-[2-(4-methylpiperazin-1-yl)ethoxy]phenyl}nicotinonitrile,
4-(1*H*-indol-5-ylamino)-5-[4-methoxy-3-(2-piperazin-1-ylethoxy)phenyl]nicotinonitrile,
4-(1*H*-indol-5-ylamino)-5-[4-methoxy-3-(2-thiomorpholin-4-ylethoxy)phenyl]nicotinonitrile,
5-{3-[2-(4-ethylpiperazin-1-yl)ethoxy]-4-methoxyphenyl}-4-(1*H-*indol-5-ylamino)nicotinonitrile,
4-(1*H*-indol-5-ylamino)-5-[4-methoxy-3-(2-piperidin-1-ylethoxy)phenyl]nicotinonitrile,
5-{3-[2-(dimethylamino)ethoxy]-4-methoxyphenyl}-4-(1*H*-indol-5-ylamino)nicotinonitrile,
5-(3-{2-[bis(2-hydroxyethyl)amino]ethoxy}-4-methoxyphenyl)-4-(1*H*-indol-5-ylamino)nicotinonitrile,
5-{3-[2-(4-hydroxypiperidin-1-yl)ethoxy]-4-methoxyphenyl}-4-(1*H*-indol-5-ylamino)nicotinonitrile,
4-(1*H-*indol-5-ylamino)-5-(4-methoxy-3-{2-[(pyridin-3-ylmethyl)amino]ethoxy}phenyl)nicotinonitrile,
4-(1*H-*indol-5-ylamino)-5-(4-methoxy-3-{2-[(pyridin-4-ylmethyl)amino]ethoxy}phenyl)nicotinonitrile,
4-(1*H*-indol-5-ylamino)-5-(4-methoxy-3-{2-[(pyridin-2-ylmethyl)amino]ethoxy}phenyl)nicotinonitrile,
4-(1*H*-indol-5-ylamino)-5-(4-methoxy-3-{2-[(2-phenylethyl)amino]ethoxy}phenyl)nicotinonitrile,
5-{3-[2-(cyclopentylamino)ethoxy]-4-methoxyphenyl}-4-(1*H*-indol-5-ylamino)nicotinonitrile,
5-{3-[2-(cyclohexylamino)ethoxy]-4-methoxyphenyl}-4-(1*H*-indol-5-ylamino)nicotinonitrile,
5-(3-{2-[(2-furylmethyl)amino]ethoxy}-4-methoxyphenyl)-4-(1*H*-indol-5-ylamino)nicotinonitrile,
5-(2-bromo-4,5-dimethoxyphenyl)-4-(1*H-*indol-5-ylamino)nicotinonitrile,
5-(2-bromo-4,5-dimethoxyphenyl)-4-[(4-methyl-1*H*-indol-5-yl)amino]nicotinonitrile,
4-(1*H*-indol-4-ylamino)-5-[4-methoxy-3-(2-piperidin-1-ylethoxy)phenyl]nicotinonitrile,
5-(3-{2-[(2-hydroxyethyl)amino]ethoxy}-4-methoxyphenyl)-4-(1*H*-indol-4-ylamino)nicotinonitrile,
4-(1*H*-indol-5-ylamino)-5-(4-methoxy-3-{2-[(3-phenylpropyl)amino]ethoxy}phenyl)nicotinonitrile,
4-(1*H-*indol-5-ylamino)-5-(4-methoxy-3-{2-[(2-pyridin-2-ylethyl)amino]ethoxy}phenyl)nicotinonitrile,
4-(1*H*-indol-5-ylamino)-5-(4-methoxy-3-{2-[(2-pyridin-3-ylethyl)amino]ethoxy}phenyl)nicotinonitrile,
4-(1*H*-indol-5-ylamino)-5-(4-methoxy-3-{2-[(2-pyridin-4-ylethyl)amino]ethoxy}phenyl)nicotinonitrile,
5-[3-(dimethylamino)phenyl]-4-(1*H*-indol-4-ylamino)nicotinonitrile,
4-(1*H*-indol-4-ylamino)-5-[3-(methylsulfonyl)phenyl]nicotinonitrile,
N-{3-[5-cyano-4-(1*H*-indol-4-ylamino)pyridin-3-yl]phenyl}methanesulfonamide,
4-(1*H*-indol-5-ylamino)-5-phenylnicotinonitrile,
4-(1*H*-indol-5-ylamino)-5-(3-thienyl)nicotinonitrile,
4-(1*H*-indol-5-ylamino)-3,3-bipyridine-5-carbonitrile,
5-(1,3-benzodioxol-5-yl)-4-(1*H*-indol-5-ylamino)nicotinonitrile,
4-(1*H*-indol-5-ylamino)-3)4,-bipyridine-5-carbonitrile,
5-(3-furyl)-4-(1*H*-indol-5-ylamino)nicotinonitrile,
5-(1*H*-indol-5-yl)-4-(1*H*-indol-5-ylamino)nicotinonitrile,
5-(2,3-dihydro-1,4-henzodioxin-6-yl)-4-(1*H*-indol-5-ylamino)nicotinonitrile,
4-(1*H*-indol-5-ylamino)-5-pyrimidin-5-ylnicotinonitrile,
4-(1*H*-indol-5-ylamino)-5 -(2-methoxypyrimidin-5-yl)nicotinonitrile, 4-(1*H*-indol-5-ylamino)-5-(2-thienyl)nicotinonitrile,
5-(1-benzofuran-2-yl)-4-(1*H*-indol-5-ylamino)nicotinonitrile,
5-(3,5-dimethylisoxazol-4-yl)-4-(1*H*-indol-5-ylamino)nicotinonitrile,
5-[3-(hydroxymethyl)phenyl]-4-(1*H*-indol-4-ylamino)nicotinonitrile,
5-{3-[(dimethylamino)methyl]phenyl}-4-(1*H*-indol-4-ylamino)nicotinonitrile,
4-(1*H*-indol-4-ylamino)-5-{5-[(prop-2-yn-1-ylamino)methyl]-1-benzothien-2-yl}nicotinonitrile,
5-{5-[(butylamino)methyl]-1-benzothien-2-yl}-4-(1*H*-indol-4-ylamino)nicotinonitrile,
5-(5-{[(2-hydroxyethyl)amino]methyl)-1-benzothien-2-yl)-4-(1*H*-indol-4-ylamino)nicotinonitrile,
5-(5-{[(3-hydroxypropyl)amino]methyl}-1-benzothien-2-yl)-4-(1*H-*indol-4-ylamino)nicotinonitrile,
4-(1*H-*indol-4-ylamino)-5-(5-{[(3-methoxypropyl)amino]methyl}-1-benzothien-2-yl)nicotinonitrile,
5-(5-{[(4-hydroxybutyl)amino]methyl}-1-benzothien-2-yl)-4-(1*H*-indol-4-ylamino)nicotinonitrile,
5-{5-[(cyclopropylamino)methyl]-1-benzothien-2-yl}-4-(1*H*-indol-4-ylamino)nicotinonitrile,
5-(5-{[(cyclopropylmethyl)amino]methyl}-1-benzothien-2-yl)-4-(1*H*-indol-4-ylamino)nicotinonitrile,
4-(1*H*-indol-4-ylamino)-5-[5-(pyrrolidin-1-ylmethyl)-1-benzothien-2-yl]nicotinonitrile,
4-(1*H*-indol-4-ylamino)-5-[5-(moxpholin-4-ylmethyl)-1-benzothien-2-yl]nicotinonitrile,
4-(1*H*-indol-4-ylamino)-5-(5-{[(2-morpholin-4-ylethyl)amino]methyl}-1-benzothien-2-yl)nicotinonitrile,
4-(1*H*-indol-4-ylamino)-5-[5-(piperidin-1-ylmethyl)-1-benzothien-2-yl]nicotinonitrile,
5-(5-{[4-(hydroxymethyl)piperidin-1-yl]methyl}-1-benzothien-2-yl)-4-(1*H-*indol-4-ylamino)nicotinonitrile,
5-[5-(hydroxymethyl)-1-benzothien-2-yl]-4-(1*H*-indol-4-ylamino)nicotinonitrile,
5-{5-[(benzylamino)methyl]-1-benzothien-2-yl}-4-(1*H*-indol-4-ylamino)nicotinonitrile,
4-(1*H*-indol-4-ylamino)-5-(5-{[(2-phenylethyl)amino]methyl}-1-benzothien-2-yl)nicotinonitrile,
4-(1*H*-indol-4-ylamino)-5-(5-{[(pyridin-2-ylmethyl)amino]methyl}-1-benzothien-2-yl)nicotinonitrile,
4-(1*H*-indol-4-ylamino)-5-(5-{[(pyridin-3-ylmethyl)amino]methyl}-1-benzothien-2-yl)nicotinonitrile,
4-(1*H*-indol-4-ylamino)-5-(5-{[(pyridin-4-ylmethyl)amino]methyl}-1-benzothien-2-yl)nicotinonitrile,
5-[4-(dimethylamino)phenyl]-4-(pyridin-3-ylamino)nicotinonitrile,
5-[4-(dimethylamino)phenyl]-4-(1*H*-indazol-5-ylamino)nicotinonitrile,
5-[4-(dimethylamino)phenyl]-4-(1*H*-indazol-6-ylamino)nicotinonitrile,
5-[4-(dimethylamino)phenyl]-4-[(5-hydroxy-1*H-*pyrazol-3-yl)amino]nicotinonitrile,
4-(1*H*-indazol-5-ylamino)-5-(3-methoxyphenyl)nicotinonitrile,
4-(1*H*-indazol-6-ylamino)-5-(3-methoxyphenyl)nicotinonitrile,
4-[(5-hydroxy-1*H*-pyrazol-3-yl)amino]-5-(3-methoxyphenyl)nicotinonitrile,
5-(3-bromophenyl)-4-[(4-methyl-1*H*-indol-5-yl)amino]nicotinonitrile,
4-(1*H*-indol-4-ylamino)-5-[4-methoxy-3-(2-morpholin-4-ylethoxy)phenyl]nicotinonitrile,
4-(1*H*-indol-4-ylamino)-5-{4-methoxy-3-[2-(4-methylpiperazin-1-yl)ethoxy]phenyl}nicotinonitrile,
5-{3-[2-(dimethylamino)ethoxy]-4-methoxyphenyl}-4-(1*H*-indol-4-ylamino)nicotinonitrile,
5-{3-[2-(4-hydroxypiperidin-1-yl)ethoxy]-4-methoxyphenyl}-4-(1*H*-indol-4-ylamino)nicotinonitrile,
5-[3-(2-chloroethoxy)-4-methoxyphenyl]-4-(1*H*-indol-4-ylamino)nicotinonitrile,
4-[(4-methyl-1*H*-indol-5-yl)amino]-5-[3-(2-thienyl)phenyl]nicotinonitrile,
5-(3,4-dimethoxyphenyl)-4-[(4-ethyl-1*H*-indol-5-yl)amino]nicotinonitrile,
5-[3-(5-formyl-2-thienyl)phenyl]-4-[(4-methyl-1*H*-indol-5-yl)amino]nicotinonitrile,
5-(3-{5-[(dimethylamino)methyl]-2-thienyl}phenyl)-4-[(4-methyl-1*H*-indol-5-yl)amino]nicotinonitrile,
3'-[5-cyano-4-(1*H*-indol-4-ylamino)pyridin-3-yl]-*N*-(4-hydroxybutyl)biphenyl-4-carboxamide,
3'-{5-cyano-4-[(4-methyl-1*H-*indol-5-yl)amino]pyridin-3-yl}-*N-*(4-hydroxybutyl)biphenyl-4-carboxamide,
4-(1*H*-indol-4-ylamino)-5-[3-(trifluoromethyl)phenyl]nicotinonitrile,
5-(3-cyanophenyl)-4-(1*H*-indol-4-ylamino)nicotinonitrile,
3-[5-cyano-4-(1*H*-indol-4-ylamino)pyridin-3-yl]-*N*,*N-*dimethylbenzamide,
3-[5-cyano-4-(1*H*-indol-4-ylamino)pyridin-3-yl]-*N,N-*dimethylbenzenesulfonamide,
3-[5-cyano-4-(1*H*-indol-4-ylamino)pyridin-3-yl]benzamide,
5-(3-{5-[(dimethylamino)methyl]-2-thienyl}phenyl)-4-(1*H*-indol-4-ylamino)nicotinonitrile,
2-[5-cyano-4-(1*H*-indol-4-ylamino)pyridin-3-yl]-*N,N-*dimethylbenzenesulfonamide,
*N*-{4-[5-cyano-4-(1*H*-indol-4-ylamino)pyridin-3-yl]phenyl}methanesulfonamide,
5-(1-benzofuran-2-yl)-4-(1*H*-indol-4-ylamino)nicotinonitrile,
5-dibenzo[*b,d*]furan-4-yl-4-(1*H*-indol-4-ylamino)nicotinonitrile,
4-(1*H*-indol-4-ylamino)-5-{1-[(4-methylphenyl)sulfonyl]-1*H*-indol-3-yl}nicotinonitrile,
5-(1-benzothien-2-yl)-4-(1*H*-indol-4-ylamino)nicotinonitrile,
4-(1*H*-indol-4-ylamino)-5-(4-methoxyphenyl)nicotinonitrile,
4-(1*H*-indol-4-ylamino)-5-(2-methoxyphenyl)nicotinonitrile,
5-(1*H*-indol-2-yl)-4-(1*H*-indol-4-ylamino)nicotinonitrile,
4-[5-cyano-4-(1*H*-indol-4-ylamino)pyridin-3-yl]-*N,N-*dimethylbenzenesulfonamide,
3-[5-cyano-4-(1*H*-indol-4-ylamino)pyridin-3-yl]benzoic acid,
5-[3-(aminomethyl)phenyl]-4-(1*H*-indol-4-ylamino)nicotinonitrile,
5-(3,4-dimethoxyphenyl)-4-[(2-oxo-2,3-dihydro-1*H*-indol-4-yl)amino]nicotinonitrile,
4-[5-cyano-4-(1*H*-indol-4-ylamino)pyridin-3-yl]-*N*-(2-methoxyethyl)benzamide,
4-(1*H*-indol-4-ylamino)-5-(4-methoxy-3-{2-[(3-phenylpropyl)amino]ethoxy}phenyl)nicotinonitrile,
5-[4-(2-chloroethoxy)phenyl]-4-(1*H*-indol-4-ylamino)nicotinonitrile,
5-[3-(5-formyl-2-thienyl)phenyl]-4-(1*H*-indol-4-ylamino)nicotinonitrile,
4-(1*H*-indol-4-ylamino)-5-[4-(2-morpholin-4-ylethoxy)phenyl]nicotinonitrile,
4-(1*H*-indol-4-ylamino)-5-{4-[2-(4-methylpiperazin-1-yl)ethoxy]phenyl}nicotinonitrile,
5-(3,4-dimethoxyphenyl)-4-[(5-methyl-1*H*-indol-4-yl)amino]nicotinonitrile, 5-(2,4-dimethoxyphenyl)-4-(1*H*-indol-4-ylamino)nicotinonitrile,
4-(1*H*-indol-4-ylamino)-5-[4-methoxy-3-(2-{[3-(2-oxopyrrolidin-1-yl)propyl]amino}ethoxy)phenyl]nicotinonitrile,
5-[3-(2-{[2-(1*H*-imidazol-4-yl)ethyl]amino}ethoxy)-4-methoxyphenyl]-4-(1*H*-indol-4-ylamino)nicotinonitrile,
4-(1*H*-indol-4-ylamino)-5-(4-methoxy-3-{2-[(3-pyrrolidin-1-ylpropyl)amino]ethoxy}phenyl)nicotinonitrile,
4-(1*H*-indol-4-ylamino)-5-[4-methoxy-3-(2-{[2-(1-methylpyrrolidin-2-yl)ethyl]amino}ethoxy)phenyl]nicotinonitrile,
5-(4-methoxy-3-{2-[(3-phenylpropyl)amino]ethoxy}phenyl)-4-[(4-methyl-1*H*-indol-5-yl)amino]nicotinonitrile,
5-{4-methoxy-3-[2-(4-methylpiperazin-1-yl)ethoxy]phenyl}-4-[(4-methyl-1*H*-indol-5-yl)amino]nicotinonitrile,
5-(3-methoxy-4-{2-[(3-phenylpropyl)amino]ethoxy}phenyl)-4-[(4-methyl-1*H*-indol-5-yl)amino]nicotinonitrile,
5-{4-[2-(dimethylamino)ethoxy]-3-methoxyphenyl}-4-[(4-methyl-1*H*-indol-5-yl)amino]nicotinonitrile,
5-(3-bromophenyl)-4-[(2-oxo-2,3-dihydro-1*H*-indol-4-yl)amino]nicotinonitrile,
4-(1*H*-indol-4-ylamino)-5-(4-{2-[(3-phenylpropyl)amino]ethoxy}phenyl)nicotinonitrile,
5-[4-(2-chloroethoxy)phenyl]-4-[(4-methyl-1*H*-indol-5-yl)amino]nicotinonitrile,
5-(3,4-dimethoxyphenyl)-4-[(2-methyl-1*H*-indol-4-yl)amino]nicotinonitrile,
4-[(4-methyl-1*H*-indol-5-yl)amino]-5-{4-[2-(4-methylpiperazin-1-yl)ethoxy]phenyl}nicotinonitrile,
4-[(4-methyl-1*H*-indol-5-yl)amino]-5-[4-(2-morpholin-4-ylethoxy)phenyl]nicotinonitrile,
5-(1-benzofuran-3-yl)-4-(1*H*-indol-4-ylamino)nicotinonitrile,
4-(1*H*-indol-4-ylamino)-5-(4-methoxy-3-{2-[(2-phenylethyl)amino]ethoxy}phenyl)nicotinonitrile,
4-(1*H*-indol-4-ylamino)-5-(4-methoxy-3-{2-[(2-pyridin-3-ylethyl)amino]ethoxy}phenyl)nicotinonitrile,
4-[(4-methyl-1*H*-indol-5-yl)amino]-5-(4-{2-[(3-phenylpropyl)amino]ethoxy}phenyl)nicotinonitrile,
6'-[3-(dimethylamino)propoxy]-4-(1*H*-indol-4-ylamino)-3,3'-bipyridine-5-carbonitrile,
6'-[3-(dimethylamino)propoxy]-4-[(4-methyl-1*H*-indol-5-yl)amino]-3,3'-bipyridine-5-carbonitrile,
5-(3-hydroxyphenyl)-4-(1*H*-indol-4-ylamino)nicotinonitrile,
4-(1*H*-indol-4-ylamino)-5-[5-(piperazin-1-ylmethyl)-1-benzothien-2-yl]nicotinonitrile,
N-({2-[5-cyano-4-(1*H*-indol-4-ylamino)pyridin-3-yl]-1-benzothien-5-yl}methyl)-b-alaninamide,
4-(1*H*-indol-4-ylamino)-6'-[(2-morpholin-4-ylethyl)amino]-3,3'-bipyridine-5-carbonitrile,
4-[(4-methyl-1*H*-indol-5-yl)amino]-6'-[(2-morpholin-4-ylethyl)amino]-3,3'-bipyridine-5-carbonitrile,
5-{2-chloro-4-[2-(dimethylamino)ethoxy)phenyl}-4-[(4-methyl-1*H*-indol-5-yl)amino]nicotinonitrile,
4-(1*H*-indol-4-ylamino)-5-(4-methoxy-3-{2-[(3-morpholin-4-ylpropyl)amino]ethoxy}phenyl)nicotinonitrile,
5-[3-(2-{[3-(1*H*-imidazol-1-yl)propyl]amino}ethoxy)-4-methoxyphenyl]-4-(1*H*-indol-4-ylamino)nicotinonitrile,
5-(3-{[(2*S*)-2-amino-3-phenylpropyl]oxy}phenyl)-4-(1*H*-indol-4-ylamino)nicotinonitrile,
5-{5-[(benzylamino)methyl]-1-benzothien-2-yl}-4-[(4-methyl-1*H*-indol-5-yl)amino]nicotinonitrile,
5-{4-[2-(4-butylpiperazin-1-yl)ethoxy]phenyl}-4-[(4-methyl-1*H*-indol-5-yl)amino]nicotinonitrile,
5-[5-(1,3-dioxan-2-yl)-1-benzofuran-2-yl]-4-(1*H*-indol-4-ylamino)nicotinonitrile,
5-[5-(1,3-dioxan-2-yl)-1-benzofuran-2-yl]-4-[(4-methyl-1*H*-indol-5-yl)amino]nicotinonitrile,
5-(2-chloro-4-methoxyphenyl)-4-[(4-methyl-1*H*-indol-5-yl)amino]nicotinonitrile,
5-[4-(2-chloroethoxy)-3-methoxyphenyl]-4-[(4-methyl-1*H*-indol-5-yl)amino] nicotinonitrile,
5-(1-benzofuran-2-yl)-4-(1*H*-indazol-5-ylamino)nicotinonitrile, 5-(4-hydroxyphenyl)-4-[(4-methyl-1*H*-indol-5-yl)amino]nicotinonitrile,
5-(2-chloro-6-methoxyphenyl)-4-[(4-methyl-1*H*-indol-5-yl)amino]nicotinonitrile,
5-[3-methoxy-4-(2-piperidin-1-ylethoxy)phenyl]-4-[(4-methyl-1*H*-indol-5-yl)amino]nicotinonitrile,
5-{3-methoxy-4-[2-(4-methylpiperazin-1-yl)ethoxy]phenyl}-4-[(4-methyl-1*H*-indol-5-yl)amino]nicotinonitrile,
5-[3-methoxy-4-(2-morpholin-4-ylethoxy)phenyl]-4-[(4-methyl-1*H*-indol-5-yl)amino] nicotinonitrile,
5-(3,4-dimethoxyphenyl)-4-(1*H*-indazol-5-ylamino)nicotinonitrile,
5-(2,3-dichlorophenyl)-4-[(4-methyl-1*H*-indol-5-yl)amino]nicotinonitrile,
5-(4-bromo-2-fluorophenyl)-4-[(4-methyl-1*H*-indol-5-yl)amino]nicotinonitrile,
5-{5-[(dimethylamino)methyl]-1-benzofuran-2-yl}-4-[(4-methyl-1*H*-indol-5-yl)amino]nicotinonitrile,
5-(5-{[(2-hydroxyethyl)amino]methyl}-1-benzofuran-2-yl)-4-[(4-methyl-1*H-*indol-5-yl)amino]nicotinonitrile,
5-(5-{[(3-hydroxypropyl)amino]methyl}-1-benzofuran-2-yl)-4-[(4-methyl-1*H*-indol-5-yl)amino]nicotinonitrile,
5-(5-{[(2,3-dihydroxypropyl)amino]methyl}-1-benzofuran-2-yl)-4-[(4-methyl-1*H*-indol-5-yl)amino]nicotinonitrile,
5-(5-{[(2,3-dihydroxypropyl)(methyl)amino]methyl}-1-benzofuran-2-yl)-4-[(4-methyl-1*H*-indol-5-yl)amino]nicotinonitrile,
5-{5-[(cyclohexylamino)methyl]-1-benzofuran-2-yl}-4-[(4-methyl-1*H*-indol-5-yl)amino]nicotinonitrile,
4-[(4-methyl-1*H*-indol-5-yl)amino]-5-[5-(morpholin-4-ylmethyl)-1-benzofuran-2-yl]nicotinonitrile,
4-[(4-methyl-1*H*-indol-5-yl)amino]-5-[5-(pyrrolidin-1-ylmethyl)-1-benzofuran-2-yl]nicotinonitrile,
4-[(4-methyl-1*H*-indol-5-yl)amino]-5-(5-{[(2-pyrrolidin-1-ylethyl)amino]methyl}-1-benzofuran-2-yl)nicotinonitrile,
4-[(4-methyl-1*H*-indol-5-yl)amino]-5-[5-({[(1-methylpiperidin-4-yl)methyl)amino}methyl)-1-benzofuran-2-yl]nicotinonitrile,
5-(5-{[4-(hydroxymethyl)piperidin-1-yl]methyl}-1-benzofuran-2-yl)-4-[(4-methyl-1*H*-indol-5-yl)amino]nicotinonitrile,
4-[(4-methyl-1*H*-indol-5-yl)amino]-5-{5-[(4-pyrrolidin-1-ylpiperidin-1-yl)methyl]-1-benzofuran-2-yl}nicotinonitrile,
5-[5-(1,4'-bipiperidin-1'-ylmethyl)-1-benzofuran-2-yl]-4-[(4-methyl-1*H-*indol-5-yl)amino]nicotinonitrile,
4-[(4-methyl-1*H*-indol-5-yl)amino]-5-{5-[(4-morpholin-4-ylpiperidin-1-yl)methyl]-1-benzofuran-2-yl}nicotinonitrile,
5-[5-({4-[2-(dimethylamino)ethyl]piperazin-1-yl)methyl)-1-benzofuran-2-yl]-4-[(4-methyl-1*H*-indol-5-yl)amino]nicotinonitrile,
4-[(4-methyl-1*H*-indol-5-yl)amino]-5-{5-[(4-pyridin-2-ylpiperazin-1-yl)methyl]-1-benzofuran-2-yl nicotinonitrile,
4-[(4-methyl-1*H*-indol-5-yl)amino]-5-(5-{[(pyridin-2-ylmethyl)amino]methyl}-1-benzofuran-2-yl)nicotinonitrile,
4-[(4-methyl-1*H*-indol-5-yl)amino]-5-(5-{[(pyridin-3-ylmethyl)amino]methyl}-1-benzofuran-2-yl)nicotinonitrile,
4-[(4-methyl-1*H*-indol-5-yl)amino]-5-(5-{[(pyridin-4-ylmethyl)amino]methyl}-1-benzofuran-2-yl)nicotinonitrile,
4-[(4-methyl-1*H*-indol-5-yl)amino]-5-[5-(morpholin-4-ylmethyl)-2-furyl]nicotinonitrile,
4-[(4-methyl-1*H*-indol-5-yl)amino]-5-[5-(piperidin-1-ylmethyl)-2-furyl]nicotinonitrile,
5-[5-(1,4'-bipiperidin-1'-ylmethyl)-2-furyl]-4-[(4-methyl-1*H*-indol-5-yl)amino]nicotinonitrile,
4-[(4-methyl-1*H*-indol-5-yl)amino]-5-{5-[(4-pyrrolidin-1-ylpiperidin-1-yl)methyl]-2-furyl} nicotinonitrile,
4-[(4-methyl-1*H*-indol-5-yl)amino]-5-{5-[(4-morpholin-4-ylpiperidin-1-yl)methyl]-2-furyl}nicotinonitrile,
5-{5-[(diethylamino)methyl]-2-furyl}-4-[(4-methyl-1*H*-indol-5-yl)amino]nicotinonitrile,
5-{5-[(dibutylamino)methyl]-2-furyl}-4-[(4-methyl-1*H*-indol-5-yl)amino]nicotinonitrile,
5-{5-[(benzylamino)methyl]-2-furyl}-4-[(4-methyl-1*H*-indol-5-yl)amino]nicotinonitrile,
4-[(4-methyl-1*H*-indol-5-yl)amino]-5-(5-{[(3-phenylpropyl)amino]methyl}-2-furyl)nicotinonitrile,
4-[(4-methyl-1*H*-indol-5-yl)amino]-5-[5-(pyrrolidin-1-ylmethyl)-2-furyl]nicotinonitrile,
4-[(4-methyl-1*H*-indol-5-yl)amino]-5-[5-(thiomorpholin-4-ylmethyl)-2-furyl]nicotinonitrile,
5-(3,4-dimethoxyphenyl)-4-[(6-methyl-1*H*-indol-5-yl)amino]nicotinonitrile, 5-(1-benzofuran-2-yl)-4-[(6-methyl-1*H*-indol-5-yl)amino]nicotinonitrile,
5-(4-{2-[(2-hydroxyethyl)amino]ethoxy}phenyl)-4-[(4-methyl-1*H*-indol-5-yl)amino]nicotinonitrile,
5-(4-{2-[(3-hydroxypropyl)amino]ethoxy}phenyl)-4-[(4-methyl-1*H*-indol-5-yl)amino]nicotinonitrile,
5-(4-{2-[(2-ethoxyethyl)amino]ethoxy}phenyl)-4-[(4-methyl-1*H*-indol-5-yl)amino]nicotinonitrile,
5-[4-(2-{[2-(dimethylamino)ethyl]amino}ethoxy)phenyl]-4-[(4-methyl-1*H-*indol-5-yl)amino]nicotinonitrile,
4-[(4-methyl-1*H*-indol-5-yl)amino]-5-[4-(2-pyrrolidin-1-ylethoxy)phenyl]nicotinonitrile,
5-{4-[2-(benzylamino)ethoxy]phenyl}-4-[(4-methyl-1*H*-indol-5-yl)amino]nicotinonitrile,
4-[(4-methyl-1*H*-indol-5-yl)amino]-5-(4-{2-[(1-methylpiperidin-4-yl)amino]ethoxy}phenyl)nicotinonitrile,
4-[(4-methyl-1*H*-indol-5-yl)amino]-5-[4-(2-{[(1-methylpiperidin-4-yl)methyl]amino}ethoxy)phenyl]nicotinonitrile,
5-(4-{2-[4-(hydroxymethyl)piperidin-1-yl]ethoxy}phenyl)-4-[(4-methyl-1*H-*indol-5-yl)amino]nicotinonitrile,
4-[(4-methyl-1*H*-indol-5-yl)amino]-5-{4-[2-(4-pyrrolidin-1-ylpiperidin-1-yl)ethoxy]phenyl}nicotinonitrile,
4-[(4-methyl-1*H*-indol-5-yl)amino]-5-{4-[2-(4-morpholin-4-ylpiperidin-1-yl)ethoxy]phenyl}nicotinonitrile,
5-{4-[2-(4-ethylpiperazin-1-yl)ethoxy]phenyl}-4-[(4-methyl-1*H*-indol-5-yl)amino]nicotinonitrile,
5-{4-[2-(4-methyl-1,4-diazepan-1-yl)ethoxy]phenyl}-4-[(4-methyl-1*H*-indol-5-yl)amino]nicotinonitrile,
5-(4-{2-[4-(2-hydroxyethyl)piperazin-1-yl]ethoxy}phenyl)-4-[(4-methyl-1*H-*indol-5-yl)amino]nicotinonitrile,
5-[4-(2-{4-[2-(dimethylamino)ethyl]piperazin-1-yl}ethoxy)phenyl] -4-[(4-methyl-1*H*-indol-5-yl)amino]nicotinonitrile,
5-[4-(2-{[3-(1*H*-imidazol-1-yl)propyl]amino}ethoxy)phenyl]-4-[(4-methyl-1*H*-indol-5-yl)amino]nicotinonitrile,
4-[(4-methyl-1*H*-indol-5-yl)amino]-5-{4-[2-(4-pyridin-2-ylpiperazin-1-yl)ethoxy]phenyl}nicotinonitrile,
4-[(4-methyl-1*H*-indol-5-yl)amino]-5-{4-[2-(4-pyridin-4-ylpiperazin-1-yl)ethoxy]phenyl}nicotinonitrile,
4-[(4-methyl-1*H-*indol-5-yl)amino]-5-(4-{2-[(pyridin-2-ylmethyl)amino]ethoxy}phenyl)nicotinonitrile,
4-[(4-methyl-1*H*-indol-5-yl)amino]-5-(4-{2-[(pyridin-3-ylmethyl)amino]ethoxy}phenyl)nicotinonitrile,
4-[(4-methyl-1*H*-indol-5-yl)amino]-5-(4-{2-[(pyridin-4-ylmethyl)amino]ethoxy} phenyl)nicotinonitrile,
4-[(4-methyl-1*H*-indol-5-yl)amino]-5-{4-[2-(4-phenylpiperidin-1-yl)ethoxy]phenyl}nicotinonitrile,
5-(5-{[4-(dimethylamino)piperidin-1-yl]methyl}-2-furyl)-4-[(4-methyl-1*H-*indol-5-yl)amino]nicotinonitrile,
5-{5-[(4-isopropylpiperazin-1-yl)methyl]-2-furyl}-4-[(4-niethyl-1*H*-indol-5-yl)amino]nicotinonitrile,
4-[(4-methyl-1*H*-indol-5-yl)amino]-5-{5-[2-(4-methylpiperazin-1-yl)ethoxy]-1-benzofuran-2-yl}nicotinonitrile,
5-(3,4-dimethoxyphenyl)-4-{[2-(4-methylpiperazin-1-yl)ethyl]amino}nicotinonitrile,
5-(3,4-dimethoxyphenyl)-4-{[3-(4-methylpiperazin-1-yl)propyl]amino}nicotinonitrile,
4-({[*trans*-4-(aminomethyl)cyclohexyl]methyl}amino)-5-(3,4-dimethoxyphenyl)nicotinonitrile,
4-{[(*trans*-4-aminocyclohexyl)methyl]amino}-5-(3,4-dimethoxyphenyl)nicotinonitrile,
4-({[*cis-*3-(aminomethyl)cyclohexyl]methyl}amino)-5-(3,4-dimethoxyphenyl)nicotinonitrile,
5-(3,4-dimethoxyphenyl)-4-[(2-piperidin-4-ylethyl)amino]nicotinonitrile,
5-(3,4-dimethoxyphenyl)-4-[(piperidin-4-ylmethyl)amino]nicotinonitrile,
4-[(*cis*-4-aminocyclohexyl)amino]-5-(3,4-dimethoxyphenyl)nicotinonitrile,
5-(3,4-dimethoxyphenyl)-4-{[2-(1-methylpipendin-4-yl)ethyl]amino}nicotinonitrile,
5-(3,4-dimethoxyphenyl)-4-{[(1-methylpiperidin-4-yl)methyl]amino}nicotinonitrile,
4-[(4-methyl-1*H*-indol-5-yl)amino]-5-{4-[(4-methylpiperazin-1-yl)methyl]phenyl}nicotinonitrile,
4-[(4-methyl-1*H*-indol-5-yl)amino]-5-[4-(pyrrolidin-1-ylmethyl)phenyl]nicotinonitrile,
4-[(4-methyl-1*H*-indol-5-yl)amino]-5-[4-(morpholin-4-ylmethyl)phenyl]nicotinonitrile,
5-{4-[(dimethylamino)methyl]phenyl}-4-[(4-methyl-1*H*-indol-5-yl)amino]nicotinonitrile,
4-[(4-methyl-1*H*-indol-5-yl)amino]-2'-(morpholin-4-ylmethyl)-3,4'-bipyridine-5-carbonitrile,
4-[(4-methyl-1*H*-indol-5-yl)amino]-5-{3-[(4-methylpiperazin-1-yl)methyl]phenyl}nicotinonitrile,
4-[(4-methyl-1*H*-indol-5-yl)amino]-5-[3-(pyrrolidin-1-ylmethyl)phenyl]nicotinonitrile,
4-[(4-methyl-1*H*-indol-5-yl)amino]-5-[3-(morpholin-4-ylmethyl)phenyl]nicotinonitrile,
5-{3-[(dimethylamino)methyl]phenyl}-4-[(4-methyl-1*H*-indol-5-yl)amino]nicotinonitrile,
5-(4-{[(2R)-2-amino-3-phenylpropyl]oxy}phenyl)-4-(1*H*-indol-4-ylamino)nicotinonitrile,
5-{2-fluoro-4-[(4-methylpiperazin-1-yl)methyl]phenyl}-4-[(4-methyl-1*H-*indol-5-yl)amino]nicotinonitrile,
5-[4-(3-chloropropoxy)phenyl]-4-[(4-methyl-1*H*-indol-5-yl)amino]nicotinonitrile,
4-[(4-methyl-1*H*-indol-5-yl)amino]-5-[5-(piperidin-1-ylmethyl)-1-benzofuran-2-yl]nicotinonitrile,
4-[(4-methyl-1*H*-indol-5-yl)amino]-5-{5-[(4-methylpiperazin-1-yl)methyl]-1-benzofuran-2-yl}nicotinonitrile,
5-(5-formyl-2-thienyl)-4-[(4-methyl-1*H*-indol-5-yl)amino]nicotinonitrile,
4-[(4-methyl-1*H*-indol-5-yl)amino]-5-{5-[(4-methylpiperazin-1-yl)methyl]-2-thienyl}nicotinonitrile,
5-(5-formyl-1-benzofuran-2-yl)-4-[(4-methyl-1*H*-indol-5-yl)amino]nicotinonitrile,
5-(3-methyl-1-benzofuran-2-yl)-4-[(4-methyl-1*H*-indol-5-yl)amino]nicotinonitrile,
4-[(4-methyl-1*H*-indol-5-yl)amino]-3,4'-bipyridine-5-carbonitrile,
4-[(4-methyl-1*H*-indol-5-yl)amino]-5-{5-[(4-methylpiperazin-1-yl)methyl]-2-furyl}nicotinonitrile,
2'-chloro-4-[(4-methyl-1*H*-indol-5-yl)amino]-3,4'-bipyridine-5-carbonitrile,
5-{2-chloro-4-[2-(4-methylpiperazin-1-yl)ethoxy]phenyl}-4-[(4-methyl-1*H-*indol-5-yl)amino]nicotinonitrile,
2'-chloro-4-[(4-methyl-1*H*-indol-5-yl)amino]-3,3'-bipyridine-5-carbonitrile,
4-[(4-methyl-1*H*-indol-5-yl)amino]-5-{4-[3-(4-methylpiperazin-1-yl)propoxy]phenyl}nicotinonitrile,
4-[(4-methyl-1*H*-indol-5-yl)amino]-5-[4-(3-morpholin-4-ylpropoxy)phenyl]nicotinonitrile,
4-[(4-methyl-1*H*-indol-5-yl)amino]-5-[4-(3-piperidin-1-ylpropoxy)phenyl]nicotinonitrile,
5-{4-[3-(dimethylamino)propoxy]phenyl}-4-[(4-methyl-1*H*-indol-5-yl)amino]nicotinonitrile,
5-[3,4-bis(2-methoxyethoxy)phenyl]-4-[(4-methyl-1*H*-indol-5-yl)amino]nicotinonitrile,
5-[3-methoxy-4-(2-methoxyethoxy)phenyl]-4-[(4-methyl-1*H*-indol-5-yl)amino]nicotinonitrile,
5-[5-(hydroxymethyl)-1-benzofuran-2-yl]-4-[(4-methyl-1*H*-indol-5-yl)amino]nicotinonitrile,
5-[4-(2-methoxyethoxy)phenyl]-4-[(4-methyl-1*H*-indol-5-yl)amino]nicotinonitrile,
5-[3-(2-methoxyethoxy)phenyl]-4-[(4-methyl-1*H*-indol-5-yl)amino]nicotinonitrile,
5-(5-formyl-1-benzofuran-2-yl)-4-(1*H*-indol-5-ylamino)nicotinonitrile,
4-(1H-indol-5-ylamino)-5-{5-[(4-methylpiperazin-1-yl)methyl]-1-benzofuran-2-yl}nicotinonitrile,
5-{5-[(4-cyclopentylpiperazin-1-yl)methyl]-2-furyl}-4-[(4-methyl-1*H*-indol-5-yl)amino]nicotinonitrile,
5-{5-[(1,1-dioxidothiomorpholin-4-yl)methyl]-2-furyl}-4-[(4-methyl-1*H-*indol-5-yl)amino]nicotinonitrile,
5-(3,4-dimethoxyphenyl)-4-(1*H*-pyrrolo[2,3-b]pyridin-5-ylamino)nicotinonitrile,
5-(5-formyl-2-furyl)-4-[(4-methyl-1*H*-indol-5-yl)amino]nicotinonitrile,
5-[4-(4-chlorobutoxy)phenyl]-4-[(4-methyl-1*H*-indol-5-yl)amino]nicotinonitrile,
4-[(4-methyl-1*H*-indol-5-yl)amino]-5-{4-[4-(4-methylpiperazin-1-yl)butoxy]phenyl}nicotinonitrile,
4-[(4-chloro-1*H*-pyrrolo[2,3-b]pyridin-5-yl)amino]-5-[3-methoxy-4-(2-methoxyethoxy)phenyl]nicotinonitrile,
5-[4-(2-chloroethoxy)phenyl]-4-[(6-methyl-1*H*-indol-5-yl)amino]nicotinonitrile,
5-(5-formyl-1-benzofuran-2-yl)-4-[(6-methyl-1*H*-indol-5-yl)amino]nicotinonitrile,
4-[(6-methyl-1*H*-indol-5-yl)amino]-5-{4-[2-(4-methylpiperazin-1-yl)ethoxy]phenyl}nicotinonitrile,
4-[(6-methyl-1*H*-indol-5-yl)amino]-5-{5-[(4-methylpiperazin-1-yl)methyl]-1-benzofuran-2-yl}nicotinonitrile,
4-[(7-chloro-4-methyl-1*H*-indol-5-yl)amino]-5-(3,4-dimethoxyphenyl)nicotinonitrile,
5-[5-(hydroxymethyl)-1-benzofuran-2-yl]-4-[(6-methyl-1*H*-indol-5-yl)amino]nicotinonitrile,
5-{5-[(diethylamino)methyl]-1-benzofuran-2-yl}-4-[(6-methyl-1*H*-indol-5-yl)amino]nicotinonitrile,
5-[3-(4-chlorobutoxy)phenyl]-4-[(4-methyl-1*H*-indol-5-yl)amino]nicotinonitrile,
4-[(4-methyl-1*H*-indol-5-yl)amino]-5-{3-[4-(4-methylpiperazin-1-yl)butoxy]phenyl}nicotinonitrile,
4-[(4-methyl-1*H*-indol-5-yl)amino]-5-{5-[(4-methylpiperazin-1-yl)methyl]-3-furyl}nicotinonitrile,
4-[(6-methyl-1*H*-indol-5-yl)amino]-5-[4-(2-piperidin-1-ylethoxy)phenyl]nicotinonitrile,
5-{4-[2-(4-hydroxypiperidin-1-yl)ethoxy]phenyl}-4-[(6-methyl-1*H*-indol-5-yl)amino]nicotinonitrile,
4-[(6-methyl-1*H*-indol-5-yl)amino]-5-{4-[2-(4-pyrrolidin-1-ylpiperidin-1-yl)ethoxy]phenyl}nicotinonitrile,
4-[(4-methyl-1*H*-indol-5-yl)amino]-6'-morpholin-4-yl-3,3'-bipyridine-5-carbonitrile,
4-[(4-methyl-1*H*-indol-5-yl)amino]-6'-piperidin-1-yl-3,3'-bipyridine-5-carbonitrile,
4-[(4-methyl-1*H*-indol-5-yl)amino]-5-pyrimidin-5-ylnicotinonitrile,
4-[(4-methyl-1*H*-indol-5-yl)amino]-5-(2-piperidin-1-ylpyrimidin-5-yl)nicotinonitrile,
4-[(4-methyl-1*H*-indol-5-yl)amino]-5-(2-morpholin-4-ylpyrimidin-5-yl)nicotinonitrile,
4-[(4-methyl-1*H*-indol-5-yl)amino]-5-(2-pyrrolidin-1-ylpyrimidin-5-yl)nicotinonitrile,
5-[2-(dimethylamino)pyrimidin-5-yl]-4-[(4-methyl-1*H*-indol-5-yl)amino]nicotinonitrile,
5-(1-benzothien-2-yl)-4-[(4-methyl-1*H*-indol-5-yl)amino]nicotinonitrile,
5-[4-(2-chloroethoxy)phenyl]-4-(1*H*-indol-5-ylamino)nicotinonitrile,
5-(5-formyl-3-thienyl)-4-[(4-methyl-1*H*-indol-5-yl)amino]nicotinonitrile,
5-(4-formyl-2-furyl)-4-[(4-methyl-1*H*-indol-5-yl)amino]nicotinonitrile,
4-[(4-methyl-1*H*-indol-5-yl)amino]-5-{5-[(4-methylpiperazin-1-yl)methyl]-3-thienyl}nicotinonitrile,
4-[(4-methyl-1*H*-indol-5-yl)amino]-3,3'-bipyridine-5-carbonitrile,
4-(1*H*-indol-5-ylamino)-5-{4-[2-(4-methylpiperazin-1-yl)ethoxy]phenyl}nicotinonitrile,
4-[(4-methyl-1*H*-indol-5-yl)amino]-5-{5-[(4-methylpiperazin-1-yl)methyl]-1-benzothien-2-yl}nicotinonitrile,
4-[(4-chloro-1*H*-pyrrolo[2,3-b]pyridin-5-yl)amino]-5-(3,4-dimethoxyphenyl)nicotinonitrile,
4-[(*trans*-4-aminocyclohexyl)amino]-5-(3,4-dimethoxyphenyl)nicotinonitrile,
1-butyl-3-(4-{5-cyano-4-[(4-methyl-1*H*-indol-5-yl)amino]pyridin-3-yl}phenyl)urea,
methyl (4-{5-cyano-4-[(4-methyl-1*H*-indol-5-yl)amino]pyridin-3-yl}phenyl)carbamate,
benzyl (4-{5-cyano-4-[(4-methyl-1*H*-indol-5-yl)amino]pyridin-3-yl}-2-fluorophenyl)carbamate,
4-methoxybenzyl (4-{5-cyano-4-[(4-methyl-1*H*-indol-5-yl)amino]pyridin-3-yl}-2-fluorophenyl)carbamate,
4-[(7-chloro-4-methyl-1*H*-indol-5-yl)amino]-5-{5-[(4-methylpiperazin-1-yl)methyl]-1-benzofuran-2-yl}nicotinonitrile,
5-(3,4-dimethoxyphenyl)-4-[(4-methyl-1*H*-indol-7-yl)amino]nicotinonitrile,
5-(1-benzofuran-2-yl)-4-[(7-chloro-4-methyl-1*H*-indol-5-yl)amino]nicotinonitrile,
4-[(7-chloro-4-methyl-1*H*-indol-5-yl)amino]-5-(5-formyl-1-benzofuran-2-yl)nicotinonitrile,
5-[4-(2-chloroethoxy)phenyl]-4-[(7-chloro-4-methyl-1*H*-indol-5-yl)amino]nicotinonitrile,
4-[(7-chloro-4-methyl-1*H*-indol-5-yl)amino]-5-{4-[2-(dimethylamino)ethoxy]phenyl}nicotinonitrile,
4-[(7-chloro-4-methyl-1*H*-indol-5-yl)amino]-5-{4-[2-(4-methylpiperazin-1-yl)ethoxy]phenyl}nicotinonitrile,
*tert*-butyl 4-[(2-{5-cyano-4-[(4-methyl-1*H*-indol-5-yl)amino]pyridin-3-yl)-1-benzofuran-5-yl)methyl]piperazine-1-carboxylate,
5-(2-formyl-1-methyl-1*H*-imidazol-5-yl)-4-[(4-methyl-1*H*-indol-5-yl)amino]nicotinonitrile,
4-[(4-methyl-1*H*-indol-5-yl)amino]-5-(5-{[4-(methylsulfonyl)piperazin-1-yl]methyl}-1-benzofuran-2-yl)nicotinonitrile,
4-[(4-methyl-1*H*-indol-5-yl)amino]-5-{1-methyl-2-[(4-methylpiperazin-1-yl)methyl]-1*H*-imidazol-5-yl}nicotinonitrile,
4-[(4-methyl-1*H*-indol-5-yl)amino]-5-[5-(piperazin-1-ylmethyl)-1-benzofuran-2-yl]nicotinonitrile,
4-[(4-methyl-1*H*-indol-5-yl)amino]-5-(1,3-thiazol-2-yl)nicotinonitrile,
5-(1-methyl-1*H*-imidazol-2-yl)-4-[(4-methyl-1*H*-indol-5-yl)amino]nicotinonitrile,
4-[(4-methyl-1*H*-indol-5-yl)amino]-5-(1,3-thiazol-4-yl)nicotinonitrile,
5-(3,4-dimethoxyphenyl)-4-(1*H*-indol-7-ylamino)nicotinonitrile,
5-(3,4-dimethoxyphenyl)-4-[(4-methoxy-1*H*-indol-5-yl)amino]nicotinonitrile,
5-(3,4-dimethoxyphenyl)-4-[(4-fluoro-1*H*-indol-5-yl)amino]nicotinonitrile,
4-[(7-chloro-4-methyl-1*H*-indol-5-yl)amino]-5-[5-(piperazin-1-ylmethyl)-1-benzofuran-2-yl]nicotinonitrile,
*tert*-butyl 4-[(2-{4-[(7-chloro-4-methyl-1*H*-indol-5-yl)amino]-5-cyanopyridin-3-yl}-1-benzofuran-5-yl)methyl]piperazine-1-carboxylate,
5-(3,4-dimethoxyphenyl)-4-[(2,4-dimethyl-1*H*-indol-5-yl)amino]nicotinonitrile,
5-{2-[(dimethylamino)methyl]phenyl}-4-[(4-methyl-1*H*-indol-5-yl)amino]nicotinonitrile,
5-(5-formyl-2-methoxyphenyl)-4-[(4-methyl-1*H*-indol-5-yl)amino]nicotinonitrile,
5-{2-methoxy-5-[(4-methylpiperazin-1-yl)methyl]phenyl}-4-[(4-methyl-1*H-*indol-5-yl)amino]nicotinonitrile,
5-{5-[(4-ethylpiperazin-1-yl)methyl]-1-benzofuran-2-yl}-4-[(4-methyl-1*H-*indol-5-yl)amino]nicotinonitrile,
4-[(4-methyl-1*H*-indol-5-yl)amino]-5-{5-[(4-methyl-4-oxidopiperazin-1-yl)methyl]-1-benzofuran-2-yl}nicotinonitrile,
5-(3,4-dimethoxyphenyl)-4-[(1,4-dimethyl-1*H*-indol-5-yl)amino]nicotinonitrile,
3-{5-cyano-4-[(4-methyl-1*H*-indol-5-yl)amino]pyridin-3-yl}benzoic acid,
5-(2-methoxyphenyl)-4-[(4-methyl-1*H*-indol-5-yl)amino]nicotinonitrile,
5-(3-methoxyphenyl)-4-[(4-methyl-1*H*-indol-5-yl)amino]nicotinonitrile,
5-(4-methoxyphenyl)-4-[(4-methyl-1*H*-indol-5-yl)amino]nicotinonitrile,
4-[(4-methyl-1*H*-indol-5-yl)amino]-5-phenylnicotinonitrile,
4-[(4-methyl-1*H*-indol-5-yl)amino]-5-(2-thienyl)nicotinonitrile,
4-[(4-methyl-1*H*-indol-5-yl)amino]-5-(3-thienyl)nicotinonitrile,
5-(3-furyl)-4-[(4-methyl-1*H*-indol-5-yl)amino]nicotinonitrile,
5-(1-methyl-1*H*-imidazol-5-yl)-4-[(4-methyl-1*H*-indol-5-yl)amino]nicotinonitrile,
4'-[(4-methyl-1*H*-indol-5-yl)amino]-2,3'-bipyridine-5'-carbonitrile,
1-(4-{5-cyano-4-[(4-methyl-1*H*-indol-5-yl)amino]pyridin-3-yl}phenyl)-3-cyclopropylurea,
1-(4-{5-cyano-4-[(4-methyl-1*H*-indol-5-yl)amino]pyridin-3-yl}phenyl)-3-methylurea,
3-(4-{5-cyano-4-[(4-methyl-1*H*-indol-5-yl)amino]pyridin-3-yl}phenyl)-1,1-dimethylurea,
N-(4-{5-cyano-4-[(4-methyl-1*H*-indol-5-yl)amino]pyridin-3-yl}phenyl)morpholine-4-carboxamide,
4-[(4-methyl-1*H*-indol-5-yl)amino]-5-(4-nitrophenyl)nicotinonitrile,
5-(4-aminophenyl)-4-[(4-methyl-1*H*-indol-5-yl)amino]nicotinonitrile,
5-(3-aminophenyl)-4-[(4-methyl-1*H*-indol-5-yl)amino]nicotinonitrile,
5-(2-aminophenyl)-4-[(4-methyl-1*H*-indol-5-yl)amino]nicotinonitrile,
5-[4-(dimethylamino)phenyl]-4-[(4-methyl-1*H*-indol-5-yl)amino]nicotinonitrile,
5-[3-(dimethylamino)phenyl]-4-[(4-methyl-1*H*-indol-5-yl)amino]nicotinonitrile,
N-(4-{5-cyano-4-[(4-methyl-1*H*-indol-5-yl)amino]pyridin-3-yl}phenyl)acetamide,
N-(2-{5-cyano-4-[(4-methyl-1*H*-indol-5-yl)amino]pyridin-3-yl}phenyl)acetamide,
N-(3-{5-cyano-4-[(4-methyl-1*H*-indol-5-yl)amino]pyridin-3-yl}phenyl)acetamide,
N-(4-{5-cyano-4-[(4-methyl-1*H*-indol-5-yl)amino]pyridin-3-yl}phenyl)-2-methylpropanamide,
4-{5-cyano-4-[(4-methyl-1*H*-indol-5-yl)amino]pyridin-3-yl}-N-methylbenzamide,
4-[(4-methyl-1*H*-indol-5-yl)amino]-5-(1-naphthyl)nicotinonitrile,
4-[(4-methyl-1*H*-indol-5-yl)amino]-5-(2-naphthyl)nicotinonitrile,
4-[(4-methyl-1*H*-indol-5-yl)amino]-5-(1-methyl-1*H*-pyrazol-5-yl)nicotinonitrile,
4-[(4-methyl-1*H*-indol-5-yl)amino]-5-(1*H*-pyrazol-4-yl)nicotinonitrile,
5-(1-benzothiophen-3-yl)-4-[(4-methyl-1*H*-indol-5-yl)amino]nicotinonitrile,
5-(1-methyl-1*H*-indol-2-yl)-4-[(4-methyl-1*H*-indol-5-yl)amino]nicotinonitrile,
5-(1*H*-indol-5-yl)-4-[(4-methyl-1*H*-indol-5-yl)amino]nicotinonitrile,
5-(1*H*-indol-6-yl)-4-[(4-methyl-1*H*-indol-5-yl)amino]nicotinonitrile,
4-[(4-methyl-1*H*-indol-5-yl)amino]-5-quinolin-3-ylnicotinonitrile,
4-[(4-methyl-1*H*-indol-5-yl)amino]-5-quinolin-8-ylnicotinonitrile,
5-(1-benzofuran-5-yl)-4-[(4-methyl-1*H*-indol-5-yl)amino]nicotinonitrile,
4-(4-methyl-1*H*-indol-5-ylamino)-5-(quinolin-5-yl)nicotinonitrile,
5-(dibenzo[b,d]thiophen-3-yl)-4-(4-methyl-1*H*-indol-5-ylamino)nicotinonitrile,
5-(benzo[b]thiophen-5-yl)-4-(4-methyl-1*H*-indol-5-ylamino)nicotinonitrile,
5-(1*H*-indol-4-yl)-4-(4-methyl-1*H*-indol-5-ylamino)nicotinonitrile,
4-[(2,4-dimethyl-1*H*-indol-5-yl)amino]-5-{5-[(4-methylpiperazin-1-yl)methyl]-1-benzofuran-2-yl}nicotinonitrile,
4-[(2,4-dimethyl-1*H*-indol-5-yl)amino]-5-[5-(piperazin-1-ylmethyl)-1-benzofuran-2-yl]nicotinonitrile,
4-[(4-methyl-1*H*-indol-5-yl)amino]-5-{6-[(4-methylpiperazin-1-yl)methyl]-1-benzofuran-2-yl}nicotinonitrile,
4-[(4-methyl-1*H*-indol-5-yl)amino]-5-[6-(piperazin-1-ylmethyl)-1-benzofuran-2-yl]nicotinonitrile,
4-[(4-methyl-1*H*-indol-5-yl)amino]-5-{4-[(piperazin-1-yl)methyl]phenyl}nicotinonitrile,
4-[(2,4-dimethyl-1*H*-indol-5-yl)amino]-5-{4-[(4-methylpiperazin-1-yl)methyl]phenyl}nicotinonitrile,
4-(2,4-dimethyl-1*H*-indol-5-ylamino)-5-{3-[2-(4-methylpiperazin-1-yl)ethoxy]phenyl}nicotinonitrile,
5-{4-[2-(dimethylamino)ethoxy]-3-methoxyphenyl}-4-[(2,4-dimethyl-1*H-*indol-5-yl)amino]nicotinonitrile,
4-[(2,4-dimethyl-1*H*-indol-5-yl)amino]-5-{4-[2-(4-methylpiperazin-1-yl)ethoxy]phenyl}nicotinonitrile,
4-[(4-methyl-1*H*-indol-5-yl)amino]-5-{4-[2-(piperazin-1-yl)ethoxy]phenyl}nicotinonitrile,
4-(4-methyl-1*H*-indol-5-ylamino)-2'-((4-methylpiperazin-1-yl)methyl)-3,4'-bipyridine-5-carbonitrile,
4-(4-methyl-1*H*-indol-5-ylamino)-2'-((piperazin-1-yl)methyl)-3,4'-bipyridine-5-carbonitrile,
4'-(4-methyl-1*H*-indol-5-ylamino)-5-((4-methylpiperazin-1-yl)methyl)-2,3'-bipyridine-5'-carbonitrile,
4'-(4-methyl-1*H*-indol-5-ylamino)-5-(morpholinomethyl)-2,3'-bipyridine-5'-carbonitrile,
4'-(4-methyl-1*H*-indol-5-ylamino)-5-((piperazin-1-yl)methyl)-2,3 bipyridine-5'-carbonitrile,
4'-(4-methyl-1*H*-indol-5-ylamino)-6-((4-methylpiperazin-1-yl)methyl)-2,3'-bipyridine-5'-carbonitrile,
4'-(4-methyl-1*H*-indol-5-ylamino)-6-(morpholinomethyl)-2,3'-bipyridine-5'-carbonitrile,
4'-(4-methyl-1*H*-indol-5-ylamino)-6-((piperazin-1-yl)methyl)-2,3'-bipyridine-5'-carbonitrile,
4'-(4-methyl-1*H*-indol-5-ylamino)-4-(morpholinomethyl)-2,3'-bipyridine-5'-carbonitrile,
4'-(4-methyl-1*H*-indol-5-ylamino)-4-((4-methylpiperazin-1-yl)methyl)-2,3'-bipyridine-5'-carbonitrile,
4'-(4-methyl-1*H*-indol-5-ylamino)-4-((piperazin-1-yl)methyl)-2,3'-bipyridine-5'-carbonitrile,
4-(4-methyl-1*H*-indol-5-ylamino)-5'-((4-methylpiperazin-1-yl)methyl)-3,3'-bipyridine-5-carbonitrile,
4-(4-methyl-1*H*-indol-5-ylamino)-5'-((piperazin-1-yl)methyl)-3,3'-bipyridine-5-carbonitrile,
4-(4-methyl-1*H*-indol-5-ylamino)-5'-(morpholinomethyl)-3,3'-bipyridine-5-carbonitrile,
4-(4-methyl-1*H*-indol-5-ylamino)-6'-((4-methylpiperazin-1-yl)methyl)-3,3'-bipyridine-5-carbonitrile,
4-(4-methyl-1*H*-indol-5-ylamino)-6'-((piperazin-1-yl)methyl)-3,3'-bipyridine-5-carbonitrile,
4-(4-methyl-1*H*-indol-5-ylamino)-6'-(morpholinomethyl)-3,3'-bipyridine-5-carbonitrile,
4-(4-methyl-1*H*-indol-5-ylamino)-5-(3-(piperazin-1-ylmethyl)phenyl)nicotinonitrile,
4-(4-methyl-1*H*-indol-5-ylamino)-5-(4-(piperazin-1-ylmethyl)phenyl)nicotinonitrile,
4-({[*cis*-4-(aminomethyl)cyclohexyl]methyl}amino)-5-(3,4-dimethoxyphenyl)nicotinonitrile, and
5-(3,4-dimethoxyphenyl)-4-(1*H*-indol-5-ylamino)nicotinonitrile 1-oxide.

21. The compound of any one of claims 1-20, wherein the compound is in the form of an enantiomer.

22. A pharmaceutical composition comprising the compound of any one of claims 1-21 and a pharmaceutically acceptable carrier or excipient.

23. A compound of any one of claims 1-21 or a pharmaceutically acceptable salt, hydrate, or ester thereof for use in the treatment or inhibition of a pathological condition or disorder mediated by a protein kinase in a mammal.

24. The compound for use of claim 23, wherein the protein kinase is protein kinase C.

25. The compound for use of claim 23 or 24, wherein the pathological condition or disorder is an inflammatory disease or an autoimmune disease selected from asthma, colitis, multiple sclerosis, psoriasis, arthritis, rheumatoid arthritis, osteoarthritis, and joint inflammation.

26. A compound as claimed in any one of claims 1 to 21 or a pharmaceutically acceptable salt, hydrate, or ester thereof for use as a medicament.

27. Use of a compound as claimed in any one of claims 1 to 21 or a pharmaceutically acceptable salt, hydrate, or ester thereof in the preparation of a medicament for the treatment or inhibition of a pathological condition or disorder mediated by a protein kinase in a mammal.

## Patentansprüche

1. Verbindung der Formel I oder der Formel I': 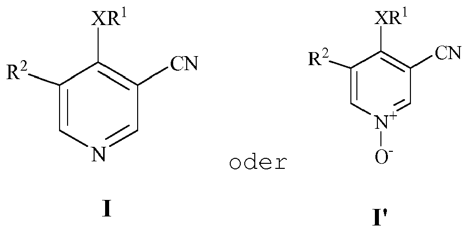 oder ein pharmazeutisch annehmbares Salz, Hydrat oder Ester davon, wobei:
X ausgewählt ist aus a) -NR³-Y-, b) -O-Y-, c) -S(O)ₘ-Y-, d) -S(O)ₘNR³-Y-, e) -NR³S(O)ₘ-Y-, f) -C(O)NR³-Y-, g)-C(S)NR³-Y-, h) -NR³C(O)-Y-, i) -NR³C(S)-Y-, j) -C(O)O-Y-, k) -OC(O)-Y-und l) einer kovalenten Bindung;
Y bei jedem Vorkommen unabhängig ausgewählt ist aus a) einer divalenten C₁₋₁₀-Alkylgruppe, b) einer divalenten C₂₋₁₀-Alkenylgruppe, c) einer divalenten C₂₋₁₀-Alkinylgruppe, d) einer divalenten C₁₋₁₀-Halogenalkylgruppe und e) einer kovalenten Bindung;
R¹ ausgewählt ist aus a) einer C₁₋₁₀-Alkylgruppe, b) einer C₃₋₁₄-Cycloalkylgruppe, c) einer 3-14-gliedrigen Cycloheteroalkylgruppe, d) einer C₈₋₁₄-polycyclischen Arylgruppe und e) einer 5-14-gliedrigen Heteroarylgruppe, wobei jede Gruppe optional substituiert ist mit 1-4 -Y-R⁴;
R² eine C₆₋₁₄-Arylgruppe oder eine 5-14-gliedrige Heteroarylgruppe ist, wobei jede Gruppe optional substituiert ist mit 1-4 Gruppen, die unabhängig ausgewählt sind aus -Y-R⁴ oder -O-Y-R⁴;
R³ ausgewählt ist aus a) H, b) einer C₁₋₁₀-Alkylgruppe, c) einer C₂₋₁₀-Alkenylgruppe, d) einer C₂₋₁₀-Alkinylgruppe und e) einer C₁₋₁₀-Halogenalkylgruppe;
R⁴ bei jedem Vorkommen unabhängig ausgewählt ist aus a) Halogen, b) -CN, c) -NO₂, d) Oxo, e) -O-Y-R⁵, f) -NR⁶-Y-R⁷, g) -N(O)R⁶-Y-R⁷, h) -S(O)ₘ-Y-R⁵, i) -S(O)ₘO-Y-R⁵, j) -S(O)ₘNR⁶-Y-R⁷, k) -C(O)-Y-R⁵, l) -C(O)O-Y-R⁵, m) -C(O)NR⁶-Y-R⁷, n) -C(S)NR⁶-Y-R⁷, o) einer C₁₋₁₀-Alkylgruppe, p) einer C₂₋₁₀-Alkenylgruppe, q) einer C₂₋₁₀-Alkinylgruppe, r) einer C₁₋₁₀-Halogenalkylgruppe, s) einer C₃₋₁₄-Cycloalkylgruppe, t) einer C₆₋₁₄-Arylgruppe, u) einer 3-14-gliedrigen Cycloheteroalkylgruppe und v) einer 5-14-gliedrigen Heteroarylgruppe, wobei jedes von o) - v) optional substituiert ist mit 1-4 -Y-R⁸-Gruppen;
R⁵ bei jedem Vorkommen unabhängig ausgewählt ist aus a) H, b) -C(O)R⁹, c) -C(O)OR⁹, d) einer C₁₋₁₀-Alkylgruppe, e) einer C₂₋₁₀-Alkenylgruppe, f) einer C₂₋₁₀-Alkinylgruppe, g) einer C₁₋₁₀-Halogenalkylgruppe, h) einer C₃₋₁₄-Cycloalkylgruppe, i) einer C₆₋₁₄-Arylgruppe, j) einer 3-14-gliedrigen Cycloheteroalkylgruppe und k) einer 5-14-gliedrigen Heteroarylgruppe, wobei jedes von d) - k) optional substituiert ist mit 1-4 -Y-R⁸-Gruppen;
R⁶ und R⁷ bei jedem Vorkommen unabhängig ausgewählt sind aus a) H, b) -O-Y-R⁹, c) -S(O)ₘ-Y-R⁹, d) -S(O)ₘO-Y-R⁹, e) -C(O)-Y-R⁹, f) -C(O)O-Y-R⁹, g) -C(O)NR¹⁰-Y-R¹¹, h) -C(S)NR¹⁰-Y-R¹³-, i) einer C₁₋₁₀-Alkylgruppe, j) einer C₂₋₁₀-Alkenylgruppe, k) einer C₂₋₁₀-Alkinylgruppe, l) einer C₁₋₁₀-Halogenalkylgruppe, m) einer C₃₋₁₄-Cycloalkylgruppe, n) einer C₆₋₁₄-Arylgruppe, o) einer 3-14-gliedrigen Cycloheteroalkylgruppe und p) einer 5-14-gliedrigen Heteroarylgruppe; wobei jedes von i) - p) optional substituiert ist mit 1-4 -Y-R⁸-Gruppen;
R⁸ bei jedem Vorkommen unabhängig ausgewählt ist aus a) Halogen, b) -CN, c) -NO₂, d) Oxo, e) -O-Y-R⁹, f) -NR¹⁰-Y-R¹¹, g) -N(O)R¹⁰-Y-R¹¹, h) -S(O)ₘ-Y-R⁹, i) -S(O)ₘO-Y-R⁹, j) -S(O)ₘNR¹⁰-Y-R¹¹, k) -C(O)-Y-R⁹, l) -C(O)O-Y-R⁹, m) -C(O)NR¹⁰-Y-R¹¹, n) -C(S)NR¹⁰-Y-R¹¹, o) einer C₁₋₁₀-Alkylgruppe, p) einer C₂₋₁₀-Alkenylgruppe, q) einer C₂₋₁₀-Alkinylgruppe, r) einer C₁₋₁₀-Halogenalkylgruppe, s) einer C₃₋₁₄-Cycloalkylgruppe, t) einer C₆₋₁₄-Arylgruppe, u) einer 3-14-gliedrigen Cycloheteroalkylgruppe und v) einer 5-14-gliedrigen Heteroarylgruppe, wobei jedes von o) - v) optional substituiert ist mit 1-4 -Y-R¹²-Gruppen;
R⁹ bei jedem Vorkommen unabhängig ausgewählt ist aus a) H, b) -C(O)-C₁₋₁₀-Alkyl, c) -C(O)OH, d) -C(O)O-C₁₋₁₀-Alkyl, e) einer C₁₋₁₀-Alkylgruppe, f) einer C₂₋₁₀-Alkenylgruppe, g) einer C₂₋₁₀-Alkinylgruppe, h) einer C₁₋₁₀-Halogenalkylgruppe, i) einer C₃₋₁₄-Cycloalkylgruppe, j) einer C₆₋₁₀-Arylgruppe, k) einer 3-14-gliedrigen Cycloheteroalkylgruppe und l) einer 5-14-gliedrigen Heteroarylgruppe, wobei jede der C₁₋₁₀-Alkylgruppen, der C₂₋₁₀-Alkenylgruppen, der C₂₋₁₀-Alkinylgruppen, der C₁₋₁₀-Halogenalkylgruppen, der C₃₋₁₄-Cycloalkylgruppen, der C₆₋₁₄-Arylgruppen, der 3-14-gliedrigen Cycloheteroalkylgruppen und der 5-14-gliedrigen Heteroarylgruppen optional substituiert ist mit 1-4 -Y-R¹²-Gruppen;
R¹⁰ und R¹¹ bei jedem Vorkommen unabhängig ausgewählt sind aus a) H, b) -OH, c) -SH, d) -NH₂, e) -NH-C₁₋₁₀-Alkyl, f) -N(C₁₋₁₀-Alkyl)₂, g) -S(O)ₘ-C₁₋₁₀-Alkyl, h) -S(O)₂OH, i) -S(O)ₘ-OC₁₋₁₀-Alkyl, j) -C(O)-C₁₋₁₀-Alkyl, k) -C(O)OH, l) -C(O)-OC₁₋₁₀-Alkyl, m) -C(O)NH₂, n) -C(O)NH-C₁₋₁₀-Alkyl, o) -C(O)N(C₁₋₁₀-Alkyl)₂, p) -C(S)NH₂, q) -C(S)NH-C₁₋₁₀-Alkyl, r) -C(S)N(C₁₋₁₀-Alkyl)₂, s) einer C₁₋₁₀-Alkylgruppe, t) einer C₂₋₁₀-Alkenylgruppe, u) einer C₂₋₁₀-Alkinylgruppe, v) einer C₁₋₁₀-Alkoxygruppe, w) einer C₁₋₁₀-Halogenalkylgruppe, x) einer C₃₋₁₄-Cycloalkylgruppe, y) einer C₆₋₁₄-Arylgruppe, z) einer 3-14-gliedrigen Cycloheteroalkylgruppe und aa) einer 5-14-gliedrigen Heteroarylgruppe, wobei jede der C₁₋₁₀-Alkylgruppen, der C₂₋₁₀-Alkenylgruppen, der C₂₋₁₀-Alkinylgruppen, der C₁₋₁₀-Alkoxygruppen, der C₁₋₁₀-Halogen-alkylgruppen, der C₃₋₁₄-Cycloalkylgruppen, der C₆₋₁₄ Arylgruppen, der 3-14-gliedrigen Cycloheteroalkylgruppen und der 5-14-gliedrigen Heteroarylgruppen optional substituiert ist mit 1-4 -Y-R¹²-Gruppen;
R¹² bei jedem Vorkommen unabhängig ausgewählt ist aus a) Halogen, b) -CN, c) -NO₂, d) Oxo, e) -OH, f) -NH₂, g) -NH(C₁₋₁₀-Alkyl), h) -N(C₁₋₁₀-Alkyl)₂, i) -SH, j) -S(O)ₘ-C₁₋₁₀-Alkyl, k) -S(O)₂OH, l) -S (O)ₘ-OC₁₋₁₀-Alkyl, m) -C (O)-C₁₋₁₀-Alkyl, n) -C(O)OH, o) -C(O)-OC₁₋₁₀-Alkyl, p) -C(O)NH₂, q) -C(O)NH-C₁₋₁₀-Alkyl, r) -C(O)N(C₁₋₁₀-Alkyl)₂, s) -C(S)NH₂, t) -C(S)NH-C₁₋₁₀-Alkyl, u) -C(S)N(C₁₋₁₀-Alkyl)₂, v) einer C₁₋₁₀-Alkylgruppe, w) einer C₂₋₁₀-Alkenylgruppe, x) einer C₂₋₁₀-Alkinylgruppe, y) einer C₁₋₁₀-Alkoxygruppe, z) einer C₁₋₁₀-Halogenalkylgruppe, aa) einer C₃₋₁₄-Cycloalkylgruppe, ab) einer C₆₋₁₄-Arylgruppe, ac) einer 3-14-gliedrigen Cycloheteroalkylgruppe und ad) einer 5-14-gliedrigen Heteroarylgruppe; und m 0, 1 oder 2 ist.

2. Verbindung nach Anspruch 1 oder ein pharmazeutisch annehmbares Salz, Hydrat oder Ester davon, wobei X ausgewählt ist aus -NH-, -N(CH₃)-, -NH-CH₂-, -NH-CH₂CH₂-, -NH-CH₂CH₂CH₂-, -0- und einer kovalenten Bindung.

3. Verbindung nach Anspruch 1 oder 2 oder ein pharmazeutisch annehmbares Salz, Hydrat oder Ester davon, wobei R¹ eine C₈₋₁₄-polycyclische Arylgruppe oder eine 5-14-gliedrige Heteroarylgruppe ist, wobei jede Gruppe optional substituiert ist mit 1-4 -Y-R⁴-Gruppen.

4. Verbindung nach einem der Ansprüche 1-3 oder pharmazeutisch annehmbares Salz, Hydrat oder Ester davon, wobei R¹ ausgewählt ist aus einer Benzimidazolylgruppe, einer Benzodioxolylgruppe, einer Benzodioxinylgruppe, einer Benzodioxanylgruppe, einer Benzofuranylgruppe, einer Benzothienylgruppe, einer Benzoxadiazolylgruppe, einer Benzoxazolylgruppe, einer Benzisoxazolylgruppe, einer Benzothiadiazolylgruppe, einer Benzothiazolylgruppe, einer Benzisothiazolylgruppe, einer Benzo[c]isothiazolylgruppe, einer Benzo[c]thienylgruppe, einer Benzotriazolylgruppe, einer Indazolylgruppe, einer Indenylgruppe, einer Indanylgruppe, einer Indolylgruppe, einer Isobenzofuranylgruppe, einer Isoindolylgruppe, einer Isochinolinylgruppe, einer Naphthylgruppe, einer Indolinylgruppe, einer Pyrazolylgruppe, einer Pyridinylgruppe, einer Pyrrolopyridinylgruppe, einer Pyrrolylgruppe, einer Chinolinylgruppe und einer Tetrahydronaphthalenylgruppe, wobei jede Gruppe optional substituiert ist mit 1-4 -Y-R⁴-Gruppen.

5. Verbindung nach einem der Ansprüche 1-4 oder ein pharmazeutisch annehmbares Salz, Hydrat oder Ester davon, wobei R¹ eine Indolylgruppe oder eine Pyrrolopyridinylgruppe ist, von denen jede optional substituiert ist mit 1-4 -Y-R⁴-Gruppen.

6. Verbindung nach einem der Ansprüche 1-5 oder ein pharmazeutisch annehmbares Salz, Hydrat oder Ester davon, wobei R¹ eine 1*H*-Indol-4-yl-Gruppe, eine 1*H*-Indol-5-yl-Gruppe, eine 1*H*-Indol-6-yl-Gruppe oder eine 1*H*-Indol-7-yl-Gruppe ist, wobei jede Gruppe optional substituiert ist mit 1-4 Gruppen, die unabhängig ausgewählt sind aus einem Halogen, einer C₁₋₄-Alkylgruppe und einer C₁₋₄-Alkoxygruppe.

7. Verbindung nach Anspruch 1 oder 2 oder ein pharmazeutisch annehmbares Salz, Hydrat oder Ester davon, wobei R¹ eine C₃₋₁₄-Cycloalkylgruppe oder eine 3-14-gliedrige Cycloheteroarylgruppe ist, wobei jede Gruppe optional substituiert ist mit 1-4 -Y-R⁴-Gruppen.

8. Verbindung nach Anspruch 7 oder ein pharmazeutisch annehmbares Salz, Hydrat oder Ester davon, wobei R¹ ausgewählt ist aus einer Cyclobutylgruppe, einer Cyclopentylgruppe, einer Cyclohexylgruppe, einer Cycloheptylgruppe, einer Pyrrolidinylgruppe, einer Piperidinylgruppe, einer Piperazinylgruppe, einer Morpholinylgruppe und einer Thiomorpholinylgruppe, wobei jede Gruppe optional substituiert ist mit 1-4 -Y-R⁴-Gruppen.

9. Verbindung nach einem der Ansprüche 1-8 oder ein pharmazeutisch annehmbares Salz, Hydrat oder Ester davon, wobei R² ausgewählt ist aus einer Phenylgruppe, einer C₈₋₁₄-Arylgruppe und einer 5-14-gliedrigen Heteroarylgruppe, wobei jede Gruppe optional substituiert ist mit 1-4 Gruppen, die unabhängig ausgewählt sind aus -Y-R⁴ und -O-Y-R⁴.

10. Verbindung nach einem der Ansprüche 1-9 oder ein pharmazeutisch annehmbares Salz, Hydrat oder Ester davon, wobei R² ist: 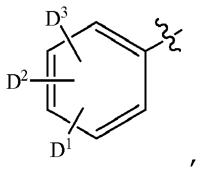 wobei D¹, D² und D³ unabhängig sind: H, eine -Y-R⁴-Gruppe oder eine -O-Y-R⁴-Gruppe.

11. Verbindung nach Anspruch 10 oder ein pharmazeutisch annehmbares Salz, Hydrat oder Ester davon, wobei mindestens eines von D¹, D² und D³ eine -Y-R⁴-Gruppe oder eine -O-Y-R⁴-Gruppe ist, wobei Y bei jedem Vorkommen unabhängig eine divalente C₁₋₄-Alkylgruppe oder eine kovalente Bindung ist und R⁴ bei jedem Vorkommen unabhängig ausgewählt ist aus einem Halogen, -CN, -NO₂, -O-Y-R⁵, -NR⁶-Y-R⁷, -S(O)₂-Y-R⁵, -S(O) ₂NR⁶-Y-R⁷, -C(O)-Y-R⁵, -C(O)O-Y-R⁵, -C (O)NR⁶-Y-R⁷, einer C₁₋₁₀-Alkylgruppe, einer C₁₋₁₀-Halogenalkylgruppe, einer C₃₋₁₄-Cycloalkylgruppe, einer C₆₋₁₄-Arylgruppe, einer 3-14-gliedrigen Cycloheteroalkylgruppe und einer 5-14-gliedrigen Heteroarylgruppe, wobei jede der C₁₋₁₀-Alkylgruppen, der C₁₋₁₀-Halogenalkylgruppen, der C₃₋₁₄-Cycloalkylgruppen, der C₆₋₁₄-Arylgruppen, der 3-14-gliedrigen Cycloheteroalkylgruppen und der 5-14-gliedrigen Heteroarylgruppen optional substituiert ist mit 1-4 -Y-R⁸-Gruppen.

12. Verbindung nach Anspruch 11 oder ein pharmazeutisch annehmbares Salz, Hydrat oder Ester davon, wobei die -Y-R⁴-Gruppe und die -O-Y-R⁴-Gruppe ausgewählt sind aus -O-(CH₂)ₙNR⁶-Y-R⁷, - (CH₂)ₙNR⁶-Y-R⁷, einer -O-(CH₂)ₙ-3-14-gliedrigen Cycloheteroalkylgruppe und einer -(CH₂)ₙ-3-14-gliedrigen Cycloheteroalkylgruppe, wobei jede der 3-14-gliedrigen Cycloheteroalkylgruppen optional substituiert ist mit 1-4 -Y-R⁸-Gruppen und n bei jedem Vorkommen unabhängig 0, 1, 2, 3 oder 4 ist.

13. Verbindung nach Anspruch 12 oder ein pharmazeutisch annehmbares Salz, Hydrat oder Ester davon, wobei die 3-14-gliedrige Cycloheteroalkylgruppe der -O-(CH₂)ₙ-3-14-gliedrigen Cycloheteroalkylgruppen und der -(CH₂)ₙ-3-14-gliedrigen Cycloheteroalkylgruppen ausgewählt ist aus einer Pyrrolidinylgruppe, einer Morpholinylgruppe, einer Piperazinylgruppe, einer Piperidinylgruppe, einer Azepanylgruppe, einer Diazepanylgruppe und einer Thiomorpholinylgruppe.

14. Verbindung nach Anspruch 11 oder ein pharmazeutisch annehmbares Salz, Hydrat oder Ester davon, wobei die -Y-R⁴-Gruppe und die -O-Y-R⁴-Gruppe sind: 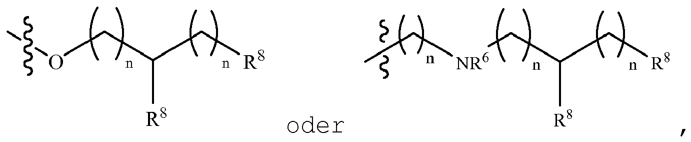 wobei R⁸ bei jedem Vorkommen unabhängig ausgewählt ist aus -O-Y-R⁹, -NR¹⁰-Y-R¹¹, einer C₆₋₁₄-Arylgruppe und einer 5-14-gliedrigen Heteroarylgruppe, wobei die C₆₋₁₄-Arylgruppe und die 5-14-gliedrige Heteroarylgruppe optional substituiert sind mit 1-4 -Y-R¹²-Gruppen und n bei jedem Vorkommen unabhängig 0, 1, 2, 3 oder 4 ist.

15. Verbindung nach einem der Ansprüche 11-14 oder ein pharmazeutisch annehmbares Salz, Hydrat oder Ester davon, wobei mindestens eines von D¹, D² und D³ ausgewählt ist aus einem Halogen, -CN, -NO₂, -S(O)₂-Y-R⁵, -S(O) ₂NR⁶-Y-R⁷, -C (O) O-Y-R⁵, -C (O) NR⁶-Y-R⁷, einer C₁₋₁₀-Alkylgruppen und einer C₁₋₁₀-Halogenalkylgruppe.

16. Verbindung nach einem der Ansprüche 11-15 oder ein pharmazeutisch annehmbares Salz, Hydrat oder Ester davon, wobei mindestens eines von D¹, D² und D³ eine C₆₋₁₄-Arylgruppe oder eine 5-14-gliedrige Heteroarylgruppe ist, wobei jede Gruppe optional substituiert ist mit 1-4 -Y-R⁸-Gruppen.

17. Verbindung nach Anspruch 16 oder ein pharmazeutisch annehmbares Salz, Hydrat oder Ester davon, wobei mindestens eines von D¹, D² und D³ ausgewählt ist aus einer Benzothienylgruppe, einer Benzofurylgruppe, einer Furylgruppe, einer Pyridylgruppe, einer Pyrimidinylgruppe, einer Pyrrolylgruppe und einer Thienylgruppe, wobei jede Gruppe optional substituiert ist mit 1-4 -Y-R⁸-Gruppen, Y bei jedem Vorkommen unabhängig eine C₁₋₄-Alkylgruppe oder eine kovalente Bindung ist und R⁸ bei jedem Vorkommen unabhängig ausgewählt ist aus Halogen, -CN, -NO₂, -O-Y-R⁹, -NR¹⁰-Y-R¹¹, -C(O)-Y-R⁹, -C(O)NR¹⁰-Y-R¹¹, -S(O)₂-Y-R⁹, -S(O)₂NR¹⁰-Y-R¹¹ und einer 3-14-gliedrigen Cycloheteroalkylgruppe, optional substituiert mit einer C₁₋₄-Alkylgruppe.

18. Verbindung nach einem der Ansprüche 1-9 oder ein pharmazeutisch annehmbares Salz, Hydrat oder Ester davon, wobei R² eine C₈₋₁₄-bicyclische Arylgruppe oder eine 5-14-gliedrige Heteroarylgruppe ist, wobei jede Gruppe optional substituiert ist mit 1-4 Gruppen, die unabhängig ausgewählt sind aus -Y-R⁴ und -O-Y-R⁴.

19. Verbindung nach Anspruch 18 oder ein pharmazeutisch annehmbares Salz, Hydrat oder Ester davon, wobei R² ausgewählt ist aus einer Pyridylgruppe, einer Pyrimidylgruppe, einer Pyrazinylgruppe, einer Furylgruppe, einer Thienylgruppe, einer Thiazolylgruppe, einer Oxazolylgruppe, einer Benzofuranylgruppe, einer Benzothienylgruppe, einer Indolylgruppe, einer Benzodioxinylgruppe, einer Benzodioxolylgruppe, einer Benzodioxanylgruppe, einer Dibenzofuranylgruppe, einer Dibenzothienylgruppe, einer Benzoindolylgruppe, einer Indanylgruppe, einer Indenylgruppe, einer Isothiazolylgruppe, einer Pyridazinylgruppe, einer Pyrazolylgruppe, einer Tetrahydronaphthylgruppe, einer Isoxazolylgruppe, einer Chinolinylgruppe, einer Naphthylgruppe, einer Imidazolylgruppe und einer Pyrrolylgruppe, wobei jede Gruppe optional substituiert ist mit 1-4 Gruppen, die unabhängig ausgewählt sind aus -(CH₂)ₙ-R⁴ und -O-(CH₂)ₙ-R⁴, wobei n bei jedem Vorkommen unabhängig 0, 1, 2, 3 oder 4 ist und R⁴ bei jedem Vorkommen unabhängig -NR⁶-Y-R⁷ oder eine 3-14-gliedrige Cycloheteroalkylgruppe ist, optional substituiert mit einer -Y-R⁸-Gruppe.

20. Verbindung nach Anspruch 1, ausgewählt aus den folgenden Verbindungen:
5-(3,4-Dimethoxyphenyl)-4-(1*H*-indol-5-ylamino)nicotinonitril,
4-(2,1,3-Benzothiadiazol-4-ylamino)-5-(3,4-dimethoxyphenyl)nicotinonitril,
5-(3,4-Dimethoxyphenyl)-4-(isochinolin-5-ylamino)nicotinonitril,
5-(3,4-Dimethoxyphenyl)-4-(chinolin-5-ylamino)nicotinonitril,
5-(3,4-Dimethoxyphenyl)-4-(5,6,7,8-tetrahydronaphthalin-1-ylamino)nicotinonitril,
4-(2,3-Dihydro-1,4-benzodioxin-6-ylamino)-5-(3,4-dimethoxyphenyl)nicotinonitril,
4-(2,3-Dihydro-1*H*-inden-5-ylamino)-5-(3,4-dimethoxyphenyl)nicotinonitril,
5-(3,4-Dimethoxyphenyl)-4-(1*H*-indol-6-ylamino)nicotinonitril,
5-(3,4-Dimethoxyphenyl)-4-(1*H*-indol-4-ylamino)nicotinonitril,
5-(3,4-Dimethoxyphenyl)-4-[(2-methyl-1*H*-indol-5-yl)amino]nicotinonitril,
4-(1,3-Benzodioxol-5-ylamino)-5-(3,4-dimethoxyphenyl)nicotinonitril,
5-(3,4-Dimethoxyphenyl)-4-(2-naphthylamino)nicotinonitril,
5-(3-Bromphenyl)-4-(1*H*-indol-5-ylamino)nicotinonitril,
5-(3-Bromphenyl)-4-(1*H*-indol-4-ylamino)nicotinonitril,
5-(2-Bromphenyl)-4-(1*H*-indol-5-ylamino)nicotinonitril,
5-(4-Bromphenyl)-4-(1*H*-indol-5-ylamino)nicotinonitril,
5-(3'-Aminobiphenyl-3-yl)-4-(1*H*-indol-5-ylamino)nicotinonitril,
5-(4'-Cyanobiphenyl-3-yl)-4-(1*H*-indol-5-ylamino)nicotinonitril,
5-(4'-Aminobiphenyl-3-yl)-4-(1*H*-indol-5-ylamino)nicotinonitril,
N-{3'-[5-Cyano-4-(1*H*-indol-5-ylamino)pyridin-3-yl]biphenyl-4-yl}acetamid,
4-(1*H*-Indol-5-ylamino)-5-(3-pyridin-4-ylphenyl)nicotinonitril,
3'-[5-Cyano-4-(1*H*-indol-5-ylamino)pyridin-3-yl]-N,N-dimethylbiphenyl-4-carboxamid,
3'-[5-Cyano-4-(1*H*-indol-5-ylamino)pyridin-3-yl]-N-cyclopentylbiphenyl-4-carboxamid,
4-(1*H*-Indol-5-ylamino)-5-[3-(1*H*-pyrrol-3-yl)phenyl]nicotinonitril,
5-(2-Bromphenyl)-4-[(7-methyl-1H-indol-5-yl)amino]nicotinonitril,
5-(2-Bromphenyl)-4-(1*H*-indol-4-ylamino)nicotinonitril,
4-(1*H*-Indol-5-ylamino)-5-(3'-methylbiphenyl-3-yl)nicotinonitril,
4-(1*H*-Indol-5-ylamino)-5-(4'-methylbiphenyl-3-yl)nicotinonitril,
5-(2'-Chlorbiphenyl-3-yl)-4-(1*H*-indol-5-ylamino)nicotinonitril,
5-(3'-Chlorbiphenyl-3-yl)-4-(1*H*-indol-5-ylamino)nicotinonitril,
5-(4'-Chlorbiphenyl-3-yl)-4-(1*H*-indol-5-ylamino)nicotinonitril,
5-(3'-Cyanobiphenyl-3-yl)-4-(1*H*-indol-5-ylamino)nicotinonitril,
3'-[5-Cyano-4-(1*H*-indol-5-ylamino)pyridin-3-yl]biphenyl-3-carbonsäure,
3'-[5-Cyano-4-(1*H*-indol-5-ylamino)pyridin-3-yl]biphenyl-4-carbonsäure,
3'-[5-Cyano-4-(1*H*-indol-4-ylamino)pyridin-3-yl]biphenyl-4-carbonsäure,
4-(1*H*-Indol-5-ylamino)-5-[3-(2-thienyl)phenyl]nicotinonitril,
4-(1*H*-Indol-5-ylamino)-5-(3-pyridin-3-ylphenyl)nicotinonitril,
4-(1*H*-Indol-5-ylamino)-5-(3-pyrimidin-2-ylphenyl)nicotinonitril,
4-(1*H*-Indol-5-ylamino)-5-[3-(4-methyl-2-thienyl)phenyl]nicotinonitril,
5-[3-(5-Acetyl-2-thienyl)phenyl]-4-(1*H*-indol-5-ylamino)nicotinonitril,
4-(1*H*-Indol-5-ylamino)-5-[3-(3-thienyl)phenyl]nicotinonitril,
5-[3-(3-Furyl)phenyl]-4-(1*H*-indol-5-ylamino)nicotinonitril,
5-(2'-Chlorbiphenyl-2-yl)-4-(1*H*-indol-5-ylamino)nicotinonitril,
5-(3'-Chlorbiphenyl-2-yl)-4-(1*H*-indol-5-ylamino)nicotinonitril,
5-(4'-Chlorbiphenyl-2-yl)-4-(1*H*-indol-5-ylamino)nicotinonitril,
4-(1*H*-Indol-5-ylamino)-5-[2-(3-thienyl)phenyl]nicotinonitril,
4-(1*H*-Indol-4-ylamino)-5-[3-(2-thienyl)phenyl]nicotinonitril,
3'-[5-Cyano-4-(1*H*-indol-4-ylamino)pyridin-3-yl]-N-cyclopentylbiphenyl-4-carboxamid,
4-(1*H*-Indol-4-ylamino)-5-[4'-(pyrrolidin-1-ylcarbonyl)biphenyl-3-yl]nicotinonitril,
5-[3-(5-Formyl-2-thienyl)phenyl]-4-(1*H*-indol-5-ylamino)nicotinonitril,
4-(1*H*-Indol-5-ylamino)-5-(3-nitrophenyl)nicotinonitril,
N-{3-[5-Cyano-4-(1*H*-indol-5-ylamino)pyridin-3-yl]phenyl}acetamid,
4-(1*H*-Indol-4-ylamino)-5-[4-methoxy-3-(2-methoxyethoxy)phenyl]nicotinonitril,
4-(1*H*-Indol-5-ylamino)-5-[4-methoxy-3-(2-methoxyethoxy)phenyl]nicotinonitril,
4-(1*H*-Indol-6-ylamino)-5-[4-methoxy-3-(2-methoxyethoxy)phenyl]nicotinonitril,
4-(1,3-Benzothiazol-6-ylamino)-5-[4-methoxy-3-(2-methoxyethoxy)phenyl]nicotinonitril,
5-[4-Methoxy-3-(2-methoxyethoxy)phenyl]-4-[(2-methyl-1*H-*indol-5-yl)amino]nicotinonitril,
4-(1*H*-1,2,3-Benzotriazol-5-ylamino)-5-[4-methoxy-3-(2-methoxyethoxy)phenyl]nicotinonitril,
4-(1*H*-Indol-4-ylamino)-5-[3-methoxy-4-(2-methoxyethoxy)phenyl]nicotinonitril,
4-(1*H*-Indol-5-ylamino)-5-[3-methoxy-4-(2-methoxyethoxy)phenyl]nicotinonitril,
4-(1*H*-Indol-6-ylamino)-5-[3-methoxy-4-(2-methoxyethoxy)phenyl]nicotinonitril,
4-(1,3-Benzothiazol-6-ylamino)-5-[3-methoxy-4-(2-methoxyethoxy)phenyl]nicotinonitril,
5-[3-Methoxy-4-(2-methoxyethoxy)phenyl]-4-[(2-methyl-1*H-*indol-5-yl)amino]nicotinonitril,
4-(1*H*-Indol-4-ylamino)-5-[3-(2-methoxyethoxy)phenyl]nicotinonitril,
5-[3-(2-Chlorethoxy)phenyl]-4-(1*H*-indol-4-ylamino)nicotinonitril,
5-[3-(2-Chlorethoxy)phenyl]-4-(1*H*-indol-6-ylamino)nicotinonitril,
5-[3-(2-Chlorethoxy)phenyl]-4-(1*H*-indol-5-ylamino)nicotinonitril,
4-(1*H*-Indol-4-ylamino)-5-{3-[2-(4-methylpiperazin-1-yl)ethoxy]phenyl}nicotinonitril,
4-(1*H*-Indol-4-ylamino)-5-[3-(2-pyrrolidin-1-ylethoxy)phenyl]nicotinonitril,
4-(1*H*-Indol-4-ylamino)-5-[3-(2-morpholin-4-ylethoxy)phenyl]nicotinonitril,
4-(1*H*-Indol-4-ylamino)-5-[3-(2-piperidin-1-ylethoxy)phenyl]nicotinonitril,
4-(1*H*-Indol-6-ylamino)-5-[3-(2-pyrrolidin-1-ylethoxy)phenyl]nicotinonitril,
4-(1*H*-Indol-6-ylamino)-5-[3-(2-morpholin-4-ylethoxy)phenyl]nicotinonitril,
4-(1*H*-Indol-5-ylamino)-5-[3-(2-pyrrolidin-1-ylethoxy)phenyl]nicotinonitril,
4-(1*H*-Indol-5-ylamino)-5-[3-(2-morpholin-4-ylethoxy)phenyl]nicotinonitril,
5-(3-{2-[(2-Hydroxyethyl)amino]ethoxy}phenyl)-4-(1*H*-indol-5-ylamino)nicotinonitril,
4-(1*H*-Indol-5-ylamino)-5-(3-{2-[(2-pyrrolidin-1-ylethyl)amino]ethoxy}phenyl)nicotinonitril,
5-[3-(2-Chlorethoxy)-4-methoxyphenyl]-4-(1*H*-indol-5-ylamino)nicotinonitril,
5-{3-[2-(Diethylamino)ethoxy]-4-methoxyphenyl}-4-(1*H*-indol-5-ylamino)nicotinonitril,
5-{3-[2-(Diisopropylamino)ethoxy]-4-methoxyphenyl}-4-(1*H-*indol-5-ylamino)nicotinonitril,
5-{3-[2-(Benzylamino)ethoxy]-4-methoxyphenyl}-4-(1*H*-indol-5-ylamino)nicotinonitril,
4-(1*H*-Indol-5-ylamino)-5-(4-methoxy-3-{2-[(2-methoxyethyl)amino]ethoxy}phenyl)nicotinonitril,
4-(1*H*-Indol-5-ylamino)-5-(4-methoxy-3-{2-[(5-methyl-1,3,4-thiadiazol-2-yl)amino]ethoxy}phenyl)nicotinonitril,
4-(1*H*-Indol-5-ylamino)-5-(3-{5-[(4-methylpiperazin-1-yl)methyl]-2-thienyl}phenyl)nicotinonitril,
4-(1*H*-Indol-5-ylamino)-5-{3-[5-(morpholin-4-ylmethyl)-2-thienyl]phenyl}nicotinonitril,
4-(1*H*-Indol-5-ylamino)-5-{3-[5-(piperidin-1-ylmethyl)-2-thienyl]phenyl}nicotinonitril,
5-(3-{5-[(Dimethylamino)methyl]-2-thienyl}phenyl)-4-(1*H-*indol-5-ylamino)nicotinonitril,
5-(3-Brom-4-methoxyphenyl)-4-(1*H*-indol-5-ylamino)nicotinonitril,
4-(1*H*-Indol-5-ylamino)-5-[4-methoxy-3-(2-thienyl)phenyl]nicotinonitril,
5-(4'-Chlor-6-methoxybiphenyl-3-yl)-4-(1*H*-indol-5-ylamino)nicotinonitril,
5-(3'-Chlor-6-methoxybiphenyl-3-yl)-4-(1*H*-indol-5-ylamino)nicotinonitril,
5'-[5-Cyano-4-(1*H*-indol-5-ylamino)pyridin-3-yl]-N-cyclopentyl-2'-methoxybiphenyl-4-carboxamid,
5-(2'-Chlor-6-methoxybiphenyl-3-yl)-4-(1*H*-indol-5-ylamino)nicotinonitril,
5-[3-(Benzyloxy)phenyl]-4-(1*H*-indol-5-ylamino)nicotinonitril,
5-[4-(Benzyloxy)phenyl]-4-(1*H*-indol-5-ylamino)nicotinonitril,
3'-[5-Cyano-4-(1*H*-indol-5-ylamino)pyridin-3-yl]-N-methylbiphenyl-4-carboxamid,
N-Butyl-3'-[5-cyano-4-(1*H*-indol-5-ylamino)pyridin-3-yl]biphenyl-4-carboxamid,
3'-[5-Cyano-4-(1*H*-indol-5-ylamino)pyridin-3-yl]-N-(1-ethylpropyl)biphenyl-4-carboxamid,
3'-[5-Cyano-4-(1*H*-indol-5-ylamino)pyridin-3-yl]-N-(2-hydroxyethyl)biphenyl-4-carboxamid,
3'-[5-Cyano-4-(1*H*-indol-5-ylamino)pyridin-3-yl]-N-(2-methoxyethyl)biphenyl-4-carboxamid,
3'-[5-Cyano-4-(1*H*-indol-5-ylamino)pyridin-3-yl]-N-cyclopropylbiphenyl-4-carboxamid,
3'-[5-Cyano-4-(1*H*-indol-5-ylamino)pyridin-3-yl]-N-cyclohexylbiphenyl-4-carboxamid,
3'-[5-Cyano-4-(1*H*-indol-5-ylamino)pyridin-3-yl]-N-(2-pyrrolidin-1-ylethyl)biphenyl-4-carboxamid,
N-Benzyl-3'-[5-cyano-4-(1*H*-indol-5-ylamino)pyridin-3-yl]biphenyl-4-carboxamid,
4-(1*H*-Indol-5-ylamino)-5-[4'-(pyrrolidin-1-ylcarbonyl)biphenyl-3-yl]nicotinonitril,
4-(1*H*-Indol-5-ylamino)-5-[4'-(morpholin-4-ylcarbonyl)biphenyl-3-yl]nicotinonitril,
4-(1*H*-Indol-5-ylamino)-5-{4'-[(4-methylpiperazin-1-yl)carbonyl]biphenyl-3-yl}nicotinonitril,
3'-[5-Cyano-4-(1*H*-indol-5-ylamino)pyridin-3-yl]-N-cyclopentylbiphenyl-3-carboxamid,
3'-[5-Cyano-4-(1*H*-indol-5-ylamino)pyridin-3-yl]-N-(4-hydroxybutyl)biphenyl-4-carboxamid,
3'-[5-Cyano-4-(1*H*-indol-5-ylamino)pyridin-3-yl]-N-(3-hydroxypropyl)biphenyl-4-carboxamid,
3'-[5-Cyano-4-(1*H*-indol-5-ylamino)pyridin-3-yl]-N-[2-(methylamino)ethyl]biphenyl-4-carboxamid,
3'-[5-Cyano-4-(1*H*-indol-5-ylamino)pyridin-3-yl]-N-(pyridin-2-ylmethyl)biphenyl-4-carboxamid,
3'-[5-Cyano-4-(1*H*-indol-5-ylamino)pyridin-3-yl]-N-(pyridin-3-ylmethyl)biphenyl-4-carboxamid,
3'-[5-Cyano-4-(1*H*-indol-5-ylamino)pyridin-3-yl]-N-(pyridin-4-ylmethyl)biphenyl-4-carboxamid,
N-Butyl-3'-[5-cyano-4-(1*H*-indol-4-ylamino)pyridin-3-yl]biphenyl-4-carboxamid,
3'-[5-Cyano-4-(1*H*-indol-4-ylamino)pyridin-3-yl]-N-(2-hydroxyethyl)biphenyl-4-carboxamid,
5-(3,4-Dimethoxyphenyl)-4-[(7-methyl-1*H*-indol-5-yl)amino]nicotinonitril,
5-(3,4-Dimethoxyphenyl)-4-[(4-methyl-1*H*-indol-5-yl)amino]nicotinonitril,
5-(3,4-Dimethoxyphenyl)-4-[1*H*-indol-5-yl(methyl)amino]nicotinonitril,
5-(3,4-Dimethoxyphenyl)-4-(1*H*-indol-5-yloxy)nicotinonitril,
5-(3,4-Dimethoxyphenyl)-4-(1*H*-indol-5-yl)nicotinonitril,
5-(1-Benzofuran-2-yl)-4-[(4-methyl-1*H*-indol-5-yl)amino]nicotinonitril,
5-(5-Formyl-1-benzothien-2-yl)-4-(1*H*-indol-4-ylamino)nicotinonitril,
5-{5-[(Dimethylamino)methyl]-1-benzothien-2-yl}-4-(1*H*-indol-4-ylamino)nicotinonitril,
5-(4-Hydroxyphenyl)-4-(1*H*-indol-4-ylamino)nicotinonitril,
5-(4-{[(2*S*)-2-Amino-3-phenylpropyl]oxy}phenyl)-4-(1*H*-indol-4-ylamino)nicotinonitril,
5-(5-Formyl-1-benzothien-2-yl)-4-[(4-methyl-1*H*-indol-5-yl)amino]nicotinonitril,
4-[(4-Methyl-1*H*-indol-5-yl)amino]-5-[5-(piperidin-1-ylmethyl)-1-benzothien-2-yl]nicotinonitril,
5-[5-(Hydroxymethyl)-1-benzothien-2-yl]-4-[(4-methyl-1*H-*indol-5-yl)amino]nicotinonitril,
4-(1*H*-Indol-5-ylamino)-5-[4-methoxy-3-(2-morpholin-4-ylethoxy)phenyl]nicotinonitril,
4-(1*H*-Indol-5-ylamino)-5-{4-methoxy-3-[2-(4-methylpiperazin-1-yl)ethoxy]phenyl}nicotinonitril,
4-(1*H*-Indol-5-ylamino)-5-[4-methoxy-3-(2-piperazin-1-ylethoxy)phenyl]nicotinonitril,
4-(1*H*-Indol-5-ylamino)-5-[4-methoxy-3-(2-thiomorpholin-4-ylethoxy)phenyl]nicotinonitril,
5-{3-[2-(4-Ethylpiperazin-1-yl)ethoxy]-4-methoxyphenyl}-4-(1*H*-indol-5-ylamino)nicotinonitril,
4-(1*H*-Indol-5-ylamino)-5-[4-methoxy-3-(2-piperidin-1-ylethoxy)phenyl]nicotinonitril,
5-{3-[2-(Dimethylamino)ethoxy]-4-methoxyphenyl}-4-(1*H*-indol-5-ylamino)nicotinonitril,
5-(3-{2-[Bis(2-hydroxyethyl)amino]ethoxy}-4-methoxyphenyl)-4-(1*H*-indol-5-ylamino)nicotinonitril,
5-{3-[2-(4-Hydroxypiperidin-1-yl)ethoxy]-4-methoxyphenyl}-4-(1*H*-indol-5-ylamino)nicotinonitril,
4-(1*H*-Indol-5-ylamino)-5-(4-methoxy-3-{2-[(pyridin-3-ylmethyl)amino]ethoxy}phenyl)nicotinonitril,
4-(1*H*-Indol-5-ylamino)-5-(4-methoxy-3-{2-[(pyridin-4-ylmethyl)amino]ethoxy}phenyl)nicotinonitril,
4-(1*H*-Indol-5-ylamino)-5-(4-methoxy-3-{2-[(pyridin-2-ylmethyl)amino]ethoxy}phenyl)nicotinonitril,
4-(1*H*-Indol-5-ylamino)-5-(4-methoxy-3-{2-[(2-phenylethyl)amino]ethoxy}phenyl)nicotinonitril,
5-{3-[2-(Cyclopentylamino)ethoxy]-4-methoxyphenyl}-4-(1*H-*indol-5-ylamino)nicotinonitril,
5-{3-[2-(Cyclohexylamino)ethoxy]-4-methoxyphenyl}-4-(1*H-*indol-5-ylamino)nicotinonitril,
5-(3-{2-[(2-Furylmethyl)amino]ethoxy}-4-methoxyphenyl)-4-(1*H-*indol-5-ylamino)nicotinonitril,
5-(2-Brom-4,5-dimethoxyphenyl)-4-(1*H*-indol-5-ylamino)nicotinonitril,
5-(2-Brom-4,5-dimethoxyphenyl)-4-[(4-methyl-1*H*-indol-5-yl)amino]nicotinonitril,
4-(1*H*-Indol-4-ylamino)-5-[4-methoxy-3-(2-piperidin-1-ylethoxy)phenyl]nicotinonitril,
5-(3-{2-[(2-Hydroxyethyl)amino]ethoxy}-4-methoxyphenyl)-4-(1*H*-indol-4-ylamino)nicotinonitril,
4-(1*H*-Indol-5-ylamino)-5-(4-methoxy-3-{2-[(3-phenylpropyl)amino]ethoxy}phenyl)nicotinonitril,
4-(1*H*-Indol-5-ylamino)-5-(4-methoxy-3-{2-[(2-pyridin-2-ylethyl)amino]ethoxy}phenyl)nicotinonitril,
4-(1*H*-Indol-5-ylamino)-5-(4-methoxy-3-{2-[(2-pyridin-3-ylethyl)amino]ethoxy}phenyl)nicotinonitril,
4-(1*H*-Indol-5-ylamino)-5-(4-methoxy-3-{2-[(2-pyridin-4-ylethyl)amino]ethoxy}phenyl)nicotinonitril,
5-[3-(Dimethylamino)phenyl]-4-(1*H*-indol-4-ylamino)nicotinonitril,
4-(1*H*-Indol-4-ylamino)-5-[3-(methylsulfonyl)phenyl]nicotinonitril,
N-{3-[5-Cyano-4-(1*H*-indol-4-ylamino)pyridin-3-yl]phenyl}methansulfonamid,
4-(1*H*-Indol-5-ylamino)-5-phenylnicotinonitril,
4-(1*H*-Indol-5-ylamino)-5-(3-thienyl)nicotinonitril,
4-(1*H*-Indol-5-ylamino)-3,3'-bipyridin-5-carbonitril,
5-(1,3-Benzodioxol-5-yl)-4-(1*H*-indol-5-ylamino)nicotinonitril,
4-(1*H*-Indol-5-ylamino)-3,4'-bipyridin-5-carbonitril,
5-(3-Furyl)-4-(1*H*-indol-5-ylamino)nicotinonitril,
5-(1*H*-Indol-5-yl)-4-(1*H*-indol-5-ylamino)nicotinonitril,
5-(2,3-Dihydro-1,4-benzodioxin-6-yl)-4-(1*H*-indol-5-ylamino)nicotinonitril,
4-(1*H*-Indol-5-ylamino)-5-pyrimidin-5-ylnicotinonitril,
4-(1*H*-Indol-5-ylamino)-5-(2-methoxypyrimidin-5-yl)nicotinonitril,
4-(1*H*-Indol-5-ylamino)-5-(2-thienyl)nicotinonitril,
5-(1-Benzofuran-2-yl)-4-(1*H*-indol-5-ylamino)nicotinonitril,
5-(3,5-Dimethylisoxazol-4-yl)-4-(1*H*-indol-5-ylamino)nicotinonitril,
5-[3-(Hydroxymethyl)phenyl]-4-(1*H*-indol-4-ylamino)nicotinonitril,
5-{3-[(Dimethylamino)methyl]phenyl}-4-(1*H*-indol-4-ylamino)nicotinonitril,
4-(1*H*-Indol-4-ylamino)-5-{5-[(prop-2-in-1-ylamino)methyl]-1-benzothien-2-yl}nicotinonitril,
5-{5-[(Butylamino)methyl]-1-benzothien-2-yl}-4-(1*H*-indol-4-ylamino)nicotinonitril,
5-(5-{[(2-Hydroxyethyl)amino]methyl}-1-benzothien-2-yl)-4-(1*H*-indol-4-ylamino)nicotinonitril,
5-(5-{[(3-Hydroxypropyl)amino]methyl}-1-benzothien-2-yl)-4-(1*H*-indol-4-ylamino)nicotinonitril,
4-(1*H*-Indol-4-ylamino)-5-(5-{[(3-methoxypropyl)amino]methyl}-1-benzothien-2-yl)nicotinonitril,
5-(5-{[(4-Hydroxybutyl)amino]methyl}-1-benzothien-2-yl)-4-(1*H*-indol-4-ylamino)nicotinonitril,
5-{5-[(Cyclopropylamino)methyl]-1-benzothien-2-yl}-4-(1*H-*indol-4-ylamino)nicotinonitril,
5-(5-{[(Cyclopropylmethyl)amino]methyl}-1-benzothien-2-yl)-4-(1*H*-indol-4-ylamino)nicotinonitril,
4-(1*H*-Indol-4-ylamino)-5-[5-(pyrrolidin-1-ylmethyl)-1-benzothien-2-yl]nicotinonitril,
4-(1*H*-Indol-4-ylamino)-5-[5-(morpholin-4-ylmethyl)-1-benzothien-2-yl]nicotinonitril,
4-(1*H*-Indol-4-ylamino)-5-(5-{[(2-morpholin-4-ylethyl)amino]methyl}-1-benzothien-2-yl)nicotinonitril,
4-(1*H*-Indol-4-ylamino)-5-[5-(piperidin-1-ylmethyl)-1-benzothien-2-yl]nicotinonitril,
5-(5-{[4-(Hydroxymethyl)piperidin-1-yl]methyl}-1-benzothien-2-yl)-4-(1*H*-indol-4-ylamino)nicotinonitril,
5-[5-(Hydroxymethyl)-1-benzothien-2-yl]-4-(1*H*-indol-4-ylamino)nicotinonitril,
5-{5-[(Benzylamino)methyl]-1-benzothien-2-yl}-4-(1*H*-indol-4-ylamino)nicotinonitril,
4-(1*H*-Indol-4-ylamino)-5-(5-{[(2-phenylethyl)amino]methyl}-1-benzothien-2-yl)nicotinonitril,
4-(1*H*-Indol-4-ylamino)-5-(5-{[(pyridin-2-ylmethyl)amino]methyl}-1-benzothien-2-yl)nicotinonitril,
4-(1*H*-Indol-4-ylamino)-5-(5-{[(pyridin-3-ylmethyl)amino]methyl}-1-benzothien-2-yl)nicotinonitril,
4-(1*H*-Indol-4-ylamino)-5-(5-{[(pyridin-4-ylmethyl)amino]methyl}-1-benzothien-2-yl)nicotinonitril,
5-[4-(Dimethylamino)phenyl]-4-(pyridin-3-ylamino)nicotinonitril,
5-[4-(Dimethylamino)phenyl]-4-(1*H*-indazol-5-ylamino)nicotinonitril,
5-[4-(Dimethylamino)phenyl]-4-(1*H*-indazol-6-ylamino)nicotinonitril,
5-[4-(Dimethylamino)phenyl]-4-[(5-hydroxy-1*H*-pyrazol-3-yl)amino]nicotinonitril,
4-(1*H*-Indazol-5-ylamino)-5-(3-methoxyphenyl)nicotinonitril,
4-(1*H*-Indazol-6-ylamino)-5-(3-methoxyphenyl)nicotinonitril,
4-[(5-Hydroxy-1*H*-pyrazol-3-yl)amino]-5-(3-methoxyphenyl)nicotinonitril,
5-(3-Bromphenyl)-4-[(4-methyl-1*H*-indol-5-yl)amino]nicotinonitril,
4-(1*H*-Indol-4-ylamino)-5-[4-methoxy-3-(2-morpholin-4-ylethoxy)phenyl]nicotinonitril,
4-(1*H*-Indol-4-ylamino)-5-{4-methoxy-3-[2-(4-methylpiperazin-1-yl)ethoxy]phenyl}nicotinonitril,
5-{3-[2-(Dimethylamino)ethoxy]-4-methoxyphenyl}-4-(1*H*-indol-4-ylamino)nicotinonitril,
5-{3-[2-(4-Hydroxypiperidin-1-yl)ethoxy]-4-methoxyphenyl}-4-(1*H*-indol-4-ylamino)nicotinonitril,
5-[3-(2-Chlorethoxy)-4-methoxyphenyl]-4-(1*H*-indol-4-ylamino)nicotinonitril,
4-[(4-Methyl-1*H*-indol-5-yl)amino]-5-[3-(2-thienyl)phenyl]nicotinonitril,
5-(3,4-Dimethoxyphenyl)-4-[(4-ethyl-1*H*-indol-5-yl)amino]nicotinonitril,
5-[3-(5-Formyl-2-thienyl)phenyl]-4-[(4-methyl-1*H*-indol-5-yl)amino]nicotinonitril,
5-(3-{5-[(Dimethylamino)methyl]-2-thienyl}phenyl)-4-[(4-methyl-1*H*-indol-5-yl)amino]nicotinonitril,
3'-[5-Cyano-4-(1*H*-indol-4-ylamino)pyridin-3-yl]-N-(4-hydroxybutyl)biphenyl-4-carboxamid,
3'-{5-Cyano-4-[(4-methyl-1*H*-indol-5-yl)amino]pyridin-3-yl}-*N-*(4-hydroxybutyl)biphenyl-4-carboxamid,
4-(1*H*-Indol-4-ylamino)-5-[3-(trifluormethyl)phenyl]nicotinonitril,
5-(3-Cyanophenyl)-4-(1*H*-indol-4-ylamino)nicotinonitril,
3-[5-Cyano-4-(1*H*-indol-4-ylamino)pyridin-3-yl]-*N*,*N-*dimethylbenzamid,
3-[5-Cyano-4-(1*H*-indol-4-ylamino)pyridin-3-yl]-*N*,*N-*dimethylbenzolsulfonamid,
3-[5-Cyano-4-(1*H*-indol-4-ylamino)pyridin-3-yl]benzamid,
5-(3-{5-[(Dimethylamino)methyl]-2-thienyl}phenyl)-4-(1*H-*indol-4-ylamino)nicotinonitril,
2-[5-Cyano-4-(1*H*-indol-4-ylamino)pyridin-3-yl]-*N*,*N-*dimethylbenzolsulfonamid,
N-{4-[5-Cyano-4-(1*H*-indol-4-ylamino)pyridin-3-yl]phenyl}methansulfonamid,
5-(1-Benzofuran-2-yl)-4-(1*H*-indol-4-ylamino)nicotinonitril,
5-Dibenzo[b,d]furan-4-yl-4-(1*H*-indol-4-ylamino)nicotinonitril,
4-(1*H*-Indol-4-ylamino)-5-{1-[(4-methylphenyl)sulfonyl]-1*H-*indol-3-yl}nicotinonitril,
5-(1-Benzothien-2-yl)-4-(1*H*-indol-4-ylamino)nicotinonitril,
4-(1*H*-Indol-4-ylamino)-5-(4-methoxyphenyl)nicotinonitril,
4-(1*H*-Indol-4-ylamino)-5-(2-methoxyphenyl)nicotinonitril,
5-(1*H*-Indol-2-yl)-4-(1*H*-indol-4-ylamino)nicotinonitril,
4-[5-Cyano-4-(1*H*-indol-4-ylamino)pyridin-3-yl]-*N*,*N-*dimethylbenzolsulfonamid,
3-[5-Cyano-4-(1*H*-indol-4-ylamino)pyridin-3-yl]benzoesäure,
5-[3-(Aminomethyl)phenyl]-4-(1*H*-indol-4-ylamino)nicotinonitril,
5-(3,4-Dimethoxyphenyl)-4-[(2-oxo-2,3-dihydro-1*H*-indol-4-yl)amino]nicotinonitril,
4-[5-Cyano-4-(1*H*-indol-4-ylamino)pyridin-3-yl]-*N*-(2-methoxyethyl)benzamid,
4-(1*H*-Indol-4-ylamino)-5-(4-methoxy-3-{2-[(3-phenylpropyl)amino]ethoxy}phenyl)nicotinonitril,
5-[4-(2-Chlorethoxy)phenyl]-4-(1*H*-indol-4-ylamino)nicotinonitril,
5-[3-(5-Formyl-2-thienyl)phenyl]-4-(1*H*-indol-4-ylamino)nicotinonitril,
4-(1*H*-Indol-4-ylamino)-5-[4-(2-morpholin-4-ylethoxy)phenyl]nicotinonitril,
4-(1*H*-Indol-4-ylamino)-5-{4-[2-(4-methylpiperazin-1-yl)ethoxy]phenyl}nicotinonitril,
5-(3,4-Dimethoxyphenyl)-4-[(5-methyl-1*H*-indol-4-yl)amino]nicotinonitril,
5-(2,4-Dimethoxyphenyl)-4-(1*H*-indol-4-ylamino)nicotinonitril,
4-(1*H*-Indol-4-ylamino)-5-[4-methoxy-3-(2-{[3-(2-oxopyrrolidin-1-yl)propyl]amino}ethoxy)phenyl]nicotinonitril,
5-[3-(2-{[2-(1*H*-Imidazol-4-yl)ethyl]amino}ethoxy)-4-methoxyphenyl]-4-(1*H*-indol-4-ylamino)nicotinonitril,
4-(1*H*-Indol-4-ylamino)-5-(4-methoxy-3-{2-[(3-pyrrolidin-1-ylpropyl)amino]ethoxy}phenyl)nicotinonitril,
4-(1*H*-Indol-4-ylamino)-5-[4-methoxy-3-(2-{[2-(1-methylpyrrolidin-2-yl)ethyl]amino}ethoxy)phenyl]nicotinonitril,
5-(4-Methoxy-3-{2-[(3-phenylpropyl)amino]ethoxy}phenyl)-4-[(4-methyl-1H-indol-5-yl)amino]nicotinonitril,
5-{4-Methoxy-3-[2-(4-methylpiperazin-1-yl)ethoxy]phenyl}-4-[(4-methyl-1*H*-indol-5-yl)amino]nicotinonitril,
5-(3-Methoxy-4-{2-[(3-phenylpropyl)amino]ethoxy}phenyl)-4-[(4-methyl-1*H*-indol-5-yl)amino]nicotinonitril,
5-{4-[2-(Dimethylamino)ethoxy]-3-methoxyphenyl}-4-[(4-methyl-1*H*-indol-5-yl)amino]nicotinonitril,
5-(3-Bromphenyl)-4-[(2-oxo-2,3-dihydro-1*H*-indol-4-yl)amino]nicotinonitril,
4-(1*H*-Indol-4-ylamino)-5-(4-{2-[(3-phenylpropyl)amino]ethoxy}phenyl)nicotinonitril,
5-[4-(2-Chlorethoxy)phenyl]-4-[(4-methyl-1*H*-indol-5-yl)amino]nicotinonitril,
5-(3,4-Dimethoxyphenyl)-4-[(2-methyl-1*H*-indol-4-yl)amino]nicotinonitril,
4-[(4-Methyl-1*H*-indol-5-yl)amino]-5-{4-[2-(4-methylpiperazin-1-yl)ethoxy]phenyl}nicotinonitril,
4-[(4-Methyl-1*H*-indol-5-yl)amino]-5-[4-(2-morpholin-4-ylethoxy)phenyl]nicotinonitril,
5-(1-Benzofuran-3-yl)-4-(1*H*-indol-4-ylamino)nicotinonitril,
4-(1*H*-Indol-4-ylamino)-5-(4-methoxy-3-{2-[(2-phenylethyl)amino]ethoxy}phenyl)nicotinonitril,
4-(1*H*-Indol-4-ylamino)-5-(4-methoxy-3-{2-[(2-pyridin-3-ylethyl)amino]ethoxy}phenyl)nicotinonitril,
4-[(4-Methyl-1*H*-indol-5-yl)amino]-5-(4-{2-[(3-phenylpropyl)amino]ethoxy}phenyl)nicotinonitril,
6'-[3-(Dimethylamino)propoxyl-4-(1*H*-indol-4-ylamino)-3,3'-bipyridin-5-carbonitril,
6'-[3-(Dimethylamino)propoxy]-4-[(4-methyl-1*H*-indol-5-yl)amino]-3,3'-bipyridin-5-carbonitril,
5-(3-Hydroxyphenyl)-4-(1*H*-indol-4-ylamino)nicotinonitril,
4-(1*H*-Indol-4-ylamino)-5-[5-(piperazin-1-ylmethyl)-1-benzothien-2-yl]nicotinonitril,
N-({2-[5-Cyano-4-(1*H*-indol-4-ylamino)pyridin-3-yl]-1-benzothien-5-yl}methyl)-b-alaninamid,
4-(1*H*-Indol-4-ylamino)-6'-[(2-morpholin-4-ylethyl)amino]-3,3'-bipyridin-5-carbonitril,
4-[(4-Methyl-1*H*-indol-5-yl)amino]-6'-[(2-morpholin-4-ylethyl)amino]-3,3'-bipyridin-5-carbonitril,
5-{2-Chlor-4-[2-(dimethylamino)ethoxy]phenyl}-4-[(4-methyl-1*H*-indol-5-yl)amino]nicotinonitril,
4-(1*H*-Indol-4-ylamino)-5-(4-methoxy-3-{2-[(3-morpholin-4-ylpropyl)amino]ethoxy}phenyl)nicotinonitril,
5-[3-(2-{[3-(1*H*-Imidazol-1-yl)propyl]amino}ethoxy)-4-methoxyphenyl]-4-(1*H*-indol-4-ylamino)nicotinonitril,
5-(3-{[(2*S*)-2-Amino-3-phenylpropyl]oxy}phenyl)-4-(1*H*-indol-4-ylamino)nicotinonitril,
5-{5-[(Benzylamino)methyl]-1-benzothien-2-yl}-4-[(4-methyl-1*H*-indol-5-yl)amino]nicotinonitril,
5-{4-[2-(4-Butylpiperazin-1-yl)ethoxy]phenyl}-4-[(4-methyl-1*H*-indol-5-yl)amino]nicotinonitril,
5-[5-(1,3-Dioxan-2-yl)-1-benzofuran-2-yl]-4-(1*H*-indol-4-ylamino)nicotinonitril,
5-[5-(1,3-Dioxan-2-yl)-1-benzofuran-2-yl]-4-[(4-methyl-1*H-*indol-5-yl)amino]nicotinonitril,
5-(2-Chlor-4-methoxyphenyl)-4-[(4-methyl-1*H*-indol-5-yl)amino]nicotinonitril,
5-[4-(2-Chlorethoxy)-3-methoxyphenyl]-4-[(4-methyl-1*H*-indol-5-yl)amino]nicotinonitril,
5-(1-Benzofuran-2-yl)-4-(1*H*-indazol-5-ylamino)nicotinonitril,
5-(4-Hydroxyphenyl)-4-[(4-methyl-1*H*-indol-5-yl)amino]nicotinonitril,
5-(2-Chlor-6-methoxyphenyl)-4-[(4-methyl-1*H*-indol-5-yl)amino]nicotinonitril,
5-[3-Methoxy-4-(2-piperidin-1-ylethoxy)phenyl]-4-[(4-methyl-1*H*-indol-5-yl)amino]nicotinonitril,
5-{3-Methoxy-4-[2-(4-methylpiperazin-1-yl)ethoxy]phenyl}-4-[(4-methyl-1*H*-indol-5-yl)amino]nicotinonitril,
5-[3-Methoxy-4-(2-morpholin-4-ylethoxy)phenyl]-4-[(4-methyl-1*H*-indol-5-yl)amino]nicotinonitril,
5-(3,4-Dimethoxyphenyl)-4-(1*H*-indazol-5-ylamino)nicotinonitril,
5-(2,3-Dichlorphenyl)-4-[(4-methyl-1*H*-indol-5-yl)amino]nicotinonitril,
5-(4-Brom-2-fluorphenyl)-4-[(4-methyl-1*H*-indol-5-yl)amino]nicotinonitril,
5-{5-[(Dimethylamino)methyl]-1-benzofuran-2-yl}-4-[(4-methyl-1*H*-indol-5-yl)amino]nicotinonitril,
5-(5-{[(2-Hydroxyethyl)amino]methyl}-1-benzofuran-2-yl)-4-[(4-methyl-1*H*-indol-5-yl)amino]nicotinonitril,
5-(5-{[(3-Hydroxypropyl)amino]methyl}-1-benzofuran-2-yl)-4-[(4-methyl-1*H*-indol-5-yl)amino]nicotinonitril,
5-(5-{[(2,3-Dihydroxypropyl)amino]methyl}-1-benzofuran-2-yl)-4-[(4-methyl-1*H*-indol-5-yl)amino]nicotinonitril,
5-(5-{[(2,3-Dihydroxypropyl)(methyl)amino]methyl}-1-benzofuran-2-yl)-4-[(4-methyl-1*H*-indol-5-yl)amino]nicotinonitril,
5-{5-[(Cyclohexylamino)methyl]-1-benzofuran-2-yl}-4-[(4-methyl-1*H*-indol-5-yl)amino]nicotinonitril,
4-[(4-Methyl-1*H*-indol-5-yl)amino]-5-[5-(morpholin-4-ylmethyl)-1-benzofuran-2-yl]nicotinonitril,
4-[(4-Methyl-1*H*-indol-5-yl)amino]-5-[5-(pyrrolidin-1-ylmethyl)-1-benzofuran-2-yl]nicotinonitril,
4-[(4-Methyl-1*H*-indol-5-yl)amino]-5-(5-{[(2-pyrrolidin-1-ylethyl)amino]methyl}-1-benzofuran-2-yl)nicotinonitril,
4-[(4-Methyl-1*H*-indol-5-yl)amino]-5-[5-({[(1-methylpiperidin-4-yl)methyl]aminolmethyl)-1-benzofuran-2-yl]nicotinonitril,
5-(5-{[4-(Hydroxymethyl)piperidin-1-yl]methyl}-1-benzofuran-2-yl)-4-[(4-methyl-1*H*-indol-5-yl)amino]nicotinonitril,
4-[(4-Methyl-1*H*-indol-5-yl)amino]-5-{5-[(4-pyrrolidin-1-ylpiperidin-1-yl)methyl]-1-benzofuran-2-yl}nicotinonitril,
5-[5-(1,4'-Bipiperidin-1'-ylmethyl)-1-benzofuran-2-yl]-4-[(4-methyl-1*H*-indol-5-yl)amino]nicotinonitril,
4-[(4-Methyl-1*H*-indol-5-yl)amino]-5-{5-[(4-morpholin-4-ylpiperidin-1-yl)methyl]-1-benzofuran-2-yl}nicotinonitril,
5-[5-({4-[2-(Dimethylamino)ethyl]piperazin-1-yl}methyl)-1-benzofuran-2-yl]-4-[(4-methyl-1*H*-indol-5-yl)amino]nicotinonitril,
4-[(4-Methyl-1*H*-indol-5-yl)amino]-5-{5-[(4-pyridin-2-ylpiperazin-1-yl)methyl]-1-benzofuran-2-yl}nicotinonitril,
4-[(4-Methyl-1*H*-indol-5-yl)amino]-5-(5-{[(pyridin-2-ylmethyl)amino]methyl}-1-benzofuran-2-yl)nicotinonitril,
4-[(4-Methyl-1*H*-indol-5-yl)amino]-5-(5-{[(pyridin-3-ylmethyl)amino]methyl}-1-benzofuran-2-yl)nicotinonitril,
4-[(4-Methyl-1*H*-indol-5-yl)amino]-5-(5-{[(pyridin-4-ylmethyl)amino]methyl}-1-benzofuran-2-yl)nicotinonitril,
4-[(4-Methyl-1*H*-indol-5-yl)amino]-5-[5-(morpholin-4-ylmethyl)-2-furyl]nicotinonitril,
4-[(4-Methyl-1*H*-indol-5-yl)amino]-5-[5-(piperidin-1-ylmethyl)-2-furyl]nicotinonitril,
5-[5-(1,4'-Bipiperidin-1'-ylmethyl)-2-furyl]-4-[(4-methyl-1*H-*indol-5-yl)amino]nicotinonitril,
4-[(4-Methyl-1*H*-indol-5-yl)amino]-5-{5-[(4-pyrrolidin-1-ylpiperidin-1-yl)methyl]-2-furyl}nicotinonitril,
4-[(4-Methyl-1*H*-indol-5-yl)amino]-5-{5-[(4-morpholin-4-ylpiperidin-1-yl)methyl]-2-furyl}nicotinonitril,
5-{5-[(Diethylamino)methyl]-2-furyl}-4-[(4-methyl-1*H*-indol-5-yl)amino]nicotinonitril,
5-{5-[(Dibutylamino)methyl]-2-furyl}-4-[(4-methyl-1*H*-indol-5-yl)amino]nicotinonitril,
5-{5-[(Benzylamino)methyl]-2-furyl}-4-[(4-methyl-1*H*-indol-5-yl)amino]nicotinonitril,
4-[(4-Methyl-1*H*-indol-5-yl)amino]-5-(5-{[(3-phenylpropyl)amino]methyl}-2-furyl)nicotinonitril,
4-[(4-Methyl-1*H*-indol-5-yl)amino]-5-[5-(pyrrolidin-1-ylmethyl)-2-furyl]nicotinonitril,
4-[(4-Methyl-1*H*-indol-5-yl)amino]-5-[5-(thiomorpholin-4-ylmethyl)-2-furyl]nicotinonitril,
5-(3,4-Dimethoxyphenyl)-4-[(6-methyl-1*H*-indol-5-yl)amino]nicotinonitril,
5-(1-Benzofuran-2-yl)-4-[(6-methyl-1*H*-indol-5-yl)amino]nicotinonitril,
5-(4-{2-[(2-Hydroxyethyl)amino]ethoxy}phenyl)-4-[(4-methyl-1*H*-indol-5-yl)amino]nicotinonitril,
5-(4-{2-[(3-Hydroxypropyl)amino]ethoxy}phenyl)-4-[(4-methyl-1*H*-indol-5-yl)amino]nicotinonitril,
5-(4-{2-[(2-Ethoxyethyl)amino]ethoxy}phenyl)-4-[(4-methyl-1*H-*indol-5-yl)amino]nicotinonitril,
5-[4-(2-{[2-(Dimethylamino)ethyl]amino}ethoxy)phenyl]-4-[(4-methyl-1*H*-indol-5-yl)amino]nicotinonitril,
4-[(4-Methyl-1*H*-indol-5-yl)amino]-5-[4-(2-pyrrolidin-1-ylethoxy)phenyl]nicotinonitril,
5-{4-[2-(Benzylamino)ethoxy]phenyl}-4-[(4-methyl-1*H*-indol-5-yl)amino]nicotinonitril,
4-[(4-Methyl-1*H*-indol-5-yl)amino]-5-(4-{2-[(1-methylpiperidin-4-yl)amino]ethoxy}phenyl)nicotinonitril,
4-[(4-Methyl-1*H*-indol-5-yl)amino]-5-[4-(2-{[(1-methylpiperidin-4-yl)methyl]amino}ethoxy)phenyl]nicotinonitril,
5-(4-{2-[4-(Hydroxymethyl)piperidin-1-yl]ethoxy}phenyl)-4-[(4-methyl-1*H*-indol-5-yl)amino]nicotinonitril,
4-[(4-Methyl-1*H*-indol-5-yl)amino]-5-{4-[2-(4-pyrrolidin-1-ylpiperidin-1-yl)ethoxy]phenyllnicotinonitril,
4-[(4-Methyl-1*H*-indol-5-yl)amino]-5-{4-[2-(4-morpholin-4-ylpiperidin-1-yl)ethoxy]phenyl}nicotinonitril,
5-{4-[2-(4-Ethylpiperazin-1-yl)ethoxy]phenyl}-4-[(4-methyl-1*H*-indol-5-yl)amino]nicotinonitril,
5-{4-[2-(4-Methyl-1,4-diazepan-1-yl)ethoxy]phenyl}-4-[(4-methyl-1*H*-indol-5-yl)amino]nicotinonitril,
5-(4-{2-[4-(2-Hydroxyethyl)piperazin-1-yl]ethoxy}phenyl)-4-[(4-methyl-1*H*-indol-5-yl)amino]nicotinonitril,
5-[4-(2-{4-[2-(Dimethylamino)ethyl]piperazin-1-yl}ethoxy)phenyl]-4-[(4-methyl-1*H*-indol-5-yl)amino]nicotinonitril,
5-[4-(2-{[3-(1*H*-Imidazol-1-yl)propyl]amino}ethoxy)phenyl]-4-[(4-methyl-1*H*-indol-5-yl)amino]nicotinonitril,
4-[(4-Methyl-1*H*-indol-5-yl)amino]-5-{4-[2-(4-pyridin-2-ylpiperazin-1-yl)ethoxy]phenyl}nicotinonitril,
4-[(4-Methyl-1*H*-indol-5-yl)amino]-5-{4-[2-(4-pyridin-4-ylpiperazin-1-yl)ethoxy]phenyllnicotinonitril,
4-[(4-Methyl-1*H*-indol-5-yl)amino]-5-(4-{2-[(pyridin-2-ylmethyl)amino]ethoxy}phenyl)nicotinonitril,
4-[(4-Methyl-1*H*-indol-5-yl)amino]-5-(4-{2-[(pyridin-3-ylmethyl)amino]ethoxy}phenyl)nicotinonitril,
4-[(4-Methyl-1*H*-indol-5-yl)amino]-5-(4-{2-[(pyridin-4-ylmethyl)amino]ethoxy}phenyl)nicotinonitril,
4-[(4-Methyl-1*H*-indol-5-yl)amino]-5-{4-[2-(4-phenylpiperidin-1-yl)ethoxylphenyl}nicotinonitril,
5-(5-{[4-(Dimethylamino)piperidin-1-yl]methyl}-2-furyl)-4-[(4-methyl-1*H*-indol-5-yl)amino]nicotinonitril,
5-{5-[(4-Isopropylpiperazin-1-yl)methyl]-2-furyl}-4-[(4-methyl-1*H*-indol-5-yl)amino]nicotinonitril,
4-[(4-Methyl-1*H*-indol-5-yl)amino]-5-{5-[2-(4-methylpiperazin-1-yl)ethoxy]-1-benzofuran-2-yl}nicotinonitril,
5-(3,4-Dimethoxyphenyl)-4-{[2-(4-methylpiperazin-1-yl)ethyl]amino}nicotinonitril,
5-(3,4-Dimethoxyphenyl)-4-{[3-(4-methylpiperazin-1-yl)propyl]amino}nicotinonitril,
4-({[*trans*-4-(Aminomethyl)cyclohexyl]methyl}amino)-5-(3,4-dimethoxyphenyl)nicotinonitril,
4-{[(*trans*-4-Aminocyclohexyl)methyl]amino}-5-(3,4-dimethoxyphenyl)nicotinonitril,
4-({[*cis*-3-(Aminomethyl)cyclohexyl]methyl}amino)-5-(3,4-dimethoxyphenyl)nicotinonitril,
5-(3,4-Dimethoxyphenyl)-4-[(2-piperidin-4-ylethyl)amino]nicotinonitril,
5-(3,4-Dimethoxyphenyl)-4-[(piperidin-4-ylmethyl)amino]nicotinonitril,
4-[(*cis*-4-Aminocyclohexyl)amino]-5-(3,4-dimethoxyphenyl)nicotinonitril,
5-(3,4-Dimethoxyphenyl)-4-{[2-(1-methylpiperidin-4-yl)ethyl]amino}nicotinonitril,
5-(3,4-Dimethoxyphenyl)-4-{[(1-methylpiperidin-4-yl)methyl]amino}nicotinonitril,
4-[(4-Methyl-1*H*-indol-5-yl)amino]-5-{4-[(4-methylpiperazin-1-yl)methyl]phenyl}nicotinonitril,
4-[(4-Methyl-1*H*-indol-5-yl)amino]-5-[4-(pyrrolidin-1-ylmethyl)phenyl]nicotinonitril,
4-[(4-Methyl-1*H*-indol-5-yl)amino]-5-[4-(morpholin-4-ylmethyl)phenyl]nicotinonitril,
5-{4-[(Dimethylamino)methyl]phenyl}-4-[(4-methyl-1H-indol-5-yl)amino]nicotinonitril,
4-[(4-Methyl-1*H*-indol-5-yl)amino]-2'-(morpholin-4-ylmethyl)-3,4'-bipyridin-5-carbonitril,
4-[(4-Methyl-1*H*-indol-5-yl)amino]-5-{3-[(4-methylpiperazin-1-yl)methyl]phenyl}nicotinonitril,
4-[(4-Methyl-1*H*-indol-5-yl)amino]-5-[3-(pyrrolidin-1-ylmethyl)phenyl]nicotinonitril,
4-[(4-Methyl-1*H*-indol-5-yl)amino]-5-[3-(morpholin-4-ylmethyl)phenyl]nicotinonitril,
5-{3-[(Dimethylamino)methyl]phenyl}-4-[(4-methyl-1*H*-indol-5-yl)amino]nicotinonitril,
5-(4-{[(2R)-2-Amino-3-phenylpropyl]oxy}phenyl)-4-(1*H*-indol-4-ylamino)nicotinonitril,
5-{2-Fluor-4-[(4-methylpiperazin-1-yl)methyl]phenyl}-4-[(4-methyl-1*H*-indol-5-yl)amino]nicotinonitril,
5-[4-(3-Chlorpropoxy)phenyl]-4-[(4-methyl-1*H*-indol-5-yl)amino]nicotinonitril,
4-[(4-Methyl-1*H*-indol-5-yl)amino]-5-[5-(piperidin-1-ylmethyl)-1-benzofuran-2-yl]nicotinonitril,
4-[(4-Methyl-1*H*-indol-5-yl)amino]-5-{5-[(4-methylpiperazin-1-yl)methyl]-1-benzofuran-2-yl}nicotinonitril,
5-(5-Formyl-2-thienyl)-4-[(4-methyl-1*H*-indol-5-yl)amino]nicotinonitril,
4-[(4-Methyl-1*H*-indol-5-yl)amino]-5-{5-[(4-methylpiperazin-1-yl)methyl]-2-thienyl}nicotinonitril,
5-(5-Formyl-1-benzofuran-2-yl)-4-[(4-methyl-1*H*-indol-5-yl)amino]nicotinonitril,
5-(3-Methyl-1-benzofuran-2-yl)-4-[(4-methyl-1*H*-indol-5-yl)amino]nicotinonitril,
4-[(4-Methyl-1*H*-indol-5-yl)amino]-3,4'-bipyridin-5-carbonitril,
4-[(4-Methyl-1*H*-indol-5-yl)amino]-5-{5-[(4-methylpiperazin-1-yl)methyl]-2-furyl}nicotinonitril,
2'-Chlor-4-[(4-methyl-1*H*-indol-5-yl)amino]-3,4'-bipyridin-5-carbonitril,
5-{2-Chlor-4-[2-(4-methylpiperazin-1-yl)ethoxy]phenyl}-4-[(4-methyl-1*H*-indol-5-yl)amino]nicotinonitril,
2'-Chlor-4-[(4-methyl-1*H*-indol-5-yl)amino]-3,3'-bipyridin-5-carbonitril,
4-[(4-Methyl-1*H*-indol-5-yl)amino]-5-{4-[3-(4-methylpiperazin-1-yl)propoxy]phenyl}nicotinonitril,
4-[(4-Methyl-1*H*-indol-5-yl)amino]-5-[4-(3-morpholin-4-ylpropoxy)phenyl]nicotinonitril,
4-[(4-Methyl-1*H*-indol-5-yl)amino]-5-[4-(3-piperidin-1-ylpropoxy)phenyl]nicotinonitril,
5-{4-[3-(Dimethylamino)propoxy]phenyl}-4-[(4-methyl-1*H*-indol-5-yl)amino]nicotinonitril,
5-[3,4-Bis(2-methoxyethoxy)phenyl]-4-[(4-methyl-1*H*-indol-5-yl)amino]nicotinonitril,
5-[3-Methoxy-4-(2-methoxyethoxy)phenyl]-4-[(4-methyl-1*H-*indol-5-yl)amino]nicotinonitril,
5-[5-(Hydroxymethyl)-1-benzofuran-2-yl]-4-[(4-methyl-1*H-*indol-5-yl)amino]nicotinonitril,
5-[4-(2-Methoxyethoxy)phenyl]-4-[(4-methyl-1*H*-indol-5-yl)amino]nicotinonitril,
5-[3-(2-Methoxyethoxy)phenyl]-4-[(4-methyl-1*H*-indol-5-yl)amino]nicotinonitril,
5-(5-Formyl-1-benzofuran-2-yl)-4-(1*H*-indol-5-ylamino)nicotinonitril,
4-(1*H*-Indol-5-ylamino)-5-{5-[(4-methylpiperazin-1-yl)methyl]-1-benzofuran-2-yl}nicotinonitril,
5-{5-[(4-Cyclopentylpiperazin-1-yl)methyl]-2-furyl}-4-[(4-methyl-1*H*-indol-5-yl)amino]nicotinonitril,
5-{5-[(1,1-Dioxidothiomorpholin-4-yl)methyl]-2-furyl}-4-[(4-methyl-1*H*-indol-5-yl)amino]nicotinonitril,
5-(3,4-Dimethoxyphenyl)-4-(1*H*-pyrrolo[2,3-b]pyridin-5-ylamino)nicotinonitril,
5-(5-Formyl-2-furyl)-4-[(4-methyl-1H-indol-5-yl)amino]nicotinonitril,
5-[4-(4-Chlorbutoxy)phenyl]-4-[(4-methyl-1*H*-indol-5-yl)amino]nicotinonitril,
4-[(4-Methyl-1*H*-indol-5-yl)amino]-5-{4-[4-(4-methylpiperazin-1-yl)butoxy]phenyl}nicotinonitril,
4-[(4-Chlor-1*H*-pyrrolo[2,3-b]pyridin-5-yl)amino]-5-[3-methoxy-4-(2-methoxyethoxy)phenyl]nicotinonitril,
5-[4-(2-Chlorethoxy)phenyl]-4-[(6-methyl-1*H*-indol-5-yl)amino]nicotinonitril,
5-(5-Formyl-1-benzofuran-2-yl)-4-[(6-methyl-1*H*-indol-5-yl)amino]nicotinonitril,
4-[(6-Methyl-1*H*-indol-5-yl)amino]-5-{4-[2-(4-methylpiperazin-1-yl)ethoxy]phenyl}nicotinonitril,
4-[(6-Methyl-1*H*-indol-5-yl)amino]-5-{5-[(4-methylpiperazin-1-yl)methyl]-1-benzofuran-2-yl}nicotinonitril,
4-[(7-Chlor-4-methyl-1*H*-indol-5-yl)amino]-5-(3,4-dimethoxyphenyl)nicotinonitril,
5-[5-(Hydroxymethyl)-1-benzofuran-2-yl]-4-[(6-methyl-1*H-*indol-5-yl)amino]nicotinonitril,
5-{5-[(Diethylamino)methyl]-1-benzofuran-2-yl}-4-[(6-methyl-1*H*-indol-5-yl)amino]nicotinonitril,
5-[3-(4-Chlorbutoxy)phenyl]-4-[(4-methyl-1*H*-indol-5-yl)amino]nicotinonitril,
4-[(4-Methyl-1*H*-indol-5-yl)amino]-5-{3-[4-(4-methylpiperazin-1-yl)butoxy]phenyl}nicotinonitril,
4-[(4-Methyl-1*H*-indol-5-yl)amino]-5-{5-[(4-methylpiperazin-1-yl)methyl]-3-furyl}nicotinonitril,
4-[(6-Methyl-1*H*-indol-5-yl)amino]-5-[4-(2-piperidin-1-ylethoxy)phenyl]nicotinonitril,
5-{4-[2-(4-Hydroxypiperidin-1-yl)ethoxy]phenyl}-4-[(6-methyl-1*H*-indol-5-yl)amino]nicotinonitril,
4-[(6-Methyl-1*H*-indol-5-yl)amino]-5-{4-[2-(4-pyrrolidin-1-ylpiperidin-1-yl)ethoxy]phenyl}nicotinonitril,
4-[(4-Methyl-1*H*-indol-5-yl)amino]-6'-morpholin-4-yl-3,3'-bipyridin-5-carbonitril,
4-[(4-Methyl-1*H*-indol-5-yl)amino]-6'-piperidin-1-yl-3,3'-bipyridin-5-carbonitril,
4-[(4-Methyl-1*H*-indol-5-yl)amino]-5-pyrimidin-5-ylnicotinonitril,
4-[(4-Methyl-1*H*-indol-5-yl)amino]-5-(2-piperidin-1-ylpyrimidin-5-yl)nicotinonitril,
4-[(4-Methyl-1*H*-indol-5-yl)amino]-5-(2-morpholin-4-ylpyrimidin-5-yl)nicotinonitril,
4-[(4-Methyl-1*H*-indol-5-yl)amino]-5-(2-pyrrolidin-1-ylpyrimidin-5-yl)nicotinonitril,
5-[2-(Dimethylamino)pyrimidin-5-yl]-4-[(4-methyl-1*H*-indol-5-yl)amino]nicotinonitril,
5-(1-Benzothien-2-yl)-4-[(4-methyl-1*H*-indol-5-yl)amino]nicotinonitril,
5-[4-(2-Chlorethoxy)phenyl]-4-(1*H*-indol-5-ylamino)nicotinonitril,
5-(5-Formyl-3-thienyl)-4-[(4-methyl-1*H*-indol-5-yl)amino]nicotinonitril,
5-(4-Formyl-2-furyl)-4-[(4-methyl-1*H*-indol-5-yl)amino]nicotinonitril,
4-[(4-Methyl-1*H*-indol-5-yl)amino]-5-{5-[(4-methylpiperazin-1-yl)methyl]-3-thienyl}nicotinonitril,
4-[(4-Methyl-1*H*-indol-5-yl)amino]-3,3'-bipyridin-5-carbonitril,
4-(1*H*-Indol-5-ylamino)-5-{4-[2-(4-methylpiperazin-1-yl)ethoxy]phenyl}nicotinonitril,
4-[(4-Methyl-1*H*-indol-5-yl)amino]-5-{5-[(4-methylpiperazin-1-yl)methyl]-1-benzothien-2-yl}nicotinonitril,
4-[(4-Chlor-1*H*-pyrrolo[2,3-b]pyridin-5-yl)amino]-5-(3,4-dimethoxyphenyl)nicotinonitril,
4-[(*trans*-4-Aminocyclohexyl)amino]-5-(3,4-dimethoxyphenyl)nicotinonitril,
1-Butyl-3-(4-{5-cyano-4-[(4-methyl-1*H*-indol-5-yl)amino]pyridin-3-yl}phenyl)harnstoff,
Methyl-(4-{5-cyano-4-[(4-methyl-1*H*-indol-5-yl)amino]pyridin-3-yl}phenyl)carbamat,
Benzyl-(4-{5-cyano-4-[(4-methyl-1*H*-indol-5-yl)amino]pyridin-3-yl}-2-fluorphenyl)carbamat,
4-Methoxybenzyl-(4-{5-cyano-4-[(4-methyl-1*H*-indol-5-yl)amino]pyridin-3-yl}-2-fluorphenyl)carbamat,
4-[(7-Chlor-4-methyl-1*H*-indol-5-yl)amino]-5-{5-[(4-methylpiperazin-1-yl)methyl]-1-benzofuran-2-yl}nicotinonitril,
5-(3,4-Dimethoxyphenyl)-4-[(4-methyl-1*H*-indol-7-yl)amino]nicotinonitril,
5-(1-Benzofuran-2-yl)-4-[(7-chlor-4-methyl-1*H*-indol-5-yl)amino]nicotinonitril,
4-[(7-Chlor-4-methyl-1*H*-indol-5-yl)amino]-5-(5-formyl-1-benzofuran-2-yl)nicotinonitril,
5-[4-(2-Chlorethoxy)phenyl]-4-[(7-chlor-4-methyl-1*H*-indol-5-yl)amino]nicotinonitril,
4-[(7-Chlor-4-methyl-1*H*-indol-5-yl)amino]-5-{4-[2-(dimethylamino)ethoxy]phenyl}nicotinonitril,
4-[(7-Chlor-4-methyl-1*H*-indol-5-yl)amino]-5-{4-[2-(4-methylpiperazin-1-yl)ethoxy]phenyl}nicotinonitril,
*tert*-Butyl-4-[(2-{5-cyano-4-[(4-methyl-1*H*-indol-5-yl)amino]pyridin-3-yl}-1-benzofuran-5-yl)methyl]piperazin-1-carboxylat,
5-(2-Formyl-1-methyl-1*H*-imidazol-5-yl)-4-[(4-methyl-1*H*-indol-5-yl)amino]nicotinonitril,
4-[(4-Methyl-1*H*-indol-5-yl)amino]-5-(5-{[4-(methylsulfonyl)piperazin-1-yl]methyl}-1-benzofuran-2-yl)nicotinonitril,
4-[(4-Methyl-1*H*-indol-5-yl)amino]-5-{1-methyl-2-[(4-methylpiperazin-1-yl)methyl]-1*H*-imidazol-5-yl}nicotinonitril,
4-[(4-Methyl-1*H*-indol-5-yl)amino]-5-[5-(piperazin-1-ylmethyl)-1-benzofuran-2-yl]nicotinonitril,
4-[(4-Methyl-1*H*-indol-5-yl)amino]-5-(1,3-thiazol-2-yl)nicotinonitril,
5-(1-Methyl-1*H*-imidazol-2-yl)-4-[(4-methyl-1*H*-indol-5-yl)amino]nicotinonitril,
4-[(4-Methyl-1*H*-indol-5-yl)amino]-5-(1,3-thiazol-4-yl)nicotinonitril,
5-(3,4-Dimethoxyphenyl)-4-(1*H*-indol-7-ylamino)nicotinonitril,
5-(3,4-Dimethoxyphenyl)-4-[(4-methoxy-1*H*-indol-5-yl)amino]nicotinonitril,
5-(3,4-Dimethoxyphenyl)-4-[(4-fluor-1*H*-indol-5-yl)amino]nicotinonitril,
4-[(7-Chlor-4-methyl-1*H*-indol-5-yl)amino]-5-[5-(piperazin-1-ylmethyl)-1-benzofuran-2-yl]nicotinonitril,
*tert*-Butyl-4-[(2-{4-[(7-chlor-4-methyl-1*H*-indol-5-yl)amino]-5-cyanopyridin-3-yl}-1-benzofuran-5-yl)methyl]piperazine-1-carboxylat,
5-(3,4-Dimethoxyphenyl)-4-[(2,4-dimethyl-1*H*-indol-5-yl)amino]nicotinonitril,
5-{2-[(Dimethylamino)methyl]phenyl}-4-[(4-methyl-1*H*-indol-5-yl)amino]nicotinonitril,
5-(5-Formyl-2-methoxyphenyl)-4-[(4-methyl-1*H*-indol-5-yl)amino]nicotinonitril,
5-{2-Methoxy-5-[(4-methylpiperazin-1-yl)methyl]phenyl}-4-[(4-methyl-1*H*-indol-5-yl)amino]nicotinonitril,
5-{5-[(4-Ethylpiperazin-1-yl)methyl]-1-benzofuran-2-yl}-4-[(4-methyl-1*H*-indol-5-yl)amino]nicotinonitril,
4-[(4-Methyl-1*H*-indol-5-yl)amino]-5-{5-[(4-methyl-4-oxidopiperazin-1-yl)methyl]-1-benzofuran-2-yl}nicotinonitril,
5-(3,4-Dimethoxyphenyl)-4-[(1,4-dimethyl-1*H*-indol-5-yl)amino]nicotinonitril,
3-{5-Cyano-4-[(4-methyl-1*H*-indol-5-yl)amino]pyridin-3-yl}benzoesäure,
5-(2-Methoxyphenyl)-4-[(4-methyl-1*H*-indol-5-yl)amino]nicotinonitril,
5-(3-Methoxyphenyl)-4-[(4-methyl-1*H*-indol-5-yl)amino]nicotinonitril,
5-(4-Methoxyphenyl)-4-[(4-methyl-1*H*-indol-5-yl)amino]nicotinonitril,
4-[(4-Methyl-1*H*-indol-5-yl)amino]-5-phenylnicotinonitril,
4-[(4-Methyl-1*H*-indol-5-yl)amino]-5-(2-thienyl)nicotinonitril,
4-[(4-Methyl-1*H*-indol-5-yl)amino]-5-(3-thienyl)nicotinonitril,
5-(3-Furyl)-4-[(4-methyl-1*H*-indol-5-yl)amino]nicotinonitril,
5-(1-Methyl-1*H*-imidazol-5-yl)-4-[(4-methyl-1*H*-indol-5-yl)amino]nicotinonitril,
4'-[(4-Methyl-1*H*-indol-5-yl)amino]-2,3'-bipyridin-5'-carbonitril,
1-(4-{5-Cyano-4-[(4-methyl-1*H*-indol-5-yl)amino]pyridin-3-yl}phenyl)-3-cyclopropylharnstoff,
1-(4-{5-Cyano-4-[(4-methyl-1*H*-indol-5-yl)amino]pyridin-3-yl}phenyl)-3-methylharnstoff,
3-(4-{5-Cyano-4-[(4-methyl-1*H*-indol-5-yl)amino]pyridin-3-yl}phenyl)-1,1-dimethylharnstoff,
N-(4-{5-Cyano-4-[(4-methyl-1*H*-indol-5-yl)amino]pyridin-3-yl}phenyl)morpholin-4-carboxamid,
4-[(4-Methyl-1*H*-indol-5-yl)amino]-5-(4-nitrophenyl)nicotinonitril,
5-(4-Aminophenyl)-4-[(4-methyl-1*H*-indol-5-yl)amino]nicotinonitril,
5-(3-Aminophenyl)-4-[(4-methyl-1*H*-indol-5-yl)amino]nicotinonitril,
5-(2-Aminophenyl)-4-[(4-methyl-1*H*-indol-5-yl)amino]nicotinonitril,
5-[4-(Dimethylamino)phenyl]-4-[(4-methyl-1*H*-indol-5-yl)amino]nicotinonitril,
5-[3-(Dimethylamino)phenyl]-4-[(4-methyl-1*H*-indol-5-yl)amino]nicotinonitril,
N-(4-{5-Cyano-4-[(4-methyl-1*H*-indol-5-yl)amino]pyridin-3-yl}phenyl)acetamid,
N-(2-{5-Cyano-4-[(4-methyl-1*H*-indol-5-yl)amino]pyridin-3-yl}phenyl)acetamid,
N-(3-{5-Cyano-4-[(4-methyl-1*H*-indol-5-yl)amino]pyridin-3-yl}phenyl)acetamid,
N-(4-{5-Cyano-4-[(4-methyl-1*H*-indol-5-yl)amino]pyridin-3-yl}phenyl)-2-methylpropanamid,
4-{5-Cyano-4-[(4-methyl-1*H*-indol-5-yl)amino]pyridin-3-yl}-N-methylbenzamid,
4-[(4-Methyl-1*H*-indol-5-yl)amino]-5-(1-naphthyl)nicotinonitril,
4-[(4-Methyl-1*H*-indol-5-yl)amino]-5-(2-naphthyl)nicotinonitril,
4-[(4-Methyl-1*H*-indol-5-yl)amino]-5-(1-methyl-1*H*-pyrazol-5-yl)nicotinonitril,
4-[(4-Methyl-1*H*-indol-5-yl)amino]-5-(1*H*-pyrazol-4-yl)nicotinonitril,
5-(1-Benzothiophen-3-yl)-4-[(4-methyl-1*H*-indol-5-yl)amino]nicotinonitril,
5-(1-Methyl-1*H*-indol-2-yl)-4-[(4-methyl-1*H*-indol-5-yl)amino]nicotinonitril,
5-(1*H*-Indol-5-yl)-4-[(4-methyl-1*H*-indol-5-yl)amino]nicotinonitril,
5-(1*H*-Indol-6-yl)-4-[(4-methyl-1*H*-indol-5-yl)amino]nicotinonitril,
4-[(4-Methyl-1*H*-indol-5-yl)amino]-5-chinolin-3-ylnicotinonitril,
4-[(4-Methyl-1*H*-indol-5-yl)amino]-5-chinolin-8-ylnicotinonitril,
5-(1-Benzofuran-5-yl)-4-[(4-methyl-1*H*-indol-5-yl)amino]nicotinonitril,
4-(4-Methyl-1*H*-indol-5-ylamino)-5-(chinolin-5-yl)nicotinonitril,
5-(Dibenzo[b,d]thiophen-3-yl)-4-(4-methyl-1*H*-indol-5-ylamino)nicotinonitril,
5-(Benzo[b]thiophen-5-yl)-4-(4-methyl-1*H*-indol-5-ylamino)nicotinonitril,
5-(1*H*-Indol-4-yl)-4-(4-methyl-1*H*-indol-5-ylamino)nicotinonitril,
4-[(2,4-Dimethyl-1*H*-indol-5-yl)amino]-5-{5-[(4-methylpiperazin-1-yl)methyl]-1-benzofuran-2-yl}nicotinonitril,
4-[(2,4-Dimethyl-1*H*-indol-5-yl)amino]-5-[5-(piperazin-1-ylmethyl)-1-benzofuran-2-yl]nicotinonitril,
4-[(4-Methyl-1*H*-indol-5-yl)amino]-5-{6-[(4-methylpiperazin-1-yl)methyl]-1-benzofuran-2-yl}nicotinonitril,
4-[(4-Methyl-1*H*-indol-5-yl)amino]-5-[6-(piperazin-1-ylmethyl)-1-benzofuran-2-yl]nicotinonitril,
4-[(4-Methyl-1*H*-indol-5-yl)amino]-5-{4-[(piperazin-1-yl)methyl]phenyl}nicotinonitril,
4-[(2,4-Dimethyl-1*H*-indol-5-yl)amino]-5-{4-[(4-methylpiperazin-1-yl)methyl]phenyl}nicotinonitril,
4-(2,4-Dimethyl-1*H*-indol-5-ylamino)-5-{3-[2-(4-methylpiperazin-1-yl)ethoxy]phenyl}nicotinonitril,
5-{4-[2-(Dimethylamino)ethoxy]-3-methoxyphenyl}-4-[(2,4-dimethyl-1*H*-indol-5-yl)amino]nicotinonitril,
4-[(2,4-Dimethyl-1*H*-indol-5-yl)amino]-5-{4-[2-(4-methylpiperazin-1-yl)ethoxy]phenyl}nicotinonitril,
4-[(4-Methyl-1*H*-indol-5-yl)amino]-5-{4-[2-(piperazin-1-yl)ethoxy]phenyl}nicotinonitril,
4-(4-Methyl-1*H*-indol-5-ylamino)-2'-((4-methylpiperazin-1-yl)methyl)-3,4'-bipyridin-5-carbonitril,
4-(4-Methyl-1*H*-indol-5-ylamino)-2'-((piperazin-1-yl)methyl)-3,4'-bipyridin-5-carbonitril,
4'-(4-Methyl-1*H*-indol-5-ylamino)-5-((4-methylpiperazin-1-yl)methyl)-2,3'-bipyridin-5'-carbonitril,
4'-(4-Methyl-1*H*-indol-5-ylamino)-5-(morpholinomethyl)-2,3'-bipyridin-5'-carbonitril,
4'-(4-Methyl-1*H*-indol-5-ylamino)-5-((piperazin-1-yl)methyl)-2,3'-bipyridin-5'-carbonitril,
4'-(4-Methyl-1*H*-indol-5-ylamino)-6-((4-methylpiperazin-1-yl)methyl)-2,3'-bipyridin-5'-carbonitril,
4'-(4-Methyl-1*H*-indol-5-ylamino)-6-(morpholinomethyl)-2,3'-bipyridin-5'-carbonitril,
4'-(4-Methyl-1*H*-indol-5-ylamino)-6-((piperazin-1-yl)methyl)-2,3'-bipyridin-5'-carbonitril,
4'-(4-Methyl-1*H*-indol-5-ylamino)-4-(morpholinomethyl)-2,3'-bipyridin-5'-carbonitril,
4'-(4-Methyl-1*H*-indol-5-ylamino)-4-((4-methylpiperazin-1-yl)methyl)-2,3'-bipyridin-5'-carbonitril,
4'-(4-Methyl-1*H*-indol-5-ylamino)-4-((piperazin-1-yl)methyl)-2,3'-bipyridin-5'-carbonitril,
4-(4-Methyl-1*H*-indol-5-ylamino)-5'-((4-methylpiperazin-1-yl)methyl)-3,3'-bipyridin-5-carbonitril,
4-(4-Methyl-1*H*-indol-5-ylamino)-5'-((piperazin-1-yl)methyl)-3,3'-bipyridin-5-carbonitril,
4-(4-Methyl-1*H*-indol-5-ylamino)-5'-(morpholinomethyl)-3,3'-bipyridin-5-carbonitril,
4-(4-Methyl-1*H*-indol-5-ylamino)-6'-((4-methylpiperazin-1-yl)methyl)-3,3'-bipyridin-5-carbonitril,
4-(4-Methyl-1*H*-indol-5-ylamino)-6'-((piperazin-1-yl)methyl)-3,3'-bipyridin-5-carbonitril,
4-(4-Methyl-1*H*-indol-5-ylamino)-6'-(morpholinomethyl)-3,3'-bipyridin-5-carbonitril,
4-(4-Methyl-1*H*-indol-5-ylamino)-5-(3-(piperazin-1-ylmethyl)phenyl)nicotinonitril,
4-(4-Methyl-1*H*-indol-5-ylamino)-5-(4-(piperazin-1-ylmethyl)phenyl)nicotinonitril,
4-({[*cis*-4-(Aminomethyl)cyclohexyl]methyl}amino)-5-(3,4-dimethoxyphenyl)nicotinonitril und
5-(3,4-Dimethoxyphenyl)-4-(1*H*-indol-5-ylamino)nicotinonitril 1-oxid.

21. Verbindung nach einem der Ansprüche 1-20, wobei die Verbindung in Form eines Enantiomers vorliegt.

22. Pharmazeutische Zusammensetzung, umfassend die Verbindung nach einem der Ansprüche 1-21 und einen pharmazeutisch annehmbaren Träger oder Hilfsstoff.

23. Verbindung nach einem der Ansprüche 1-21 oder ein pharmazeutisch annehmbares Salz, Hydrat oder Ester davon zur Verwendung bei der Behandlung oder Inhibierung eines pathologischen Zustands oder einer Erkrankung, der/die durch eine Proteinkinase vermittelt wird bei einem Säugetier.

24. Verbindung zur Verwendung nach Anspruch 23, wobei die Proteinkinase Proteinkinase C ist.

25. Verbindung zur Verwendung nach Anspruch 23 oder 24, wobei der pathologische Zustand oder die Erkrankung eine entzündliche Erkrankung oder eine Autoimmunerkrankung ist, ausgewählt aus Asthma, Colitis, multipler Sklerose, Psoriasis, Arthritis, rheumatoider Arthritis, Osteoarthritis und Gelenkentzündung.

26. Verbindung wie in einem der Ansprüche 1 bis 21 beansprucht oder ein pharmazeutisch annehmbares Salz, Hydrat oder Ester davon zur Verwendung als Medikament.

27. Verwendung einer Verbindung wie in einem der Ansprüche 1 bis 21 beansprucht oder eines pharmazeutisch annehmbaren Salzes, Hydrats oder Esters davon zur Herstellung eines Medikaments zur Behandlung oder Inhibierung eines pathologischen Zustands oder einer Erkrankung, der/die durch eine Proteinkinase vermittelt wird bei einem Säugetier.

## Revendications

1. Composé de formule I ou de formule I' : 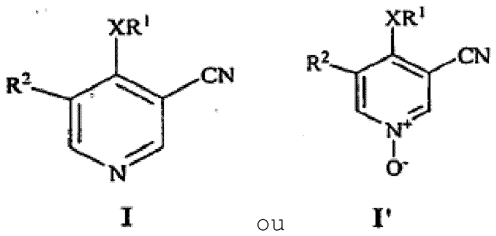 ou un sel, hydrate ou ester de celui-ci pharmaceutiquement acceptable, dans lesquelles :
X est sélectionné parmi a) -NR³-Y-, b) -O-Y-, c) -S(O)ₘ-Y-, d) -S(O)ₘNR³-Y-, e) -NR³S(O)ₘ-Y-, f) -C(O)NR³-Y-, g) -C(S)NR³-Y- , h) -NR³C(O)-Y-, i) -NR³C(S)-Y-, j) -C(O)O-Y-, k) -OC(O)-Y- et 1) une liaison covalente ;
Y, à chaque occurrence, est indépendamment sélectionné parmi a) un groupe alkyle divalent en C₁ à C₁₀, b) un groupe alcényle divalent en C₂ à C₁₀, c) un groupe alcynyle divalent en C₂ à C₁₀, d) un groupe halogénoalkyle divalent en C₁ à C₁₀ et e) une liaison covalente ;
R¹ est sélectionné parmi a) un groupe alkyle en C₁ à C₁₀, b) un groupe cycloalkyle en C₃ à C₁₄, c) un groupe cyclohétéroalkyle de 3 à 14 chaînons, d) un groupe aryle polycyclique en C₈ à C₁₄ et e) un groupe hétéroaryle de 5 à 14 chaînons, dans lequel chaque groupe est optionnellement substitué par 1 à 4 -Y-R⁴ ;
R² est un groupe aryle en C₆ à C₁₄ ou un groupe hétéroaryle de 5 à 14 chaînons, dans lequel chaque groupe est optionnellement substitué par 1 à 4 groupes sélectionnés indépendamment parmi -Y-R⁴ ou -O-Y-R⁴ ;
R³ est sélectionné parmi a) H, b) un groupe alkyle en C₁ à C₁₀, c) un groupe alcényle en C₂ à C₁₀, d) un groupe alcynyle en C₂ à C₁₀ et e) un groupe halogénoalkyle en C₁ à C₁₀ ;
R⁴, à chaque occurrence, est indépendamment sélectionné parmi a) un halogène, b) -CN, c) -NO₂, d) un oxo, e) -O-Y-R⁵, f) -NR⁶-Y-R⁷, g) -N(O)R⁶Y-R⁷, h) -S(O)ₘ-Y-R⁵, i) -S(O)ₘO-Y-R⁵, j)-S(O)ₘNR⁶-Y-R⁷, k) -C(O)-Y-R⁵, l) -C(O)O-Y-R⁵, m) -C(O)NR⁶-Y-R⁷, n) -C(S)NR⁶-Y-R⁷, o) un groupe alkyle en C₁ à C₁₀, p) un groupe alcényle en C₂ à C₁₀, q) un groupe alcynyle en C₂ à C₁₀, r) un groupe halogénoalkyle en C₁ à C₁₀, s) un groupe cycloalkyle en C₃ à C₁₄, t) un groupe aryle en C₆ à C₁₄, u) un groupe cyclohétéroalkyle de 3 à 14 chaînons et v) un groupe hétéroaryle de 5 à 14 chaînons, dans lequel chacun parmi o) à v) est optionnellement substitué par 1 à 4 groupes -Y-R⁸;
R⁵, à chaque occurrence, est indépendamment sélectionné parmi a) H, b) -C(O)R⁹, c) -C(O)OR⁹, d) un groupe alkyle en C₁ à C₁₀, e) un groupe alcényle en C₂ à C₁₀, f) un groupe alcynyle en C₂ à C₁₀, g) un groupe halogénoalkyle en C₁ à C₁₀, h) un groupe cycloalkyle en C₃ à C₁₄, i) un groupe aryle en C₆ à C₁₄, j) un groupe cyclohétéroalkyle de 3 à 14 chaînons et k) un groupe hétéroaryle de 5 à 14 chaînons, dans lequel chacun parmi d) à k) est optionnellement substitué par 1 à 4 groupes -Y-R⁸ ;
R⁶ et R⁷, à chaque occurrence, sont sélectionnés indépendamment parmi a) H, b) -O-Y-R⁹, c) -S(O)ₘ-Y-R⁹, d) -S(O)ₘO-Y-R⁹, e) -C(O)-Y-R⁹, f) -C(O)O-Y-R⁹, g) -C(O)NR¹⁰-Y-R¹¹, h) -C(S)NR¹⁰-Y-R¹¹, i) un groupe alkyle en C₁ à C₁₀, j) un groupe alcényle en C₂ à C₁₀, k) un groupe alcynyle en C₂ à C₁₀, 1) un groupe halogénoalkyle en C₁ à C₁₀, m) un groupe cycloalkyle en C₃ à C₁₄, n) un groupe aryle en C₆ à C₁₄, o) un groupe cyclohétéroalkyle de 3 à 14 chaînons et p) un groupe hétéroaryle de 5 à 14 chaînons ; dans lequel chacun parmi i) à p) est optionnellement substitué par 1 à 4 groupes -Y-R⁸;
R⁸, à chaque occurrence, est indépendamment sélectionné parmi a) un halogène, b) -CN, c) -NO₂, d) un oxo, e) -O-Y-R⁹, f) -NR¹⁰-Y-R¹¹, g) -N(O)R¹⁰-Y-R¹¹, h) -S(O)ₘ-Y-R⁹, i) -S(O)ₘO-Y-R⁹, j) -S(O)ₘNR¹⁰-Y-R¹¹, k) -C(O)-Y-R⁹, 1) -C(O)O-Y-R⁹, m) -C(O)NR¹⁰-YR¹¹, n) -C(S)NR¹⁰-Y-R¹¹, o) un groupe alkyle en C₁ à C₁₀, p) un groupe alcényle en C₂ à C₁₀, q) un groupe alcynyle en C₂ à C₁₀, r) un groupe halogénoalkyle en C₁ à C₁₀, s) un groupe cycloalkyle en C₃ à C₁₄, t) un groupe aryle en C₆ à C₁₄, u) un groupe cyclohétéroalkyle de 3 à 14 chaînons et v) un groupe hétéroaryle de 5 à 14 chaînons, dans lequel chacun parmi o) à v) est optionnellement substitué par 1 à 4 groupes -Y-R¹² ;
R⁹, à chaque occurrence, est indépendamment sélectionné parmi a) H, b) un -C(O)-alkyle en C₁ à C₁₀, c) -C(O)OH, d) un -C(O)O-alkyle en C₁ à C₁₀, e) un groupe alkyle en C₁ à C₁₀, f) un groupe alcényle en C₂ à C₁₀, g) un groupe alcynyle en C₂ à C₁₀, h) un groupe halogénoalkyle en C₁ à C₁₀, i) un groupe cycloalkyle en C₃ à C₁₄, j) un groupe aryle en C₆ à C₁₄, k) un groupe cyclohétéroalkyle de 3 à 14 chaînons et 1) un groupe hétéroaryle de 5 à 14 chaînons, dans lequel chacun parmi le groupe alkyle en C₁ à C₁₀, le groupe alcényle en C₂ à C₁₀, le groupe alcynyle en C₂ à C₁₀, le groupe halogénoalkyle en C₁ à C₁₀, le groupe cycloalkyle en C₃ à C₁₄, le groupe aryle en C₆ à C₁₄, le groupe cyclohétéroalkyle de 3 à 14 chaînons et le groupe hétéroaryle de 5 à 14 chaînons est optionnellement substitué par 1 à 4 groupes -Y-R¹²;
R¹⁰ et R¹¹, à chaque occurrence, sont sélectionnés indépendamment parmi a) H, b) -OH, c) -SH, d) -NH₂, e) un -NH-alkyle en C₁ à C₁₀, f) un -N (alkyle en C₁ à C₁₀)₂, g) un -S(O)ₘ-alkyle en C₁ à C₁₀, h) -S(O)₂OH, i) un -S(O)ₘ-Oalkyle en C₁ à C₁₀, j) un -C(O)-alkyle en C₁ à C₁₀, k) -C(O)OH, 1) un -C(O)-Oalkyle en C₁ à C₁₀, m) -C(O)NH₂, n) un -C(O)NH-alkyle en C₁ à C₁₀, o) un -C(O)N(alkyle en C₁ à C₁₀)₂, p) un -C(S)NH₂, q) un -C (S) NH-alkyle en C₁ à C₁₀, r) un -C (S) N (alkyle en C₁ à C₁₀)₂, s) un groupe alkyle en C₁ à C₁₀, t) un groupe alcényle en C₂ à C₁₀, u) un groupe alcynyle en C₂ à C₁₀, v) un groupe alcoxy en C₁ à C₁₀, w) un groupe halogénoalkyle en C₁ à C₁₀, x) un groupe cycloalkyle en C₃ à C₁₄, y) un groupe aryle en C₆ à C₁₄, z) un groupe cyclohétéroalkyle de 3 à 14 chaînons et aa) un groupe hétéroaryle de 5 à 14 chaînons, dans lequel chacun parmi le groupe alkyle en C₁ à C₁₀, le groupe alcényle en C₂ à C₁₀, le groupe alcynyle en C₂ à C₁₀, le groupe alcoxy en C₁ à C₁₀, le groupe halogénoalkyle en C₁ à C₁₀, le groupe cycloalkyle en C₃ à C₁₄, le groupe aryle en C₆ à C₁₄, le groupe cyclohétéroalkyle de 3 à 14 chaînons et le groupe hétéroaryle de 5 à 14 chaînons est optionnellement substitué par 1 à 4 groupes -Y-R¹²;
R¹², à chaque occurrence, est indépendamment sélectionné parmi a) un halogène, b) -CN, c) -NO₂, d) un oxo, e) -OH, f) -NH₂, g) un -NH (alkyle en C₁ à C₁₀), h) un -N (alkyle en C₁ à C₁₀)₂, i) -SH, j) un -S(O)ₘ-alkyle en C₁ à C₁₀, k) -S(O)₂OH, l) un -S(O)ₙ-Oalkyle en C₁ à C₁₀, m) un -C(O)-alkyle en C₁ à C₁₀, n) -C(O)OH, o) un -C(O)-Oalkyle en C₁ à C₁₀, p) -C(O)NH₂, q) un -C(O)NH-alkyle en C₁ à C₁₀, r) un -C(O)N(alkyle en C₁ à C₁₀)₂, s) -C(S)NH₂, t) un -C(S)NH-alkyle en C₁ à C₁₀, u) un -C (S) N (alkyle en C₁ à C₁₀)₂, v) un groupe alkyle en C₁ à C₁₀, w) un groupe alcényle en C₂ à C₁₀, x) un groupe alcynyle en C₂ à C₁₀, y) un groupe alcoxy en C₁ à C₁₀, z) un groupe halogénoalkyle en C₁ à C₁₀, aa) un groupe cycloalkyle en C₃ à C₁₄, ab) un groupe aryle en C₆ à C₁₄, ac) un groupe cyclohétéroalkyle de 3 à 14 chaînons et ad) un groupe hétéroaryle de 5 à 14 chaînons ; et m est 0, 1 ou 2.

2. Composé selon la revendication 1 ou sel, hydrate ou ester de celui-ci pharmaceutiquement acceptable, dans lequel X est sélectionné parmi -NH-, -N(CH₃)-, -NH-CH₂-, -NH-CH₂CH₂-, -NH-CH₂CH₂CH₂-, -O- et une liaison covalente.

3. Composé selon la revendication 1 ou 2 ou sel, hydrate ou ester de celui-ci pharmaceutiquement acceptable, dans lequel R¹ est un groupe aryle polycyclique en C₈ à C₁₄ ou un groupe hétéroaryle de 5 à 14 chaînons, dans lequel chaque groupe est optionnellement substitué par 1 à 4 groupes -Y-R⁴.

4. Composé selon l'une quelconque des revendications 1 à 3 ou sel, hydrate ou ester de celui-ci pharmaceutiquement acceptable, dans lequel R¹ est sélectionné parmi un groupe benzimidazolyle, un groupe benzodioxolyle, un groupe benzodioxinyle, un groupe benzodioxanyle, un groupe benzofuranyle, un groupe benzothiényle, un groupe benzoxadiazolyle, un groupe benzoxazolyle, un groupe benzisoxazolyle, un groupe benzothiadiazolyle, un groupe benzothiazolyle, un groupe benzisothiazolyle, un groupe benzo[c]iso-thiazolyle, un groupe benzo[c]thiényle, un groupe benzotriazolyle, un groupe indazolyle, un groupe indényle, un groupe indanyle, un groupe indolyle, un groupe isobenzofuranyle, un groupe isoindolyle, un groupe isoquinolinyle, un groupe naphtyle, un groupe indolinyle, un groupe pyrazolyle, un groupe pyridinyle, un groupe pyrrolopyridinyle, un groupe pyrrolyle, un groupe quinolinyle et un groupe tétrahydronaphtalényle, dans lequel chaque groupe est optionnellement substitué par 1 à 4 groupes -Y-R⁴.

5. Composé selon l'une quelconque des revendications 1 à 4 ou sel, hydrate ou ester de celui-ci pharmaceutiquement acceptable, dans lequel R¹ est un groupe indolyle ou un groupe pyrrolopyridinyle, chacun étant optionnellement substitué par 1 à 4 groupes -Y-R⁴.

6. Composé selon l'une quelconque des revendications 1 à 5 ou sel, hydrate ou ester de celui-ci pharmaceutiquement acceptable, dans lequel R¹ est un groupe 1*H*-indol-4-yle, un groupe 1*H*-indol-5-yle, un groupe 1*H*-indol-6-yle ou un groupe 1*H*-indol-7-yle, dans lequel chaque groupe est optionnellement substitué par 1 à 4 groupes sélectionnés indépendamment parmi un halogène, un groupe alkyle en C₁ à C₄ et un groupe alcoxy en C₁ à C₄.

7. Composé selon la revendication 1 ou 2 ou sel, hydrate ou ester de celui-ci pharmaceutiquement acceptable, dans lequel R¹ est un groupe cycloalkyle en C₃ à C₁₄ ou un groupe cyclohétéroalkyle de 3 à 14 chaînons, dans lequel chaque groupe est optionnellement substitué par 1 à 4 groupes -Y-R⁴.

8. Composé selon la revendication 7 ou sel, hydrate ou ester de celui-ci pharmaceutiquement acceptable, dans lequel R¹ est sélectionné parmi un groupe cyclobutyle, un groupe cyclopentyle, un groupe cyclohexyle, un groupe cycloheptyle, un groupe pyrrolidinyle, un groupe pipéridinyle, un groupe pipérazinyle, un groupe morpholinyle et un groupe thiomorpholinyle, dans lequel chaque groupe est optionnellement substitué par 1 à 4 groupes -Y-R⁴.

9. Composé selon l'une quelconque des revendications 1 à 8 ou sel, hydrate ou ester de celui-ci pharmaceutiquement acceptable, dans lequel R² est sélectionné parmi un groupe phényle, un groupe aryle en C₈ à C₁₄ et un groupe hétéroaryle de 5 à 14 chaînons, dans lequel chaque groupe est optionnellement substitué par 1 à 4 groupes sélectionnés indépendamment parmi -Y-R⁴ et -O-Y-R⁴.

10. Composé selon l'une quelconque des revendications 1 à 9 ou sel, hydrate ou ester de celui-ci pharmaceutiquement acceptable, dans lequel R² est : 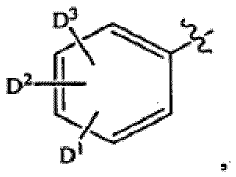 dans lequel D¹, D² et D³ sont indépendamment H, un groupe -Y-R⁴ ou un groupe -O-Y-R⁴.

11. Composé selon la revendication 10 ou sel, hydrate ou ester de celui-ci pharmaceutiquement acceptable, dans lequel au moins un parmi D¹, D² et D³ est un groupe -Y-R⁴ ou un groupe -O-Y-R⁴, dans lesquels Y, à chaque occurrence, est indépendamment un groupe alkyle divalent en C₁ à C₄ ou une liaison covalente, et R⁴, à chaque occurrence, est indépendamment sélectionné parmi un halogène, -CN, -NO₂, -O-Y-R⁵, -NR⁶-Y-R⁷, -S(O)₂Y-R⁵, -S(O)₂NR⁶-Y-R⁷, -C(O)-Y-R⁵, -C(O)O-Y-R⁵, -C(O)NR⁶-Y-R⁷, un groupe alkyle en C₁ à C₁₀, un groupe halogénoalkyle en C₁ à C₁₀, un groupe cycloalkyle en C₃ à C₁₄, un groupe aryle en C₆ à C₁₄, un groupe cyclohétéroalkyle de 3 à 14 chaînons et un groupe hétéroaryle de 5 à 14 chaînons, dans lequel chacun parmi le groupe alkyle en C₁ à C₁₀, le groupe halogénoalkyle en C₁ à C₁₀, le groupe cycloalkyle en C₃ à C₁₄, le groupe aryle en C₆ à C₁₄, le groupe cyclohétéroalkyle de 3 à 14 chaînons et le groupe hétéroaryle de 5 à 14 chaînons est optionnellement substitué par 1 à 4 groupes -Y-R⁸.

12. Composé selon la revendication 11 ou sel, hydrate ou ester de celui-ci pharmaceutiquement acceptable, dans lequel le groupe -Y-R⁴ et le groupe -O-Y-R⁴ sont sélectionnés parmi -O-(CH₂)ₙNR⁶-Y-R⁷, -(CH₂)ₙNR⁶-Y-R⁷, un groupe -O-(CH₂)-cyclohétéroalkyle de 3 à 14 chaînons et un groupe -(CH₂)ₙ-cyclohétéroalkyle de 3 à 14 chaînons, dans lequel chacun parmi le groupe cyclohétéroalkyle de 3 à 14 chaînons est optionnellement substitué par 1 à 4 groupes -Y-R⁸, et n, à chaque occurrence, est indépendamment 0, 1, 2, 3 ou 4.

13. Composé selon la revendication 12 ou sel, hydrate ou ester de celui-ci pharmaceutiquement acceptable, dans lequel le groupe cyclohétéroalkyle de 3 à 14 chaînons du groupe -O-(CH₂)ₙ-cyclohétéroalkyle de 3 à 14 chaînons et du groupe -(CH₂)ₙ-cyclohétéro-alkyle de 3 à 14 chaînons est sélectionné parmi un groupe pyrrolidinyle, un groupe morpholinyle, un groupe pipérazinyle, un groupe pipéridinyle, un groupe azépanyle, un groupe diazépanyle et un groupe thiomorpholinyle.

14. Composé selon la revendication 11 ou sel, hydrate ou ester de celui-ci pharmaceutiquement acceptable, dans lequel le groupe -Y-R⁴ et le groupe -O-Y-R⁴ sont 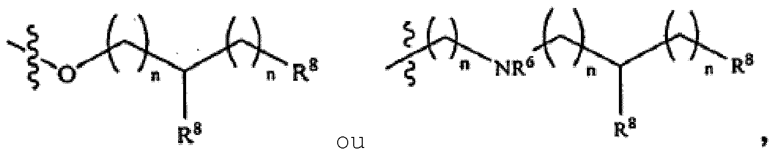 dans lesquels R⁸, à chaque occurrence, est indépendamment sélectionné parmi -O-Y-R⁹, -NR¹⁰-Y-R¹¹, un groupe aryle en C₆ à C₁₄ et un groupe hétéroaryle de 5 à 14 chaînons, dans lequel le groupe aryle en C₆ à C₁₄ et le groupe hétéroaryle de 5 à 14 chaînons sont optionnellement substitués par 1 à 4 groupes -Y-R¹², et n, à chaque occurrence, est indépendamment 0, 1, 2, 3 ou 4.

15. Composé selon l'une quelconque des revendications 11 à 14 ou sel, hydrate ou ester de celui-ci pharmaceutiquement acceptable, dans lequel au moins un parmi D¹, D² et D³ est sélectionné parmi un halogène, -CN, -NO₂, -S(O)₂-Y-R⁵, -S(O)₂NR⁶-Y-R⁷, -C(O)O-Y-R⁵, -C (0) NR⁶-Y-R⁷, un groupe alkyle en C₁ à C₁₀ et un groupe halogénoalkyle en C₁ à C₁₀.

16. Composé selon l'une quelconque des revendications 11 à 15 ou sel, hydrate ou ester de celui-ci pharmaceutiquement acceptable, dans lequel au moins un parmi D¹, D² et D³ est un groupe aryle en C₆, à C₁₄ ou un groupe hétéroaryle de 5 à 14 chaînons, dans lequel chaque groupe est optionnellement substitué par 1 à 4 groupes -Y-R⁸.

17. Composé selon la revendication 16 ou sel, hydrate ou ester de celui-ci pharmaceutiquement acceptable, dans lequel au moins un parmi D¹, D² et D³ est sélectionné parmi un groupe benzothiényle, un groupe benzofuryle, un groupe furyle, un groupe pyridyle, un groupe pyrimidinyle, un groupe pyrrolyle et un groupe thiényle, dans lequel chaque groupe est optionnellement substitué par 1 à 4 groupes -Y-R⁸, Y, à chaque occurrence, est indépendamment un groupe alkyle en C₁ à C₄ ou une liaison covalente, et R⁸, à chaque occurrence, est indépendamment sélectionné parmi un halogène, -CN, -NO₂, -O-Y-R⁹, -NR¹⁰-Y-R¹¹, -C(O)-Y-R⁹, -C(O)NR¹⁰-Y-R¹¹,-S(O)₂-Y-R⁹, -S(O)₂NR¹⁰-Y-R¹¹ et un groupe cyclohétéroalkyle de 3 à 14 chaînons optionnellement substitué par un groupe alkyle en C₁ à C₄.

18. Composé selon l'une quelconque des revendications 1 à 9 ou sel, hydrate ou ester de celui-ci pharmaceutiquement acceptable, dans lequel R² est un groupe aryle bicyclique en C₈ à C₁₄ ou un groupe hétéroaryle de 5 à 14 chaînons, dans lequel chaque groupe est optionnellement substitué par 1 à 4 groupes sélectionnés indépendamment parmi -Y-R⁴ et -O-Y-R⁴.

19. Composé selon la revendication 18 ou sel, hydrate ou ester de celui-ci pharmaceutiquement acceptable, dans lequel R² est sélectionné parmi un groupe pyridyle, un groupe pyrimidyle, un groupe pyrazinyle, un groupe furyle, un groupe thiényle, un groupe thiazolyle, un groupe oxazolyle, un groupe benzofuranyle, un groupe benzothiényle, un groupe indolyle, un groupe benzodioxinyle, un groupe benzodioxolyle, un groupe benzodioxanyle, un groupe dibenzofuranyle, un groupe dibenzothiényle, un groupe benzoindolyle, un groupe indanyle, un groupe indényle, un groupe isothiazolyle, un groupe pyridazinyle, un groupe pyrazolyle, un groupe tétrahydronaphtyle, un groupe isoxazolyle, un groupe quinolinyle, un groupe naphtyle, un groupe imidazolyle et un groupe pyrrolyle, dans lequel chaque groupe est optionnellement substitué par 1 à 4 groupes sélectionnés indépendamment parmi -(CH₂)ₙ-R⁴ et -O-(CH₂)ₙ-R⁴, dans lequel n, à chaque occurrence, est indépendamment 0, 1, 2, 3 ou 4, et R⁴, à chaque occurrence, est indépendamment -NR⁶-Y-R⁷ ou un groupe cyclohétéroalkyle de 3 à 14 chaînons optionnellement substitué par un groupe -Y-R⁸.

20. Composé selon la revendication 1, sélectionné parmi les composés suivantes :
le 5-(3,4-diméthoxyphényl)-4-(1*H*-indol-5-ylamino)-nicotinonitrile,
le 4-(2,1,3-benzothiadiazol-4-ylamino)-5-(3,4-diméthoxyphényl)nicotinonitrile,
le 5-(3,4-diméthoxyphényl)-4-(isoquinolin-5-yl-amino)nicotinonitrile,
le 5-(3,4-diméthoxyphényl)-4-(quinolin-5-ylamino)-nicotinonitrile,
le 5-(3,4-diméthoxyphényl)-4-(5,6,7,8-tétrahydro-naphtalèn-1-ylamino)nicotinonitrile,
le 4-(2,3-dihydro-1,4-benzodioxin-6-ylamino)-5-(3,4-diméthoxyphényl)nicotinonitrile,
le 4-(2,3-dihydro-1*H*-indèn-5-ylamino)-5-(3,4-diméthoxyphényl)nicotinonitrile,
le 5-(3,4-diméthoxyphényl)-4-(1*H*-indol-6-ylamino)-nicotinonitrile,
le 5-(3,4-diméthoxyphényl)-4-(1*H*-indol-4-ylamino)-nicotinonitrile,
le 5-(3,4-diméthoxyphényl)-4-[(2-méthyl-1*H*-indol-5-yl)amino]nicotinonitrile,
le 4-(1,3-benzodioxol-5-ylamino)-5-(3,4-diméthoxy-phényl)nicotinonitrile,
le 5-(3,4-diméthoxyphényl)-4-(2-naphtylamino)-nicotinonitrile,
le 5-(3-bromophényl)-4-(1*H*-indol-5-ylamino)-nicotinonitrile,
le 5-(3-bromophényl)-4-(1*H*-indol-4-ylamino)-nicotinonitrile,
le 5-(2-bromophényl)-4-(1*H*-indol-5-ylamino)-nicotinonitrile,
le 5-(4-bromophényl)-4-(1*H*-indol-5-ylamino)-nicotinonitrile,
le 5-(3'-aminobiphényl-3-yl)-4-(1*H*-indol-5-yl-amino)nicotinonitrile,
le 5-(4'-cyanobiphényl-3-yl)-4-(1*H*-indol-5-yl-amino)nicotinonitrile,
le 5-(4'-aminobiphényl-3-yl)-4-(1*H*-indol-5-yl-amino)nicotinonitrile,
le N-{3'-[5-cyano-4-(1*H*-indol-5-ylamino)pyridin-3-yl]biphényl-4-yl}acétamide,
le 4-(1*H*-indol-5-ylamino)-5-(3-pyridin-4-yl-phényl)nicotinonitrile,
le 3'-[5-cyano-4-(1*H*-indol-5-ylamino)pyridin-3-yl]-N,N-diméthylbiphényl-4-carboxamide,
le 3'-[5-cyano-4-(1*H*-indol-5-ylamino)pyridin-3-yl]-N-cyclopentylbiphényl-4-carboxamide,
le 4-(1*H*-indol-5-ylamino)-5-[3-(1*H*-pyrrol-3-yl)-phényl]nicotinonitrile,
le 5-(2-bromophényl)-4-[(7-méthyl-1*H*-indol-5-yl)-amino]nicotinonitrile,
le 5-(2-bromophényl)-4-(1*H*-indol-4-ylamino)-nicotinonitrile,
le 4-(1*H-*indol-5-ylamino)-5-(3'-méthylbiphényl-3-yl)nicotinonitrile,
le 4-(1*H*-indol-5-ylamino)-5-(4'-méthylbiphényl-3-yl)nicotinonitrile,
le 5-(2'-chlorobiphényl-3-yl)-4-(1*H*-indol-5-yl-amino)nicotinonitrile,
le 5-(3'-chlorobiphényl-3-yl)-4-(1*H*-indol-5-yl-amino)nicotinonitrile,
le 5-(4'-chlorobiphényl-3-yl)-4-(1*H*-indol-5-yl-amino)nicotinonitrile,
le 5-(3'-cyanobiphényl-3-yl)-4-(1*H*-indol-5-yl-amino)nicotinonitrile,
l'acide 3'-[5-cyano-4-(1*H*-indol-5-ylamino)pyridin-3-yl]biphényl-3-carboxylique,
l'acide 3'-[5-cyano-4-(1*H*-indol-5-ylamino)pyridin-3-yl]biphényl-4-carboxylique,
l'acide 3'-[5-cyano-4-(1*H*-indol-4-ylamino)pyridin-3-yl]biphényl-4-carboxylique,
le 4-(1*H*-indol-5-ylamino)-5-[3-(2-thiényl)phényl]-nicotinonitrile,
le 4-(1*H*-indol-5-ylamino)-5-(3-pyridin-3-yl-phényl)nicotinonitrile,
le 4-(1*H*-indol-5-ylamino)-5-(3-pyrimidin-2-yl-phényl)nicotinonitrile,
le 4-(1*H*-indol-5-ylamino)-5-[3-(4-méthyl-2-thiényl)phényl]nicotinonitrile,
le 5-[3-(5-acétyl-2-thiényl)phényl]-4-(1*H*-indol-5-ylamino)nicotinonitrile,
le 4-(1*H*-indol-5-ylamino)-5-[3-(3-thiényl)phényl]-nicotinonitrile,
le 5-[3-(3-furyl)phényl]-4-(1*H*-indol-5-ylamino)-nicotinonitrile,
le 5-(2'-chlorobiphényl-2-yl)-4-(1*H*-indol-5-yl-amino)nicotinonitrile,
le 5-(3'-chlorobiphényl-2-yl)-4-(1*H*-indol-5-yl-amino)nicotinonitrile,
le 5-(4'-chlorobiphényl-2-yl)-4-(1*H*-indol-5-yl-amino)nicotinonitrile,
le 4-(1*H*-indol-5-ylamino)-5-[2-(3-thiényl)phényl]-nicotinonitrile,
le 4-(1*H*-indol-4-ylamino)-5-[3-(2-thiényl)phényl]-nicotinonitrile,
le 3'-[5-cyano-4-(1*H*-indol-4-ylamino)pyridin-3-yl]-N-cyclopentylbiphényl-4-carboxamide,
le 4-(1*H*-indol-4-ylamino)-5-[4'-(pyrrolidin-1-yl-carbonyl)biphényl-3-yl]nicotinonitrile,
le 5-[3-(5-formyl-2-thiényl)phényl]-4-(1*H*-indol-5-ylamino)nicotinonitrile,
le 4-(1*H*-indol-5-ylamino)-5-(3-nitrophényl)-nicotinonitrile,
le N-{3-[5-cyano-4-(1*H*-indol-5-ylamino)pyridin-3-yl]phényl}acétamide,
le 4-(1*H*-indol-4-ylamino)-5-[4-méthoxy-3-(2-méthoxyéthoxy)phényl]nicotinonitrile,
le 4-(1*H*-indol-5-ylamino)-5-[4-méthoxy-3-(2-méthoxyéthoxy)phényl]nicotinonitrile,
le 4-(1*H*-indol-6-ylamino)-5-[4-méthoxy-3-(2-méthoxyéthoxy)phényl]nicotinonitrile,
le 4-(1,3-benzothiazol-6-ylamino)-5-[4-méthoxy-3-(2-méthoxyéthoxy)phényl]nicotinonitrile,
le 5-[4-méthoxy-3-(2-méthoxyéthoxy)phényl]-4-[(2-méthyl-1*H*-indol-5-yl)amino]nicotinonitrile,
le 4-(1*H*-1,2,3-benzotriazol-5-ylamino)-5-[4-méthoxy-3-(2-méthoxyéthoxy)phényl]nicotinonitrile,
le 4-(1*H*-indol-4-ylamino)-5-[3-méthoxy-4-(2-méthoxyéthoxy)phényl]nicotinonitrile,
le 4-(1*H*-indol-5-ylamino)-5-[3-méthoxy-4-(2-méthoxyéthoxy)phényl]nicotinonitrile,
le 4-(1*H*-indol-6-ylamino)-5-[3-méthoxy-4-(2-méthoxyéthoxy)phényl]nicotinonitrile,
le 4-(1,3-benzothiazol-6-ylamino)-5-[3-méthoxy-4-(2-méthoxyéthoxy)phényl]nicotinonitrile,
le 5-[3-méthoxy-4-(2-méthoxyéthoxy)phényl]-4-[(2-méthyl-1*H*-indol-5-yl)amino]nicotinonitrile,
le 4-(1*H*-indol-4-ylamino)-5-[3-(2-méthoxyéthoxy)-phényl]nicotinonitrile,
le 5-[3-(2-chloroéthoxy)phényl]-4-(1*H*-indol-4-yl-amino)nicotinonitrile,
le 5-[3-(2-chloroéthoxy)phényl]-4-(1*H*-indol-6-yl-amino)nicotinonitrile,
le 5-[3-(2-chloroéthoxy)phényl]-4-(1*H*-indol-5-ylamino)nicotinonitrile,
le 4-(1*H*-indol-4-ylamino)-5-{3-[2-(4-méthyl-pipérazin-1-yl)éthoxy]phényl}nicotinonitrile,
le 4-(1*H*-indol-4-ylamino)-5-[3-(2-pyrrolidin-1-yl-éthoxy)phényl]nicotinonitrile,
le 4-(1*H*indol-4-ylamino)-5-[3-(2-morpholin-4-yl-éthoxy)phényl]nicotinonitrile,
le 4-(1*H*-indol-4-ylamino)-5-[3-(2-pipéridin-1-yl-éthoxy)phényl]nicotinonitrile,
le 4-(1*H*-indol-6-ylamino)-5-[3-(2-pyrrolidin-1-yl-éthoxy)phényl]nicotinonitrile,
le 4-(1*H*-indol-6-ylamino)-5-[3-(2-morpholin-4-yl-éthoxy)phényl]nicotinonitrile,
le 4-(1*H*-indol-5-ylamino)-5-[3-(2-pyrrolidin-1-yl-éthoxy)phényl]nicotinonitrile,
le 4-(1*H*-indol-5-ylamino)-5-[3-(2-morpholin-4-yl-éthoxy)phényl]nicotinonitrile,
le 5-(3-{2-[(2-hydroxyéthyl)amino]éthoxy}phényl)-4-(1*H*-indol-5-ylamino)nicotinonitrile,
le 4-(1*H*-indol-5-ylamino)-5-(3- {2-[(2-pyrrolidin-1-yléthyl)amino]éthoxy}phényl)nicotinonitrile,
le 5-[3-(2-chloroéthoxy)-4-méthoxyphényl]-4-(1*H*-indol-5-ylamino)nicotinonitrile,
le 5-{3-[2-(diéthylamino)éthoxy]-4-méthoxyphényl}-4-(1*H*-indol-5-ylamino)nicotinonitrile,
le 5-{3-[2-(diisopropylamino)éthoxy]-4-méthoxy-phényl}-4-(1*H*-indol-5-ylamino)nicotinonitrile,
le 5-{3-[2-(benzylamino)éthoxy]-4-méthoxyphényl}-4-(1*H*-indol-5-ylamino)nicotinonitrile,
le 4-(1*H*-indol-5-ylamino)-5-(4-méthoxy-3-{2-[(2-méthoxyéthyl)amino]éthoxy}phényl)nicotinonitrile,
le 4-(1*H*-indol-5-ylamino)-5-(4-méthoxy-3-{2-[(5-méthyl-1,3,4-thiadiazol-2-yl)amino]éthoxy}phényl)-nicotinonitrile,
le 4-(1*H*-indol-5-ylamino)-5-(3-{5-[(4-méthyl-pipérazin-1-yl)méthyl]-2-thiényl}phényl)nicotinonitrile,
le 4-(1*H*-indol-5-ylamino)-5-{3-[5-(morpholin-4-yl-méthyl)-2-thiényl]phényl}nicotinonitrile,
le 4-(1*H*-indol-5-ylamino)-5-{3-[5-(pipéridin-1-yl-méthyl)-2-thiényl]phényl}nicotinonitrile,
le 5-(3-{5-[(diméthylamino)méthyl]-2-thiényl}-phényl)-4-(1*H*-indol-5-ylamino)nicotinonitrile,
le 5-(3-bromo-4-méthoxyphényl)-4-(1*H*-indol-5-yl-amino)nicotinonitrile,
le 4-(1*H*-indol-5-ylamino)-5-[4-méthoxy-3-(2-thiényl)phényl]nicotinonitrile,
le 5-(4'-chloro-6-méthoxybiphényl-3-yl)-4-(1*H*-indol-5-ylamino)nicotinonitrile,
le 5-(3'-chloro-6-méthoxybiphényl-3-yl)-4-(1*H*-indol-5-ylamino)nicotinonitrile,
le 5'-[5-cyano-4-(1*H*-indol-5-ylamino)pyridin-3-yl]-N-cyclopentyl-2'-méthoxybiphényl-4-carboxamide,
le 5-(2'-chloro-6-méthoxybiphényl-3-yl)-4-(1*H*-indol-5-ylamino)nicotinonitrile,
le 5-[3-(benzyloxy)phényl]-4-(1*H*-indol-5-ylamino)-nicotinonitrile,
le 5-[4-(benzyloxy)phényl]-4-(1*H*-indol-5-ylamino)-nicotinonitrile,
le 3'-[5-cyano-4-(1*H*-indol-5-ylamino)pyridin-3-yl]-N-méthylbiphényl-4-carboxamide,
le N-butyl-3'-[5-cyano-4-(1*H*-indol-5-ylamino)-pyridin-3-yl]biphényl-4-carboxamide,
le 3'-[5-cyano-4-(1*H*-indol-5-ylamino)pyridin-3-yl]-N-(1-éthylpropyl)biphényl-4-carboxamide,
le 3'-[5-cyano-4-(1*H*-indol-5-ylamino)pyridin-3-yl]-N-(2-hydroxyéthyl)biphényl-4-carboxamide,
le 3'-[5-cyano-4-(1*H*-indol-5-ylamino)pyridin-3-yl]-N-(2-méthoxyéthyl)biphényl-4-carboxamide,
le 3'-[5-cyano-4-(1*H*-indol-5-ylamino)pyridin-3-yl]-N-cyclopropylbiphényl-4-carboxamide,
le 3'-[5-cyano-4-(1*H*-indol-5-ylamino)pyridin-3-yl]-N-cyclohexylbiphényl-4-carboxamide,
le 3'-[5-cyano-4-(1*H*-indol-5-ylamino)pyridin-3-yl]-N-(2-pyrrolidin-1-yléthyl)biphényl-4-carboxamide,
le N-benzyl-3'-[5-cyano-4-(1*H*-indol-5-ylamino)-pyridin-3-yl]biphényl-4-carboxamide,
le 4-(1*H*-indol-5-ylamino)-5-[4'-(pyrrolidin-1-yl-carbonyl)biphényl-3-yl]nicotinonitrile,
le 4-(1*H*-indol-5-ylamino)-5-[4'-(morpholin-4-yl-carbonyl)biphényl-3-yl]nicotinonitrile,
le 4-(1*H*-indol-5-ylamino)-5-{4'-[(4-méthyl-pipérazin-1-yl)carbonyl]biphényl-3-yl}nicotinonitrile,
le 3'-[5-cyano-4-(1*H*-indol-5-ylamino)pyridin-3-yl]-N-cyclopentylbiphényl-3-carboxamide,
le 3'-[5-cyano-4-(1*H*-indol-5-ylamino)pyridin-3-yl]-N-(4-hydroxybutyl)biphényl-4-carboxamide,
le 3'-[5-cyano-4-(1*H*-indol-5-ylamino)pyridin-3-yl]-N-(3-hydroxypropyl)biphényl-4-carboxamide,
le 3'-[5-cyano-4-(1*H*-indol-5-ylamino)pyridin-3-yl]-N-[2-(méthylamino)éthyl]biphényl-4-carboxamide,
le 3'-[5-cyano-4-(1*H*-indol-5-ylamino)pyridin-3-yl]-N-(pyridin-2-ylméthyl)biphényl-4-carboxamide,
le 3'-[5-cyano-4-(1*H*-indol-5-ylamino)pyridin-3-yl]-N-(pyridin-3-ylméthyl)biphényl-4-carboxamide,
le 3'-[5-cyano-4-(1*H*-indol-5-ylamino)pyridin-3-yl]-N-(pyridin-4-ylméthyl)biphényl-4-carboxamide,
le N-butyl-3'-[5-cyano-4-(1*H*-indol-4-ylamino)-pyridin-3-yl]biphényl-4-carboxamide,
le 3'-[5-cyano-4-(1*H*-indol-4-ylamino)pyridin-3-yl]-N-(2-hydroxyéthyl)biphényl-4-carboxamide,
le 5-(3,4-diméthoxyphényl)-4-[(7-méthyl-1*H*-indol-5-yl)amino]nicotinonitrile,
le 5-(3,4-diméthoxyphényl)-4-[(4-méthyl-1*H*-indol-5-yl)amino]nicotinonitrile,
le 5-(3,4-diméthoxyphényl)-4-[1*H*-indol-5-yl-(méthyl)amino]nicotinonitrile,
le 5-(3,4-diméthoxyphényl)-4-(1*H*-indol-5-yloxy)-nicotinonitrile,
le 5-(3,4-diméthoxyphényl)-4-(1*H*-indol-5-yl)-nicotinonitrile,
le 5-(1-benzofuran-2-yl)-4-[(4-méthyl-1*H*-indol-5-yl)amino]nicotinonitrile,
le 5-(5-formyl-1-benzothién-2-yl)-4-(1*H*-indol-4-ylamino)nicotinonitrile,
le 5-{5-[(diméthylamino)méthyl]-1-benzothién-2-yl}-4-(1*H*-indol-4-ylamino)nicotinonitrile,
le 5-(4-hydroxyphényl)-4-(1*H*-indol-4-ylamino)-nicotinonitrile,
le 5-(4-{[(2S)-2-amino-3-phénylpropyl]oxy}phényl)-4-(1*H*-indol-4-ylamino)nicotinonitrile,
le 5-(5-formyl-1-benzothién-2-yl)-4-[(4-méthyl-1*H-*indol-5-yl)amino]nicotinonitrile,
le 4-[(4-méthyl-1*H*-indol-5-yl)amino]-5-[5-(pipéridin-1-ylméthyl)-1-benzothién-2-yl]-nicotinonitrile,
le 5-[5-(hydroxyméthyl)-1-benzothién-2-yl]-4-[(4-méthyl-1*H*-indol-5-yl)amino]nicotinonitrile,
le 4-(1*H*-indol-5-ylamino)-5-[4-méthoxy-3-(2-morpholin-4-yléthoxy)phényl]nicotinonitrile,
le 4-(1*H*-indol-5-ylamino)-5-{4-méthoxy-3-[2-(4-méthylpipérazin-1-yl)éthoxy]phényl}nicotinonitrile,
le 4-(1*H*-indol-5-ylamino)-5-[4-méthoxy-3-(2-pipérazin-1-yléthoxy)phényl]nicotinonitrile,
le 4-(1*H*-indol-5-ylamino)-5-[4-méthoxy-3-(2-thiomorpholin-4-yléthoxy)phényl]nicotinonitrile,
le 5-{3-[2-(4-éthylpipérazin-1-yl)éthoxy]-4-méthoxyphényl}-4-(1*H*-indol-5-ylamino)nicotinonitrile,
le 4-(1*H*-indol-5-ylamino)-5-[4-méthoxy-3-(2-pipéridin-1-yléthoxy)phényl]nicotinonitrile,
le 5-{3-[2-(diméthylamino)éthoxy]-4-méthoxy-phényl}-4-(1*H*-indol-5-ylamino)nicotinonitrile,
le 5-(3-{2-[bis(2-hydroxyéthyl)amino]éthoxy}-4-méthoxyphényl)-4-(1*H*-indol-5-ylamino)nicotinonitrile,
le 5-{3-[2-(4-hydroxypipéridin-1-yl)éthoxy]-4-méthoxyphényl}-4-(1*H*-indol-5-ylamino)nicotinonitrile,
le 4-(1*H*-indol-5-ylamino)-5-(4-méthoxy-3-{2-[(pyridin-3-ylméthyl)amino]éthoxy}phényl)-nicotinonitrile,
le 4-(1*H*-indol-5-ylamino)-5-(4-méthoxy-3-{2-[(pyridin-4-ylméthyl)amino]éthoxy}phényl)-nicotinonitrile,
le 4-(1*H*-indol-5-ylamino)-5-(4-méthoxy-3-{2-[(pyridin-2-ylméthyl)amino]éthoxy}phényl)-nicotinonitrile,
le 4-(1*H*-indol-5-ylamino)-5-(4-méthoxy-3-{2-[(2-phényléthyl)amino]éthoxy}phényl)nicotinonitrile,
le 5-{3-[2-(cyclopentylamino)éthoxy]-4-méthoxy-phényl}-4-(1*H*-indol-5-ylamino)nicotinonitrile,
le 5-{3-[2-(cyclohexylamino)éthoxy]-4-méthoxy-phényl}-4-(1*H*-indol-5-ylamino)nicotinonitrile,
le 5-(3-{2-[(2-furylméthyl)amino]éthoxy}-4-méthoxyphényl)-4-(1*H*-indol-5-ylamino)nicotinonitrile,
le 5-(2-bromo-4,5-diméthoxyphényl)-4-(1*H*-indol-5-ylamino)nicotinonitrile,
le 5-(2-bromo-4,5-diméthoxyphényl)-4-[(4-méthyl-1*H-*indol-5-yl)amino]nicotinonitrile,
le 4-(1*H*-indol-4-ylamino)-5-[4-méthoxy-3-(2-pipéridin-1-yléthoxy)phényl]nicotinonitrile,
le 5-(3-{2-[(2-hydroxyéthyl)amino]éthoxy}-4-méthoxyphényl)-4-(1*H*-indol-4-ylamino)nicotinonitrile,
le 4-(1*H*-indol-5-ylamino)-5-(4-méthoxy-3-{2-[(3-phénylpropyl)amino]éthoxy}phényl)nicotinonitrile,
le 4-(1*H*-indol-5-ylamino)-5-(4-méthoxy-3-{2-[(2-pyridin-2-yléthyl)amino]éthoxy}phényl)nicotinonitrile,
le 4-(1*H*-indol-5-ylamino)-5-(4-méthoxy-3-{2-[(2-pyridin-3-yléthyl)amino]éthoxy}phényl)nicotinonitrile,
le 4-(1*H*-indol-5-ylamino)-5-(4-méthoxy-3-{2-[(2-pyridin-4-yléthyl)amino]éthoxy}phényl)nicotinonitrile,
le 5-[3-(diméthylamino)phényl]-4-(1*H*-indol-4-yl-amino)nicotinonitrile,
le 4-(1*H*-indol-4-ylamino)-5-[3-(méthylsulfonyl)-phényl]nicotinonitrile,
le N-{3-[5-cyano-4-(1*H*-indol-4-ylamino)pyridin-3-yl]phényl}méthanesulfonamide,
le 4-(1*H*-indol-5-ylamino)-5-phénylnicotinonitrile,
le 4-(1*H*-indol-5-ylamino)-5-(3-thiényl)-nicotinonitrile,
le 4-(1*H*-indol-5-ylamino)-3,3'-bipyridine-5-carbonitrile,
le 5-(1,3-benzodioxol-5-yl)-4-(1*H*-indol-5-yl-amino)nicotinonitrile,
le 4-(1*H*-indol-5-ylamino)-3,4'-bipyridine-5-carbonitrile,
le 5-(3-furyl)-4-(1*H*-indol-5-ylamino)-nicotinonitrile,
le 5-(1*H*-indol-5-yl)-4-(1*H*-indol-5-ylamino)-nicotinonitrile,
le 5-(2,3-dihydro-1,4-benzodioxin-6-yl)-4-(1*H*-indol-5-ylamino)nicotinonitrile,
le 4-(1*H*-indol-5-ylamino)-5-pyrimidin-5-yl-nicotinonitrile,
le 4-(1*H*-indol-5-ylamino)-5-(2-méthoxypyrimidin-5-yl)nicotinonitrile,
le 4-(1*H*-indol-5-ylamino)-5-(2-thiényl)-nicotinonitrile,
le 5-(1-benzofuran-2-yl)-4-(1*H*-indol-5-ylamino)nicotinonitrile,
le 5-(3,5-diméthylisoxazol-4-yl)-4-(1*H*-indol-5-ylamino)nicotinonitrile,
le 5-[3-(hydroxyméthyl)phényl]-4-(1*H*-indol-4-yl-amino)nicotinonitrile,
le 5-{3-[(diméthylamino)méthyl]phényl}-4-(1*H*-indol-4-ylamino)nicotinonitrile,
le 4-(1*H*-indol-4-ylamino)-5-{5-[(prop-2-yn-1-yl-amino)méthyl]-1-benzothién-2-yl}nicotinonitrile,
le 5-{5-[(butylamino)méthyl]-1-benzothién-2-yl}-4-(1*H-*indol-4-ylamino)nicotinonitrile,
le 5-(5-{[(2-hydroxyéthyl)amino]méthyl}-1-benzothién-2-yl)-4-(1*H*-indol-4-ylamino)nicotinonitrile,
le 5-(5-{[(3-hydroxypropyl)amino]méthyl}-1-benzothién-2-yl)-4-(1*H*-indol-4-ylamino)nicotinonitrile,
le 4-(1*H*-indol-4-ylamino)-5-(5-{[(3-méthoxy-propyl)amino]méthyl}-1-benzothién-2-yl)nicotinonitrile,
le 5-(5-{[(4-hydroxybutyl)amino]méthyl}-1-benzothién-2-yl)-4-(1*H*-indol-4-ylamino)nicotinonitrile,
le 5-{5-[(cyclopropylamino)méthyl]-1-benzothién-2-yl}-4-(1*H*-indol-4-ylamino)nicotinonitrile,
le 5-(5-{[(cyclopropylméthyl)amino]méthyl}-1-benzothién-2-yl)-4-(1*H*-indol-4-ylamino)nicotinonitrile,
le 4-(1*H*-indol-4-ylamino)-5-[5-(pyrrolidin-1-yl-méthyl)-1-benzothién-2-yl]nicotinonitrile,
le 4-(1*H*-indol-4-ylamino)-5-[5-(morpholin-4-yl-méthyl)-1-benzothién-2-yl]nicotinonitrile,
le 4-(1*H*-indol-4-ylamino)-5-(5-{[(2-morpholin-4-yléthyl)amino]méthyl}-1-benzothién-2-yl)nicotinonitrile,
le 4-(1*H*-indol-4-ylamino)-5-[5-(pipéridin-1-yl-méthyl)-1-benzothién-2-yl]nicotinonitrile,
le 5-(5-{[4-(hydroxyméthyl)pipéridin-1-yl]méthyl}-1-benzothién-2-yl)-4-(1*H*-indol-4-ylamino)-nicotinonitrile,
le 5-[5-(hydroxyméthyl)-1-benzothién-2-yl]-4-(1*H-*indol-4-ylamino)nicotinonitrile,
le 5-{5-[(benzylamino)méthyl]-1-benzothién-2-yl}-4-(1*H*-indol-4-ylamino)nicotinonitrile,
le 4-(1*H*-indol-4-ylamino)-5-(5-{[(2-phényléthyl)-amino]méthyl}-1-benzothién-2-yl)nicotinonitrile,
le 4-(1*H*-indol-4-ylamino)-5-(5-{[(pyridin-2-yl-méthyl)amino]méthyl}-1-benzothién-2-yl)nicotinonitrile,
le 4-(1*H*-indol-4-ylamino)-5-(5-{[(pyridin-3-yl-méthyl)amino]méthyl}-1-benzothién-2-yl)nicotinonitrile,
le 4-(1*H*-indol-4-ylamino)-5-(5-{[(pyridin-4-yl-méthyl)amino]méthyl}-1-benzothién-2-yl)nicotinonitrile,
le 5-[4-(diméthylamino)phényl]-4-(pyridin-3-yl-amino)nicotinonitrile,
le 5-[4-(diméthylamino)phényl]-4-(1*H*-indazol-5-yl-amino)nicotinonitrile,
le 5-[4-(diméthylamino)phényl]-4-(1*H*-indazol-6-yl-amino)nicotinonitrile,
le 5-[4-(diméthylamino)phényl]-4-[(5-hydroxy-1*H-*pyrazol-3-yl)amino]nicotinonitrile,
le 4-(1*H*-indazol-5-ylamino)-5-(3-méthoxyphényl)-nicotinonitrile,
le 4-(1*H*-indazol-6-ylamino)-5-(3-méthoxyphényl)-nicotinonitrile,
le 4-[(5-hydroxy-1*H*-pyrazol-3-yl)amino]-5-(3-méthoxyphényl)nicotinonitrile,
le 5-(3-bromophényl)-4-[(4-méthyl-1*H*-indol-5-yl)-amino]nicotinonitrile,
le 4-(1*H*-indol-4-ylamino)-5-[4-méthoxy-3-(2-morpholin-4-yléthoxy)phényl]nicotinonitrile,
le 4-(1*H*-indol-4-ylamino)-5-{4-méthoxy-3-[2-(4-méthylpipérazin-1-yl)éthoxy]phényl}nicotinonitrile,
le 5-{3-[2-(diméthylamino)éthoxy]-4-méthoxy-phényl}-4-(1*H*-indol-4-ylamino)nicotinonitrile,
le 5-{3-[2-(4-hydroxypipéridin-1-yl)éthoxy]-4-méthoxyphényl}-4-(1*H*-indol-4-ylamino)nicotinonitrile,
le 5-[3-(2-chloroéthoxy)-4-méthoxyphényl]-4-(1*H*-indol-4-ylamino)nicotinonitrile,
le 4-[(4-méthyl-1*H*-indol-5-yl)amino]-5-[3-(2-thiényl)phényl]nicotinonitrile,
le 5-(3,4-diméthoxyphényl)-4-[(4-éthyl-1*H*-indol-5-yl)amino]nicotinonitrile,
le 5-[3-(5-formyl-2-thiényl)phényl]-4-[(4-méthyl-1H-indol-5-yl)amino]nicotinonitrile,
le 5-(3-{5-[(diméthylamino)méthyl]-2-thiényl}-phényl)-4-[(4-méthyl-1*H*-indol-5-yl)amino]-nicotinonitrile,
le 3'-[5-cyano-4-(1*H*-indol-4-ylamino)pyridin-3-yl]-N-(4-hydroxybutyl)biphényl-4-carboxamide,
le 3'-{5-cyano-4-[(4-méthyl-1*H*-indol-5-yl)amino]-pyridin-3-yl}-N-(4-hydroxybutyl)biphényl-4-carboxamide,
le 4-(1*H*-indol-4-ylamino)-5-[3-(trifluorométhyl)-phényl]nicotinonitrile,
le 5-(3-cyanophényl)-4-(1*H*-indol-4-ylamino)-nicotinonitrile,
le 3-[5-cyano-4-(1*H*-indol-4-ylamino)pyridin-3-yl]-N,N-diméthylbenzamide,
le 3-[5-cyano-4-(1*H*-indol-4-ylamino)pyridin-3-yl]-N,N-diméthylbenzènesulfonamide,
le 3-[5-cyano-4-(1*H*-indol-4-ylamino)pyridin-3-yl]benzamide,
le 5-(3-{5-[(diméthylamino)méthyl]-2-thiényl}-phényl)-4-(1*H*-indol-4-ylamino)nicotinonitrile,
le 2-[5-cyano-4-(1*H*-indol-4-ylamino)pyridin-3-yl]-N,N-diméthylbenzènesulfonamide,
le N-{4-[5-cyano-4-(1*H*-indol-4-ylamino)pyridin-3-yl]phényl}méthanesulfonamide,
le 5-(1-benzofuran-2-yl)-4-(1*H*-indol-4-ylamino)-nicotinonitrile,
le 5-dibenzo[b,d]furan-4-yl-4-(1*H*-indol-4-yl-amino)nicotinonitrile,
le 4-(1*H*-indol-4-ylamino)-5-{1-[(4-méthylphényl)-sulfonyl]-1*H*-indol-3-yl}nicotinonitrile,
le 5-(1-benzothién-2-yl)-4-(1*H*-indol-4-ylamino)-nicotinonitrile,
le 4-(1*H*-indol-4-ylamino)-5-(4-méthoxyphényl)-nicotinonitrile,
le 4-(1*H*-indol-4-ylamino)-5-(2-méthoxyphényl)-nicotinonitrile,
le 5-(1*H*-indol-2-yl)-4-(1*H*-indol-4-ylamino)-nicotinonitrile,
le 4-[5-cyano-4-(1*H*-indol-4-ylamino)pyridin-3-yl]-N,N-diméthylbenzènesulfonamide,
l'acide 3-[5-cyano-4-(1*H*-indol-4-ylamino)pyridin-3-yl]benzoïque,
le 5-[3-(aminométhyl)phényl]-4-(1*H*-indol-4-yl-amino)nicotinonitrile,
le 5-(3,4-diméthoxyphényl)-4-[(2-oxo-2,3-dihydro-1*H-*indol-4-yl)amino]nicotinonitrile,
le 4-[5-cyano-4-(1*H*-indol-4-ylamino)pyridin-3-yl]-N-(2-méthoxyéthyl)benzamide,
le 4-(1*H*-indol-4-ylamino)-5-(4-méthoxy-3-{2-[(3-phénylpropyl)amino]éthoxy}phényl)nicotinonitrile,
le 5-[4-(2-chloroéthoxy)phényl]-4-(1*H*-indol-4-yl-amino)nicotinonitrile,
le 5-[3-(5-formyl-2-thiényl)phényl]-4-(1*H*-indol-4-ylamino)nicotinonitrile,
le 4-(1*H*-indol-4-ylamino)-5-[4-(2-morpholin-4-yl-éthoxy)phényl]nicotinonitrile,
le 4-(1*H*-indol-4-ylamino)-5-{4-[2-(4-méthyl-pipérazin-1-yl)éthoxy]phényl}nicotinonitrile,
le 5-(3,4-diméthoxyphényl)-4-[(5-méthyl-1*H*-indol-4-yl)amino]nicotinonitrile,
le 5-(2,4-diméthoxyphényl)-4-(1*H*-indol-4-ylamino)-nicotinonitrile,
le 4-(1*H*-indol-4-ylamino)-5-[4-méthoxy-3-(2-{[3-(2-oxopyrrolidin-1-yl)propyl]amino}éthoxy)phényl]-nicotinonitrile,
le 5-[3-(2-{[2-(1*H*-imidazol-4-yl)éthyl]amino}-éthoxy)-4-méthoxyphényl]-4-(1*H*-indol-4-ylamino)-nicotinonitrile,
le 4-(1*H*-indol-4-ylamino)-5-(4-méthoxy-3-{2-[(3-pyrrolidin-1-ylpropyl)amino]éthoxy}phényl)-nicotinonitrile,
le 4-(1*H*-indol-4-ylamino)-5-[4-méthoxy-3-(2-{[2-(1-méthylpyrrolidin-2-yl)éthyl]amino}éthoxy)phényl]-nicotinonitrile,
le 5-(4-méthoxy-3-{2-[(3-phénylpropyl)amino]-éthoxy}phényl)-4-[(4-méthyl-1*H*-indol-5-yl)amino]-nicotinonitrile,
le 5-{4-méthoxy-3-[2-(4-méthylpipérazin-1-yl)-éthoxy]phényl}-4-[(4-méthyl-1*H*-indol-5-yl)amino]-nicotinonitrile,
le 5-(3-méthoxy-4-{2-[(3-phénylpropyl)amino]-éthoxy}phényl)-4-[(4-méthyl-1*H*-indol-5-yl)amino]-nicotinonitrile,
le 5-{4-[2-(diméthylamino)éthoxy]-3-méthoxy-phényl}-4-[(4-méthyl-1*H*-indol-5-yl)amino]-nicotinonitrile,
le 5-(3-bromophényl)-4-[(2-oxo-2,3-dihydro-1*H*-indol-4-yl)amino]nicotinonitrile,
le 4-(1*H*-indol-4-ylamino)-5-(4-{2-[(3-phényl-propyl)amino]éthoxy}phényl)nicotinonitrile,
le 5-[4-(2-chloroéthoxy)phényl]-4-[(4-méthyl-1*H*-indol-5-yl)amino]nicotinonitrile,
le 5-(3,4-diméthoxyphényl)-4-[(2-méthyl-1*H*-indol-4-yl)amino]nicotinonitrile,
le 4-[(4-méthyl-1*H*-indol-5-yl)amino]-5-{4-[2-(4-méthylpipérazin-1-yl)éthoxy]phényl}nicotinonitrile,
le 4-[(4-méthyl-1*H*-indol-5-yl)amino]-5-[4-(2-morpholin-4-yléthoxy)phényl]nicotinonitrile,
le 5-(1-benzofuran-3-yl)-4-(1*H*-indol-4-ylamino)-nicotinonitrile,
le 4-(1*H*-indol-4-ylamino)-5-(4-méthoxy-3-{2-[(2-phényléthyl)amino]éthoxy}phényl)nicotinonitrile,
le 4-(1*H*-indol-4-ylamino)-5-(4-méthoxy-3-{2-[(2-pyridin-3-yléthyl)amino]éthoxy}phényl)nicotinonitrile,
le 4-[(4-méthyl-1*H*-indol-5-yl)amino]-5-(4-{2-[(3-phénylpropyl)amino]éthoxy}phényl)nicotinonitrile,
le 6'-[3-(diméthylamino)propoxy]-4-(1*H*-indol-4-ylamino)-3,3'-bipyridine-5-carbonitrile,
le 6'-[3-(diméthylamino)propoxy]-4-[(4-méthyl-1*H-*indol-5-yl)amino]-3,3'-bipyridine-5-carbonitrile,
le 5-(3-hydroxyphényl)-4-(1*H*-indol-4-ylamino)-nicotinonitrile,
le 4-(1*H*-indol-4-ylamino)-5-[5-(pipérazin-1-yl-méthyl)-1-benzothién-2-yl]nicotinonitrile,
le N-({2-[5-cyano-4-(1*H*-indol-4-ylamino)pyridin-3-yl]-1-benzothién-5-yl}méthyl)-b-alaninamide,
le 4-(1*H*-indol-4-ylamino)-6'-[(2-morpholin-4-yl-éthyl)amino]-3,3'-bipyridine-5-carbonitrile,
le 4-[(4-méthyl-1*H*-indol-5-yl)amino]-6'-[(2-morpholin-4-yléthyl)amino]-3,3'-bipyridine-5-carbonitrile,
le 5-{2-chloro-4-[2-(diméthylamino)éthoxy]phényl}-4-[(4-méthyl-1*H*-indol-5-yl)amino]nicotinonitrile,
le 4-(1*H*-indol-4-ylamino)-5-(4-méthoxy-3-{2-[(3-morpholin-4-ylpropyl)amino]éthoxy}phényl)-nicotinonitrile,
le 5-[3-(2-{[3-(1*H*-imidazol-1-yl)propyl]amino}-éthoxy)-4-méthoxyphényl]-4-(1*H*-indol-4-ylamino)-nicotinonitrile,
le 5-(3-{[(2S)-2-amino-3-phénylpropyl]oxy}phényl)-4-(1*H*-indol-4-ylamino)nicotinonitrile,
le 5-{5-[(benzylamino)méthyl]-1-benzothién-2-yl}-4-[(4-méthyl-1*H*-indol-5-yl)amino]nicotinonitrile,
le 5-{4-[2-(4-butylpipérazin-1-yl)éthoxy]phényl}-4-[(4-méthyl-1*H*-indol-5-yl)amino]nicotinonitrile,
le 5-[5-(1,3-dioxan-2-yl)-1-benzofuran-2-yl]-4-(1*H-*indol-4-ylamino)nicotinonitrile,
le 5-[5-(1,3-dioxan-2-yl)-1-benzofuran-2-yl]-4-[(4-méthyl-1*H*-indol-5-yl)amino]nicotinonitrile,
le 5-(2-chloro-4-méthoxyphényl)-4-[(4-méthyl-1*H*-indol-5-yl)amino]nicotinonitrile,
le 5-[4-(2-chloroéthoxy)-3-méthoxyphényl]-4-[(4-méthyl-1*H*-indol-5-yl)amino]nicotinonitrile,
le 5-(1-benzofuran-2-yl)-4-(1*H*-indazol-5-yl-amino)nicotinonitrile,
le 5-(4-hydroxyphényl)-4-[(4-méthyl-1*H*-indol-5-yl)amino]nicotinonitrile,
le 5-(2-chloro-6-méthoxyphényl)-4-[(4-méthyl-1*H*-indol-5-yl)amino]nicotinonitrile,
le 5-[3-méthoxy-4-(2-pipéridin-1-yléthoxy)phényl]-4-[(4-méthyl-1*H*-indol-5-yl)amino]nicotinonitrile,
le 5-{3-méthoxy-4-[2-(4-méthylpipérazin-1-yl)-éthoxy]phényl}-4-[(4-méthyl-1*H*-indol-5-yl)amino]-nicotinonitrile,
le 5-[3-méthoxy-4-(2-morpholin-4-yléthoxy)phényl]-4-[(4-méthyl-1*H*-indol-5-yl)amino]nicotinonitrile,
le 5-(3,4-diméthoxyphényl)-4-(1*H*-indazol-5-yl-amino)nicotinonitrile,
le 5-(2,3-dichlorophényl)-4-[(4-méthyl-1*H*-indol-5-yl)amino]nicotinonitrile,
le 5-(4-bromo-2-fluorophényl)-4-[(4-méthyl-1*H*-indol-5-yl)amino]nicotinonitrile,
le 5-{5-[(diméthylamino)méthyl]-1-benzofuran-2-yl}-4-[(4-méthyl-1*H*-indol-5-yl)amino]nicotinonitrile,
le 5-(5-{[(2-hydroxyéthyl)amino]méthyl}-1-benzofuran-2-yl)-4-[(4-méthyl-1*H*-indol-5-yl)amino]-nicotinonitrile,
le 5-(5-{[(3-hydroxypropyl)amino]méthyl}-1-benzofuran-2-yl)-4-[(4-méthyl-1*H*-indol-5-yl)amino]-nicotinonitrile,
le 5-(5-{[(2,3-dihydroxypropyl)amino]méthyl}-1-benzofuran-2-yl)-4-[(4-méthyl-1*H*-indol-5-yl)amino]-nicotinonitrile,
le 5-(5-{[(2,3-dihydroxypropyl)(méthyl)amino]-méthyl}-1-benzofuran-2-yl)-4-[(4-méthyl-1*H*-indol-5-yl)amino]nicotinonitrile,
le 5-{5-[(cyclohexylamino)méthyl]-1-benzofuran-2-yl}-4-[(4-méthyl-1*H*-indol-5-yl)amino]nicotinonitrile,
le 4-[(4-méthyl-1*H*-indol-5-yl)amino]-5-[5-(morpholin-4-ylméthyl)-1-benzofuran-2-yl]-nicotinonitrile,
le 4-[(4-méthyl-1*H*-indol-5-yl)amino]-5-[5-(pyrrolidin-1-ylméthyl)-1-benzofuran-2-yl]-nicotinonitrile,
le 4-[(4-méthyl-1*H*-indol-5-yl)amino]-5-(5-{[(2-pyrrolidin-1-yléthyl)amino]méthyl}-1-benzofuran-2-yl)nicotinonitrile,
le 4-[(4-méthyl-1*H*-indol-5-yl)amino]-5-[5-({[(1-méthylpipéridin-4-yl)méthyl]amino}méthyl)-1-benzofuran-2-yl]nicotinonitrile,
le 5-(5-{[4-(hydroxyméthyl)pipéridin-1-yl]méthyl}-1-benzofuran-2-yl)-4-[(4-méthyl-1*H*-indol-5-yl)amino]-nicotinonitrile,
le 4-[(4-méthyl-1*H*-indol-5-yl)amino]-5-{5-[(4-pyrrolidin-1-ylpipéridin-1-yl)méthyl]-1-benzofuran-2-yl}nicotinonitrile,
le 5-[5-(1,4'-bipipéridin-1'-ylméthyl)-1-benzofuran-2-yl]-4-[(4-méthyl-1*H*-indol-5-yl)amino]-nicotinonitrile,
le 4-[(4-méthyl-1*H*-indol-5-yl)amino]-5-{5-[(4-morpholin-4-ylpipéridin-1-yl)méthyl]-1-benzofuran-2-yl}nicotinonitrile,
le 5-[5-({4-[2-(diméthylamino)éthyl]pipérazin-1-yl} méthyl)-1-benzofuran-2-yl]-4-[(4-méthyl-1*H*-indol-5-yl)-amino]nicotinonitrile,
le 4-[(4-méthyl-1*H*-indol-5-yl)amino]-5-{5-[(4-pyridin-2-ylpipérazin-1-yl)méthyl]-1-benzofuran-2-yl}nicotinonitrile,
le 4-[(4-méthyl-1*H*-indol-5-yl)amino]-5-(5-{[(pyridin-2-ylméthyl)amino]méthyl}-1-benzofuran-2-yl)nicotinonitrile,
le 4-[(4-méthyl-1*H*-indol-5-yl)amino]-5-(5-{[(pyridin-3-ylméthyl)amino]méthyl}-1-benzofuran-2-yl)nicotinonitrile,
le 4-[(4-méthyl-1*H*-indol-5-yl)amino]-5-(5-{[(pyridin-4-ylméthyl)amino]méthyl}-1-benzofuran-2-yl)nicotinonitrile,
le 4-[(4-méthyl-1*H*-indol-5-yl)amino]-5-[5-(morpholin-4-ylméthyl)-2-furyl]nicotinonitrile,
le 4-[(4-méthyl-1*H*-indol-5-yl)amino]-5-[5-(pipéridin-1-ylméthyl)-2-furyl]nicotinonitrile,
le 5-[5-(1,4'-bipipéridin-1'-ylméthyl)-2-furyl]-4-[(4-méthyl-1*H*-indol-5-yl)amino]nicotinonitrile,
le 4-[(4-méthyl-1*H*-indol-5-yl)amino]-5-{5-[(4-pyrrolidin-1-ylpipéridin-1-yl)méthyl]-2-furyl}-nicotinonitrile,
le 4-[(4-méthyl-1*H*-indol-5-yl)amino]-5-{5-[(4-morpholin-4-ylpipéridin-1-yl)méthyl]-2-furyl}-nicotinonitrile,
le 5-{5-[(diéthylamino)méthyl]-2-furyl}-4-[(4-méthyl-1*H*-indol-5-yl)amino]nicotinonitrile,
le 5-{5-[(dibutylamino)méthyl]-2-furyl}-4-[(4-méthyl-1*H*-indol-5-yl)amino]nicotinonitrile,
le 5-{5-[(benzylamino)méthyl]-2-furyl}-4-[(4-méthyl-1*H*-indol-5-yl)amino]nicotinonitrile,
le 4-[(4-méthyl-1*H*-indol-5-yl)amino]-5-(5-{[(3-phénylpropyl)amino]méthyl}-2-furyl)nicotinonitrile,
le 4-[(4-méthyl-1*H*-indol-5-yl)amino]-5-[5-(pyrrolidin-1-ylméthyl)-2-furyl]nicotinonitrile,
le 4-[(4-méthyl-1*H*-indol-5-yl)amino]-5-[5-(thiomorpholin-4-ylméthyl)-2-furyl]nicotinonitrile,
le 5-(3,4-diméthoxyphényl)-4-[(6-méthyl-1*H*-indol-5-yl)amino]nicotinonitrile,
le 5-(1-benzofuran-2-yl)-4-[(6-méthyl-1*H*-indol-5-yl)amino]nicotinonitrile,
le 5-(4-{2-[(2-hydroxyéthyl)amino]éthoxy}phényl)-4-[(4-méthyl-1*H*-indol-5-yl)amino]nicotinonitrile,
le 5-(4-{2-[(3-hydroxypropyl)amino]éthoxy}phényl)-4-[(4-méthyl-1*H*-indol-5-yl)amino]nicotinonitrile,
le 5-(4-{2-[(2-éthoxyéthyl)amino]éthoxy}phényl)-4-[(4-méthyl-1*H*-indol-5-yl)amino]nicotinonitrile,
le 5-[4-(2-{[2-(diméthylamino)éthyl]amino}éthoxy)-phényl]-4-[(4-méthyl-1*H*-indol-5-yl)amino]-nicotinonitrile,
le 4-[(4-méthyl-1*H*-indol-5-yl)amino]-5-[4-(2-pyrrolidin-1-yléthoxy)phényl]nicotinonitrile,
le 5-{4-[2-(benzylamino)éthoxy]phényl}-4-[(4-méthyl-1H-indol-5-yl)amino]nicotinonitrile,
le 4-[(4-méthyl-1*H*-indol-5-yl)amino]-5-(4-{2-[(1-méthylpipéridin-4-yl)amino]éthoxy}phényl)-nicotinonitrile,
le 4-[(4-méthyl-1*H*-indol-5-yl)amino]-5-[4-(2-{[(1-méthylpipéridin-4-yl)méthyl]amino}éthoxy)phényl]-nicotinonitrile,
le 5-(4-{2-[4-(hydroxyméthyl)pipéridin-1-yl]-éthoxy}phényl)-4-[(4-méthyl-1*H*-indol-5-yl)amino]-nicotinonitrile,
le 4-[(4-méthyl-1*H*-indol-5-yl)amino]-5-{4-[2-(4-pyrrolidin-1-ylpipéridin-1-yl)éthoxy]phényl}-nicotinonitrile,
le 4-[(4-méthyl-1*H*-indol-5-yl)amino]-5-{4-[2-(4-morpholin-4-ylpipéridin-1-yl)éthoxy]phényl}-nicotinonitrile,
le 5-{4-[2-(4-éthylpipérazin-1-yl)éthoxy]phényl}-4-[(4-méthyl-1*H*-indol-5-yl)amino]nicotinonitrile,
le 5-{4-[2-(4-méthyl-1,4-diazépan-1-yl)éthoxy]-phényl}-4-[(4-méthyl-1*H*-indol-5-yl)amino]-nicotinonitrile,
le 5-(4-{2-[4-(2-hydroxyéthyl)pipérazin-1-yl]-éthoxy}phényl)-4-[(4-méthyl-1*H*-indol-5-yl)amino]-nicotinonitrile,
le 5-[4-(2-{4-[2-(diméthylamino)éthyl]pipérazin-1-yl}éthoxy)phényl]-4-[(4-méthyl-1*H*-indol-5-yl)amino]-nicotinonitrile,
le 5-[4-(2-{[3-(1*H*-imidazol-1-yl)propyl]amino}-éthoxy)phényl]-4-[(4-méthyl-1*H*-indol-5-yl)amino]-nicotinonitrile,
le 4-[(4-méthyl-1*H*-indol-5-yl)amino]-5-{4-[2-(4-pyridin-2-ylpipérazin-1-yl)éthoxy]phényl}-nicotinonitrile,
le 4-[(4-méthyl-1*H*-indol-5-yl)amino]-5-{4-[2-(4-pyridin-4-ylpipérazin-1-yl)éthoxy]phényl}-nicotinonitrile,
le 4-[(4-méthyl-1*H*-indol-5-yl)amino]-5-(4-{2-[(pyridin-2-ylméthyl)amino]éthoxy}phényl)-nicotinonitrile,
le 4-[(4-méthyl-1*H*-indol-5-yl)amino]-5-(4-{2-[(pyridin-3-ylméthyl)amino]éthoxy}phényl)-nicotinonitrile,
le 4-[(4-méthyl-1*H*-indol-5-yl)amino]-5-(4-{2-[(pyridin-4-ylméthyl)amino]éthoxy}phényl)-nicotinonitrile,
le 4-[(4-méthyl-1*H*-indol-5-yl)amino]-5-{4-[2-(4-phénylpipéridin-1-yl)éthoxy]phényl}nicotinonitrile,
le 5-(5-{[4-(diméthylamino)pipéridin-1-yl]méthyl}-2-furyl)-4-[(4-méthyl-1*H*-indol-5-yl)amino]-nicotinonitrile,
le 5-{5-[(4-isopropylpipérazin-1-yl)méthyl]-2-furyl}-4-[(4-méthyl-1*H*-indol-5-yl)amino]nicotinonitrile,
le 4-[(4-méthyl-1*H*-indol-5-yl)amino]-5-{5-[2-(4-méthylpipérazin-1-yl)éthoxy]-1-benzofuran-2-yl}-nicotinonitrile,
le 5-(3,4-diméthoxyphényl)-4-{[2-(4-méthyl-pipérazin-1-yl)éthyl]amino}nicotinonitrile,
le 5-(3,4-diméthoxyphényl)-4-{[3-(4-méthyl-pipérazin-1-yl)propyl]amino}nicotinonitrile,
le 4-({[*trans*-4-(aminométhyl)cyclohexyl]méthyl}-amino)-5-(3,4-diméthoxyphényl)nicotinonitrile,
le 4-{[(*trans*-4-aminocyclohexyl)méthyl]amino}-5-(3,4-diméthoxyphényl)nicotinonitrile,
le 4-({[*cis*-3-(aminométhyl)cyclohexyl]méthyl}-amino)-5-(3,4-diméthoxyphényl)nicotinonitrile,
le 5-(3,4-diméthoxyphényl)-4-[(2-pipéridin-4-yléthyl)amino]nicotinonitrile,
le 5-(3,4-diméthoxyphényl)-4-[(pipéridin-4-ylméthyl)amino]nicotinonitrile,
le 4-[(*cis*-4-aminocyclohexyl)amino]-5-(3,4-diméthoxyphényl)nicotinonitrile,
le 5-(3,4-diméthoxyphényl)-4-{[2-(1-méthyl-pipéridin-4-yl)éthyl]amino}nicotinonitrile,
le 5-(3,4-diméthoxyphényl)-4-{[(1-méthylpipéridin-4-yl)méthyl]amino}nicotinonitrile,
le 4-[(4-méthyl-1*H*-indol-5-yl)amino]-5-{4-[(4-méthylpipérazin-1-yl)méthyl]phényl}nicotinonitrile,
le 4-[(4-méthyl-1*H*-indol-5-yl)amino]-5-[4-(pyrrolidin-1-ylméthyl)phényl]nicotinonitrile,
le 4-[(4-méthyl-1*H*-indol-5-yl)amino]-5-[4-(morpholin-4-ylméthyl)phényl]nicotinonitrile,
le 5-{4-[(diméthylamino)méthyl]phényl}-4-[(4-méthyl-1*H*-indol-5-yl)amino]nicotinonitrile,
le 4-[(4-méthyl-1*H*-indol-5-yl)amino]-2'-(morpholin-4-ylméthyl)-3,4'-bipyridine-5-carbonitrile,
le 4-[(4-méthyl-1*H*-indol-5-yl)amino]-5-{3-[(4-méthylpipérazin-1-yl)méthyl]phényl}nicotinonitrile,
le 4-[(4-méthyl-1*H*-indol-5-yl)amino]-5-[3-(pyrrolidin-1-ylméthyl)phényl]nicotinonitrile,
le 4-[(4-méthyl-1*H*-indol-5-yl)amino]-5-[3-(morpholin-4-ylméthyl)phényl]nicotinonitrile,
le 5-{3-[(diméthylamino)méthyl]phényl}-4-[(4-méthyl-1*H*-indol-5-yl)amino]nicotinonitrile,
le 5-(4-{[(2*R*)-2-amino-3-phénylpropyl]oxy}phényl)-4-(1*H*-indol-4-ylamino)nicotinonitrile,
le 5-{2-fluoro-4-[(4-méthylpipérazin-1-yl)méthyl]-phényl}-4-[(4-méthyl-1*H-*indol-5-yl)amino]-nicotinonitrile,
le 5-[4-(3-chloropropoxy)phényl]-4-[(4-méthyl-1H-indol-5-yl)amino]nicotinonitrile,
le 4-[(4-méthyl-1*H*-indol-5-yl)amino]-5-[5-(pipéridin-1-ylméthyl)-1-benzofuran-2-yl]-nicotinonitrile,
le 4-[(4-méthyl-1*H*-indol-5-yl)amino]-5-{5-[(4-méthylpipérazin-1-yl)méthyl]-1-benzofuran-2-yl}-nicotinonitrile,
le 5-(5-formyl-2-thiényl)-4-[(4-méthyl-1*H*-indol-5-yl)amino]nicotinonitrile,
le 4-[(4-méthyl-1*H*-indol-5-yl)amino]-5-{5-[(4-méthylpipérazin-1-yl)méthyl]-2-thiényl}nicotinonitrile,
le 5-(5-formyl-1-benzofuran-2-yl)-4-[(4-méthyl-1*H-*indol-5-yl)amino]nicotinonitrile,
le 5-(3-méthyl-1-benzofuran-2-yl)-4-[(4-méthyl-1*H-*indol-5-yl)amino]nicotinonitrile,
le 4-[(4-méthyl-1*H*-indol-5-yl)amino]-3,4'-bipyridine-5-carbonitrile,
le 4-[(4-méthyl-1*H*-indol-5-yl)amino]-5-{5-[(4-méthylpipérazin-1-yl)méthyl]-2-furyl}nicotinonitrile,
le 2'-chloro-4-[(4-méthyl-1*H*-indol-5-yl)amino]-3,4'-bipyridine-5-carbonitrile,
le 5-{2-chloro-4-[2-(4-méthylpipérazin-1-yl)-éthoxy]phényl}-4-[(4-méthyl-1*H*-indol-5-yl)amino]-nicotinonitrile,
le 2'-chloro-4-[(4-méthyl-1*H*-indol-5-yl)amino]-3,3'-bipyridine-5-carbonitrile,
le 4-[(4-méthyl-1*H*-indol-5-yl)amino]-5-{4-[3-(4-méthylpipérazin-1-yl)propoxy]phényl}nicotinonitrile,
le 4-[(4-méthyl-1*H*-indol-5-yl)amino]-5-[4-(3-morpholin-4-ylpropoxy)phényl]nicotinonitrile,
le 4-[(4-méthyl-1*H*-indol-5-yl)amino]-5-[4-(3-pipéridin-1-ylpropoxy)phényl]nicotinonitrile,
le 5-{4-[3-(diméthylamino)propoxy]phényl}-4-[(4-méthyl-1*H*-indol-5-yl)amino]nicotinonitrile,
le 5-[3,4-bis(2-méthoxyéthoxy)phényl]-4-[(4-méthyl-1*H-*indol-5-yl)amino]nicotinonitrile,
le 5-[3-méthoxy-4-(2-méthoxyéthoxy)phényl]-4-[(4-méthyl-1*H*-indol-5-yl)amino]nicotinonitrile,
le 5-[5-(hydroxyméthyl)-1-benzofuran-2-yl]-4-[(4-méthyl-1*H*-indol-5-yl)amino]nicotinonitrile,
le 5-[4-(2-méthoxyéthoxy)phényl]-4-[(4-méthyl-1H-indol-5-yl)amino]nicotinonitrile,
le 5-[3-(2-méthoxyéthoxy)phényl]-4-[(4-méthyl-1H-indol-5-yl)amino]nicotinonitrile,
le 5-(5-formyl-1-benzofuran-2-yl)-4-(1*H*-indol-5-ylamino)nicotinonitrile,
le 4-(1*H*-indol-5-ylamino)-5-{5-[(4-méthyl-pipérazin-1-yl)méthyl]-1-benzofuran-2-yl}-nicotinonitrile,
le 5-{5-[(4-cyclopentylpipérazin-1-yl)méthyl]-2-furyl}-4-[(4-méthyl-1*H*-indol-5-yl)amino]nicotinonitrile,
le 5-{5-[(1,1-dioxydothiomorpholin-4-yl)méthyl]-2-furyl}-4-[(4-méthyl-1*H*-indol-5-yl)amino]nicotinonitrile,
le 5-(3,4-diméthoxyphényl)-4-(1*H*-pyrrolo[2,3-b] pyridin-5-ylamino)nicotinonitrile,
le 5-(5-formyl-2-furyl)-4-[(4-méthyl-1*H*-indol-5-yl)amino]nicotinonitrile,
le 5-[4-(4-chlorobutoxy)phényl]-4-[(4-méthyl-1*H*-indol-5-yl)amino]nicotinonitrile,
le 4-[(4-méthyl-1*H*-indol-5-yl)amino]-5-{4-[4-(4-méthylpipérazin-1-yl)butoxy]phényl}nicotinonitrile,
le 4-[(4-chloro-1*H*-pyrrolo[2,3-b]pyridin-5-yl)-amino]-5-[3-méthoxy-4-(2-méthoxyéthoxy)phényl]-nicotinonitrile,
le 5-[4-(2-chloroéthoxy)phényl]-4-[(6-méthyl-1*H*-indol-5-yl)amino]nicotinonitrile,
le 5-(5-formyl-1-benzofuran-2-yl)-4-[(6-méthyl-1*H-*indol-5-yl)amino]nicotinonitrile,
le 4-[(6-méthyl-1*H*-indol-5-yl)amino]-5-{4-[2-(4-méthylpipérazin-1-yl)éthoxy]phényl}nicotinonitrile,
le 4-[(6-méthyl-1*H*-indol-5-yl)amino]-5-{5-[(4-méthylpipérazin-1-yl)méthyl]-1-benzofuran-2-yl}-nicotinonitrile,
le 4-[(7-chloro-4-méthyl-1*H*-indol-5-yl)amino]-5-(3,4-diméthoxyphényl)nicotinonitrile,
le 5-[5-(hydroxyméthyl)-1-benzofuran-2-yl]-4-[(6-méthyl-1*H*-indol-5-yl)amino]nicotinonitrile,
le 5-{5-[(diéthylamino)méthyl]-1-benzofuran-2-yl}-4-[(6-méthyl-1*H*-indol-5-yl)amino]nicotinonitrile,
le 5-[3-(4-chlorobutoxy)phényl]-4-[(4-méthyl-1*H*-indol-5-yl)amino]nicotinonitrile,
le 4-[(4-méthyl-1*H*-indol-5-yl)amino]-5-{3-[4-(4-méthylpipérazin-1-yl)butoxy]phényl}nicotinonitrile,
le 4-[(4-méthyl-1*H*-indol-5-yl)amino]-5-{5-[(4-méthylpipérazin-1-yl)méthyl]-3-furyl}nicotinonitrile,
le 4-[(6-méthyl-1*H*-indol-5-yl)amino]-5-[4-(2-pipéridin-1-yléthoxy)phényl]nicotinonitrile,
le 5-{4-[2-(4-hydroxypipéridin-1-yl)éthoxy]-phényl}-4-[(6-méthyl-1*H*-indol-5-yl)amino]-nicotinonitrile,
le 4-[(6-méthyl-1*H*-indol-5-yl)amino]-5-{4-[2-(4-pyrrolidin-1-ylpipéridin-1-yl)éthoxy]phényl}-nicotinonitrile,
le 4-[(4-méthyl-1*H*-indol-5-yl)amino]-6'-morpholin-4-yl-3,3'-bipyridine-5-carbonitrile,
le 4-[(4-méthyl-1*H*-indol-5-yl)amino]-6'-pipéridin-1-yl-3,3'-bipyridine-5-carbonitrile,
le 4-[(4-méthyl-1*H*-indol-5-yl)amino]-5-pyrimidin-5-ylnicotinonitrile,
le 4-[(4-méthyl-1*H*-indol-5-yl)amino]-5-(2-pipéridin-1-ylpyrimidin-5-yl)nicotinonitrile,
le 4-[(4-méthyl-1*H*-indol-5-yl)amino]-5-(2-morpholin-4-ylpyrimidin-5-yl)nicotinonitrile,
le 4-[(4-méthyl-1*H*-indol-5-yl)amino]-5-(2-pyrrolidin-1-ylpyrimidin-5-yl)nicotinonitrile,
le 5-[2-(diméthylamino)pyrimidin-5-yl]-4-[(4-méthyl-1*H*-indol-5-yl)amino]nicotinonitrile,
le 5-(1-benzothién-2-yl)-4-[(4-méthyl-1*H*-indol-5-yl)amino]nicotinonitrile,
le 5-[4-(2-chloroéthoxy)phényl]-4-(1*H*-indol-5-yl-amino)nicotinonitrile,
le 5-(5-formyl-3-thiényl)-4-[(4-méthyl-1*H*-indol-5-yl)amino]nicotinonitrile,
le 5-(4-formyl-2-furyl)-4-[(4-méthyl-1*H*-indol-5-yl)amino]nicotinonitrile,
le 4-[(4-méthyl-1*H*-indol-5-yl)amino]-5-{5-[(4-méthylpipérazin-1-yl)méthyl]-3-thiényl}nicotinonitrile,
le 4-[(4-méthyl-1*H*-indol-5-yl)amino]-3,3'-bipyridine-5-carbonitrile,
le 4-(1*H*-indol-5-ylamino)-5-{4-[2-(4-méthyl-pipérazin-1-yl)éthoxy]phényl}nicotinonitrile,
le 4-[(4-méthyl-1*H*-indol-5-yl)amino]-5-{5-[(4-méthylpipérazin-1-yl)méthyl]-1-benzothién-2-yl}-nicotinonitrile,
le 4-[(4-chloro-1*H*-pyrrolo[2,3-b]pyridin-5-yl)-amino]-5-(3,4-diméthoxyphényl)nicotinonitrile,
le 4-[(*trans*-4-aminocyclohexyl)amino]-5-(3,4-diméthoxyphényl)nicotinonitrile,
la 1-butyl-3-(4-{5-cyano-4-[(4-méthyl-1*H*-indol-5-yl)amino]pyridin-3-yl}phényl)urée,
le (4-{5-cyano-4-[(4-méthyl-1*H*-indol-5-yl)amino]-pyridin-3-yl}phényl)carbamate de méthyle,
le (4-{5-cyano-4-[(4-méthyl-1*H*-indol-5-yl)amino]-pyridin-3-yl}-2-fluorophényl)carbamate de benzyle,
le (4-{5-cyano-4-[(4-méthyl-1*H*-indol-5-yl)amino]-pyridin-3-yl}-2-fluorophényl)carbamate de 4-méthoxy-benzyle,
le 4-[(7-chloro-4-méthyl-1*H*-indol-5-yl)amino]-5-{5-[(4-méthylpipérazin-1-yl)méthyl]-1-benzofuran-2-yl}nicotinonitrile,
le 5-(3,4-diméthoxyphényl)-4-[(4-méthyl-1*H*-indol-7-yl)amino]nicotinonitrile,
le 5-(1-benzofuran-2-yl)-4-[(7-chloro-4-méthyl-1*H-*indol-5-yl)amino]nicotinonitrile,
le 4-[(7-chloro-4-méthyl-1*H*-indol-5-yl)amino]-5-(5-formyl-1-benzofuran-2-yl)nicotinonitrile,
le 5-[4-(2-chloroéthoxy)phényl]-4-[(7-chloro-4-méthyl-1*H*-indol-5-yl)amino]nicotinonitrile,
le 4-[(7-chloro-4-méthyl-1*H*-indol-5-yl)amino]-5-{4-[2-(diméthylamino)éthoxy]phényl}nicotinonitrile,
le 4-[(7-chloro-4-méthyl-1*H*-indol-5-yl)amino]-5-{4-[2-(4-méthylpipérazin-1-yl)éthoxy]phényl}-nicotinonitrile,
le 4-[(2-{5-cyano-4-[(4-méthyl-1*H*-indol-5-yl)-amino]pyridin-3-yl}-1-benzofuran-5-yl)méthyl]-pipérazine-1-carboxylate de tert-butyle,
le 5-(2-formyl-1-méthyl-1H-imidazol-5-yl)-4-[(4-méthyl-1*H*-indol-5-yl)amino]nicotinonitrile,
le 4-[(4-méthyl-1*H*-indol-5-yl)amino]-5-(5-{[4-(méthylsulfonyl)pipérazin-1-yl]méthyl}-1-benzofuran-2-yl)nicotinonitrile,
le 4-[(4-méthyl-1*H*-indol-5-yl)amino]-5-{1-méthyl-2-[(4-méthylpipérazin-1-yl)méthyl]-1*H*-imidazol-5-yl}-nicotinonitrile,
le 4-[(4-méthyl-1*H*-indol-5-yl)amino]-5-[5-(pipérazin-1-ylméthyl)-1-benzofuran-2-yl]-nicotinonitrile,
le 4-[(4-méthyl-1*H*-indol-5-yl)amino]-5-(1,3-thiazol-2-yl)nicotinonitrile,
le 5-(1-méthyl-1*H*-imidazol-2-yl)-4-[(4-méthyl-1*H-*indol-5-yl)amino]nicotinonitrile,
le 4-[(4-méthyl-1*H*-indol-5-yl)amino]-5-(1,3-thiazol-4-yl)nicotinonitrile,
le 5-(3,4-diméthoxyphényl)-4-(1*H*-indol-7-ylamino)-nicotinonitrile,
le 5-(3,4-diméthoxyphényl)-4-[(4-méthoxy-1*H*-indol-5-yl)amino]nicotinonitrile,
le 5-(3,4-diméthoxyphényl)-4-[(4-fluoro-1*H*-indol-5-yl)amino]nicotinonitrile,
le 4-[(7-chloro-4-méthyl-1*H*-indol-5-yl)amino]-5-[5-(pipérazin-1-ylméthyl)-1-benzofuran-2-yl]-nicotinonitrile,
le 4-[(2-{4-[(7-chloro-4-méthyl-1*H*-indol-5-yl)-amino]-5-cyanopyridin-3-yl}-1-benzofuran-5-yl)méthyl]-pipérazine-1-carboxylate de tert-butyle,
le 5-(3,4-diméthoxyphényl)-4-[(2,4-diméthyl-1*H*-indol-5-yl)amino]nicotinonitrile,
le 5-{2-[(diméthylamino)méthyl]phényl}-4-[(4-méthyl-1*H*-indol-5-yl)amino]nicotinonitrile,
le 5-(5-formyl-2-méthoxyphényl)-4-[(4-méthyl-1*H*-indol-5-yl)amino]nicotinonitrile,
le 5-{2-méthoxy-5-[(4-méthylpipérazin-1-yl)-méthyl]phényl}-4-[(4-méthyl-1*H*-indol-5-yl)amino]-nicotinonitrile,
le 5-{5-[(4-éthylpipérazin-1-yl)méthyl]-1-benzofuran-2-yl}-4-[(4-méthyl-1*H*-indol-5-yl)amino]-nicotinonitrile,
le 4-[(4-méthyl-1*H*-indol-5-yl)amino]-5-{5-[(4-méthyl-4-oxydopipérazin-1-yl)méthyl]-1-benzofuran-2-yl}nicotinonitrile,
le 5-(3,4-diméthoxyphényl)-4-[(1,4-diméthyl-1*H*-indol-5-yl)amino]nicotinonitrile,
l'acide 3-{5-cyano-4-[(4-méthyl-1*H*-indol-5-yl)amino]-pyridin-3-yl}benzoïque,
le 5-(2-méthoxyphényl)-4-[(4-méthyl-1*H*-indol-5-yl)amino]nicotinonitrile,
le 5-(3-méthoxyphényl)-4-[(4-méthyl-1*H*-indol-5-yl)amino]nicotinonitrile,
le 5-(4-méthoxyphényl)-4-[(4-méthyl-1*H*-indol-5-yl)amino]nicotinonitrile,
le 4-[(4-méthyl-1*H*-indol-5-yl)amino]-5-phényl-nicotinonitrile,
le 4-[(4-méthyl-1*H*-indol-5-yl)amino]-5-(2-thiényl)nicotinonitrile,
le 4-[(4-méthyl-1*H*-indol-5-yl)amino]-5-(3-thiényl)nicotinonitrile,
le 5-(3-furyl)-4-[(4-méthyl-1*H*-indol-5-yl)amino]-nicotinonitrile,
le 5-(1-méthyl-1*H*-imidazol-5-yl)-4-[(4-méthyl-1*H-*indol-5-yl)amino]nicotinonitrile,
le 4'-[(4-méthyl-1*H*-indol-5-yl)amino]-2,3'-bipyridine-5'-carbonitrile,
la 1-(4-{5-cyano-4-[(4-méthyl-1*H*-indol-5-yl)-amino]pyridin-3-yl}phényl)-3-cyclopropylurée,
la 1-(4-{5-cyano-4-[(4-méthyl-1*H*-indol-5-yl)-amino]pyridin-3-yl}phényl)-3-méthylurée,
la 3-(4-{5-cyano-4-[(4-méthyl-1*H*-indol-5-yl)-amino]pyridin-3-yl}phényl)-1,1-diméthylurée,
le N-(4-{5-cyano-4-[(4-méthyl-1*H*-indol-5-yl)-amino]pyridin-3-yl}phényl)morpholine-4-carboxamide,
le 4-[(4-méthyl-1*H*-indol-5-yl)amino]-5-(4-nitro-phényl)nicotinonitrile,
le 5-(4-aminophényl)-4-[(4-méthyl-1*H*-indol-5-yl)-amino]nicotinonitrile,
le 5-(3-aminophényl)-4-[(4-méthyl-1*H*-indol-5-yl)-amino]nicotinonitrile,
le 5-(2-aminophényl)-4-[(4-méthyl-1*H*-indol-5-yl)-amino]nicotinonitrile,
le 5-[4-(diméthylamino)phényl]-4-[(4-méthyl-1*H*-indol-5-yl)amino]nicotinonitrile,
le 5-[3-(diméthylamino)phényl]-4-[(4-méthyl-1*H*-indol-5-yl)amino]nicotinonitrile,
le N-(4-{5-cyano-4-[(4-méthyl-1*H*-indol-5-yl)-amino]pyridin-3-yl}phényl)acétamide,
le N-(2-{5-cyano-4-[(4-méthyl-1*H*-indol-5-yl)-amino]pyridin-3-yl}phényl)acétamide,
le N-(3-{5-cyano-4-[(4-méthyl-1*H*-indol-5-yl)-amino]pyridin-3-yl}phényl)acétamide,
le N-(4-{5-cyano-4-[(4-méthyl-1*H*-indol-5-yl)-amino]pyridin-3-yl}phényl)-2-méthylpropanamide,
le 4-{5-cyano-4-[(4-méthyl-1*H*-indol-5-yl)amino]-pyridin-3-yl}-N-méthylbenzamide,
le 4-[(4-méthyl-1*H*-indol-5-yl)amino]-5-(1-naphtyl)nicotinonitrile,
le 4-[(4-méthyl-1*H*-indol-5-yl)amino]-5-(2-naphtyl)nicotinonitrile,
le 4-[(4-méthyl-1*H*-indol-5-yl)amino]-5-(1-méthyl-1*H-*pyrazol-5-yl)nicotinonitrile,
le 4-[(4-méthyl-1*H*-indol-5-yl)amino]-5-(1*H*-pyrazol-4-yl)nicotinonitrile,
5-(1-benzothiophén-3-yl)-4-[(4-méthyl-1*H*-indol-5-yl)amino]nicotinonitrile,
le 5-(1-méthyl-1*H*-indol-2-yl)-4-[(4-méthyl-1*H*-indol-5-yl)amino]nicotinonitrile,
le 5-(1*H*-indol-5-yl)-4-[(4-méthyl-1*H*-indol-5-yl)-amino]nicotinonitrile,
le 5-(1*H*-indol-6-yl)-4-[(4-méthyl-1*H*-indol-5-yl)-amino]nicotinonitrile,
le 4-[(4-méthyl-1*H*-indol-5-yl)amino]-5-quinolin-3-ylnicotinonitrile,
le 4-[(4-méthyl-1*H*-indol-5-yl)amino]-5-quinolin-8-ylnicotinonitrile,
le 5-(1-benzofuran-5-yl)-4-[(4-méthyl-1*H*-indol-5-yl)amino]nicotinonitrile,
le 4-(4-méthyl-1*H*-indol-5-ylamino)-5-(quinolin-5-yl)nicotinonitrile,
le 5-(dibenzo[b,d]thiophén-3-yl)-4-(4-méthyl-1*H*-indol-5-ylamino)nicotinonitrile,
le 5-(benzo[b]thiophén-5-yl)-4-(4-méthyl-1*H*-indol-5-ylamino)nicotinonitrile,
le 5-(1*H*-indol-4-yl)-4-(4-méthyl-1*H*-indol-5-yl-amino)nicotinonitrile,
le 4-[(2,4-diméthyl-1*H*-indol-5-yl)amino]-5-{5-[(4-méthylpipérazin-1-yl)méthyl]-1-benzofuran-2-yl}-nicotinonitrile,
le 4-[(2,4-diméthyl-1*H*-indol-5-yl)amino]-5-[5-(pipérazin-1-ylméthyl)-1-benzofuran-2-yl]-nicotinonitrile,
le 4-[(4-méthyl-1*H*-indol-5-yl)amino]-5-{6-[(4-méthylpipérazin-1-yl)méthyl]-1-benzofuran-2-yl}-nicotinonitrile,
le 4-[(4-méthyl-1*H*-indol-5-yl)amino]-5-[6-(pipérazin-1-ylméthyl)-1-benzofuran-2-yl]-nicotinonitrile,
le 4-[(4-méthyl-1*H*-indol-5-yl)amino]-5-{4-[(pipérazin-1-yl)méthyl]phényl}nicotinonitrile,
le 4-[(2,4-diméthyl-1*H*-indol-5-yl)amino]-5-{4-[(4-méthylpipérazin-1-yl)méthyl]phényl}nicotinonitrile,
le 4-(2,4-diméthyl-1*H*-indol-5-ylamino)-5-{3-[2-(4-méthylpipérazin-1-yl)éthoxy]phényl}nicotinonitrile,
le 5-{4-[2-(diméthylamino)éthoxy]-3-méthoxy-phényl}-4-[(2,4-diméthyl-1*H*-indol-5-yl)amino]-nicotinonitrile,
le 4-[(2,4-diméthyl-1*H*-indol-5-yl)amino]-5-{4-[2-(4-méthylpipérazin-1-yl)éthoxy]phényl}nicotinonitrile,
le 4-[(4-méthyl-1*H*-indol-5-yl)amino]-5-{4-[2-(pipérazin-1-yl)éthoxy]phényl}nicotinonitrile,
le 4-(4-méthyl-1*H*-indol-5-ylamino)-2'-((4-méthyl-pipérazin-1-yl)méthyl)-3,4'-bipyridine-5-carbonitrile,
le 4-(4-méthyl-1*H*-indol-5-ylamino)-2'-((pipérazin-1-yl)méthyl)-3,4'-bipyridine-5-carbonitrile,
le 4'-(4-méthyl-1*H*-indol-5-ylamino)-5-((4-méthyl-pipérazin-1-yl)méthyl)-2,3'-bipyridine-5'-carbonitrile,
le 4'-(4-méthyl-1*H*-indol-5-ylamino)-5-(morpholino-méthyl)-2,3'-bipyridine-5'-carbonitrile,
le 4'-(4-méthyl-1*H*-indol-5-ylamino)-5-((pipérazin-1-yl)méthyl)-2,3'-bipyridine-5'-carbonitrile,
le 4'-(4-méthyl-1*H*-indol-5-ylamino)-6-((4-méthyl-pipérazin-1-yl)méthyl)-2,3'-bipyridine-5'-carbonitrile,
le 4'-(4-méthyl-1*H*-indol-5-ylamino)-6-(morpholino-méthyl)-2,3'-bipyridine-5'-carbonitrile,
le 4'-(4-méthyl-1*H*-indol-5-ylamino)-6-((pipérazin-1-yl)méthyl)-2,3'-bipyridine-5'-carbonitrile,
le 4'-(4-méthyl-1*H*-indol-5-ylamino)-4-(morpholino-méthyl)-2,3'-bipyridine-5'-carbonitrile,
le 4'-(4-méthyl-1*H*-indol-5-ylamino)-4-((4-méthylpipérazin-1-yl)méthyl)-2,3'-bipyridine-5'-carbonitrile,
le 4'-(4-méthyl-1*H*-indol-5-ylamino)-4-((pipérazin-1-yl)méthyl)-2,3'-bipyridine-5'-carbonitrile,
le 4-(4-méthyl-1*H*-indol-5-ylamino)-5'-((4-méthylpipérazin-1-yl)méthyl)-3,3'-bipyridine-5-carbonitrile,
le 4-(4-méthyl-1*H*-indol-5-ylamino)-5'-((pipérazin-1-yl)méthyl)-3,3'-bipyridine-5-carbonitrile,
le 4-(4-méthyl-1*H*-indol-5-ylamino)-5'-(morpholino-méthyl)-3,3'-bipyridine-5-carbonitrile,
le 4-(4-méthyl-1*H*-indol-5-ylamino)-6'-((4-méthyl-pipérazin-1-yl)méthyl)-3,3'-bipyridine-5-carbonitrile,
le 4-(4-méthyl-1*H*-indol-5-ylamino)-6'-((pipérazin-1-yl)méthyl)-3,3'-bipyridine-5-carbonitrile,
le 4-(4-méthyl-1*H*-indol-5-ylamino)-6'-(morpholino-méthyl)-3,3'-bipyridine-5-carbonitrile,
le 4-(4-méthyl-1*H*-indol-5-ylamino)-5-(3-(pipérazin-1-ylméthyl)phényl)nicotinonitrile,
le 4-(4-méthyl-1*H*-indol-5-ylamino)-5-(4-(pipérazin-1-ylméthyl)phényl)nicotinonitrile,
le 4-({[*cis*-4-(aminométhyl)cyclohexyl]méthyl}-amino)-5-(3,4-diméthoxyphényl)nicotinonitrile, et
le 1-oxyde de 5-(3,4-diméthoxyphényl)-4-(1*H*-indol-5-ylamino)-nicotinonitrile.

21. Composé selon l'une quelconque des revendications 1 à 20, dans lequel le composé est sous la forme d'un énantiomère.

22. Composition pharmaceutique, comprenant le composé selon l'une quelconque des revendications 1 à 21 et un support ou excipient pharmaceutiquement acceptable.

23. Composé selon l'une quelconque des revendications 1 à 21 ou sel, hydrate ou ester de celui-ci pharmaceutiquement acceptable, pour son utilisation dans le traitement ou l'inhibition d'une affection ou d'un trouble pathologique dans laquelle/lequel une protéine-kinase intervient comme médiateur chez un mammifère.

24. Composé pour son utilisation selon la revendication 23, dans lequel la protéine-kinase est la protéine-kinase C.

25. Composé pour son utilisation selon la revendication 23 ou 24, dans lequel l'affection ou le trouble pathologique est une maladie inflammatoire ou une maladie auto-immune sélectionnée parmi l'asthme, la colite, la sclérose en plaques, le psoriasis, l'arthrite, la polyarthrite rhumatoïde, l'arthrose et l'inflammation des articulations.

26. Composé selon l'une quelconque des revendications 1 à 21 ou sel, hydrate ou ester de celui-ci pharmaceutiquement acceptable, pour son utilisation comme médicament.

27. Utilisation d'un composé selon l'une quelconque des revendications 1 à 21 ou d'un sel, hydrate ou ester de celui-ci pharmaceutiquement acceptable, dans la préparation d'un médicament pour le traitement ou l'inhibition d'une affection ou d'un trouble pathologique dans laquelle/lequel une protéine-kinase intervient comme médiateur chez un mammifère.
